# EUROPEAN PATENT APPLICATION

(11) **EP 2 594 270 A2**
(43) Date of publication of application: **22.05.2013**
(21) Application number: 12193025.9
(22) Date of filing: 16.11.2012
(51) Int. Cl.: A61K 31/427, A61K 31/4418, A61K 31/498, A61K 31/4985, A61K 31/506, A61K 31/519, A61K 31/53, A61K 45/06, A61P 11/00

(54) **The use of sGC stimulators, sGC activators, alone and combinations with PDE5 inhibitors for the treatment of systemic sclerosis (SSc)**

(30) Priority: 18.11.2011 EP 11189652
(71) Applicant: BIP Patents, 40789 Monheim (DE)
(72) Inventor: Hirth-Dietrich, Claudia, 42115 Wuppertal (DE); Sandner, Peter, 42113 Wuppertal (DE); Stasch, Johannes-Peter, 42651 Solingen (DE); Hahn, Michael, 40764 Langenfeld (DE); Follmann, Markus, 42489 Wülfrath (DE)

(57) **Abstract**

The use of sGC stimulators, sGC activators alone, or in combination with PDE5 inhibitors for the prevention and treatment of fibrotic diseases, such as systemic sclerosis, scleroderma, and the concomitant fibrosis of internal organs.

## Description

The use of sGC stimulators, sGC activators alone, or in combination with PDE5 inhibitors for the prevention and treatment of fibrotic diseases, such as systemic sclerosis, scleroderma, and the concomitant fibrosis of internal organs.

### Background of the invention

### Systemic Sclerosis

The pathogenesis of Systemic Sclerosis (SSc) is still unclear and remains elusive. However, scleroderma is a non-inherited, noninfectious disease and thought to be an autoimmune disease. SSc has a broad variety of symptoms triggered by excessive deposition of extracellular matrix in the dermis resulting in skin fibrosis. In later stages SSc is characterized by progressive tissue fibrosis affecting other internal organs as the gut, the lung or the kidneys. Therefore scleroderma is the hallmark of the disease comprising also e.g. lung fibrosis, renal fibrosis, fibrosis of the heart, the gut or the blood vessels. It is suggested that inflammation, autoimmune disorders or vascular damage activates fibroblasts. Fibroproliferation is accompanied by excessive extracellular matrix production, dominated by Collagen type I resulting in progressive tissue fibrosis which can cause end organ failure and lead to high morbidity and mortality in patients with end-stage SSc (Harris et al. 2005 - Kelley's Textbook of Rhematology 7th edition. Elsevier Saunders, Philadelphia PA).

There is still no causative treatment for Systemic Sclerosis (SSc) available and the current therapy is based on suppression of the immune system via corticosteroids, cyclophosphamide, methotrexate. More recently kinase inhibitors are under investigation as immunosuppressant and antifibrotic agents in SSc, but tolerability is limited in SSc patients (Khanna and Denton 2010 - Best. Pract. Res. Clin. Rheumatol. 24:387-400, Ong and Denton 2010 - Curr. Opin. Rheumatol. 22:264-272, Spiera 2011 - Ann. Rheum. Dis. Epub Mar 2011). These therapies either used as stand alone treatment or combined are of limited efficacy and exhibited considerable side effects. Therefore alternative treatment options in SSc which are efficacious and safe are urgently needed.

### Antifibrotic effects of cGMP:

The cyclic nucleotides, cyclic adenosine monophosphate (cAMP) and cyclic guanosine monophosphate (cGMP), were discovered decades ago and represent one of the most important second messenger pathway within cells. It is well established that the regulation of intra-cellular cGMP pools have substantial impact on physiology, and pathophysiology and is one basic principle of pharmacological intervention (Evgenov et al. 2006 - Nat. Rev. Drug. Discov. 5(9):755-768). Besides the treatment of cardiovascular, lung or CNS-disorders there is ample evidence that an increase in cGMP is a very effective treatment option for urological disorders as well (Sandner et al. 2009 - Handbook Exper. Pharmacol. 191:507-531). PDE5 inhibitors are the gold-standard for the treatment of erectile dysfunction (ED) but it was shown that PDE5 inhibitors could be useful for the treatment of symptomatic BPH which is characterized by Overactive Bladder (OAB) and Lower Urinary Tract Symptoms (LUTS) (Porst et al. 2008 - Curr. Urol. Rep. 9:295-301; .McVary et al. 2007 - J. Urol. 177:1071-1077, J Urol. 177:1401-1407, Kaplan and Gonzalez. 2007 - Rev. Urol. 9:73-77). The antifibrotic effects of Vardenafil, sGC stimulators and sGC activators is not understood yet. There are some descriptions about antifibrotic effects of Nitric-Oxide which are presumably mediated by cGMP in other organs and PDE5 inhibitors or guanylate cyclase stimulators have shown efficacy in penile fibrosis (Peyronie's disease) (Ferrini et al. 2006 - B. J. Urol. 97:625-633) and liver fibrosis (Knorr et al. 2008 - Arzneimittelforschung 58:71-80) respectively.

It is not known if the NO/cGMP system is involved in SSc and if cGMP increase provides a treatment option for this disease. We hypothetized that - independent from endogenous NO/cGMP production - sGC stimulators and activators might be an effective treatment option for Systemic Sclerosis (SSc).

We therefore investigated sGC stimulators and sGC activators, i.e. example 1A according to compound of the formula 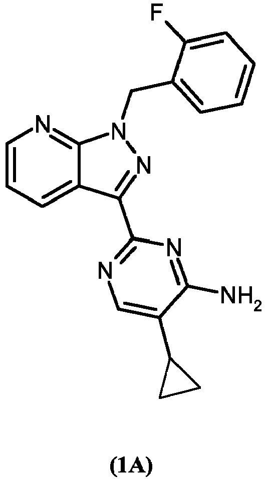 and combinations with PDE5 inhibitors thereof in vitro and in vivo in animal models for SSc. The in vivo experiments are including studies in bleomycin-induced skin and lung fibrosis in mice and studies on skin fibrosis in TSK-mice. In addition, the dose range tested on antifibrotic potential was also analyzed in mice with telemetric implants for blood-pressure and heart rate analysis.

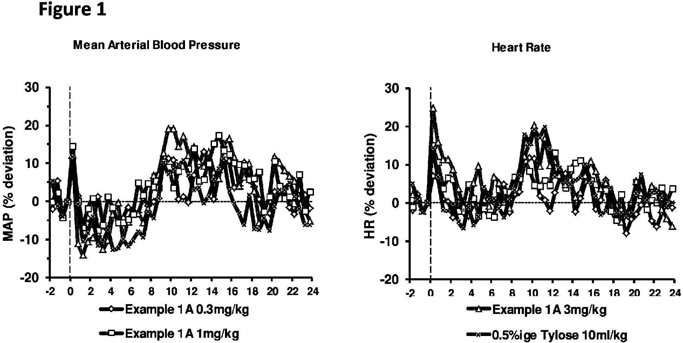

We found in vivo in our animal models that:
● sGC stimulators or sGC activators, i.e. example 1A and examples of compound classes I to X significantly reduced dermal thickness, hydroxyproline content of the skin and the number of dermal myofibroblasts in bleomycin-induced SSc in mice when administered in a preventive dose-regimen. (Example A: Table 1). These data suggest an antifibrotic effect in Systemic Sclerosis when these compounds are given preventively.
● sGC stimulators or sGC activators, i.e. example 1A and examples of compound classes I to X significantly reduced dermal thickness, hydroxyproline content of the skin and the number of dermal myofibroblasts in bleomycin-induced SSc in mice when administered in a therapeutic dose-regimen after established fibrosis. (Example B: Table 3). These data suggest an antifibrotic effect and regression of established fibrosis in Systemic Sclerosis when the compounds are given therapeutically.
● sGC stimulators or sGC activators, i.e. example 1A and examples of compound classes I to X significantly reduced dermal thickness, hydroxyproline content of the skin and the number of dermal myofibroblasts in TSK mice. Since TSK mice already exhibited established fibrosis before start of the treatment example 1A caused fibrosis-regression (Example C: Table 4). These data suggest an antifibrotic effect and regression of established fibrosis in Systemic Sclerosis when the compounds are given therapeutically.
● sGC stimulators of sGC activators, i.e. example 1A and examples of compound classes I to X were investigated in conscious mice with telemetric implants and blood pressure and heart rate was monitored (Example D). Example 1A did not or only moderately, change the heamodynamic profile of the mice in dosages with antifibrotic properties (Example D: Figure 1. These data suggest a direct antifibrotic mode of action of sGC stimulators and sGC activators independent of blood pressure reduction by these compounds.
● sGC stimulators or sGC activators, i.e. example 1A and examples of compound classes I to X, alone and in combination with PDE5 inhibitors (i.e. Vardenafil) blocked TGF-induced collagen gene expression in vitro in human dermal fibroblasts (Example E). These data suggest a direct antifibrotic effect on the level of collagen production

Thus, we found completely unexpected and for the first time that sGC stimulators or sGC activators i.e. example 1A, prevent fibrosis and regress established fibrosis in different animal models of inflammatory and non-inflammatory SSc including bleomycin-induced fibrosis models and the TSK-mouse model.

In addition there was no significant effect seen on systemic blood pressure which for the first time shows that these sGC stimulators have direct antifibrotic properties in SSc independent from blood pressure reduction.

Moreover sGC stimulators and sGC activators could block TGF-beta induce collagen synthesis implying a broad antifibrotic effect in other fibrotic disorders beyond SSc.

Taken together this data indicate for the first time that sGC stimulators and sGC activators, i.e. Example 1A could represent a future treatment option for SSc.

### Disclosure of the invention

Fibrotic disorders addressed by therapeutic agents of the invention which in particular and with substantial advantage can be treated by the above mentioned sGC stimulators or sGC activators alone or in combination with PDE5 inhibitors comprise but are not limited to Systemic Sclerosis (SSc), Systemic Sclerosis (SSc) concomitant fibrosis and fibrotic diseases.

Systemic Sclerosis (SSc) refers to but is not limited to diffuse Systemic Sclerosis (dSSc), limited Systemic Sclerosis (ISSc), overlap type of Systemic Sclerosis, undifferentiated type of Systemic Sclerosis, Systemic Sclerosis sine scleroderma, skin fibrosis, scleroderma, nephrogenic fibrosing dermopathy (NFD), nephrogenic systemic fibrosis (NSF), keloid formation.

SSc concomitant fibrosis refers to fibrosis of internal organs, comprising but not limited to the gut, the lung, the kidney and the blood vessels.

Fibrotic diseases comprises but are not limited to a condition in which collagen excess - independent of the etiology i.e. autoimmune disorders, radiation therapy, intoxications, diabetes, surgery - lead to fibrosis of the skin, gut, liver, lung, heart, bladder, prostate, blood vessels or any other localized or generalized fibrotic condition in tissues.

A preferred embodiment of the invention is compounds according to formulae of compound classes I to X for the prevention and treatment of fibrotic diseases, such as systemic sclerosis, scleroderma, and the concomitant fibrosis of internal organs, as shown below:

### Compound class I

Examples 1 to 111 of compound class I are disclosed in WO2010/065275.

| **Example** | **IUPAC NAME** |
|---|---|
| 1 | 4-amino-2-[5-chloro-3-(3,3,3-trifluoropropyl)-1*H*-indazol-1-yl]-5,5-dimethyl-5,7-dihydro-6*H*-pyrrolo[2,3-*d*]pyrimidin-6-one |
| 2 | 4-amino-5,5-dimethyl-2-[3-(3,3,3-trifluoropropyl)-1*H*-indazol-1-yl]-5,7-dihydro-6*H-*pyrrolo[2,3-*d*]pyrimidin-6-one |
| 3 | 4-amino-2-[5-chloro-3-(2,3,6-trifluorobenzyl)-1*H*-indazol-1-yl]-5,5-dimethyl-5,7-dihydro-6*H*-pyrrolo[2,3-*d*]pyrimidin-6-one |
| 4 | 4-amino-5,5-dimethyl-2-[3-(2,3,6-trifluorobenzyl)-1*H*-indazol-1-yl]-5,7-dihydro-6*H-*pyrrolo[2,3-*d*]pyrimidin-6-one |
| 5 | 4-amino-2-(5-fluoro-3-hexyl-1*H*-indazol-1-yl)-5,5-dimethyl-5,7-dihydro-6*H*-pyrrolo[2 *d*]pyrimidin-6-one |
| 6 | 4-amino-2-[5-bromo-3-(2,3,6-trifluorobenzyl)-1*H*-indazol-1-yl]-5,5-dimethyl-5,7-dihydro-6*H*-pyrrolo[2,3-*d*]pyrimidin-6-one |
| 7 | 4-amino-5,5-dimethyl-2-[5-pyridin-4-yl-3-(2,3,6-trifluorobenzyl)-1*H*-indazol-1-yl]-5,7 dihydro-6*H*-pyrrolo[2,3-*d*]pyrimidin-6-one |
| 8 | 4-amino-5,5-dimethyl-2-[3-(4,4,4-trifluorobutyl)-1*H*-thieno[3,4-*c*]pyrazol-1-yl]-5,7-dihydro-6*H*-pyrrolo[2,3-*d*]pyrimidin-6-one |
| 9 | 4-amino-5,5-dimethyl-2-[3-(2,3,6-trifluorobenzyl)-1*H*-thieno[3,4-*c*]pyrazol-1-yl]-5,7-dihydro-6*H*-pyrrolo[2,3-*d*]pyrimidin-6-one |
| 10 | 4-amino-5,5-dimethyl-2-[3-(2,3,6-trifluorobenzyl)-4,6-dihydro-1*H*-thieno[3,4-*c*]pyrazol-1-yl]-5,7-dihydro-6*H*-pyrrolo[2,3-*d*]pyrimidin-6-one |
| 11 | 4-amino-2-[3-(2-cyclopentylethyl)-1*H*-indazol-1-yl]-5,5-dimethyl-5,7-dihydro-6*H-*pyrrolo[2,3-*d*]pyrimidin-6-one |
| 12 | 4-amino-2-[3-(2-fluorobenzyl)-1*H*-indazol-1-yl]-5,5-dimethyl-5,7-dihydro-6*H-*pyrrolo[2,3-*d*]pyrimidin-6-one |
| 13 | 4-amino-2-[5-chloro-3-(2-fluorobenzyl)-1*H*-indazol-1-yl]-5,5-dimethyl-5,7-dihydro-6*H-*pyrrolo[2,3-*d*]pyrimidin-6-one |
| 14 | 4-amino-2-[5-fluoro-3-(2-fluorobenzyl)-1*H*-indazol-1-yl]-5,5-dimethyl-5,7-dihydro-6*H-*pyrrolo[2,3-*d*]pyrimidin-6-one |
| 15 | 4-amino-2-[5-chloro-3-(2,3-difluorobenzyl)-1*H*-indazol-1-yl]-5,5-dimethyl-5,7-dihydro-6*H*-pyrrolo[2,3-*d*]pyrimidin-6-one |
| 16 | 4-amino-2-[3-(2,3-difluorobenzyl)-5-fluoro-1*H*-indazol-1-yl]-5,5-dimethyl-5,7-dihydro-6*H*-pyrrolo[2,3-*d*]pyrimidin-6-one |
| 17 | 4-amino-2-[3-(2,3-difluorobenzyl)-1*H*-indazol-1-yl]-5,5-dimethyl-5,7-dihydro-6*H-*pyrrolo[2,3-*d*]pyrimidin-6-one |
| 18 | 4-amino-2-[3-(2-fluorobenzyl)-5-phenyl-1*H*-indazol-1-yl]-5,5-dimethyl-5,7-dihydro-6*H-*pyrrolo[2,3-*d*]pyrimidin-6-one |
| 19 | 4-amino-2-[5-fluoro-3-(2,3,6-trifluorobenzyl)-1*H*-indazol-1-yl]-5,5-dimethyl-5,7-dihydro-6*H*-pyrrolo[2,3-*d*]pyrimidin-6-one |
| 20 | 4-amino-5,5-dimethyl-2-[5-pyridin-3-yl-3(2,3,6-trifluorobenzyl)1*H*-indazol-1-yl]-5,7-dihydro-6*H*-pyrrolo[2,3-*d*]pyrimidin-6-one |
| 21 | 4-amino-5,5-dimethyl-2-[5-(1-methyl-1*H*-pyrazol-4-yl)-3-(2,3,6-trifluorobenzyl)-1*H-*indazol-1-yl]-5,7-dihydro-6*H*-pyrrolo[2,3-*d*]pyrimidin-6-one |
| 22 | 4-amino-2-[5-(3,5-dimethyl-1*H*-pyrazol-4-yl)-3-(2,3,6-trifluorobenzyl)-1*H*-indazol-1-yl]-5,5-dimethyl-5,7-dihydro-6*H*-pyrrolo[2,3-*d*]pyrimidin-6-one |
| 23 | 4-amino-2-[5-(3-furyl)-3-(2,3,6-trifluorobenzyl)-1*H*-indazol-1-yl]-5,5-dimethyl-5,7-dihydro-6*H*-pyrrolo[2,3-*d*]pyrimidin-6-one |
| 24 | 4-amino-5,5-dimethyl-2-[5-(4-methyl-3-thienyl)-3-(2,3,6-trifluorobenzyl)-1*H*-indazol-yl]-5,7-dihydro-6*H*-pyrrolo[2,3-*d*]pyrimidin-6-one |
| 25 | 4-amino-2-[5-cyclopropyl-3-(2,3,6-trifluorobenzyl)-1*H*-indazol-1-yl]-5,5-dimethyl-5,7-dihydro-6*H*-pyrrolo[2,3-*d*]pyrimidin-6-one |
| 26 | 4-amino-5,5-dimethyl-2-[5-pyridin-4-yl-3-(2,3,6-trifluorobenzyl)-1*H*-indazol-1-yl]-5,7-dihydro-6*H*-pyrrolo[2,3-*d*]pyrimidin-6-one |
| 27 | 4-amino-5,5-dimethyl-2-[5-phenyl-3-(2,3,6-trifluorobenzyl)-1*H*-indazol-1-yl]-5,7-dihydro-6*H*-pyrrolo[2,3-*d*]pyrimidin-6-one |
| 28 | 4-amino-2-[5-chloro-3-(pyrimidin-5-ylmethyl)-1*H*-indazol-1-yl]-5,5-dimethyl-5,7-dihydro-6*H*-pyrrolo[2,3-*d*]pyrimidin-6-one |
| 29 | 4-amino-5,5-dimethyl-2-[5-(3-thienyl)-3-(2,3,6-trifluorobenzyl)-1*H*-indazol-1-yl]-5,7-dihydro-6*H*-pyrrolo[2,3-*d*]pyrimidin-6-one |
| 30 | 4-amino-2-[5-(5-fluoropyridin-3-yl)-3-(2,3,6-trifluorobenzyl)-1*H*-indazol-1-yl]-5,5-dimethyl-5,7-dihydro-6*H*-pyrrolo[2,3-*d*]pyrimidin-6-one |
| 31 | 4-amino-2-[5-(6-fluoropyridin-3-yl)-3-(2,3,6-trifluorobenzyl)-1*H*-indazol-1-yl]-5,5-dimethyl-5,7-dihydro-6*H*-pyrrolo[2,3-*d*]pyrimidin-6-one |
| 32 | 4-amino-5,5-dimethyl-2-{3-(2,3,6-trifluorobenzyl)-5-[5-(trifluoromethyl)pyridin-3-yl]-1*H*-indazol-1-yl}-5,7-dihydro-6*H*-pyrrolo[2,3-*d*]pyrimidin-6-one |
| 33 | 4-amino-2-[3-(6-bromo-2,3-difluorobenzyl)-5-chloro-1*H*-indazol-1-yl]-5,5-dimethyl-5-dihydro-6*H*-pyrrolo[2,3-*d*]pyrimidin-6-one |
| 34 | 4-amino-2-[3-(2-cyclopentylethyl)-1*H*-indazol-1-yl]-5,5-dimethyl-5,7-dihydro-6*H-*pyrrolo[2,3-*d*]pyrimidin-6-one |
| 35 | 4-amino-2-(5-fluoro-3-pentyl-1*H*-indazol-1-yl)-5,5-dimethyl-5,7-dihydro-6*H-*pyrrolo[2,3-*d*]pyrimidin-6-one |
| 36 | 4-amino-2-[5-fluoro-3-(3,3,3-trifluoropropyl)-1*H*-indazol-1-yl]-5,5-dimethyl-5,7-dihydro-6*H*-pyrrolo[2,3-*d*]pyrimidin-6-one |
| 37 | 4-amino-2-[3-(2-cyclopentylethyl)-5-fluoro-1*H*-indazol-1-yl]-5,5-dimethyl-5,7-dihydro-dihydro-6*H*-pyrrolo[2,3-*d*]pyrimidin-6-one |
| 38 | 4-amino-2-[3-(2-cyclopentylethyl)-5-fluoro-1*H*-indazol-1-yl]-5,5-dimethyl-5,7-dihydro-6*H*-pyrrolo[2,3-*d*]pyrimidin-6-one |
| 39 | 4-amino-2-[5-fluoro-3-(4,4,4-trifluorobutyl)-1*H*-indazol-1-yl]-5,5-dimethyl-5,7-dihydro-6*H*-pyrrolo[2,3-*d*]pyrimidin-6-one |
| 40 | 4-amino-2-(5-chloro-3-pentyl-1*H*-indazol-1-yl)-5,5-dimethyl-5,7-dihydro-6H-pyrrolo[2,3-*d*]pyrimidin-6-one |
| 41 | 4-amino-2-(3-butyl-5-chloro-1*H*-indazol-1-yl)-5,5-dimethyl-5,7-dihydro-6*H*-pyrrolo[2 *d*]pyrimidin-6-one |
| 42 | 4-amino-2-[5-chloro-3-(4,4,4-trifluorobutyl)-1*H*-indazol-1-yl]-5,5-dimethyl-5,7-dihydro 6*H*-pyrrolo[2,3-*d*]pyrimidin-6-one |
| 43 | 4-amino-2-(5-chloro-3-pent-4-en-1-yl-1*H*-indazol-1-yl)-5,5-dimethyl-5,7-dihydro-6*H-*pyrrolo[2,3-*d*]pyrimidin-6-one |
| 44 | 4-amino-2-(3-but-3-en-1-yl-5-chloro-1*H*-indazol-1-yl)-5,5-dimethyl-5,7-dihydro-6*H-*pyrrolo[2,3-*d*]pyrimidin-6-one |
| 45 | 4-amino-2-(5-chloro-3-propyl-1*H*-indazol-1-yl)-5,5-dimethyl-5,7-dihydro-6*H-*pyrrolo[2,3-*d*]pyrimidin-6-one |
| 46 | ethyl 3-[1-(4-amino-5,5-dimethyl-6-oxo-6,7-dihydro-5*H*-pyrrolo[2,3-*d*]pyrimidin-2-yl) 5-chloro-1*H*-indazol-3-yl-]propanoate |
| 47 | 4-amino-2-[5-chloro-3-(3,3-dimethylbutyl)-1*H*-indazol-1-yl]-5,5-dimethyl-5,7-dihydro-6*H*-pyrrolo[2,3-*d*]pyrimidin-6-one |
| 48 | 4-amino-2-[3-(2,3-diflurobenzyl)-1*H*-thieno[3,4-*c*]pyrazol-1-yl]-5,5-dimethyl-5,7-dihydro-6*H*-pyrrolo[2,3-*d*]pyrimidin-6-one |
| 49 | 4-amino-2-[6-chloro-3-(2,3-difluorobenzyl)-1*H*-thieno[3,4-*c*]pyrazol-1-yl]-5,5-dimethyl-5,7-dihydro-6*H*-pyrrolo[2,3-*d*]pyrimidin-6-one |
| 50 | 4-amino-2-[5-chloro-3-(2,3-difluorobenzyl)-1*H*-thieno[2,3-*c*]pyrazol-1-yl]-5,5-dimethyl-5,7-dihydro-6*H*-pyrrolo[2,3-*d*]pyrimidin-6-one |
| 51 | 4-amino-2-[3-(2,3-difluorobenzyl)-1*H*-thieno[3,2-*c*]pyrazol-1-yl]-5,5-dimethyl-5,7-dihydro-6*H*-pyrrolo[2,3-*d*]pyrimidin-6-one |
| 52 | 4-amino-2-[5-chloro-3-(2,3,6-trifluorobenzyl)-1*H*-thieno[2,3-*c*]pyrazol-1-yl]-5,5-dimethyl-5,7-dihydro-6*H*-pyrrolo[2,3-*d*]pyrimidin-6-one |
| 53 | 4-amino-5,5-dimethyl-2-[3-(2,3,6-trifluorobenzyl)-1H-thieno[3,2-c]pyrazol-1-yl]-5,7-dihydro-6H-pyrrolo[2,3-d]pyrimidin-6-one |
| 54 | 4-amino-dimethyl-2-[5-methyl-3-(2,3,6-trifluorobenzyl)pyrazolo[4,3-c]pyrazol-1(5H)-5,7-dihydro-6H-pyrrolo[2,3-d]pyrimidin-6-one |
| 55 | 4-amino-5,5-dimethyl-2-[3-(2,3,6-trifluorobenzyl)-1H-thieno[2,3-c]pyrazol-1-yl]-5,7-dihydro-6H-pyrrolo[2,3-d]pyrimidin-6-one |
| 56 | 4-amino-5,5-dimethyl-2-[6-methyl-3-(2,3,6-trifluorobenzyl)pyrazolo[3,4-c]pyrazol-1(6H)-yl]-5,7-dihydro-6H-pyrrolo[2,3-d]pyrimidin-6-one |
| 57 | 4-amino-5,5-dimethyl-2-[3-(2,3,6-trifluorobenzyl)-1H-pyrazolo[4,3-c]pyrilidin-1-yl]-5, dihydro-6H-pyrrolo[2,3-d]pyrimidin-6-one |
| 58 | 4-amino-5,5-dimethyl-2-[7-(2,3,6-trifluorobenzyl)imidazo[1,5-b]pyridazin-5-yl]-5,7-dihydro-6H-pyrrolo[2,3-d]pyrimidin-6-one |
| 59 | 4-amino-2-[6-chloro-3-(2,3,6-trifluorobenzyl)imidazo][1,5-a]pyridin-1-yl]-5,5-dimethy 5,7-dihydro-6H-pyrrolo[2,3-d]pyrimidin-6-one |
| 60 | 4-amino-2-[fluoro-3-(2,3,6-trifluorobenzyl)imidazo[1,5-a]pyridin-1-yl]-5,5-dimethyl 5,7-dihydro-6H-pyrrolo[2,3-d]pyrimidin-6-one |
| 61 | 4-amino-5,5-dimethyl-2-[5-(2,3,6-trifluorobenzyl)imidazo[5,1-b][1,3]thiazol-7-yl]-5,7 dihydro-6H-pyrrolo[2,3-d]pyrimidin-6-one |
| 62 | 4-amino-5,5-dimethyl-2-[1-(2,3,6-trifluorobenzyl)imidazo[1,5-a]pyridin-3-yl]5,7-dihydro-6H-pyrrolo[2,3-d]pyrimidin-6-one |
| 63 | 4-amino-2-[3-(2,3-difluorobenzyl)imidazo[1,5-a]pyridin-1-yl]-5,5-dimethyl-5,7-dihyd 6H-pyrrolo[2,3-d]pyrimidin-6-one |
| 64 | 4-amino-2-[7-(2,3-difluorobenzyl)imidazo[1,5-b]pyridazin-5-yl]-5,5-dimethyl-5,7-dihydro-6H-pyrrolo[2,3-d]pyrimidin-6-one |
| 65 | 4-amino-2-[3-(2,3-difluorobenzyl)-6-fluoromidazo[1,5-a]pyridin-1-yl]-5,5-dimethyl-5,7-dihydro-6H-pyrrolo[2,3-d]pyrimidin-6-one |
| 66 | 4-amino-2-[7-(2-fluorobenzul)imidazo[1,5-b]pyridazin-5-yl]-5,5-dimethyl-5,7-dihydro 6H-pyrrolo[2,3-d]pyrimidin-6-one |
| 67 | 4-amino-2-[3-(2-fluorobenzyl)imidazo[1,5-a]pyridin-1-yl]-5,5-dimethyl-5,7-dihydro-6 pyrrolo[2,3-d]pyrimidin-6-one |
| 68 | 4-amino-2-[6-fluoro-3-(2-fluorobenzyl)imidazo[1,5-a]pyrimidin-1-yl]5,5-dimethyl-5,7-dihydro-6H-pyrrolo[2,3-d]pyrimidin-6-one |
| 69 | 4-amino-2-[6-chloro-3-(2-fluorobenzyl)imidazo[1,5-a]pyridin-1-yl]5,5-dimethyl-5,7-dihydro-6H-pyrrolo[2,3-d]pyrimidin-6-one |
| 70 | 4-amino-2-[7-(2,3-difluorobenzyl)-2-methylimidazo[1,5-b]pyridazin-5-yl]-5,5-dimethy 5,7-dihydro-6H-pyrrolp[2,3-d]pyrimidin-6-one |
| 71 | 4-amino-2-[6-chloro-3-(2,3-difluorobenzyl)imidazo[1,5-a]pyridin-1-yl]-5,5-dimethyl-5,7-dihydro-6H-pyrrolo[2,3-d]pyrimidin-6-one |
| 72 | 4-amino-2-(3-benzylimidazo[1,5-a]pyridin-1-yl)-5,5-dimethyl-5,7-dihydro-6H-pyrrolo[2,3-d]pyrimidin-6-one |
| 73 | 4-amino-5,5-dimethyl-2-[3-(2,3,6-trifluorobenzyl)imidazo[1,5-a]pyriklin-1-yl]-5,7-dihydro-6H-pyrrolo[2,3-d]pyrimidin-6-one |
| 74 | 4-amino-5,5-dimethyl-2-[6-phenyl-3-(2,3,6-trifluorobenzyl)imidazo[1,5-a]pyridin-1-yl 5,7-dihydro-6H-pyrrolo[2,3-d]pyrimidin-6-one |
| 75 | 4-amino-2-[6-(2-fluorophenyl)-3-(2,3,6-trifluorobenzyl)imidazo[1,5-a]pyridin-1-yl]-5, dimethyl-5,7-dihydro-6H-pyrrolo[2,3-d]pyrimidin-6-one |
| 76 | 4-amino-2-[6-(3-fluorophenyl)-3-(2,3,6-trifluorobenzyl)imidazo[2,3-a]pyridin-1-yl]-5, dimethyl-5,7-dihydro-6H-pyrrolo[2,3-d]pyrimidin-6-one |
| 77 | 4-amino-2-[6-(4-fluorophenyl)-3-(2,3,6-trifluorobenzyl)imidazo[1,5-a]pyridin-1-yl]-5, dimethyl-5,7-dihydro-6H-pyrrolo[2,3-d]pyrimidin-6-one |
| 78 | 4-amino-2-[6-(3-chlorophenyl)-3-(2,3,6-trifluorobenzyl)imidazo[1,5-a]pyridin-1-yl]-5, dimethyl-5,7dihydro-6H-pyrrolo[2,3-d]pyrimidin-6-one |
| 79 | 4-amino-5,5-dimethyl-2-[6-(3-thienyl)-3-(2,3,6-trifluorobenzyl)imidazo[1,5-a]pyridin-yl]-5,7-dihydro-6H-pyrrolo[2,3-d]pyrimidin-6-one |
| 80 | 4-amino-2-[6-cyclopropyl-3-(2,3,6-trifluorobenzyl)imidazo[1,5-a]pyridin-1-yl]-5,5-dimethyl-5,7-dihydro-6H-pyrrolo[2,3-d]pyrimidin-6-one |
| 81 | 4-amino-5,5-dimethyl-2-[7-(3,3,3-trifluoropropyl)imidazo[1,5-b]pyridazin-5-yl]-5,7-dihydro-6H-pyrrolo[2,3-d]pyrimidin-6-one |
| 82 | 4-amino-5,5-dimethyl-2-[3-(2,4,6-trifluorobenzyl)imidazo[1,5-a]pyridin-1-yl]-5,7-dihydro-6H-pyrrolo[2,3-d]pyrimidin-6-one |
| 83 | 4-amino-2-[3-(2-chloro-6-fluoro-3-methylbenzyl)imidazo[1,5-a]pyridin-1-yl]5,5-dimethyl-5,7-dihydro-6H-pyrrolo[2,3-d]pyrimidin-6-one |
| 84 | 4-amino-2-[3-(2-cyclopentylethyl)imidazo[1,5-a]pyridin-1-yl]5,5-dimethyl-5,7-dihydro 6H-pyrrolo[2,3-d]lpyrimidin-6-one |
| 85 | 4-amino-5,5-dimethyl-2-[7-(4,4,4-trifluorobutyl)imidazo[1,5-b]pyridazin-5-yl]-5,7-dihydro-6H-pyrrolo[2,3-d]pyrimidin-6-one |
| 86 | 4-amino-5,5-dimethyl-2-[3-(4,4,4-trifluorobutyl)imidazo[1,5-a]pyridin-1-yl]-5,7-dihydro-6H-pyrrolo[2,3-d]pyrimidin-6-one |
| 87 | 4-amino-5,5-dimethyl-2-{3-[2-(2-thienyl)ethyl]imidazo[1,5-a]pyridin-1-yl}-5,7-dihydro 6H-pyrrolo[2,3-d]pyrimidin-6-one |
| 88 | 4-amino-2-[3-(2-cyclopropylethyl)imidazo[1,5-a]pyridin-1-yl]-5,5-dimethyl-5,7-dihydro 6*H*-pyrrolo[2,3-*d*]pyrimidin-6-one |
| 89 | 4-amino-5,5-dimethyl-2-(3-pentylimidazo[1,5-*a*]pyridin-1-yl)-5,7-dihydro-6*H-*pyrrolo[2,3-*d*]pyrimidin-6-one |
| 90 | 4-amino-5,5-dimethyl-2-(7-pentylimidazo[1,5-*b*]pyridazin-5-yl)-5,7-dihydro-6*H-*pyrrolo[2,3-*d*]pyrimidin-6-one |
| 91 | 4-amino-5,5-dimethyl-2-[3-(3-methylbutyl)imidazo[1,5-a]pyridin-1-yl]-5,7-dihydro-6*H* pyrrolo[2,3-*d*]pyrimidin-6-one |
| 92 | 4-amino-5,5-dimethyl-2-[3-methyl-5-(2,3,6-trifluorobenzyl)imidazo[5,1-*b*][1,3]thiazol 7-yl]-5,7-dihydro-6*H*-pyrrolo[2,3-*d*]pyrimidin-6-one |
| 93 | 4-amino-5,5-dimethyl-2-[2-methyl-5-(2,3,6-trifluorobenzyl)imidazo[5,1-*b*][1,3]thiazol 7-yl]-5,7-dihydro-6*H*-pyrrolo[2,3-*d*]pyrimidin-6-one |
| 94 | 4-amino-2-[5-(2-fluorobenzyl)imidazo[5,1-*b*][1,3]thiazol-7-yl]-5,5-dimethyl-5,7-dibydro-6H-pyrrolo[2,3-*d*]pyrimidin-6-one |
| 95 | 4-amino-5,5-dimethyl-2-[1-(3,3,3-trifluoropropyl)-1*H*-indazol-3-yl]-5,7-dihydro-6*H-*pyrrolo[2,3-*d*]pyrimidin-6-one |
| 96 | 4-amino-2-[5-chloro-3-(2,3,6-trifluorobenzyl)-1*H*-indazol-1-yl]5,5-dimethyl-5,8-dihydropyrido[2,3-d*]*pyrimidin-7(6*H*)-one |
| 97 | 4-amino-5,5-dimethyl-2-[3-(2,3,6-trifluorobenzyl)-1*H*-indazol-1-yl]-5,8-dihydropyrido[2,3-*d*]pyrimidin-7(6*H*)-one |
| 98 | 4-amino-5,5-dimethyl-2-[3-(2,3,6-trifluorobenzyl)-1*H*-thieno[3,4-*c*]pyrazol-1-yl-5,8-dihydropyrido[2,3-*d*]pyrimidin-7(6*H*)-one |
| 99 | 4-amino-5,5-dimethyl-2-[3-(2,3,6-trifluorobenzyl)-4,6-dihydro-1*H*-thieno[3,4-*c*]pyrazo 1-yl]-5,8-dihydropyrido[2,3-d]pyrimidin-7(6*H*)-one |
| 100 | 4-amino-2-[5-chloro-3-(3,3,3-trifluoropropyl)-1*H*-indazol-1-yl]-5-ethyl-5-methyl-5,7-dihydro-6*H*-pyrrolo[2,3-*d*]pyrimidin-6-one |
| 101 | 4-amino-2-[5-chloro-3-(3,3,3-trifluoropropyl)-1*H*-indazol-1-yl]-5-methyl-5-propyl-5,7 dihydro-6*H*-pyrrolo[2,3-*d*]pyrimidin-6-one |
| 102 | 4-amino-2-[5-chloro-3-(3,3-dimethylbutyl)-1*H*-indazol-1-yl]-5-ethyl-5-methyl-5,7-dihydro-6*H*-pyrrolo[2,3-d]pyrimidin-6-one |
| 103 | 4amino-5-ethyl-2-[3-(2-fluorobenzyl)imidazo[1,5-*a*]pyridin-1-yl]-5-methyl-5,7-dihyd 6*H*-pyrrolo[2,3-*d*]pyrimidin-6-one |
| 104 | 4-amino-5,5-dimethyl-2-[3-(3,3,4,4,4-pentafluorobutyl)-1*H*-indazol-1-yl]-5,7-dihydro-6H-pyrrolo[2,3-d]pyrimidin-6-one |
| 105 | 4-amino-2-[5-fluoro-3-(3,3,4,4,4-pentafluorobutyl)-1H-indazol-1-yl]-5,5-dimethyl-5,7 dihydro-6H-pyrrolo[2,3-d]pyrimidin-6-one |
| 106 | 4-amino-2-[5-chloro-3-(3,3,4,4,4-pentafluorobutyl)-1H-indazol-1-yl]-5,5-dimethyl-5,7 dihydro-6H-pyrrolo[2,3-d]pyrimidin-6-one |
| 107 | 4-amino-5,5-dimethyl-2-[3-(3,3,4,4,4-pentafluorobutyl)-1H-pyrazolo[4,3-b]pyridin-1-yl]-5,7-dihydro-6H-pyrrolo[2,3-d]pyrimidin-6-one |
| | |
| 108 | 4-amino-5,5-dimethyl-2-[3-(3,3,4,4,4-pentafluorobutyl)imidazo[1,5-a]pyridin-1-yl]-5, dihydro-6H-pyrrolo[2,3-d]pyrimidin-6-one |
| 109 | 4-amino-2-[6-fluoro-3-(3,3,4,4,4-pentafluorobutyl)imidazo[1,5-a]pyridin-1-yl]-5,5-dimethyl-5,7-dihydro-6H-pyrrolo[2,3-d]pyrimidin-6-one |
| 110 | 4-amino-2-[6-chloro-3-(3,3,4,4,4-pentafluorobutyl)imidazo[1,5-a]pyridin-1-yl]-5,5-dimethyl-5,7-dihydro-6H-pyrrolo[2,3-d]pyrimidin-6-one |
| 111 | 4-amino-2-[6-chloro-1-(3,3,4,4,4-pentafluorobutyl)-1H-indazol-3-yl]-5,5-dimethyl-5,7 dihydro-6H-pyrrolo[2,3-d]pyrimidin-6-one |

### Compound class II:

### Examples 1 to 45 of compound class II are disclosed in WO2009/123316.

### Example 1:

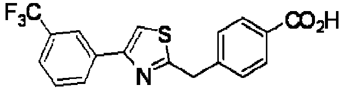

### Example 2:

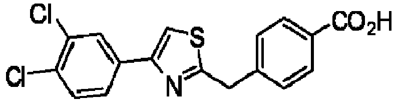

### Example 3:

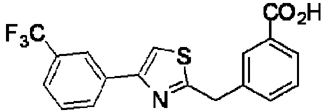

### Example 4:

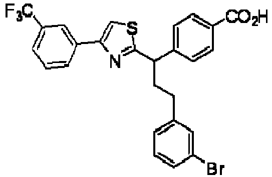

### Example 5:

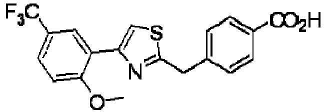

### Example 6:

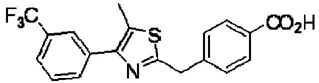

### Example 7:

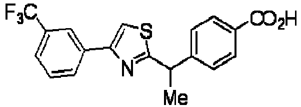

### Example 8:

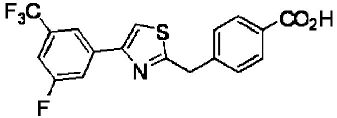

### Example 9:

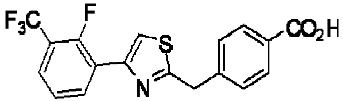

### Example 10:

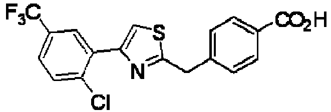

### Example 11:

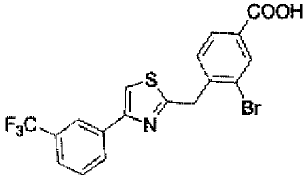

### Example 12:

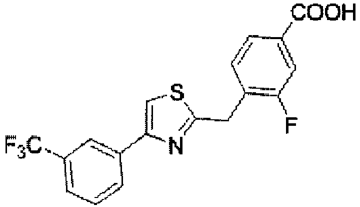

### Example 13:

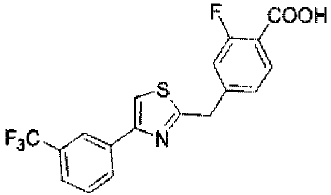

### Example 14:

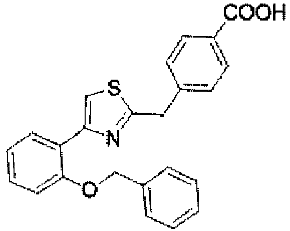

### Example 15:

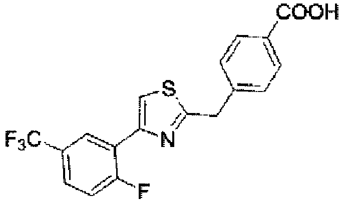

### Example 16:

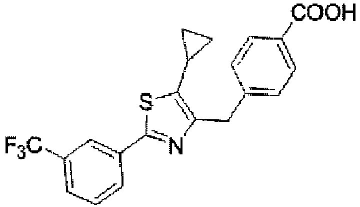

### Example 17:

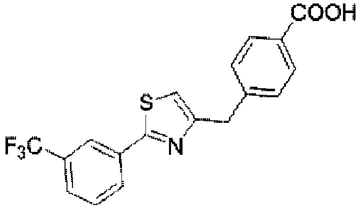

### Example 18:

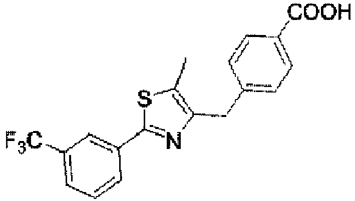

### Example 19:

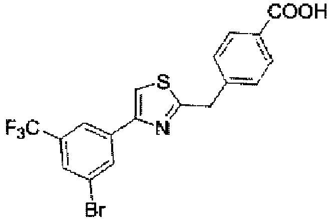

### Example 20:

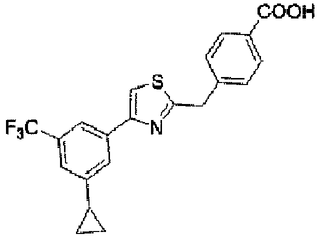

### Example 21:

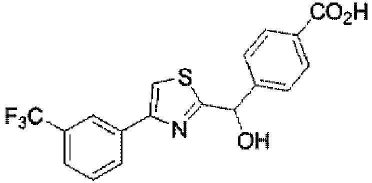

### Example 22:

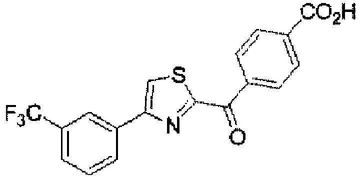

### Example 23:

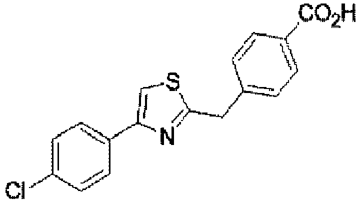

### Example 24:

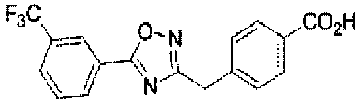

### Example 25:

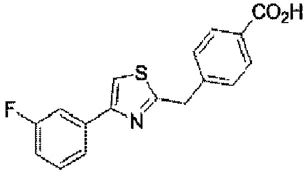

### Example 26:

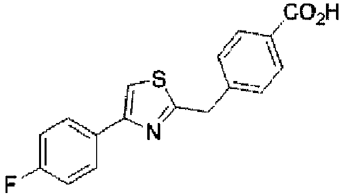

### Example 27:

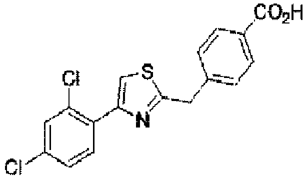

### Example 28:

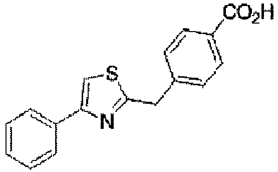

### Example 29:

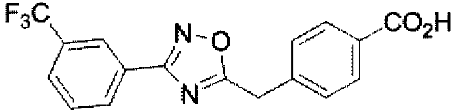

### Example 30:

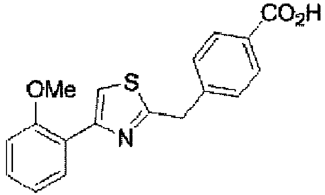

### Example 31:

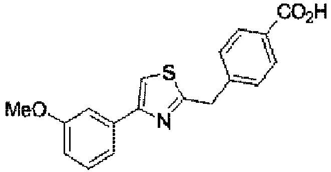

### Example 32:

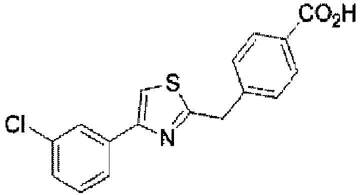

### Example 33:

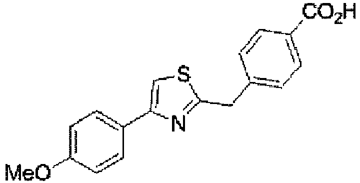

### Example 34:

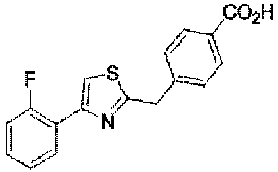

### Example 35:

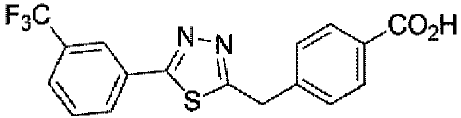

### Example 36:

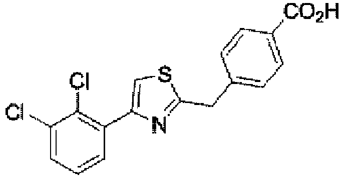

### Example 37:

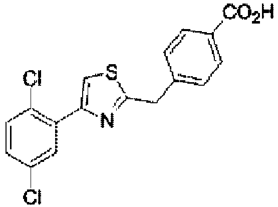

### Example 38:

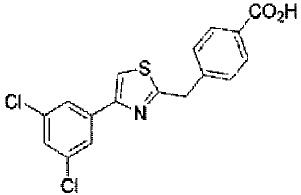

### Example 39:

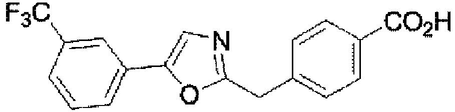

### Example 40:

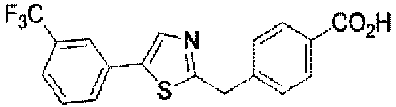

### Example 41:

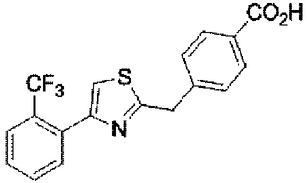

### Example 42:

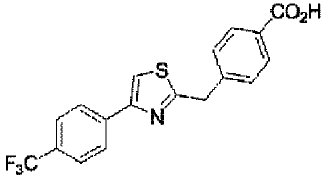

### Example 43:

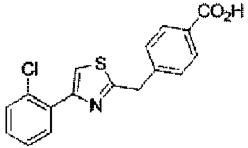

### Example 44:

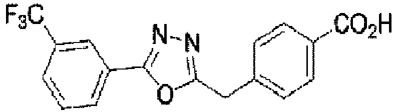

### Example 45:

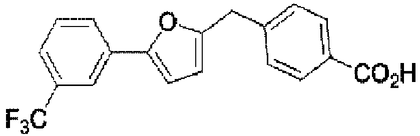

### Compound class III:

Examples 1 to 334 of compound class III are disclosed in WO2009/032249.

### Example 1

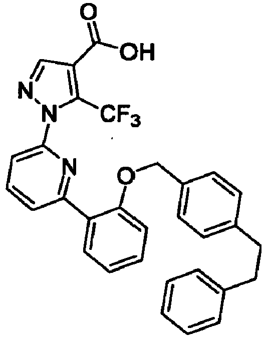

### Example 2

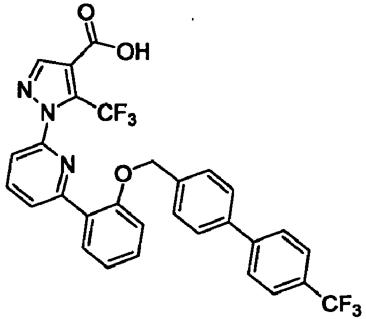

### Example 3

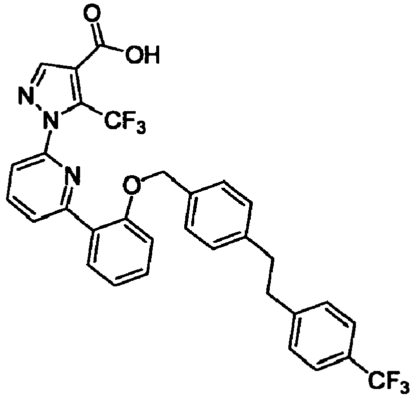

### Example 4

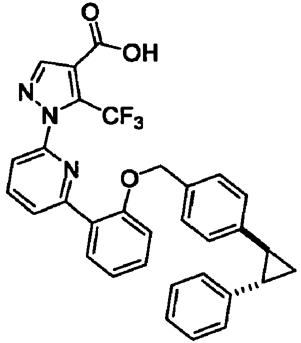

### Example 5

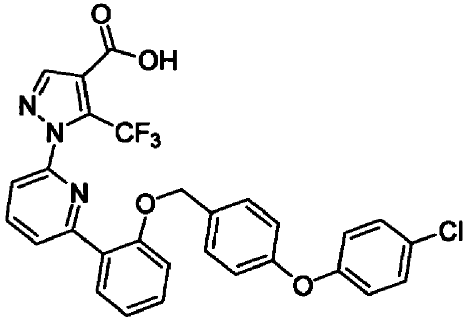

### Example 6

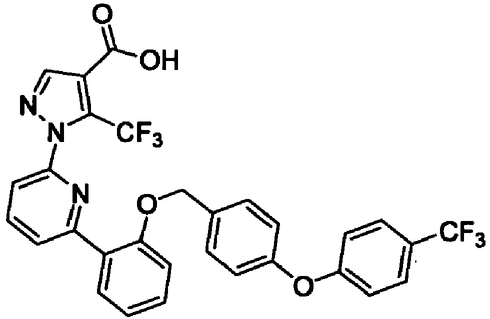

### Example 7

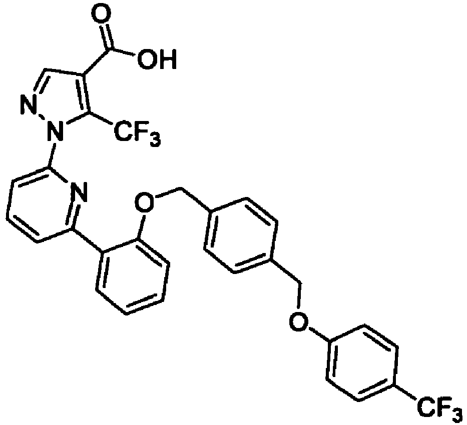

### Example 8

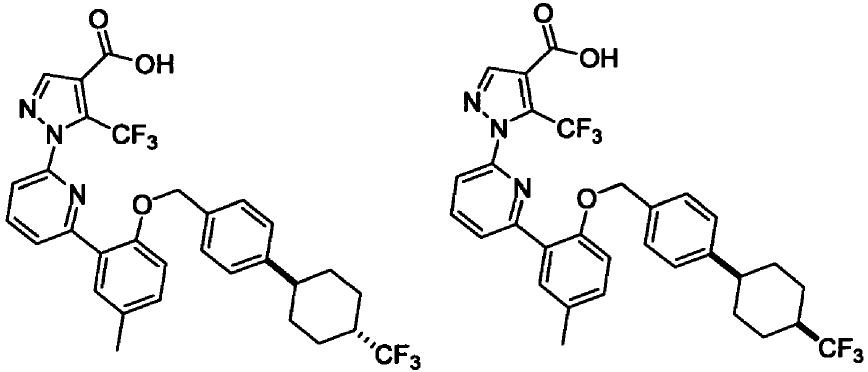

### Example 9

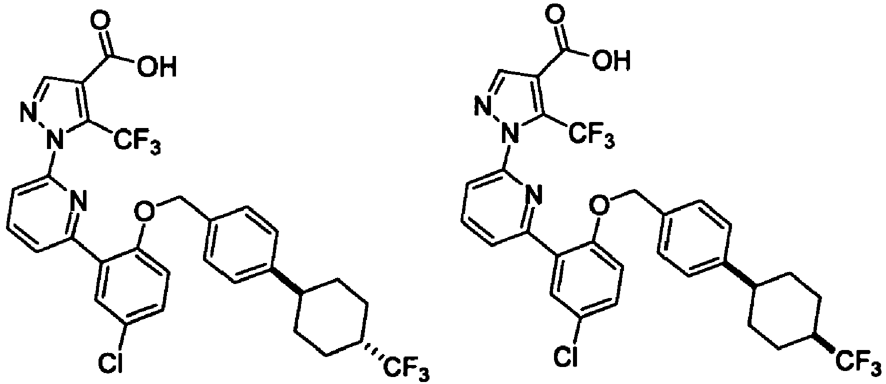

### Example 10

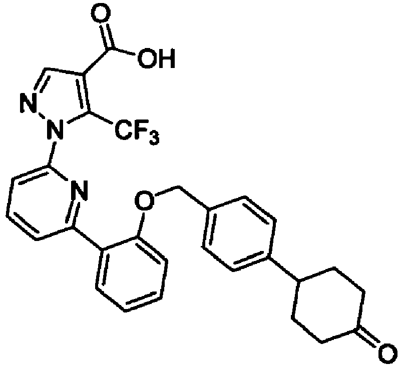

### Example 11

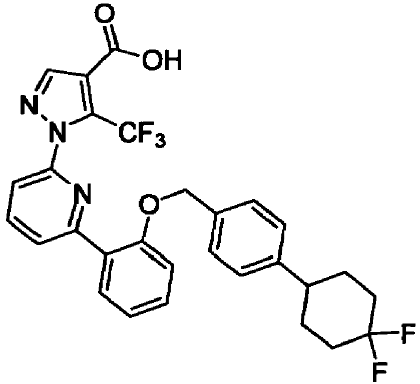

### Example 12

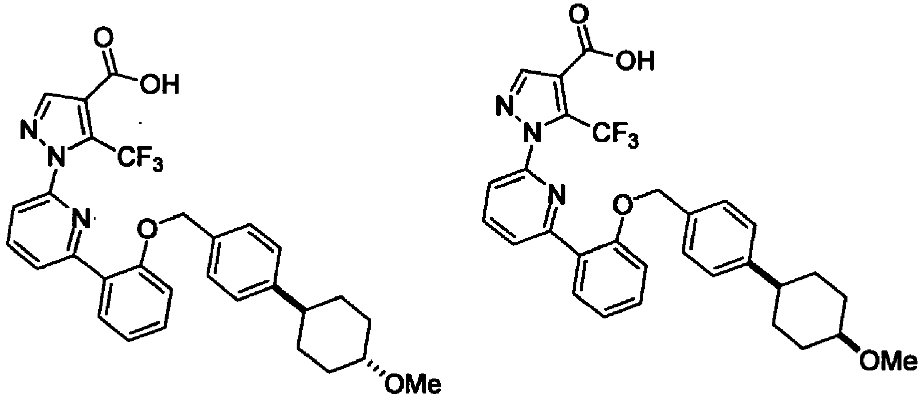

### Example 13

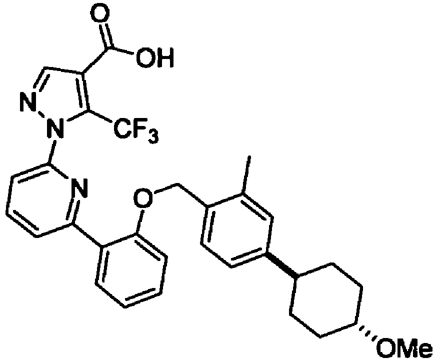

### Example 14

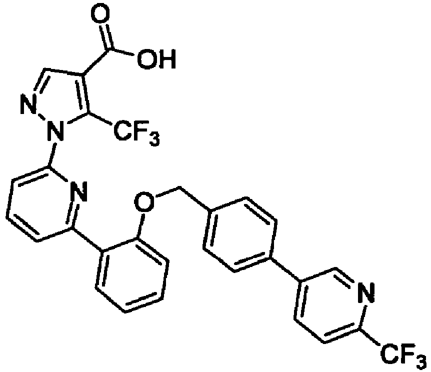

### Example 15

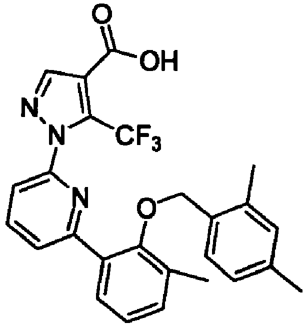

### Example 16

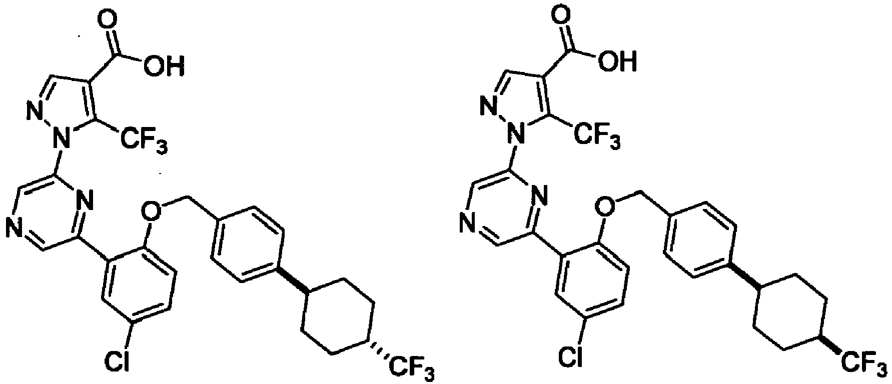

### Example 17

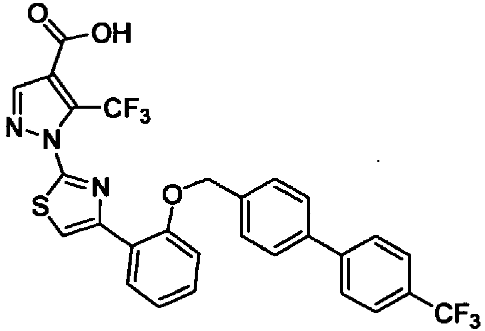

### Example 18

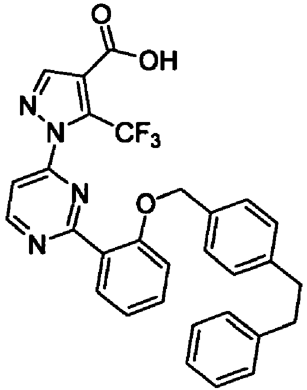

### Example 19

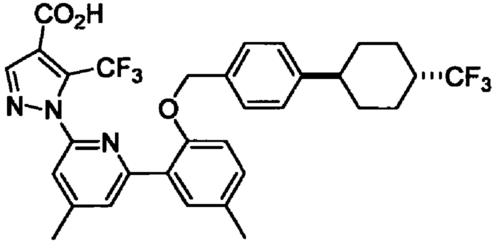

The compounds in **TABLE 1** were prepared using the chemistry described in **Examples 1-19.**

**TABLE 1**

| | | | | | |
|---|---|---|---|---|---|
| | | | | 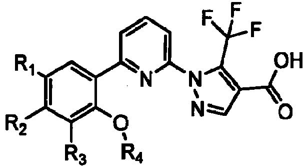 | |

| Entry | R1 | R2 | R3 | R4 | MS |
|---|---|---|---|---|---|
| 20 | -CF₃ | H | H | 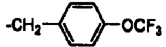 | 592.1 [M + H]⁺ |
| 21 | H | H | H | 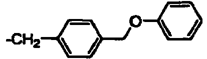 | 546.2 [M + H]⁺ |
| 22 | H | H | H | 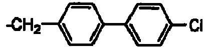 | 572.1 [M+Na]⁺ |
| 23 | H | H | H | 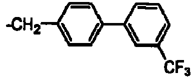 | 584.1 [M+H]⁺ |
| 24 | Me | H | H | 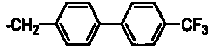 | 598.7 [M + H]⁺ |
| 25 | Me | H | H | 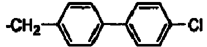 | 564.6 [M + H]⁺ |
| 26 | Me | H | H | 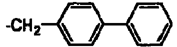 | 528.4 [M - H]⁻ |
| 27 | Br | H | H | 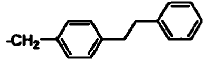 | 624.5 [M + H]⁺ |
| 29 | cPr | H | H | 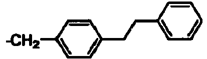 | 584.5 [M + H]⁺ |
| 30 | CF₃ | H | H | 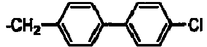 | 618.2 [M + H]⁺ |
| 31 | CF₃ | H | H | 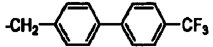 | 652.3 [M + H]⁺ |
| 32 | CF₃ | H | H | 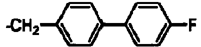 | 602.2 [M + H]⁺ |
| 33 | H | H | H | 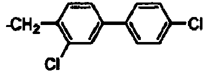 | 584.4 [M + H]⁺ |
| 34 | H | H | H | 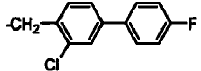 | 568.1 [M + H]⁺ |
| 35 | H | H | H | 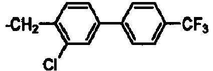 | 618.0 [M + H]⁺ |
| 36 | H | H | H | 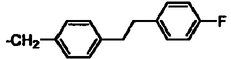 | 562.5 [M + H]⁺ |
| 37 | H | H | H | 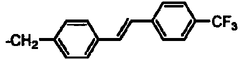 | 610.5 [M+H]⁺ |
| 38 | Me | H | H | 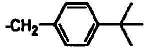 | 510.6 [M + H]⁺ |
| 39 | Me | H | H | 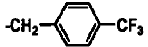 | 522.5 [M + H]⁺ |
| 40 | H | H | H | 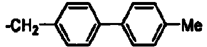 | 552.2 [M + Na]⁺ |
| 41 | H | H | H | 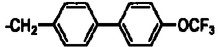 | 600.2 [M+H]⁺ |
| 42 | Me | H | H | 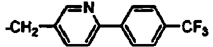 | 599.6 [M+ H]⁺ |
| 43 | H | H | H | 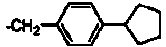 | 508.6 [M + H]⁺ |
| 44 | H | H | H | 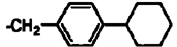 | 522.6 [M+ H]⁺ |
| 45 | H | H | H | 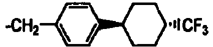 | 590.5 [M+H]⁺ |
| 46 | H | H | H | 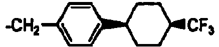 | 590.5 [M+H]⁺ |
| 47 | Me | H | I | 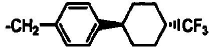 | 730.6 [M + H]⁺ |
| 48 | Cl | H | I | 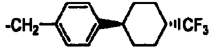 | 750.6 [M + H]⁺ |
| 49 | H | H | I | 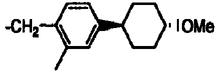 | 692.9 [M + H]⁺ |
| 50 | cPr | H | H | 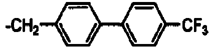 | 624.2 [M+H]⁺ |
| 51 | H | H | H | 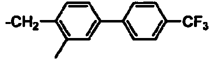 | 598.2 [M + H]⁺ |
| 52 | H | H | H | 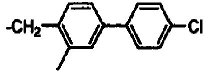 | 586.2 [M+Na]⁺ |
| 53 | H | H | H | 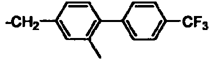 | 598.2 [M+H]⁺ |
| 54 | H | H | H | 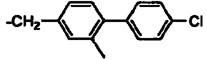 | 564.2 [M+H]⁺ |
| 55 | H | H | H | 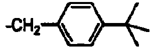 | 496.6 [M +H]⁺ |
| 56 | H | H | H | 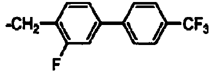 | 602.4 [M + H]⁺ |
| 57 | H | H | H | 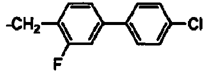 | 568.5 [M + H]⁺ |
| 58 | H | H | H | 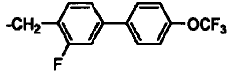 | 618.4 [M+H]⁺ |
| 59 | H | H | H | 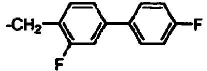 | 552.4 [M+H]⁺ |
| 60 | H | H | H | 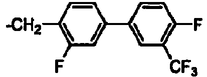 | 620.4 [M + H]⁺ |
| 61 | H | H | H | 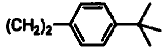 | 510.5 [M+H]⁺ |
| 62 | F | H | H | 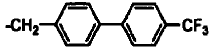 | 602.3 [M+ H]⁺ |
| 63 | F | H | H | 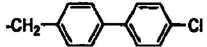 | 566.3 [M-H]- |
| 64 | F | H | H | 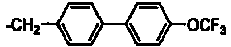 | 618.3 [M+H]⁺ |
| 65 | F | H | H | 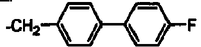 | 552.4 [M + H]⁺ |
| 66 | F | H | H | 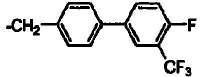 | 620.4 [M + H]⁺ |
| 67 | F | H | H | 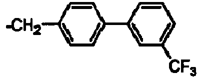 | 602.4 [M + H]⁺ |
| 68 | H | H | H | 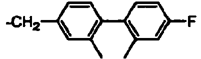 | 562.6 [M + H]⁺ |
| 69 | H | H | H | 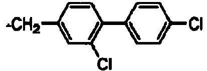 | 584.0 [M + H]⁺ |
| 70 | H | H | H | 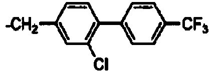 | 618.0 [M + H]⁺ |
| 71 | Me | H | H | 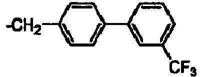 | 598.2 [M + H]⁺ |
| 72 | H | H | H | 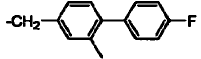 | 548.2 [M + H]⁺ |
| 73 | H | H | H | 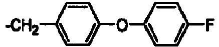 | 548.5 [M - H]⁻ |
| 74 | H | H | H | 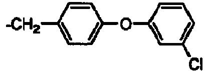 | 564.4 [M-H]⁻ |
| 75 | H | H | H | 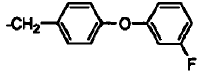 | 548.5 [M-H]⁻ |
| 76 | H | H | H | 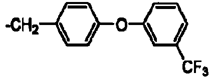 | 598.4 [M-H]⁻ |
| 77 | H | H | H | 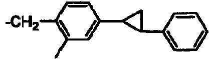 | 568.5 [M - H]⁻ |
| 78 | H | H | H | 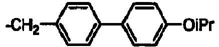 | 596.2 [M + Na]⁺ |
| 79 | H | H | H | 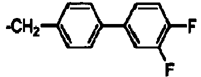 | 552.2 [M + H]⁺ |
| 80 | H | H | H | 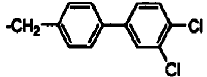 | 584.0 [M + H]⁺ |
| 81 | H | H | H | 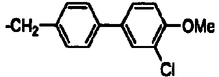 | 602.1 [M+Na]⁺ |
| 82 | H | H | H | 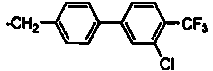 | 617.8 [M+H]⁺ |
| 83 | H | H | H | 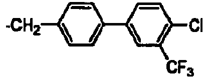 | 617.8 [M + H]⁺ |
| 84 | Me | H | H | 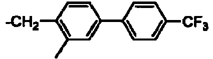 | 612.2 [M + H]⁺ |
| 85 | Me | H | H | 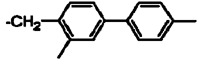 | 580.1 [M + Na]⁺ |
| 86 | Me | H | H | 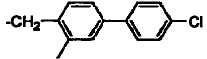 | 600.0 [M + Na]⁺ |
| 87 | Me | H | H | 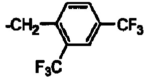 | 590.0 [M + H]⁺ |
| 88 | Me | H | H | 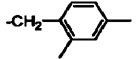 | 482.1 [M + H]⁺ |
| 89 | H | H | H | 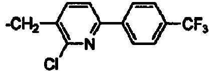 | 619.1 [M + H]⁺ |
| 90 | H | H | H | 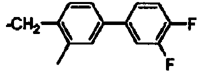 | 566.1 [M + H]⁺ |
| 91 | H | H | H | 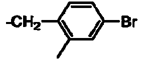 | 534.0 [M + H]⁺ |
| 92 | H | H | H | 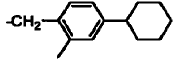 | 558.1 [M + Na]⁺ |
| 93 | H | H | H | 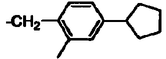 | 544.1 [M + Na]⁺ |
| 94 | H | H | H | 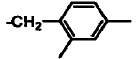 | 468.0 [M + H]⁺ |
| 95 | Me | H | H | 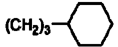 | 488.0 [M + H]⁺ |
| 96 | Me | H | H | 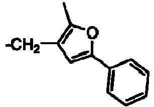 | 556.1 [M + Na]⁺ |
| 97 | Me | H | H | 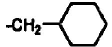 | 460.0 [M + H]⁺ |
| 98 | Me | H | H | 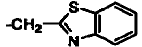 | 511.1 [M+H]⁺ |
| 99 | H | H | H | 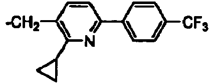 | 625.4 [M+H]⁺ |
| 100 | H | H | H | 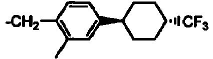 | 604.4 [M + H]⁺ |
| 101 | H | H | H | 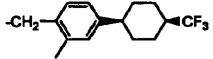 | 604.5 [M + H]⁺ |
| 102 | Me | H | H | 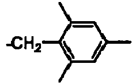 | 518.0 [M + Na]⁺ |
| 103 | CF3 | H | H | 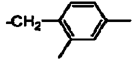 | 536.6 [M + H]⁺ |
| 104 | CF3 | H | H | 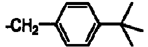 | 564.6 [M + H]⁺ |
| 105 | Me | H | H | 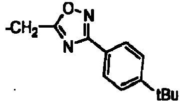 | 578.6 [M + H]⁺ |
| 106 | Me | H | H | 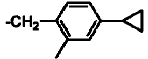 | 508.6 [M + H]⁺ |
| 107 | CF3 | H | H | 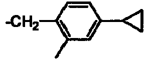 | 584.5 [M + H]⁺ |
| 108 | H | H | H | 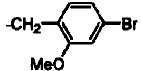 | 548.0 [M + H]⁺ |
| 109 | H | H | H | 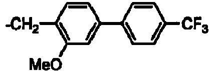 | 614.1 [M+H]⁺ |
| 110 | H | H | H | 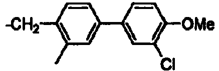 | 616.2 [M + Na]⁺ |
| 111 | H | H | H | 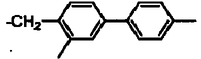 | 566.2 [M + Na]⁺ |
| 112 | H | H | H | 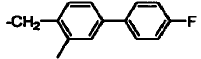 | 570.0 [M + Na]⁺ |
| 113 | H | H | H | 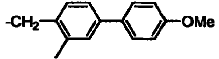 | 582.0 [M + Na]⁺ |
| 114 | H | H | H | 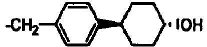 | 538.8 [M + H]⁺ |
| 115 | H | H | H | 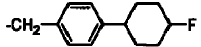 | 540.9 [M + H]⁺ |
| 116 | H | H | H | 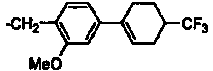 | 618.1 [M + H]⁺ |
| 117 | H | H | H | 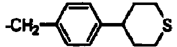 | 540.5 [M + H]⁺ |
| 118 | H | H | H | 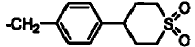 | 572.5 [M + H]⁺ |
| 119 | H | H | H | 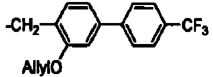 | 640.2 [M + H]⁺ |
| 120 | Me | H | H | 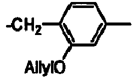 | 524.6 [M + H]⁺ |
| 121 | H | H | H | 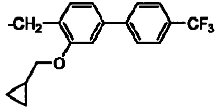 | 564.2 [M + H]⁺ |
| 122 | H | H | H | 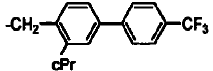 | 624.2 [M + H]⁺ |
| 123 | H | H | H | 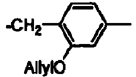 | 510.6 [M + H]⁺ |
| 124 | Me | H | H | 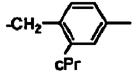 | 530.7 [M + H]⁺ |
| 125 | H | H | H | 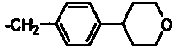 | 546.6 [M + Na]⁺ |
| 126 | Cl | H | H | 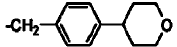 | 580.6 [M + Na]⁺ |
| 127 | H | H | H | 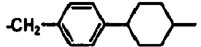 | 536.7 [M+H]⁺ |
| 128 | H | H | H | 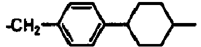 | 536.9 [M+ H]⁺ |
| 129 | Cl | H | H | 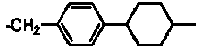 | 568.7 [M-H]⁻ |
| 130 | Cl | H | H | 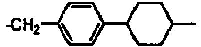 | 568.8 [M-H]⁻ |
| 131 | H | H | H | 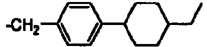 | 548.8 [M - H]⁻ |
| 132 | H | H | H | 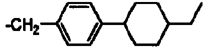 | 548.8 [M-H]⁻ |
| 133 | Cl | H | H | 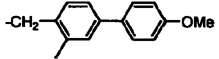 | 616.6 [M + Na]⁺ |
| 134 | Cl | H | H | 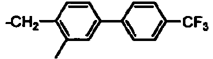 | 630.8 [M - H]⁻ |
| 135 | Cl | H | H | 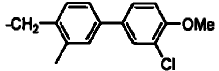 | 626.7 [M - H]⁻ |
| 136 | Me | H | H | 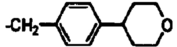 | 538.8 [M + H]⁺ |
| 137 | Me | H | H | 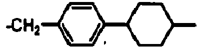 | 550.9 [M + H]⁺ |
| 138 | Me | H | H | 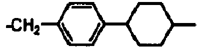 | 550.9 [M+ H]⁺ |
| 139 | H | H | H | 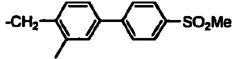 | 608.8 [M+ H]⁺ |
| 140 | Me | H | H | 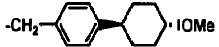 | 566.6 [M+ H]⁺ |
| 141 | Me | H | H | 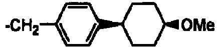 | 586.6 [M + H]⁺ |
| 142 | H | H | H | 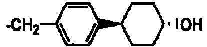 | 550.9 [M - H]⁻ |
| 143 | cPr | H | H | 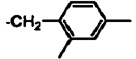 | 508.8 [M + H]⁺ |
| 144 | Cl | H | H | 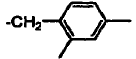 | 500.7 [M - H]⁻ |
| 145 | H | H | H | 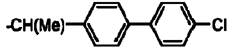 | 562.4 [M - H]⁻ |
| 146 | H | Cl | H | 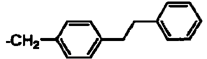 | 578.5 [M + H]⁺ |
| 147 | H | F | H | 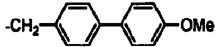 | 586.5 [M+Na]⁺ |
| 148 | H | F | H | 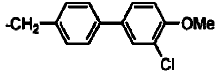 | 620.5 [M+Na]⁺ |
| 149 | H | F | H | 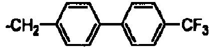 | 602.6 [M+H]⁺ |
| 150 | H | H | cPr | 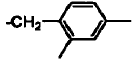 | 508.8 [M + H]⁺ |
| 151 | H | H | Cl | 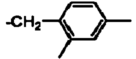 | 500.7 [M - H]⁻ |
| 152 | Me | H | Cl | 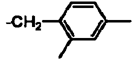 | 516.7 [M+H]⁺ |
| 153 | I | H | H | 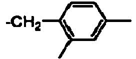 | 592.7 [M-H]⁻ |
| 154 | H | H | I | 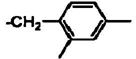 | 592.7 [M-H]⁻ |
| 155 | Me | H | Cl | 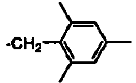 | 530.7 [M + H]⁺ |
| 156 | Me | H | Cl | 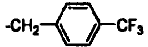 | 556.7 [M+H]⁺ |
| 157 | Me | H | Cl | 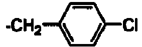 | 522.6 [M + H]⁺ |
| 158 | H | H | H | 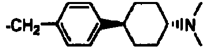 | 580.0 [M + H]⁺ |
| 159 | H | H | Me | 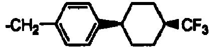 | 604.9 [M + H]⁺ |
| 160 | H | H | Me | 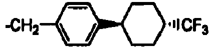 | 604.9 [M + H]⁺ |
| 161 | -CF₃ | H | H | 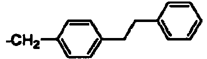 | 612.6 [M+ H]⁺ |
| 162 | Cl | H | H | 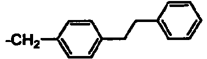 | 558.5 [M + H]⁺ |
| 163 | H | H | H | 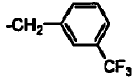 | 508.6 [M + H]⁺ |
| 164 | H | H | H | 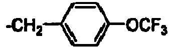 | 524.5 [M + H]⁺ |
| 165 | H | H | H | 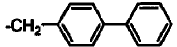 | 516.6 [M+H]⁺ |
| 166 | Cl | H | H | 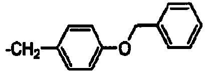 | 578.4 [M-H]⁻ |
| 167 | Cl | H | H | 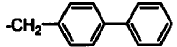 | 550.2 [M + H]⁺ |
| 168 | Cl | H | H | 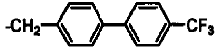 | 618.2 [M + H]⁺ |
| 169 | Cl | H | H | 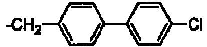 | 584.2 [M + H]⁺ |
| 170 | Cl | H | H | 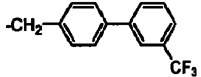 | 618.2 [M + H]⁺ |
| 171 | H | H | H | 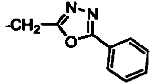 | 508.5 [M + H]⁺ |
| 172 | H | H | H | 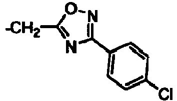 | 542.4 [M + H]⁺ |
| 173 | H | H | H | 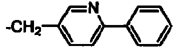 | 517.5 [M + H]⁺ |
| 174 | H | H | H | 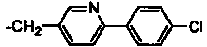 | 551.4 [M + H]⁺ |
| 175 | H | H | H | 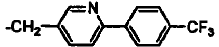 | 585.4 [M + H]⁺ |
| 176 | Me | H | H | 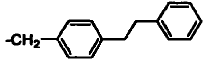 | 558.5 [M+H]⁺ |
| 177 | F | H | H | 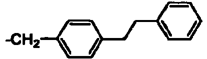 | 562.5 [M+ H]⁺ |

The compounds in TABLE 2 were prepared using the chemistry described in Examples 1-9.

**TABLE2**

| | | | | | |
|---|---|---|---|---|---|
| | | | | 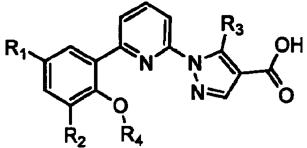 | |

| Entry | R1 | R2 | R3 | R4 | MS |
|---|---|---|---|---|---|
| 178 | H | H | Me | 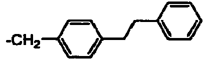 | 490.2 [M + H]⁺ |
| 179 | Cl | H | Me | 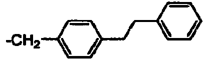 | 524.1 [M + H]⁺ |
| 180 | H | H | Me | 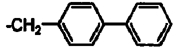 | 462.2 [M + H]⁺ |
| 181 | Cl | H | Me | 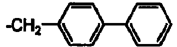 | 496.1 [M + H]⁺ |
| 182 | H | H | Me | 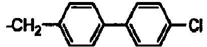 | 496.1 [M+ H]⁺ |
| 183 | H | H | Me | 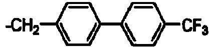 | 529.9 [M + H]⁺ |
| 184 | H | H | CF2H | 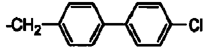 | 532.0 [M + H]⁺ |
| 185 | H | H | CF2H | 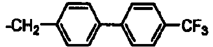 | 566.2 [M + H]⁺ |
| 186 | H | H | CF2H | 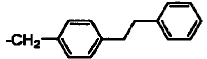 | 526.2 [M + H]⁺ |
| 187 | Cl | H | CF2H | 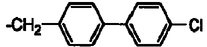 | 566.0 [M + H]⁺ |
| 188 | Cl | H | CF2H | 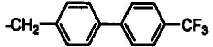 | 600.0 [M + H]⁺ |
| 189 | H | H | CF2H | 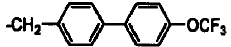 | 582.0 [M+ H]⁺ |
| 190 | H | H | CF2H | 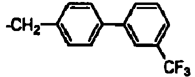 | 566.1 [M+H]⁺ |
| 191 | Me | H | CF2H | 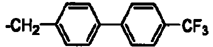 | 580.5 [M + H]⁺ |
| 192 | Me | H | CF2H | 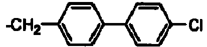 | 546.5 [M + H]⁺ |
| 193 | H | H | CF2H | 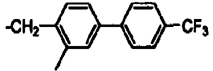 | 580.5 [M + H]⁺ |
| 194 | H | H | CF2H | 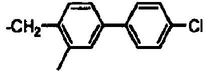 | 546.5 [M + H]⁺ |
| 195 | H | H | CF2H | 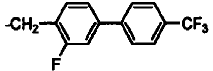 | 584.4 [M + H]⁺ |
| 196 | H | H | CF2H | 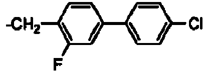 | 550.4 [M + H]⁺ |
| 197 | H | H | Et | 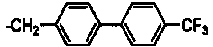 | 544.5 [M + H]⁺ |
| 198 | H | H | iPr | 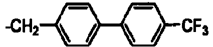 | 558.5 [M + H]⁺ |
| 199 | H | H | cPr | 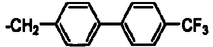 | 556.5 [M + H]⁺ |
| 200 | H | H | -CH₂OMe | 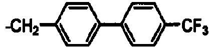 | 560.5 [M + H]⁺ |
| 201 | H | H | Et | 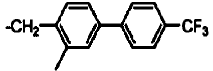 | 558.5 [M + H]⁺ |
| 202 | H | H | iPr | 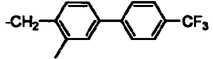 | 572.5 [M + H]⁺ |
| 203 | H | H | cPr | 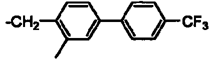 | 570.5 [M + H]⁺ |
| 204 | H | H | -CH₂OMe | 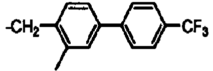 | 574.5 [M + H]⁺ |
| 205 | H | H | CF2H | 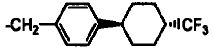 | 572.6 [M + H]⁺ |
| 206 | H | H | CF2H | 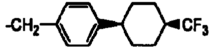 | 572.6 [M + H]⁺ |
| 207 | H | H | Pr | 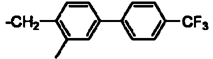 | 572.6 [M + H]⁺ |
| 208 | H | H | Ph | 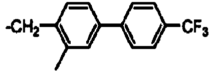 | 606.6 [M + H]⁺ |
| 209 | Cl | Cl | CF2H | 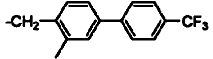 | 648.4 [M + H]⁺ |
| 210 | Cl | H | CF2H | 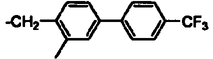 | 614.5 [M + H]⁺ |
| 211 | Cl | H | CF2H | 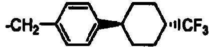 | 606.5 [M + H]⁺ |
| 212 | Cl | H | CF2H | 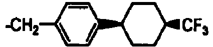 | 606.5 [M + H]⁺ |
| 213 | Cl | Cl | CF2H | 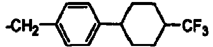 640.5 | 640.5 [M+ H]⁺ |
| 214 | H | H | CF2H | 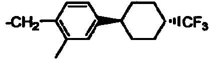 | 587.0 [M + H]⁺ |
| 215 | H | H | CF2H | 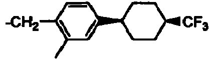 | 587.0 [M + H]⁺ |

The compounds in TABLE 3 were synthesized using chemistry described in Examples 1-19.

**TABLE 3**

| Entry | R | MS |
|---|---|---|
| 216 | 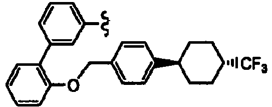 | 589.5 [M+ H]⁺ |
| 217 | 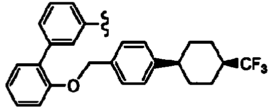 | 5 89.5 [M + H]⁺ |
| 218 | 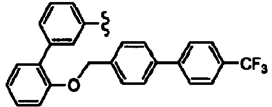 | 5 83.5 [M+ H]⁺ |
| 219 | 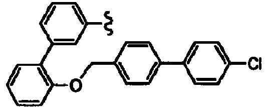 | 549.5 [M+ H]⁺ |
| 220 | 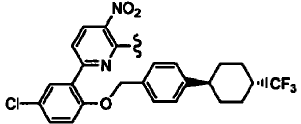 | 669.9 [M + H]⁺ |
| 221 | 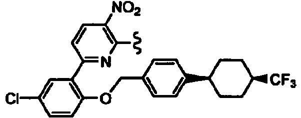 | 669.9 [M + H]⁺ |
| 222 | 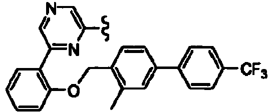 | 599.5 [M+ H]⁺ |
| 223 | 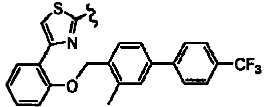 | 604.5 [M+ H]⁺ |
| 224 | 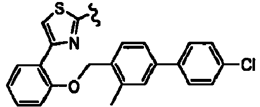 | 570.4 [M + H]⁺ |
| 225 | 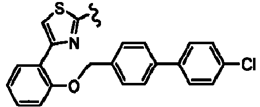 | 556.5 [M + H]⁺ |
| 226 | 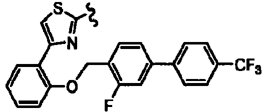 | 608.5 [M + H]⁺ |
| 227 | 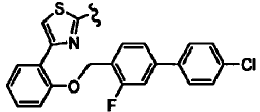 | 574.4 [M + H]⁺ |
| 228 | 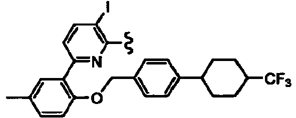 | 730.8 [M + H]⁺ |
| 229 | 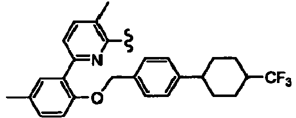 | 618.8 [M + H]⁺ |
| 230 | 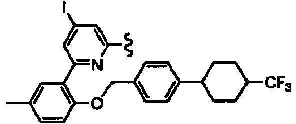 | 730.8 [M + H]⁺ |
| 231 | 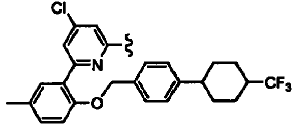 | 638.8 [M+H]⁺ |
| 232 | 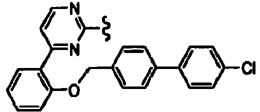 | 551.1 [M+H]⁺ |
| 233 | 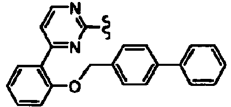 | 517.2 [M+ H]⁺ |
| 234 | 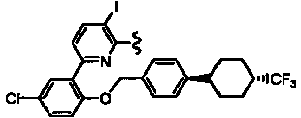 | 750.9 [M+ H]⁺ |
| 235 | 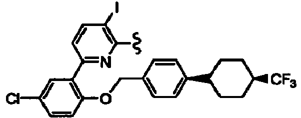 | 750.9 [M+H]⁺ |

### Example 236

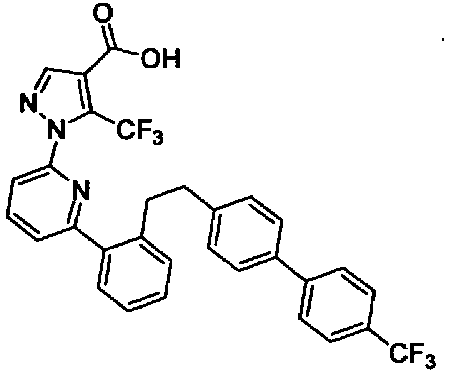

### Example 237

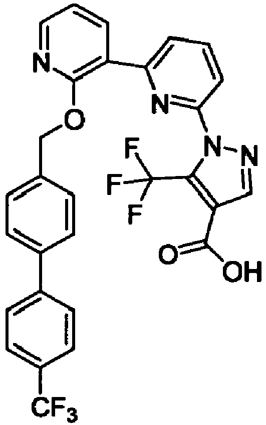

### Example 238

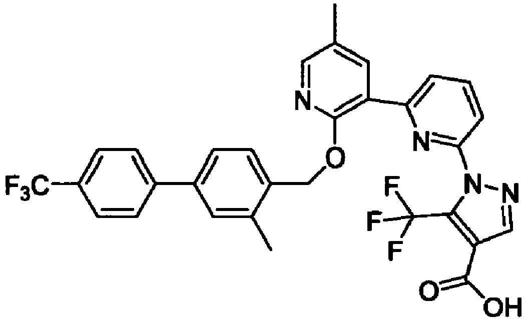

### Example 239

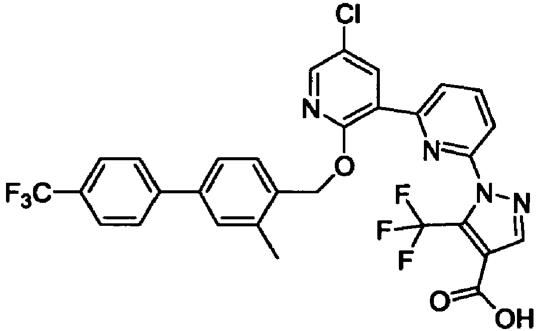

### Example 240

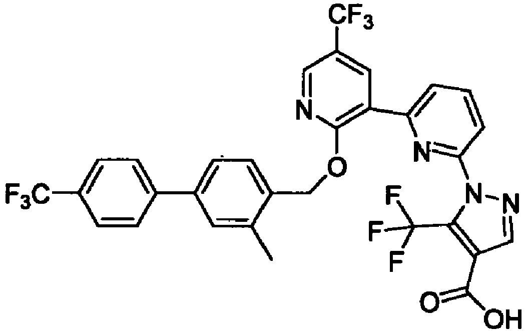

### Example 241

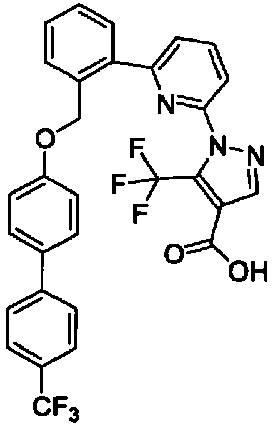

### Example 242

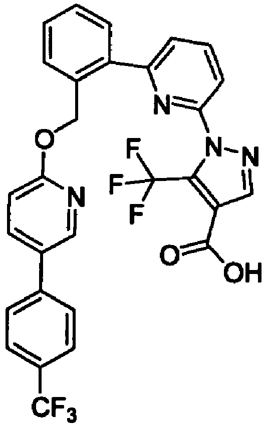

### Example 243

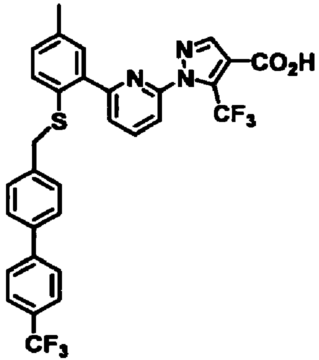

The compounds listed in **TABLE 4** were prepared using chemistry described in **Examples 236-243.**

**TABLE 4**

| | | | | |
|---|---|---|---|---|
| | | | 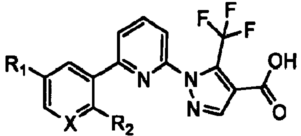 | |

| Entry | X | R1 | R2 | MS ([M+H]⁺) |
|---|---|---|---|---|
| 244 | CH | H | 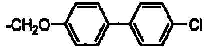 | 549.9 |
| 245 | CH | H | 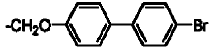 | 595.8 |
| 246 | CH | Cl | 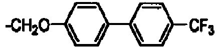 | 617.8 |
| 247 | CH | H | 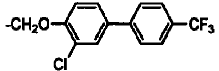 | 617.8 |
| 248 | CH | H | 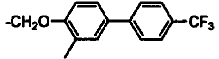 | 597.9 |
| 249 | CH | Me | 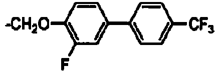 | 615.8 |
| 250 | CH | Me | 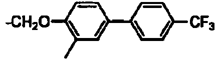 | 611.8 |
| 251 | CH | H | 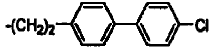 | 548.4 |
| 252 | N | H | 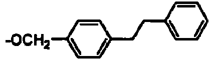 | 544.9 |
| 253 | N | H | 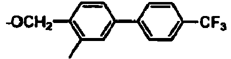 | 599.0 |
| 254 | N | Cl | 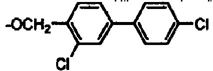 | 620.8 |
| 255 | N | Cl | 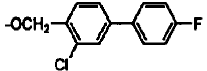 | 602.8 |
| 256 | N | Me | 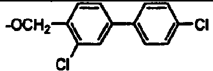 | 598.8 |
| 257 | N | Me | 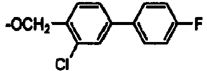 | 582.9 |
| 258 | N | Cl | 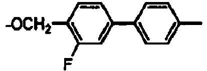 | 582.9 |
| 259 | N | Cl | 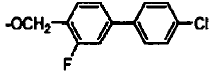 | 602.8 |
| 260 | N | Cl | 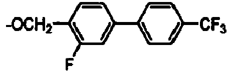 | 636.7 |
| 261 | N | Cl | 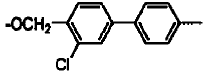 | 598.8 |
| 262 | N | Cl | 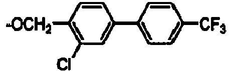 | 652.8 |
| 263 | N | Cl | 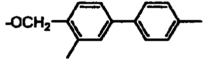 | 578.9 |
| 264 | N | Cl | 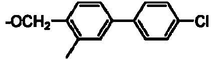 | 598.8 |
| 265 | N | Cl | 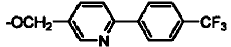 | 619.9 |
| 266 | N | Me | 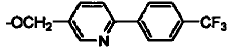 | 600.0 |
| 267 | N | CF₃ | 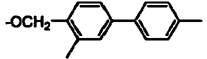 | 613.0 |
| 268 | CH | H | 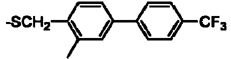 | 614.9 |
| 269 | CH | H | 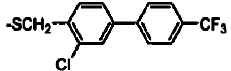 | 634.9 |
| 270 | CH | H | 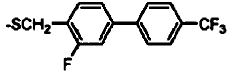 | 618.9 |
| 271 | CH | H | 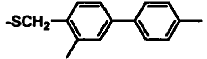 | 560.9 |
| 272 | CH | H | 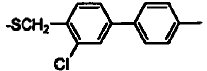 | 580.9 |
| 273 | CH | H | 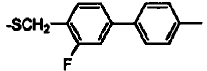 | 564.9 |
| 274 | CH | H | 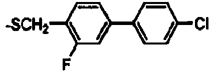 | 584.8 |
| 275 | CH | Me | 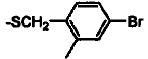 | 562.7 |
| 276 | CH | Me | 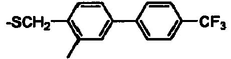 | 628.9 |
| 277 | CH | Me | 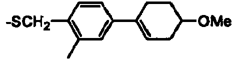 | 595.0 |
| 278 | CH | Me | 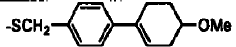 | 580.9 |
| 279 | CH | Me | 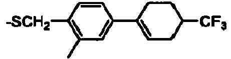 | 632.9 |
| 280 | CH | Me | 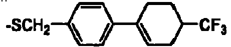 | 618.9 |
| 281 | CH | Me | 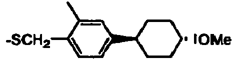 | 596.8 |
| 282 | CH | Me | 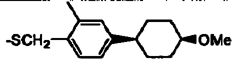 | 596.8 |
| 283 (isom er A) | CH | Me | 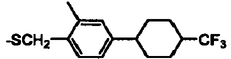 | 635.0 |
| 284 (isom er B) | CH | Me | 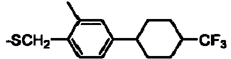 | 635.0 |
| 285 | CH | Me | 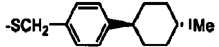 | 566.9 |
| 286 | CH | Me | 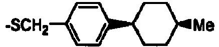 | 566.9 |
| 287 | CH | Me | 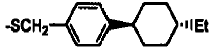 | 580.9 |
| 288 | CH | Me | 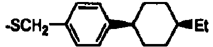 | 580.9 |
| 289 | CH | Me | 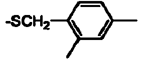 | 498.7 |
| 290 | CH | Me | 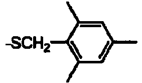 | 512.7 |
| 291 | CH | Cl | 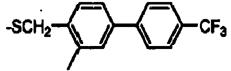 | 648.7 |
| 292 | CH | Cl | 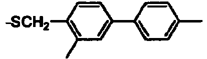 | 594.7 |
| 293 (isom er A) | CH | Cl | 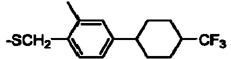 | 654.8 |
| 294 (isom er B) | CH | Cl | 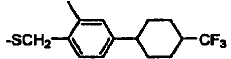 | 654.7 |
| 295 | CH | Cl | 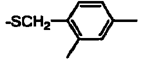 | 518.7 |
| 296 | CH | Cl | 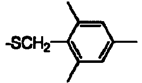 | 532.8 |
| 297 | CH | Cl | 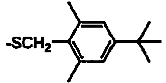 | 574.9 |
| 298 | CH | Cl | 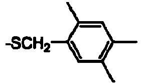 | 532.8 |
| 299 | CH | Cl | 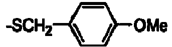 | 520.7 |

### Example 300

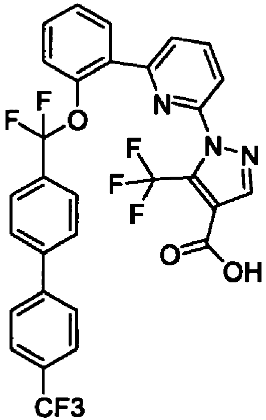

### Example 301

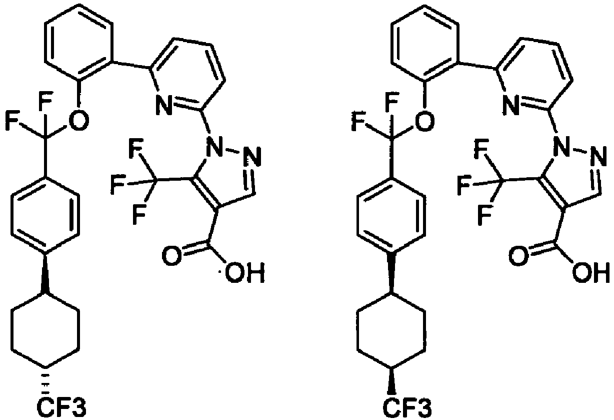

### Example 302

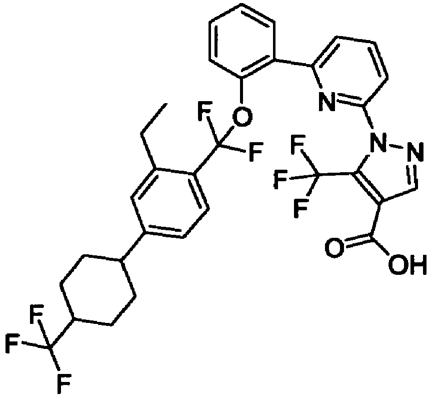

### Example 303

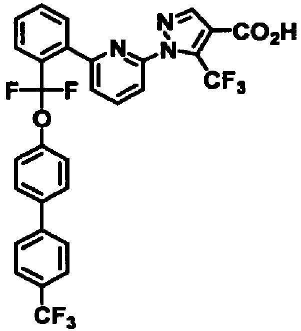

The compounds in **TABLE 5** were prepared using chemistry described in **Examples 300-303.**

**TABLE 5**

| | | | | |
|---|---|---|---|---|
| | | | 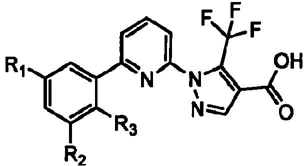 | |

| Entry | R1 | R2 | R3 | MS ([M-F]⁺) |
|---|---|---|---|---|
| 304 | Me | H | 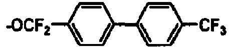 | 613.9 |
| 305 | H | H | 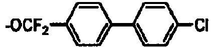 | 565.9 |
| 306 | H | H | 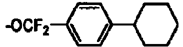 | 538.0 |
| 307 | H | H | 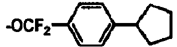 | 524.0 |
| 308 | H | H | 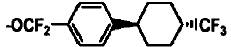 | 605.8 |
| 309 | H | H | 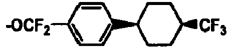 | 605.8 |
| 310 | H | H | 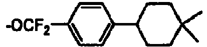 | 566.0 |
| 311 | Me | H | 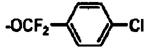 | 504.0 |
| 312 | Me | H | 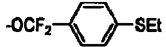 | 530.0 |
| 313 | Me | H | 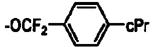 | 510.1 |
| 314 | Me | H | 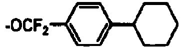 | 552.0 |
| 315 | H | H | 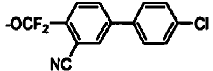 | 590.8 |
| 316 | H | H | 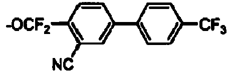 | 624.8 |
| 317 | Cl | H | 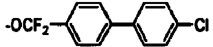 | 599.8 |
| 318 | H | H | 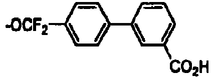 | 575.9 |
| 319 | Cl | H | 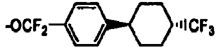 | 639.9 |
| 320 | Cl | H | 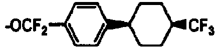 | 639.9 |
| 321 | Cl | H | 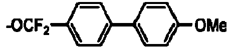 | 595.9 |
| 322 | H | H | 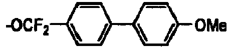 | 561.9 |
| 323 | Me | H | 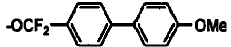 | 576.0 |
| 324 | Cl | H | 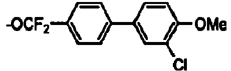 | 629.9 |
| 325 | H | H | 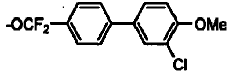 | 595.9 |
| 326 | Me | H | 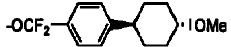 | 582.6 |
| 327 | Me | Br | 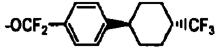 | 698.5 |
| 328 | H | H | 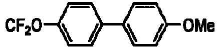 | 562.9 |
| 329 | H | H | 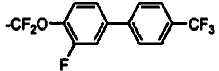 | 618.9 |

### Example 330

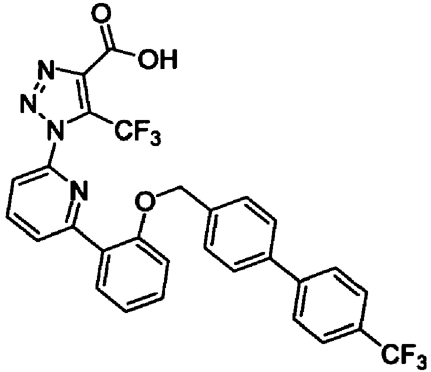

The compounds in **TABLE 6** were prepared using chemistry described in **Examples 1** and **330.**

**TABLE 6**

| | | | |
|---|---|---|---|
| | | 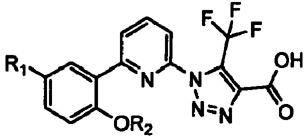 | |

| Entry | R1 | R2 | MS |
|---|---|---|---|
| 331 | H | 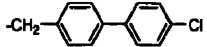 | 551.0 [M + H]⁺ |
| 332 | Me | 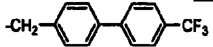 | 599.5 [M + H]⁺ |
| | | | |
| 333 | Me | 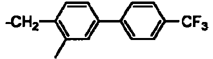 | 613.5 [M + H]⁺ |
| 334 | Me | 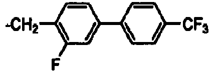 | 617.4 [M + H]⁺ |

### Compound Class IV:

Examples 1 to 186 of compound class IV are disclosed in W02010/099054.

### Example 1

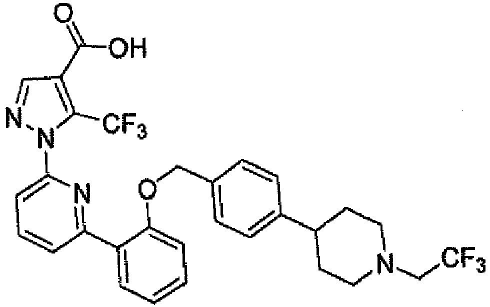

### Example 2

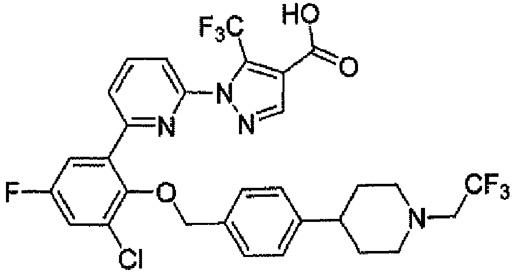

### Example 3

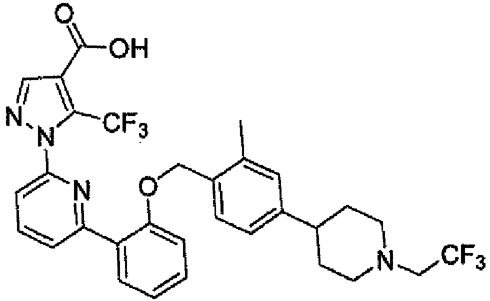

### Example 4

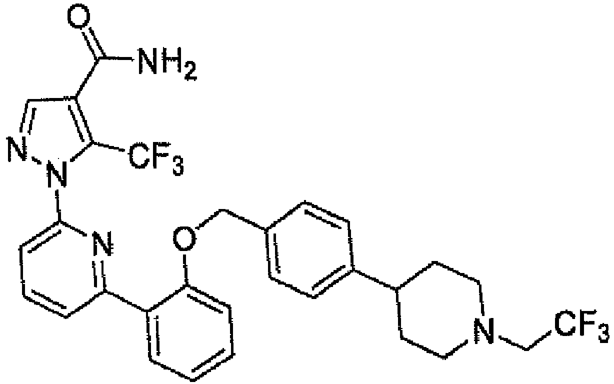

### Example 5

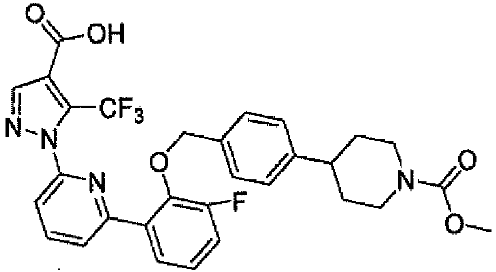

### Example 6

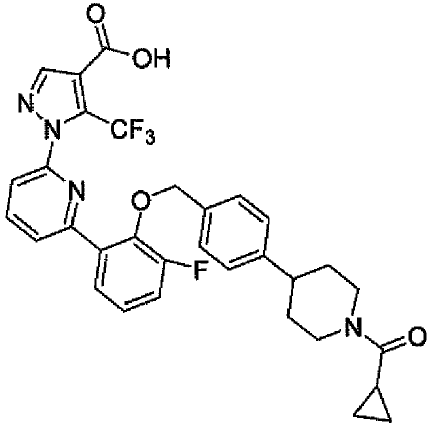

### Example 7

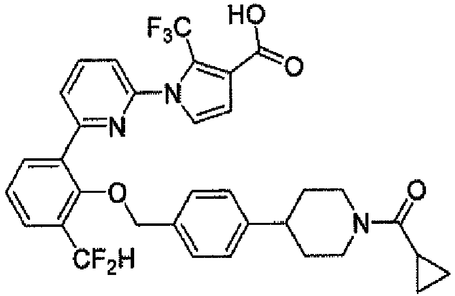

### Example 8

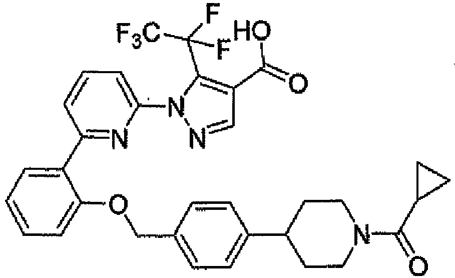

### Example 9

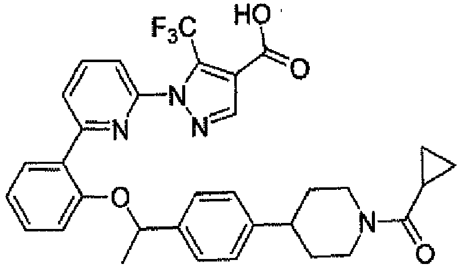

### Example 10

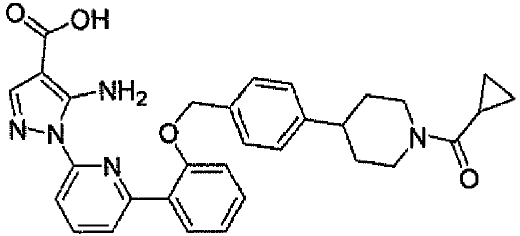

### Example 11

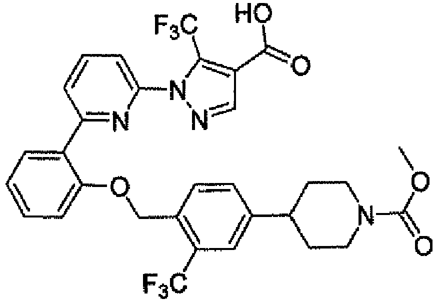

### Example 12

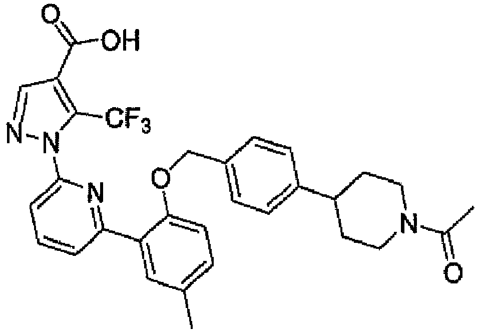

### Example 13

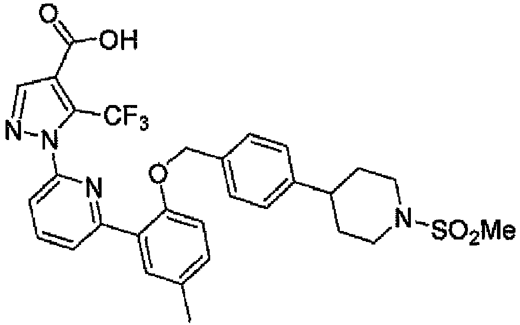

### Example 14

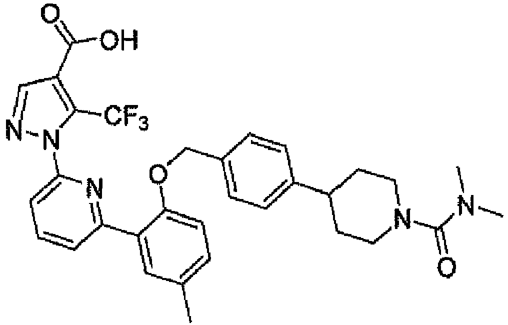

The compounds in **Table 1** were prepared using chemistry described in **Examples 1-14.**

**Table 1.**

| Example | Structure | IUPAC name |
|---|---|---|
| 15 | 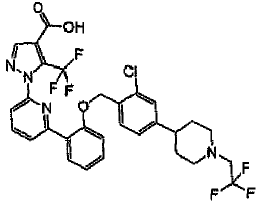 | 1-(6-(2-((2-chloro-4-(1-(2,2,2-trifluoroethyl)piperidin-4-yl)benzyl)oxy)phenyl)pyridin-2-yl)-5-(trifluoromethyl)-1H-pyrazole-4-carboxylic acid |
| 16 | 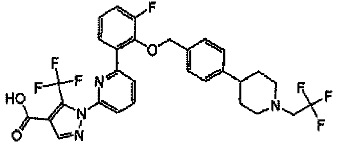 | 1-(6-(3-fluoro-2-((4-(1-(2,2,2-trifluoroethyl)piperidin-4-yl)benzyl)oxy)phenyl)pyridin-2-yl)-5-(trifluoromethyl)-1H-pyrazole-4-carboxylic acid |
| 17 | 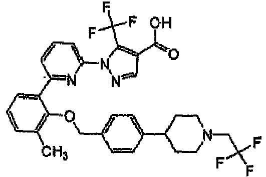 | 1-(6-(3-methyl-2-((4-(1-(2,2,2-trifluoroethyl)piperidin-4-yl)benzyl)oxy)phenyl)pyridin-2-yl)-5-(trifluoromethyl)-1H-pyrazole-4-carboxylic acid |
| 18 | 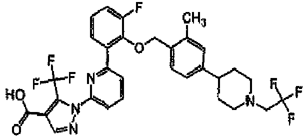 | 1-(6-(3-fluoro-2-((2-methyl-4-(1-(2,2,2-trifluoroethyl)piperidin-4-yl)benzyl)oxy)phenyl)pyridin-2-yl)-5-(trifluoromethyl)-1H-pyrazole-4-carboxylic acid |
| 19 | 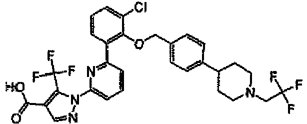 | 1-(6-(3-chloro-2-((4-(1-(2,2,2-trifluoroethyl)piperidin-4-yl)benzyl)oxy)phenyl)pyridin-2-yl)-5-(trifluoromethyl)-1H-pyrazole-4-carboxylic acid |
| 20 | 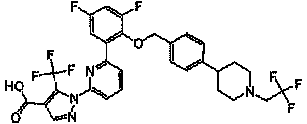 | 1-(6-(3,5-difluoro-2-((4-(1-(2,2,2-trifluoroethyl)piperidin-4-yl)benzyl)oxy)phenyl)pyridin-2-yl)-5-(trifluoromethyl)-1H-pyrazole-4-carboxylic acid |
| 21 | 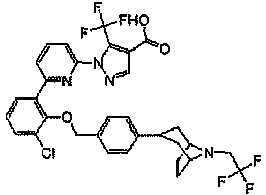 | 1-(6-(3-chloro-2-((4-(8-(2,2,2-trifluoroethyl)-8-azabicyclo[3.2.1]oct-3-yl)benzyl)oxy)phenyl)pyridin-2-yl)-5-(trifluoromethyl)-1H-pyrazole-4-carboxylic acid |
| 22 | 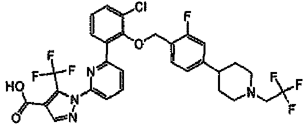 | 1-(6-(3-chloro-2-((2-fluoro-4-(1-(2,2,2-trifluoroethyl)piperidin-4-yl)benzyl)oxy)phenyl)pyridin-2-yl)-5-(trifluoromethyl)-1H-pyrazole-4-carboxylic acid |
| 23 | 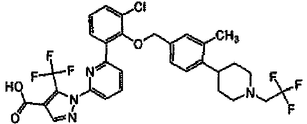 | 1-(6-(3-chloro-2-((3-methyl-4-(1-(2,2,2-trifluoroethyl)piperidin-4-yl)benzyl)oxy)phenyl)pyridin-2-yl)-5-(trifluoromethyl)-1H-pyrazole-4-carboxylic acid |
| 24 | 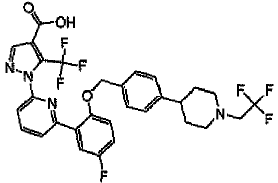 | 1-(6-(5-fluoro-2-((4-(1-(2,2,2-trifluoroethyl)piperidin-4-yl)benzyl)oxy)phenyl)pyridin-2-yl)-5-(trifluoromethyl)-1H-pyrazole-4-carboxylic acid |
| 25 | 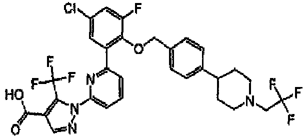 | 1-(6-(5-chloro-3-fluoro-2-((4-(1-(2,2,2-trifluoroethyl)piperidin-4-yl)benzyl)oxy)phenyl)pyridin-2-yl)-5-(trifluoromethyl)-1H-pyrazole-4-carboxylic acid |
| 26 | 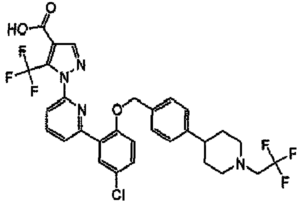 | 1-(6-(5-chloro-2-((4-(1-(2,2,2-trifluoroethyl)piperidin-4-yl)benzyl)oxy)phenyl)pyridin-2-yl)-5-(trifluoromethyl)-1H-pyrazole-4-carboxylic acid |
| 27 | 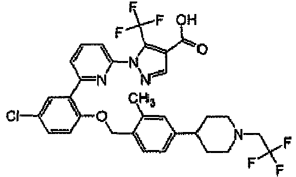 | 1-(6-(5-chloro-2-((2-methyl-4-(1-(2,2,2-trifluoroethyl)piperidin-4-yl)benzyl)oxy)phenyl)pyridin-2-yl)-5-(trifluoromethyl)-1H-pyrazole-4-carboxylic acid |
| 28 | 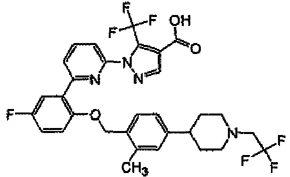 | 1-(6-(5-fluoro-2-((2-methyl-4-(1-(2,2,2-trifluoroethyl)piperidin-4-yl)benzyl)oxy)phenyl)pyridin-2-yl)-5-(trifluoromethyl)-1H-pyrazole-4-carboxylic acid |
| 29 | 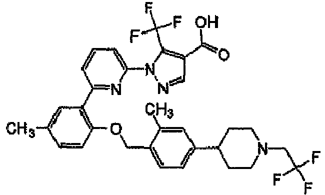 | 1-(6-(5-methyl-2-((2-methyl-4-(1-(2,2,2-trifluoroethyl)piperidin-4-yl)benzyl)oxy)phenyl)pyridin-2-yl)-5-(trifluoromethyl)-1H-pyrazole-4-carboxylic acid |
| 30 | 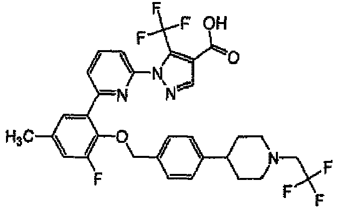 | 1-(6-(3-fluoro-5-methyl-2-((4-(1-(2,2,2-trifluoroethyl)piperidin-4-yl)benzyl)oxy)phenyl)pyridin-2-yl)-5-(trifluoromethyl)-1H-pyrazole-4-carboxylic acid |
| 31 | 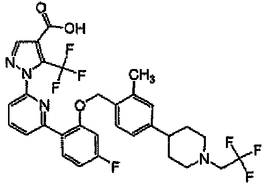 | 1-(6-(4-fluoro-2-((2-methyl-4-(1-(2,2,2-trifluoroethyl)piperidin-4-yl)benzyl)oxy)phenyl)pyridin-2-yl)-5-(trifluoromethyl)-1H-pyrazole-4-carboxylic acid |
| 32 | 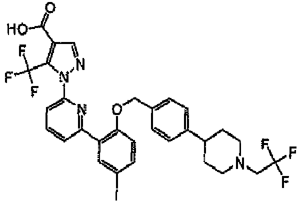 | 1-(6-(5-iodo-2-((4-(1-(2,2,2-trifluoroethyl)piperidin-4-yl)benzyl)oxy)phenyl)pyridin-2-yl)-5-(trifluoromethyl)-1H-pyrazole-4-carboxylic acid |
| 33 | 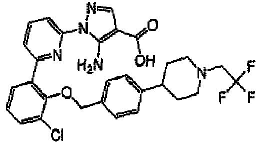 | 5-amino-1-(6-(3-chloro-2-((4-(1-(2,2,2-trifluoroethyl)piperidin-4-yl)benzyl)oxy)phenyl)pyridin-2-yl)-1H-pyrazole-4-carboxylic acid |
| 34 | 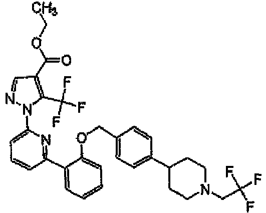 | ethyl 1-(6-(2-((4-(1-(2,2,2-trifluoroethyl)piperidin-4-yl)benzyl)oxy)phenyl)pyridin-2-yl)-5-(trifluoromethyl)-1H-pyrazole-4-carboxylate |
| 35 | 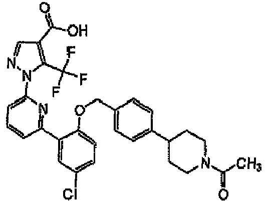 | 1-(6-(2-((4-(1-acetylpiperidin-4-yl)benzyl)oxy)-5-chlorophenyl)pyridin-2-yl)-5-(trifluoromethyl)-1H-pyrazole-4-carboxylic acid |
| 36 | 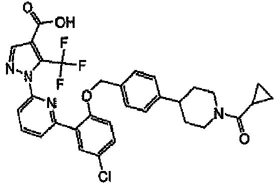 | 1-(6-(5-chloro-2-((4-(1-(cyclopropylcarbonyl)piperidin-4-yl)benzyl)oxy)phenyl)pyridin-2-yl)-5-(trifluoromethyl)-1H-pyrazole-4-carboxylic acid |
| 37 | 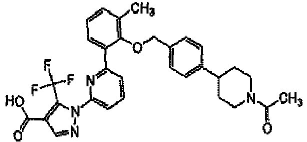 | 1-(6-(2-((4-(1-acetylpiperidin-4-yl)benzyl)oxy)-3-methylphenyl)pyridin-2-yl)-5-(trifluoromethyl)-1H-pyrazole-4-carboxylic acid |
| 38 | 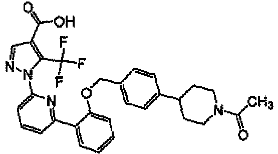 | 1-(6-(2-((4-(1-acetylpiperidin-4-yl)benzyl)oxy)phenyl)pyridin-2-yl)-5-(trifluoromethyl)-1H-pyrazole-4-carboxylic acid |
| 39 | 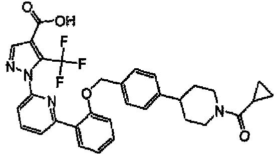 | 1-(6-(2-((4-(1-(cyclopropylcarbonyl)piperidin-4-yl)benzyl)oxy)phenyl)pyridin-2-yl)-5-(trifluoromethyl)-1H-pyrazole-4-carboxylic acid |
| 40 | 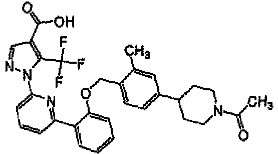 | 1-(6-(2-((4-(1-acetylpiperidin-4-yl)-2-methylbenzyl)oxy)phenyl)pyridi n-2-yl)-5-(trifluoromethyl)-1H-pyrazole-4-carboxylic acid |
| 41 | 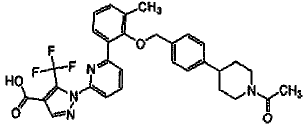 | 1-(6-(2-((4-(1-acetylpiperidin-4-yl)benzyl)oxy)-3-methylphenyl)pyridin-2-yl)-5-(trifluoromethyl)-1H-pyrazole-4-carboxylic acid |
| 42 | 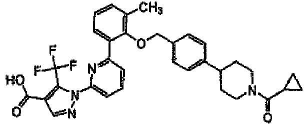 | 1-(6(2-((4-(1-(cyclopropylcarbonyl)piperidin-4-yl)benzyl)oxy)-3-methylphenyl)pyridin-2-yl)-5-(trifluoromethyl)-1H-pyrazole-4-carboxylic acid |
| 43 | 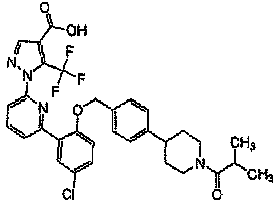 | 1-(6-(5-chloro-2-((4-(1-isobutyrylpiperidin-4-yl)benzyl)oxy)phenyl)pyridin-2-yl)-5-(trifluoromethyl)-1H-pyrazole-4-carboxylic acid |
| 44 | 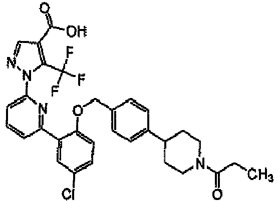 | 1-(6-(5-chloro-2-((4-(1-propionylpiperidin-4-yl)benzyl)oxy)phenyl)pyridin-2-yl)-5-(trifluoromethyl)-1H-pyrazole-4-carboxylic acid |
| 45 | 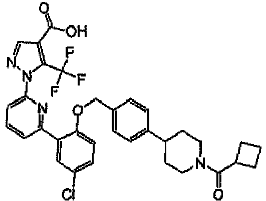 | 1-(6-(5-chloro-2-((4-(1-(cyclobutylcarbonyl)piperidin-4-yl)benzyl)oxy)phenyl)pyridin-2-yl)-5-(trifluoromethyl)-1H-pyrazole-4-carboxylic acid |
| 46 | 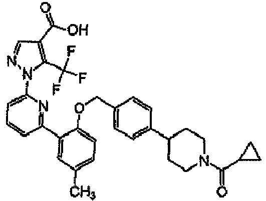 | 1-(6-(2-((4-(1-(cyclopropylcarbonyl)piperidin-4-yl)benzyl)oxy)-5-methylphenyl)pyridin-2-yl)-5-(trifluoromethyl)-1H-pyrazole-4-carboxylic acid |
| 47 | 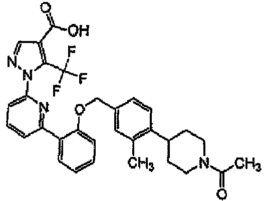 | 1-(6-(2-((4-(1-acetylpiperidin-4-yl)-3-methylbenzyl)oxy)phenyl)pyridi n-2-yl)-5-(trifluoromethyl)-1H-pyrazole-4-carboxylic acid |
| 48 | 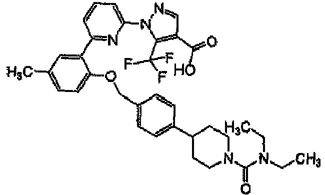 | 1-(6-(2-((4-(1-((diethylamino)carbonyl)piperid in-4-yl)benzyl)oxy)-5-methylphenyl)pyridin-2-yl)-5-(trifluoromethyl)-1H-pyrazole-4-carboxylic acid |
| 49 | 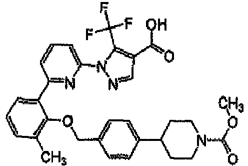 | 1-(6-(2-((4-(1-(methoxycarbonyl)piperidin-4-yl)benzyl)oxy)-3-methylphenyl)pyridin-2-yl)-5-(trifluoromethyl)-1H-pyrazole-4-carboxylic acid |
| 50 | 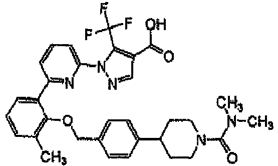 | 1-(6-(2-((4-(1-((dimethylamino)carbonyl)piper idin-4-yl)benzyl)oxy)-3-methylphenyl)pyridin-2-yl)-5-(trifluoromethyl)-1H-pyrazole-4-carboxylic acid |
| 51 | 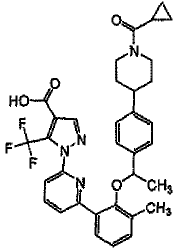 | 1-(6-(2-(1-(4-(1-(cyclopropylcarbonyl)piperidin-4-yl)phenyl)ethoxy)-3-methylphenyl)pyridin-2-yl)-5-(trifluoromethyl)-1H-pyrazole-4-carboxylic acid |
| 52 | 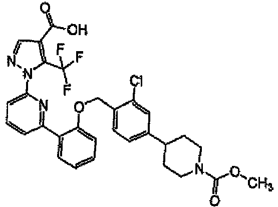 | 1-(6-(2-((2-chloro-4-(1-(methoxycarbonyl)piperidin-4-yl)benzyl)oxy)phenyl)pyridin-2-yl)-5-(trifluoromethyl)-1H-pyrazole-4-carboxylic acid |
| 53 | 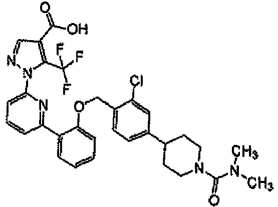 | 1-(6-(2-((2-chloro-4-(1-((dimethylamino)carbonyl)piper idin-4-yl)benzyl)oxy)phenyl)pyridin-2-yl)-5-(trifluoromethyl)-1H-pyrazole-4-carboxylic acid |
| 54 | 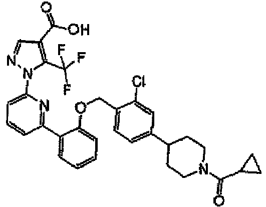 | 1-(6-(2-((2-chloro-4-(1-(cyclopropylcarbonyl)piperidin-4-yl)benzyl)oxy)phenyl)pyridin-2-yl)-5-(trifluoromethyl)-1H-pyrazole-4-carboxylic acid |
| 55 | 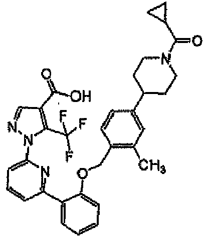 | 1-(6-(2-((4-(1-(cyclopropylcarbonyl)piperidin-4-yl)-2-methylbenzyl)oxy)phenyl)pyridi n-2-yl)-5-(trifluoromethyl)-1H-pyrazole-4-carboxylic acid |
| 56 | 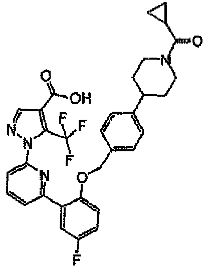 | 1-(6-(2-((4-(1-(cyclopropylcarbonyl)piperidin-4-yl)benzyl)oxy)-5-fluorophenyl)pyridin-2-yl)-5-(trifluoromethyl)-1H-pyrazole-4-carboxylic acid |
| 57 | 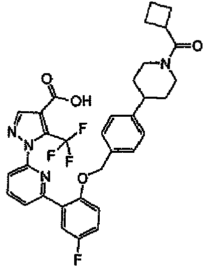 | 1-(6-(2-((4-(1-(cyclobutylcarbonyl)piperidin-4-yl)benzyl)oxy)-5-fluorophenyl)pyridin-2-yl)-5-(trifluoromethyl)-1H-pyrazole-4-carboxylic acid |
| 58 | 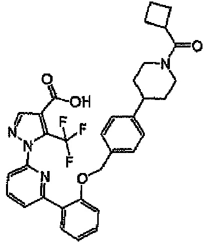 | 1-(6-(2-((4-(1-(cyclobutylcarbonyl)piperidin-4-yl)benzyl)oxy)phenyl)pyridin-2-yl)-5-(trifluoromethyl)-1H-pyrazole-4-carboxylic acid |
| 59 | 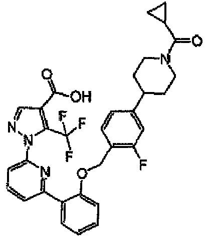 | 1-(6-(2-((4-(1-(cyclopropylcarbonyl)piperidin-4-yl)-2-fluorobenzyl)oxy)phenyl)pyridin -2-yl)-5-(trifluoromethyl)-1H-pyrazole-4-carboxylic acid |
| 60 | 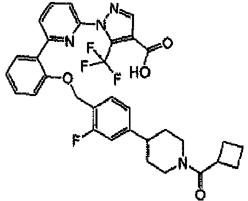 | 1-(6-(2-((4-(1-(cyclobutylcarbonyl)piperidin-4-yl)-2-fluorobenzyl)oxy)phenyl)pyridin -2-yl)-5-(trifluoromethyl)-1H-pyrazole-4-carboxylic acid |
| 61 | 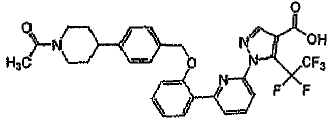 | 1-(6-(2-((4-(1-acetylpiperidin-4-yl)benzyl)oxy)phenyl)pyridin-2-yl)-5-(pentafluoroethyl)-1H-pyrazole-4-carboxylic acid |
| 62 | 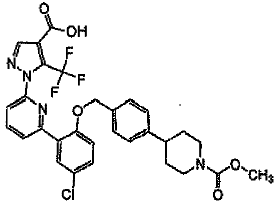 | 1-(6-(5-chloro-2-((4-(1-(methoxycarbonyl)piperidin-4-yl)benzyl)oxy)phenyl)pyridin-2-yl)-5-(trifluoromethyl)-1H-pyrazole-4-carboxylic acid |
| 63 | 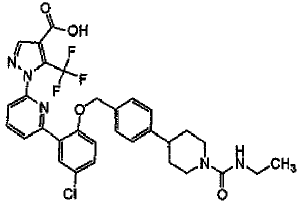 | 1-(6-(5-chloro-2-((4-(1-((ethylamino)carbonyl)piperidin -4-yl)benzyl)oxy)phenyl)pyridin-2-yl)-5-(trifluoromethyl)-1H-pyrazole-4-carboxylic acid |
| 64 | 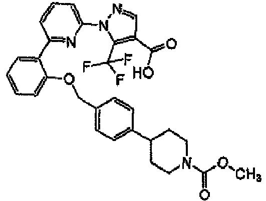 | 1-(6-(2-((4-(1-(methoxycarbonyl)piperidin-4-yl)benzyl)oxy)phenyl)pyridin-2-yl)-5-(trifluoromethyl)-1H-pyrazole-4-carboxylic acid |
| 65 | 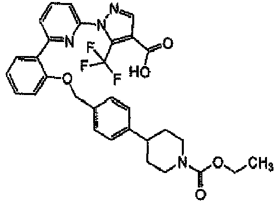 | 1-(6-(2-((4-(1-(ethoxycarbonyl)piperidin-4-yl)benzyl)oxy)phenyl)pyridin-2-yl)-5-(trifluoromethyl)-1H-pyrazole-4-carboxylic acid |
| 66 | 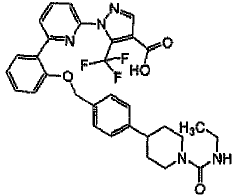 | 1-(6-(2-((4-(1-((ethylamino)carbonyl)piperidin -4-yl)benzyl)oxy)phenyl)pyridin-2-yl)-5-(trifluoromethyl)-1H-pyrazole-4-carboxylic acid |
| 67 | 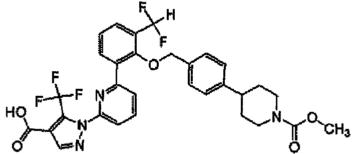 | 1-(6-(3-(difluoromethyl)-2-((4-(1-(methoxycarbonyl)piperidin-4-yl)benzyl)oxy)phenyl)pyridin-2-yl)-5-(trifluoromethyl)-1H-pyrazole-4-carboxylic acid |
| 68 | 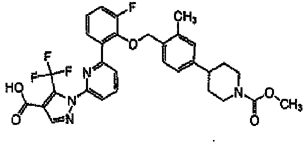 | 1-(6-(3-fluoro-2-((4-(1-(methoxycarbonyl)piperidin-4-yl)-2-methylbenzyl)oxy)phenyl)pyridi n-2-yl)-5-(trifluoromethyl)-1H-pyrazole-4-carboxylic acid |
| 69 | 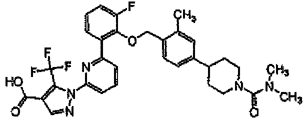 | 1-(6-(2-((4-(1-((dimethylamino)carbonyl)piper idin-4-yl)-2-methylbenzyl)oxy)-3-fluorophenyl)pyridin-2-yl)-5-(trifluoromethyl)-1H-pyrazole-4-carboxylic acid |
| 70 | 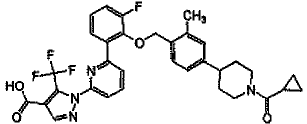 | 1-(6-(2-((4-(1-(cyclopropylcarbonyl)piperidin-4-yl)-2-methylbenzyl)oxy)-3-fluorophenyl)pyridin-2-yl)-5-(trifluoromethyl)-1H-pyrazole-4-carboxylic acid |
| 71 | 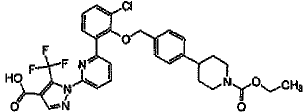 | 1-(6-(3-chloro-2-((4-(1-(ethoxycarbonyl)piperidin-4-yl)benzyl)oxy)phenyl)pyridin-2-yl)-5-(trifluoromethyl)-1H-pyrazole-4-carboxylic acid |
| 72 | 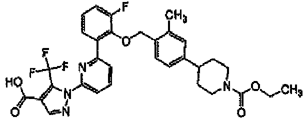 | 1-(6-(2-((4-(1-(ethoxycarbonyl)piperidin-4-yl)-2-methylbenzyl)oxy)-3-fluorophenyl)pyridin-2-yl)-5-(trifluoromethyl)-1H-pyrazole-4-carboxylic acid |
| 73 | 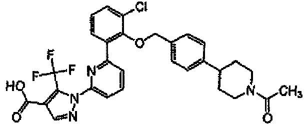 | 1-(6-(2-((4-(1-acetylpiperidin-4-yl)benzyl)oxy)-3-chlorophenyl)pyridin-2-yl)-5-(trifluoromethyl)-1H-pyrazole-4-carboxylic acid |
| 74 | 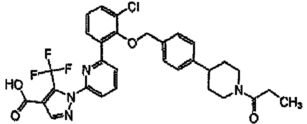 | 1-(6-(3-chloro-2-((4-(1-propionylpiperidin-4-yl)benzyl)oxy)phenyl)pyridin-2-yl)-5-(trifluoromethyl)-1H-pyrazole-4-carboxylic acid |
| 75 | 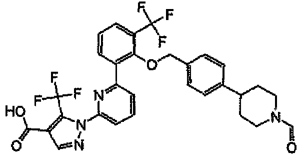 | 1-(6-(2-((4-(1-formylpiperidin-4-yl)benzyl)oxy)-3-(trifluoromethyl)phenyl)pyridin-2-yl)-5-(trifluoromethyl)-1H-pyrazole-4-carboxylic acid |
| 76 | 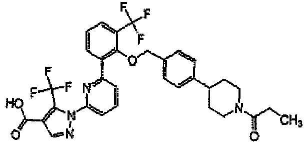 | 1-(6-(2-((4-(1-propionylpiperidin-4-yl)benzyl)oxy)-3-(trifluoromethyl)phenyl)pyridin-2-yl)-5-(trifluoromethyl)-1H-pyrazole-4-carboxylic acid |
| 77 | 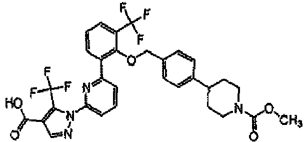 | 1-(6-(2-((4-(1-(methoxycarbonyl)piperidin-4-yl)benzyl)oxy)-3-(trifluoromethyl)phenyl)pyridin-2-yl)-5-(trifluoromethyl)-1H-pyrazole-4-carboxylic acid |
| 78 | 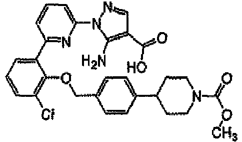 | 5-amino-1-(6-(3-chloro-2-((4-(1-(methoxycarbonyl)piperidin-4-yl)benzyl)oxy)phenyl)pyridin-2-yl)-1H-pyrazole-4-carboxylic acid |
| 79 | 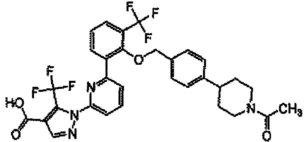 | 1-(6-(2-((4-(1-acetylpiperidin-4-yl)benzyl)oxy)-3-(trifluoromethyl)phenyl)pyridin-2-yl)-5-(trifluoromethyl)-1H-pyrazole-4-carboxylic acid |
| 80 | 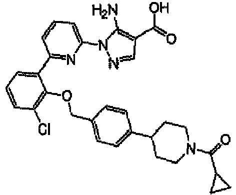 | 5-amino-1-(6-(3-chloro-2-((4-(1-(cyclopropylcarbonyl)piperidin-4-yl)benzyl)oxy)phenyl)pyridin-2-yl)-1H-pyrazole-4-carboxylic acid |
| 81 | 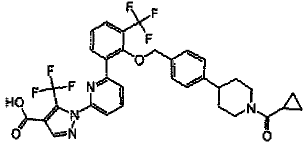 | 1-(6-(2-((4-(1-(cyclopropylcarbonyl)piperidin-4-yl)benzyl)oxy)-3-(trifluoromethyl)phenyl)pyridin-2-yl)-5-(trifluoromethyl)-1H-pyrazole-4-carboxylic acid |
| 82 | 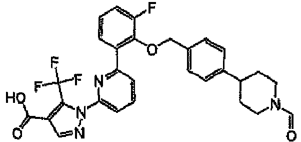 | 1-(6-(3-fluoro-2-((4-(1-formylpiperidin-4-yl)benzyl)oxy)phenyl)pyridin-2-yl)-5-(trifluoromethyl)-1H-pyrazole-4-carboxylic acid |
| 83 | 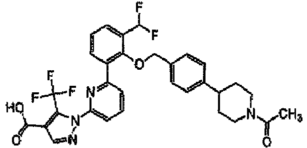 | 1-(6-(2-((4-(1-acetylpiperidin-4-yl)benzyl)oxy)-3-(difluoromethyl)phenyl)pyridin-2-yl)-5-(trifluoromethyl)-1H-pyrazole-4-carboxylic acid |
| 84 | 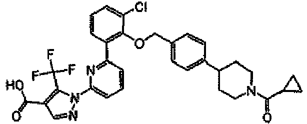 | 1-(6-(3-chloro-2-((4-(1-(cyclopropylcarbonyl)piperidin-4-yl)benzyl)oxy)phenyl)pyridin-2-yl)-5-(trifluoromethyl)-1H-pyrazole-4-carboxylic acid |
| 85 | 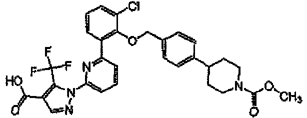 | 1-(6-(3-chloro-2-((4-(1-(methoxycarbonyl)piperidin-4-yl)benzyl)oxy)phenyl)pyridin-2-yl)-5-(trifluoromethyl)-1H-pyrazole-4-carboxylic acid |
| 86 | 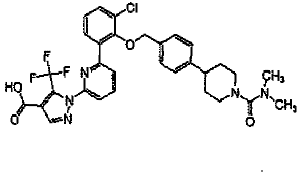 | 1-(6-(3-chloro-2-((4-(1-((dimethylamino)carbonyl)piper idin-4-yl)benzyl)oxy)phenyl)pyridin-2-yl)-5-(trifluoromethyl)-1H-pyrazole-4-carboxylic acid |
| 87 | 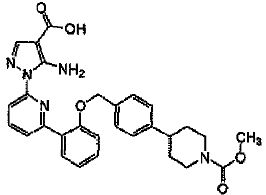 | 5-amino-1-(6-(2-((4-(1-(methoxycarbonyl)piperidin-4-yl)benzyl)oxy)phenyl)pyridin-2-yl)-1H-pyrazole-4-carboxylic acid |
| 88 | 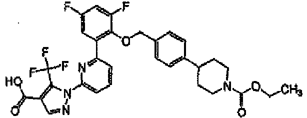 | 1-(6-(2-((4-(1-(ethoxycarbonyl)piperidin-4-yl)benzyl)oxy)-3,5-difluorophenyl)pyridin-2-yl)-5-(trifluoromethyl)-1H-pyrazole-4-carboxylic acid |
| 89 | 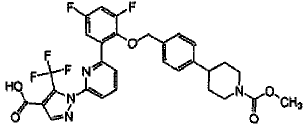 | 1-(6-(2-((4-(1-(methoxycarbonyl)piperidin-4-yl)benzyl)oxy)-3,5-difluorophenyl)pyridin-2-yl)-5-(trifluoromethyl)-1H-pyrazole-4-carboxylic acid |
| 90 | 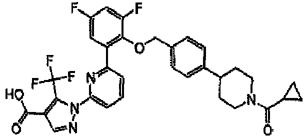 | 1-(6-(2-((4-(1-(cyclopropylcarbonyl)piperidin-4-yl)benzyl)oxy)-3,5-difluorophenyl)pyridin-2-yl)-5-(trifluoromethyl)-1H-pyrazole-4-carboxylic acid |
| 91 | 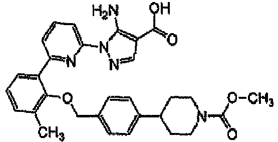 | 5-amino-1-(6-(2-((4-(1-(methoxycarbonyl)piperidin-4-yl)benzyl)oxy)-3-methylphenyl)pyridin-2-yl)-1H-pyrazole-4-carboxylic acid |
| 92 | 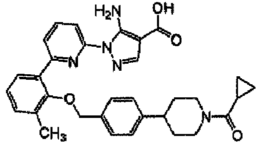 | 5-amino-1-(6-(2-((4-(1-(cyclopropylcarbonyl)piperidin-4-yl)benzyl)oxy)-3-methylphenyl)pyridin-2-yl)-1H-pyrazole-4-carboxylic acid |
| 93 | 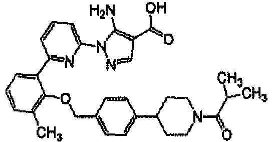 | 5-amino-1-(6-(2-((4-(1-isobutyrylpiperidin-4-yl)benzyl)oxy)-3-methylphenyl)pyridin-2-yl)-1H-pyrazole-4-carboxylic acid |
| 94 | 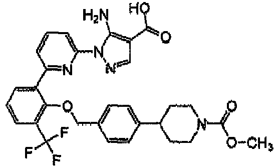 | 5-amino-1-(6-(2-((4-(1-(methoxycarbonyl)piperidin-4-yl)benzyl)oxy)-3-(trifluoromethyl)phenyl)pyridin-2-yl)-1H-pyrazole-4-carboxylic acid |
| 95 | 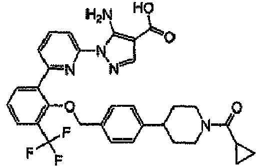 | 5-amino-1-(6-(2-((4-(1-(cyclopropylcarbonyl)piperidin-4-yl)benzyl)oxy)-3-(trifluoromethyl)phenyl)pyridin-2-yl)-1H-pyrazole-4-carboxylic acid |
| 96 | 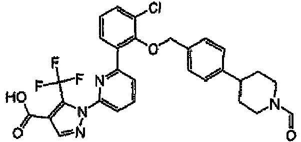 | 1-(6-(3-chloro-2-((4-(1-formylpiperidin-4-yl)benzyl)oxy)phenyl)pyridin-2-yl)-5-(trifluoromethyl)-1H-pyrazole-4-carboxylic acid |
| 97 | 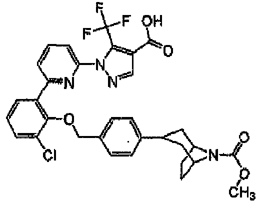 | 1-(6-(3-chloro-2-((4-(8-(methoxycarbonyl)-8-azabicyclo[3.2.1]oct-3-yl)benzyl)oxy)phenyl)pyridin-2-yl)-5-(trifluoromethyl)-1H-pyrazole-4-carboxylic acid |
| 98 | 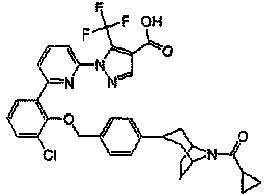 | 1-(6-(3-chloro-2-((4-(8-(cyclopropylcarbonyl)-8-azabicyclo[3.2.1]oct-3-yl)benzyl)oxy)phenyl)pyridin-2-yl)-5-(trifluoromethyl)-1H-pyrazole-4-carboxylic acid |
| 99 | 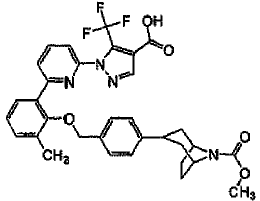 | 1-(6-(2-((4-(8-(methoxycarbonyl)-8-azabicyclo[3.2.1]oct-3-yl)benzyl)oxy)-3-methylphenyl)pyridin-2-yl)-5-(trifluoromethyl)-1H-pyrazole-4-carboxylic acid |
| 100 | 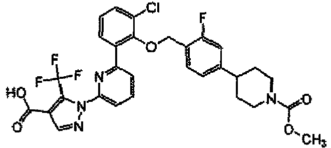 | 1-(6-(3-chloro-2-((2-fluoro-4-(1-(methoxycarbonyl)piperidin-4-yl)benzyl)oxy)phenyl)pyridin-2-yl)-5-(trifluoromethyl)-1H-pyrazole-4-carboxylic acid |
| 101 | 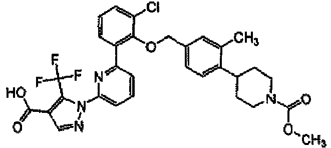 | 1-(6-(3-chloro-2-((4-(1-(methoxycarbonyl)piperidin-4-yl)-3-methylbenzyl)oxy)phenyl)pyridi n-2-yl)-5-(trifluoromethyl)-1H-pyrazole-4-carboxylic acid |
| 102 | 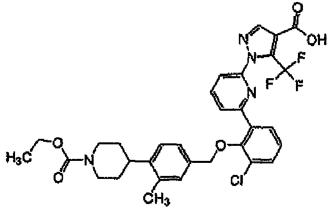 | 1-(6-(3-chloro-2-((4-(1-(ethoxycarbonyl)piperidin-4-yl)-3-methylbenzyl)oxy)phenyl)pyridi n-2-yl)-5-(trifluoromethyl)-1H-pyrazole-4-carboxylic acid |
| 103 | 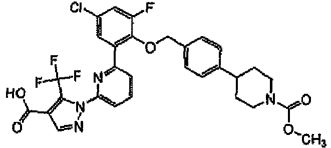 | 1-(6-(5-chloro-3-fluoro-2-((4-(1-(methoxycarbonyl)piperidin-4-yl)benzyl)oxy)phenyl)pyridin-2-yl)-5-(trifluoromethyl)-1H-pyrazole-4-carboxylic acid |
| 104 | 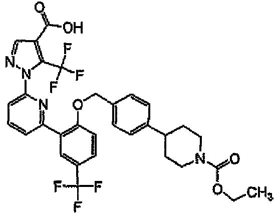 | 1-(6-(2-((4-(1-(ethoxycarbonyl)piperidin-4-yl)benzyl)oxy)-5-(trifluoromethyl)phenyl)pyridin-2-yl)-5-(trifluoromethyl)-1H-pyrazole-4-carboxylic acid |
| 105 | 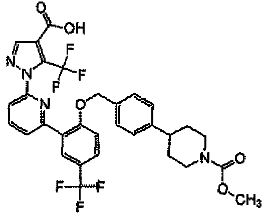 | 1-(6-(2-((4-(1-(methoxycarbonyl)piperidin-4-yl)benzyl)oxy)-5-(trifluoromethyl)phenyl)pyridin-2-yl)-5-(trifluoromethyl)-1H-pyrazole-4-carboxylic acid |
| 106 | 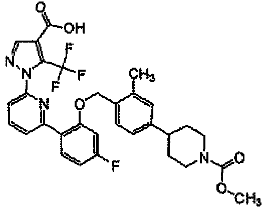 | 1-(6-(4-fluoro-2-((4-(1-(methoxycarbonyl)piperidin-4-yl)-2-methylbenzyl)oxy)phenyl)pyridi n-2-yl)-5-(trifluoromethyl)-1H-pyrazole-4-carboxylic acid |
| 107 | 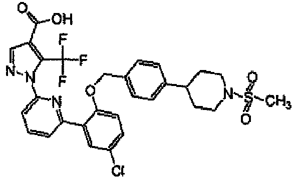 | 1-(6-(5-chloro-2-((4-(1-(methylsulfonyl)piperidin-4-yl)benzyl)oxy)phenyl)pyridin-2-yl)-5-(trifluoromethyl)-1H-pyrazole-4-carboxylic acid |
| 108 | 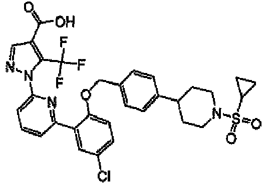 | 1-(6-(5-chloro-2-((4-(1-(cyclopropylsulfonyl)piperidin-4-yl)benzyl)oxy)phenyl)pyridin-2-yl)-5-(trifluoromethyl)-1H-pyrazole-4-carboxylic acid |
| 109 | 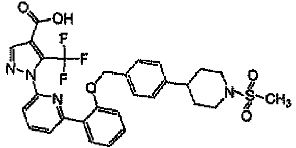 | 1-(6-(2-((4-(1-(methylsulfonyl)piperidin-4-yl)benzyl)oxy)phenyl)pyridin-2-yl)-5-(trifluoromethyl)-1H-pyrazole-4-carboxylic acid |
| 110 | 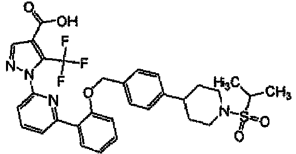 | 1-(6-(2-((4-(1-(isopropylsulfonyl)piperidin-4-yl)benzyl)oxy)phenyl)pyridin-2-yl)-5-(trifluoromethyl)-1H-pyrazole-4-carboxylic acid |
| 111 | 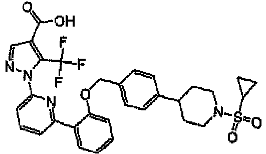 | 1-(6-(2-((4-(1-(cyclopropylsulfonyl)piperidin-4-yl)benzyl)oxy)phenyl)pyridin-2-yl)-5-(trifluoromethyl)-1H-pyrazole-4-carboxylic acid |
| 112 | 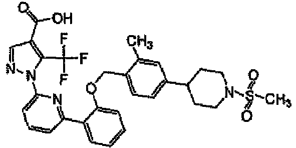 | 1-(6-(2-((2-methyl-4-(1-(methylsulfonyl)piperidin-4-yl)benzyl)oxy)phenyl)pyridin-2-yl)-5-(trifluoromethyl)-1H-pyrazole-4-carboxylic acid |
| 113 | 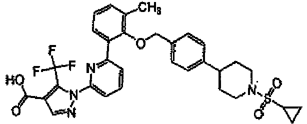 | 1-(6-(2-((4-(1-(cyclopropylsulfonyl)piperidin-4-yl)benzyl)oxy)-3-methylphenyl)pyridin-2-yl)-5-(trifluoromethyl)-1H-pyrazole-4-carboxylic acid |
| 114 | 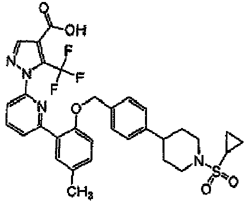 | 1-(6-(2-((4-(1-(cyclopropylsulfonyl)piperidin-4-yl)benzyl)oxy)-5-methylphenyl)pyridin-2-yl)-5-(trifluoromethyl)-1H-pyrazole-4-carboxylic acid |
| 115 | 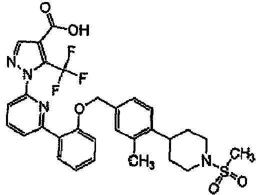 | 1-(6-(2-((3-methyl-4-(1-(methylsulfonyl)piperidin-4-yl)benzyl)oxy)phenyl)pyridin-2-yl)-5-(trifluoromethyl)-1H-pyrazole-4-carboxylic acid |
| 116 | 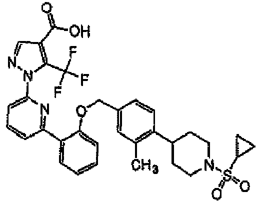 | 1-(6-(2-((4-(1-(cyclopropylsulfonyl)piperidin-4-yl)-3-methylbenzyl)oxy)phenyl)pyridi n-2-yl)-5-(trifluoromethyl)-1H-pyrazole-4-carboxylic acid |
| 117 | 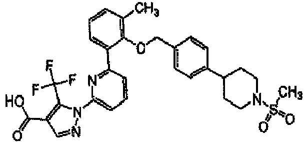 | 1-(6-(3-methyl-2-((4-(1-(methylsulfonyl)piperidin-4-yl)benzyl)oxy)phenyl)pyridin-2-yl)-5-(trifluoromethyl)-1H-pyrazole-4-carboxylic acid |
| 118 | 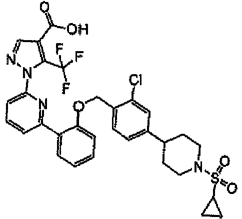 | 1-(6-(2-((2-chloro-4-(1-(cyclopropylsulfonyl)piperidin-4-yl)benzyl)oxy)phenyl)pyridin-2-yl)-5-(trifluoromethyl)-1H-pyrazole-4-carboxylic acid |
| 119 | 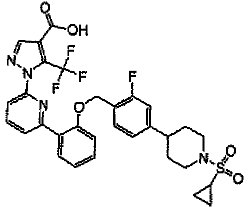 | 1-(6-(2-((4-(1-(cyclopropylsulfonyl)piperidin-4-yl)-2-fluorobenzyl)oxy)phenyl)pyridin -2-yl)-5-(trifluoromethyl)-1H-pyrazole-4-carboxylic acid |
| 120 | 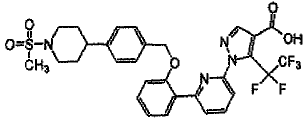 | 1-(6-(2-((4-(1-(methylsulfonyl)piperidin-4-yl)benzyl)oxy)phenyl)pyridin-2-yl)-5-(pentafluoroethyl)-1H-pyrazole-4-carboxylic acid |
| 121 | 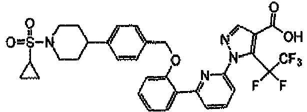 | 1-(6-(2-((4-(1-(cyclopropylsulfonyl)piperidin-4-yl)benzyl)oxy)phenyl)pyridin-2-yl)-5-(pentafluoroethyl)-1H-pyrazole-4-carboxylic acid |
| 122 | 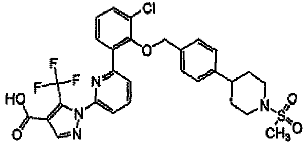 | 1-(6-(3-chloro-2-((4-(1-(methylsulfonyl)piperidin-4-yl)benzyl)oxy)phenyl)pyridin-2-yl)-5-(trifluoromethyl)-1H-pyrazole-4-carboxylic acid |
| 123 | 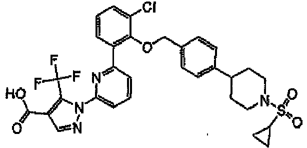 | 1-(6-(3-chloro-2-((4-(1-(cyclopropylsulfonyl)piperidin-4-yl)benzyl)oxy)phenyl)pyridin-2-yl)-5-(trifluoromethyl)-1H-pyrazole-4-carboxylic acid |
| 124 | 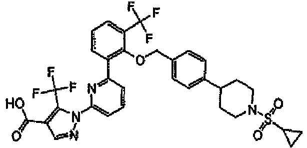 | 1-(6-(2-((4-(1-(cyclopropylsulfonyl)piperidin-4-yl)benzyl)oxy)-3-(trifluoromethyl)phenyl)pyridin-2-yl)-5-(trifluoromethyl)-1H-pyrazole-4-carboxylic acid |
| 125 | 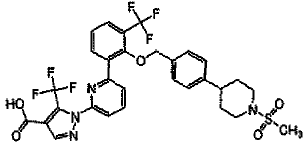 | 1-(6-(2-((4-(1-(methylsulfonyl)piperidin-4-yl)benzyl)oxy)-3-(trifluoromethyl)phenyl)pyridin-2-yl)-5-(trifluoromethyl)-1H-pyrazole-4-carboxylic acid |
| 126 | 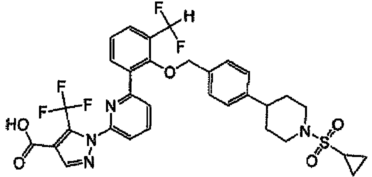 | 1-(6-(2-((4-(1-(cyclopropylsulfonyl)piperidin-4-yl)benzyl)oxy)-3-(difluoromethyl)phenyl)pyridin-2-yl)-5-(trifluoromethyl)-1H-pyrazole-4-carboxylic acid |
| 127 | 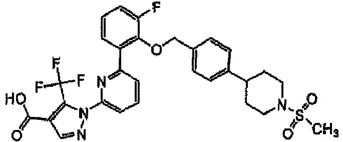 | 1-(6-(3-fluoro-2-((4-(1-(methylsulfonyl)piperidin-4-yl)benzyl)oxy)phenyl)pyridin-2-yl)-5-(trifluoromethyl)-1H-pyrazole-4-carboxylic acid |
| 128 | 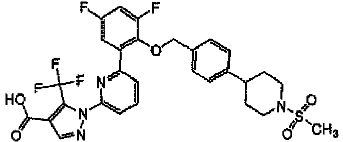 | 1-(6-(3,5-difluoro-2-((4-(1-(methylsulfonyl)piperidin-4-henyl)pyridin-2-yl)-5-(trifluoromethyl)-1H-pyrazole-4-carboxylic acid |
| 129 | 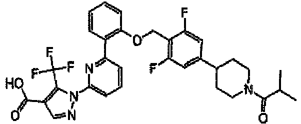 | 1-(6-(2-((2,6-difluoro-4-(1-isobutyrylpiperidin-4-yl)benzyl)oxy)phenyl)pyridin-2-yl)-5-(trifluoromethyl)-1H-pyrazole-4-carboxylic acid |
| 130 | 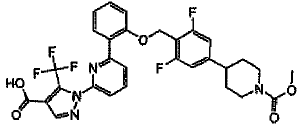 | 1-(6-(2-((2,6-difluoro-4-(1-(methoxycarbonyl)piperidin-4-yl)benzyl)oxy)phenyl)pyridin-2-yl)-5-(trifluoromethyl)-1H-pyrazole-4-carboxylic acid |
| 131 | 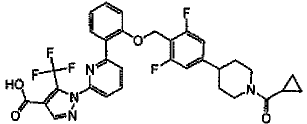 | 1-(6-(2-((4-(1-(cyclopropylcarbonyl)piperidin-4-yl)-2,6-difluorobenzyl)oxy)phenyl)pyrid in-2-yl)-5-(trifluoromethyl)-1H-pyrazole-4-carboxylic acid |
| 132 | 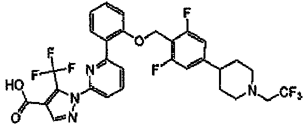 | 1-(6-(2-((2,6-difluoro-4-(1-(2,2,2-trifluoroethyl)piperidin-4-yl)benzyl)oxy)phenyl)pyridin-2-yl)-5-(trifluoromethyl)-1H-pyrazole-4-carboxylic acid |
| 133 | 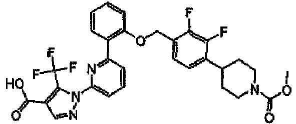 | 1-(6-(2-((2,3-difluoro-4-(1-(methoxycarbonyl)piperidin-4-yl)benzyl)oxy)phenyl)pyridin-2-yl)-5-(trifluoromethyl)-1H-pyrazole-4-carboxylic acid |
| 134 | 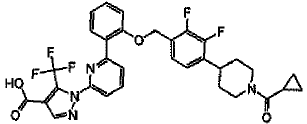 | 1-(6-(2-((4-(1-(cyclopropylcarbonyl)piperidin-4-yl)-2,3-difluorobenzyl) oxy)phenyl)pyridin-2-yl)-5-(trifluoromethyl)-1H-pyrazole-4-carboxylic acid |
| 135 | 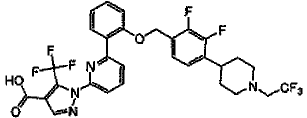 | 1-(6-(2-((2,3-difluoro-4-(1-(2,2,2-trifluoroethyl)piperidin-4-yl)benzyl)oxy)phenyl)pyridin-2-yl)-5-(trifluoromethyl)-1H-pyrazole-4-carboxylic acid |

### Example 136

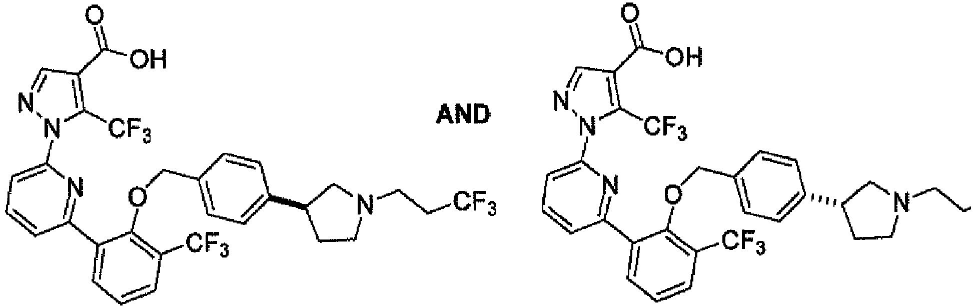

The compounds in **Table 2** were prepared using chemistry described in **Example 136,** or by analogy to chemistry described in **Examples 1-14.**

**Table 2**

| Example | Structure | IUPAC |
|---|---|---|
| 137 | 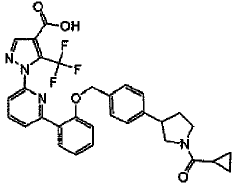 | 1-(6-(2-((4-(1-(cyclopropylcarbonyl)pyrrolidin-3-yl)benzyl)oxy)phenyl)pyridin-2-yl)-5-(trifluoromethyl)-1H-pyrazole-4-carboxylic acid |
| 138, 139 | 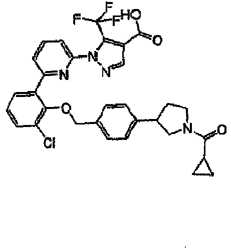 | 1-(6-(3-chloro-2-((4-((3R)-1-(cyclopropylcarbonyl)pyrrolidin-3-yl)benzyl)oxy)phenyl)pyridin-2-yl)-5-(trifluoromethyl)-1H-pyrazole-4-carboxylic acid And 1-(6-(3-chloro-2-((4-((3S)-1-(cyclopropylcarbonyl)pyrrolidin-3-yl)benzyl)oxy)phenyl)pyridin-2-yl)-5-(trifluoromethyl)-1H-pyrazole-4-carboxylic acid |
| 140, 141 | 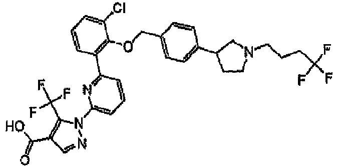 | 1-(6-(3-chloro-2-((4-(3R)-(1-(4,4,4-trifluorobutyl)pyrrolidin-3-yl)benzyl)oxy)phenyl)pyridin-2-yl)-5-(trifluoromethyl)-1H-pyrazole-4-carboxylic acid And 1-(6-(3-chloro-2-((4-(3S)-(1-(4,4,4-trifluorobutyl)pyrrolidin-3-yl)benzyl)oxy)phenyl)pyridin-2-yl)-5-(trifluoromethyl)-1H-pyrazole-4-carboxylic acid |
| 142 | 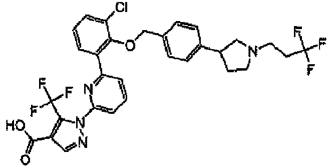 | 1-(6-(3-chloro-2-((4-(1-(3,3,3-trifluoropropyl)pyrrolidin-3-yl)benzyl)oxy)phenyl)pyridin-2-yl)-5-(trifluoromethyl)-1H-pyrazole-4-carboxylic acid |
| 143, 144 | 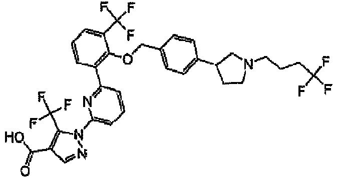 | 1-(6-(2-((4-(3R)-(1-(4,4,4-trifluorobutyl)pyrrolidin-3-yl)benzyl)oxy)-3-(trifluoromethyl)phenyl)pyridin-2-yl)-5-(trifluoromethyl)-1H-pyrazole-4-carboxylic acid AND 1-(6-(2-((4-(3S)-(1-(4,4,4-trifluorobutyl)pyrrolidin-3-yl)benzyl)oxy)-3-(trifluoromethyl)phenyl)pyridin-2-yl)-5-(trifluoromethyl)-1H-pyrazole-4-carboxylic acid |
| 145 | 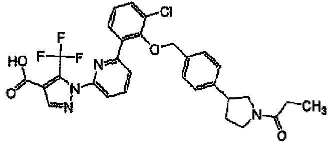 | 1-(6-(3-chloro-2-((4-(1-propionylpyrrolidin-3-yl)benzyl)oxy)phenyl)pyridin-2-yl)-5-(trifluoromethyl)-1H-pyrazole-4-carboxylic acid |
| 146 | 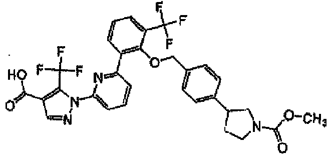 | 1-(6-(2-((4-(1-(methoxycarbonyl)pyrrolidin-3-yl)benzyl)oxy)-3-(trifluoromethyl)phenyl)pyridin-2-yl)-5-(trifluoromethyl)-1H-pyrazole-4-carboxylic acid |
| 147 | 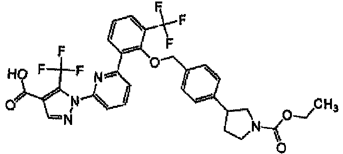 | 1-(6-(2-((4-(1-(ethoxycarbonyl)pyrrolidin-3-yl)benzyl)oxy)-3-(trifluoromethyl)phenyl)pyridin-2-yl)-5-(trifluoromethyl)-1H-pyrazole-4-carboxylic acid |

### Example 148

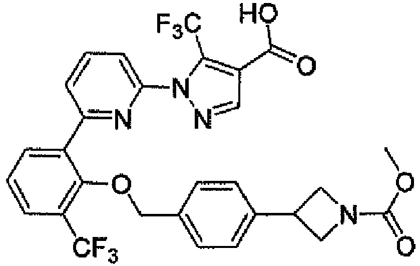

The compounds listed in **Table 3** were prepared using chemistry described in **Example 148,** and/or by analogy to chemistry described in **Examples 1-14** and **Example 136.**

**Table 3**

| Example | Structure | IUPAC |
|---|---|---|
| 149 | 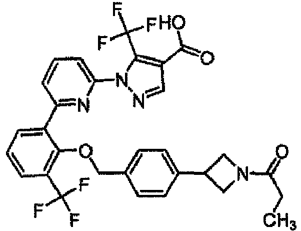 | 1-(6-(2-((4-(1-propionylazetidin-3-yl)benzyl)oxy)-3-(trifluoromethyl)phenyl)pyridin-2-yl)-5-(trifluoromethyl)-1H-pyrazole-4-carboxylic acid |
| 150 | 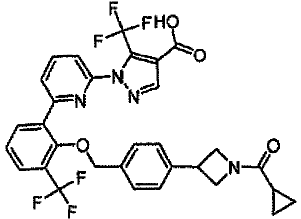 | 1-(6-(2-((4-(1-(cyclopropylcarbonyl)azetidin-3-yl)benzyl)oxy)-3-(trifluoromethyl)phenyl)pyridin-2-yl)-5-(trifluoromethyl)-1H-pyrazole-4-carboxylic acid |
| 151 | 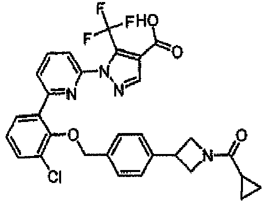 | 1-(6-(3-chloro-2-((4-(1-(cyclopropylcarbonyl)azetidin-3-yl)benzyl)oxy)phenyl)pyridin-2-yl)-5-(trifluoromethyl)-1H-pyrazole-4-carboxylic acid |
| 152 | 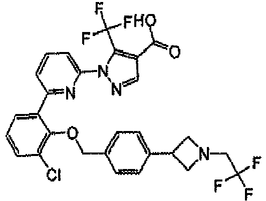 | 1-(6-(3-chloro-2-((4-(1-(2,2,2-trifluoroethyl)azetidin-3-yl)benzyl)oxy)phenyl)pyridin-2-yl)-5-(trifluoromethyl)-1H-pyrazole-4-carboxylic acid |
| 153 | 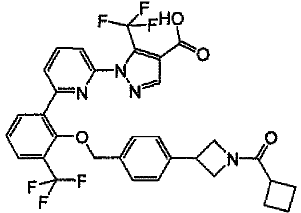 | 1-(6-(2-((4-(1-(cyclobutylcarbonyl)azetidin-3-yl)benzyl)oxy)-3-(trifluoromethyl)phenyl)pyridin-2-yl)-5-(trifluoromethyl)-1H-pyrazole-4-carboxylic acid |
| 154 | 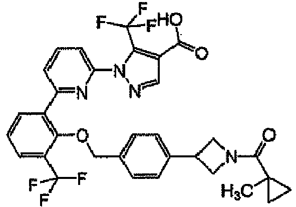 | 1-(6-(2-((4-(1-((1-methylcyclopropyl)carbonyl)aze tidin-3-yl)benzyl)oxy)-3-(trifluoromethyl)phenyl)pyridin-2-yl)-5-(trifluoromethyl)-1H-pyrazole-4-carboxylic acid |
| 155 | 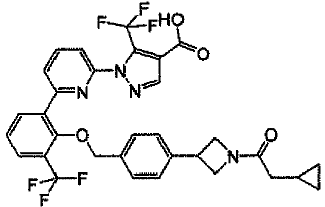 | 1-(6-(2-((4-(1-(cyclopropylacetyl)azetidin-3-yl)benzyl)oxy)-3-(trifluoromethyl)phenyl)pyridin-2-yl)-5-(trifluoromethyl)-1H-pyrazole-4-carboxylic acid |
| 156 | 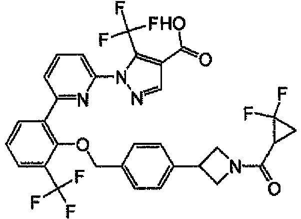 | 1-(6-(2-((4-(1-((2,2-difluorocyclopropyl)carbonyl)az etidin-3-yl)benzyl)oxy)-3-(trifluoromethyl)phenyl)pyridin-2-yl)-5-(trifluoromethyl)-1H-pyrazole-4-carboxylic acid |
| 157 | 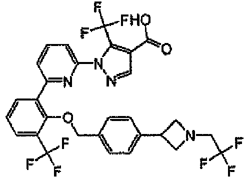 | 1-(6-(2-((4-(1-(2,2,2-trifluoroethyl)azetidin-3-yl)benzyl)oxy)-3-(trifluoromethyl)phenyl)pyridin-2-yl)-5-(trifluoromethyl)-1H-pyrazole-4-carboxylic acid |
| 158 | 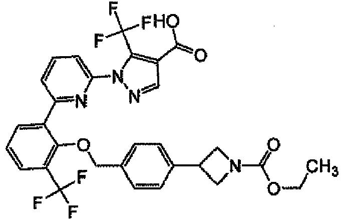 | 1-(6-(2-((4-(1-(ethoxycarbonyl)azetidin-3-yl)benzyl)oxy)-3-(trifluoromethyl)phenyl)pyridin-2-yl)-5-(trifluoromethyl)-1H-pyrazole-4-carboxylic acid |
| 159 | 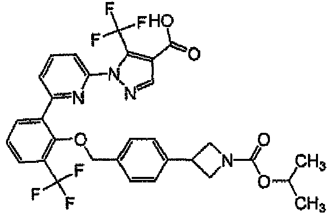 | 1-(6-(2-((4-(1-(isopropoxycarbonyl)azetidin-3-yl)benzyl)oxy)-3-(trifluoromethyl)phenyl)pyridin-2-yl)-5-(trifluoromethyl)-1H-pyrazole-4-carboxylic acid |
| 160 | 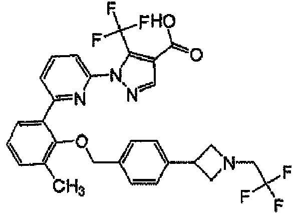 | 1-(6-(3-methyl-2-((4-(1-(2,2,2-trifluoroethyl)azetidin-3-yl)benzyl)oxy)phenyl)pyridin-2-yl)-5-(trifluoromethyl)-1H-pyrazole-4-carboxylic acid |
| 161 | 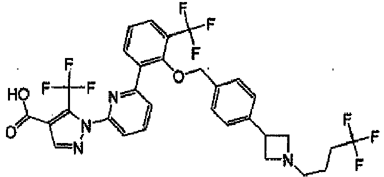 | 1-(6-(2-((4-(1-(4,4,4-trifluorobutyl)azetidin-3-yl)benzyl)oxy)-3-(trifluoromethyl)phenyl)pyridin-2-yl)-5-(trifluoromethyl)-1H-pyrazole-4-carboxylic acid |

### Example 162

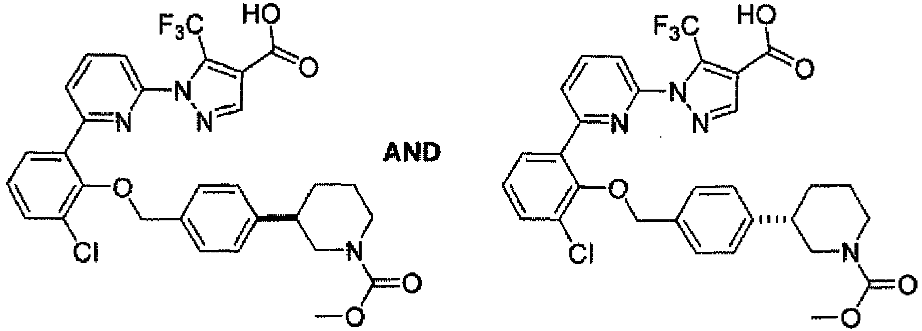

The compounds listed in **Table 4** were prepared using chemistry described in **Example 162,** and/or by analogy to chemistry described in **Examples 1-14, Example 136,** and **Example 148.**

**Table 4**

| Example | Structure | IUPAC |
|---|---|---|
| 163, 164 | 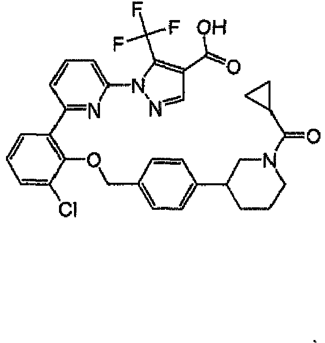 | 1-(6-(3-chloro-2-((4-(3R)-(1-(cyclopropylcarbonyl)piperidin-3-yl)benzyl)oxy)phenyl)pyridin-2-yl)-5-(trifluoromethyl)-1H-pyrazole-4-carboxylic acid **AND** 1-(6-(3-chloro-2-((4-(3S)-(1-(cyclopropylcarbonyl)piperidin-3-yl)benzyl)oxy)phenyl)pyridin-2-yl)-5-(trifluoromethyl)-1H-pyrazole-4-carboxylic acid |
| 165, 166 | 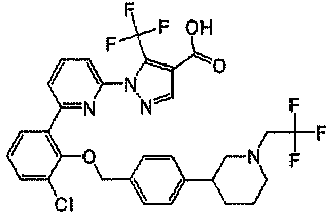 | 1-(6-(3-chloro-2-((4-(3R)-(1-(2,2,2-trifluoroethyl)piperidin-3-yl)benzyl)oxy)phenyl)pyridin-2-yl)-5-(trifluoromethyl)-1H-pyrazole-4-carboxylic acid **AND** 1-(6-(3-chloro-2-((4-(3S)-(1-(2,2,2-trifluoroethyl)piperidin-3-yl)benzyl)oxy)phenyl)pyridin-2-yl)-5-(trifluoromethyl)-1H-pyrazole-4-carboxylic acid |

### Example 167

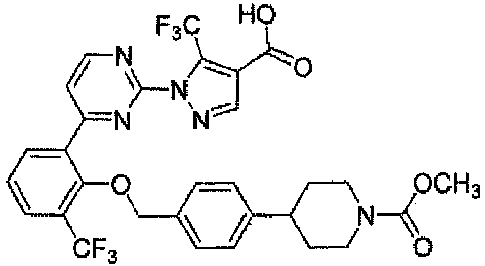

The compounds listed in **Table** 5 were prepared using chemistry described in **Example 167,** and/or by analogy to chemistry described in **Examples 1-14, Examples 136, 148,** and **162.**

**Table 5.**

| Example | Structure | IUPAC |
|---|---|---|
| 168 | 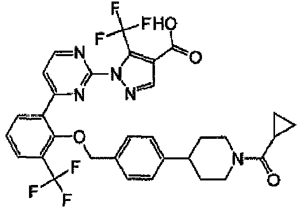 | 1-(4-(2-((4-(1-(cyclopropylcarbonyl)piperidin-4-yl)benzyl)oxy)-3-(trifluoromethyl)phenyl)pyrimidin-2-yl)-5-(trifluoromethyl)-1H-pyrazole-4-carboxylic acid |
| 169 | 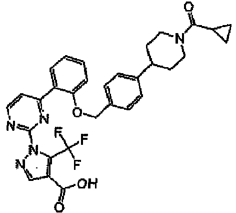 | 1-(4-(2-((4-(1-(cyclopropylcarbonyl)piperidin-4-yl)benzyl)oxy)phenyl)pyrimidin-2-yl)-5-(trifluoromethyl)-1H-pyrazole-4-carboxylic acid |
| 170 | 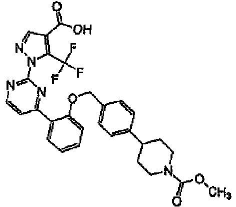 | 1-(4-(2-((4-(1-(methoxycarbonyl)piperidin-4-yl)benzyl)oxy)phenyl)pyrimidin-2-yl)-5-(trifluoromethyl)-1H-pyrazole-4-carboxylic acid |
| 171 | 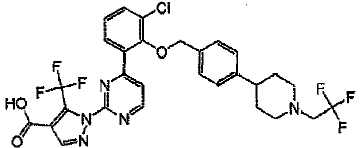 | 1-(4-(3-chloro-2-((4-(1-(2,2,2-trifluoroethyl)piperidin-4-yl)benzyl)oxy)phenyl)pyrimidin-2-yl)-5-(trifluoromethyl)-1H-pyrazole-4-carboxylic acid |
| 172 | 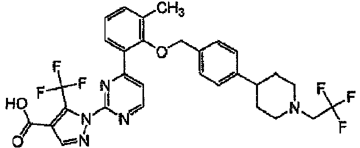 | 1-(4-(3-methyl-2-((4-(1-(2,2,2-trifluoroethyl)piperidin-4-yl)benzyl)oxy)phenyl)pyrimidin-2-yl)-5-(trifluoromethyl)-1H-pyrazole-4-carboxylic acid |
| 173 | 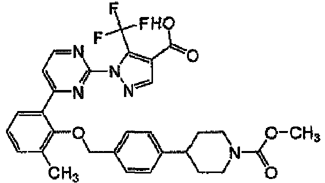 | 1-(4-(2-((4-(1-(methoxycarbonyl)piperidin-4-yl)benzyl)oxy)-3-methylphenyl)pyrimidin-2-yl)-5-(trifluoromethyl)-1H-pyrazole-4-carboxylic acid |
| 174 | 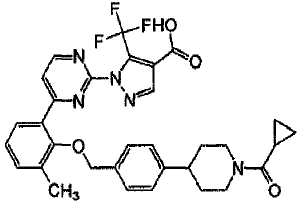 | 1-(4-(2-((4-(1-(cyclopropylcarbonyl)piperidin-4-yl)benzyl)oxy)-3-methy)phenyl)pyrimidin-2-yl)-5-(trifluoromethyl)-1H-pyrazole-4-carboxylic acid |
| 175 | 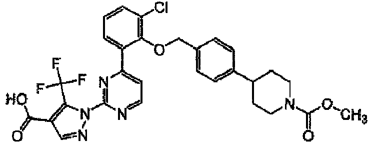 | 1-(4-(3-chloro-2-((4-(1-(methoxycarbonyl)piperidin-4-yl)benzyl)oxy)phenyl)pyrimidin-2-yl)-5-(trifluoromethyl)-1H-pyrazole-4-carboxylic acid |
| 176 | 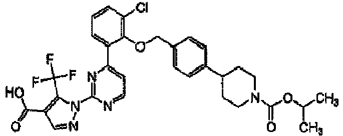 | 1-(4-(3-chloro-2-((4-(1-(isopropoxycarbonyl)piperidin-4-yl)benzyl)oxy)phenyl)pyrimidin-2-yl)-5-(trifluoromethyl)-1H-pyrazole-4-carboxylic acid |
| 177 | 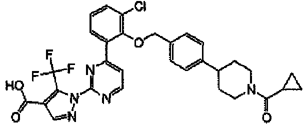 | 1-(4-(3-chloro-2-((4-(1-(cyclopropylcarbonyl)piperidin-4-yl)benzyl)oxy)phenyl)pyrimidin-2-yl)-5-(trifluoromethyl)-1H-pyrazole-4-carboxylic acid |
| 178 | 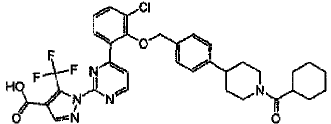 | 1-(4-(3-chloro-2-((4-(1-(cyclohexylcarbonyl)piperidin-4-yl)benzyl)oxy)phenyl)pyrimidin-2-yl)-5-(trifluoromethyl)-1H-pyrazole-4-carboxylic acid |
| 179 | 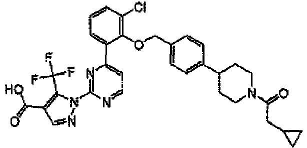 | 1-(4-(3-chloro-2-((4-(1-(cyclopropylacetyl)piperidin-4-yl)benzyl)oxy)phenyl)pyrimidin-2-yl)-5-(trifluoromethyl)-1H-pyrazole-4-carboxylic acid |

### Example 180

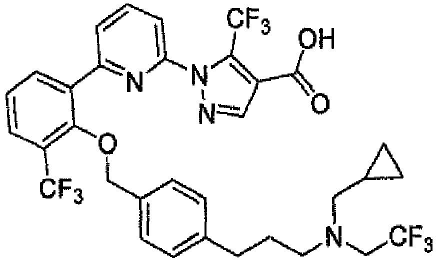

The compounds listed in Table 6 were prepared using chemistry described in Example 180, and/or by analogy to chemistry described in Examples **1-14, 136,** 148, 162, and **167.**

**Table 6**

| Example | Structure | IUPAC |
|---|---|---|
| 181 | 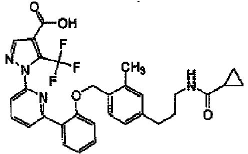 | 1-(6-(2-((4-(3-((cyclopropylcarbonyl)amino)prop yl)-2-methylbenzyl)ox y)phenyl)pyridin-2-yl)-5-(trifluoromethyl)-1H-pyrazole-4-carboxylic acid |
| 182 | 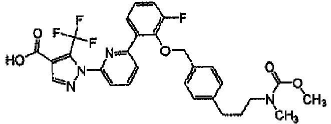 | 1-(6-(3-fluoro-2-((4-(3-((methoxycarbonyl)-(methyl)amino)propyl)benzyl)oxy) -phenyl)pyridin-2-yl)-5-(trifluoromethyl)--pyrazole-4-carboxylic acid |
| 183 | 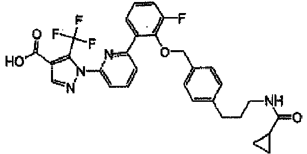 | 1-(6-(2-((4-(3-((cyclopropylcarbonyl)amino)prop yl) benzyl)oxy)-3-fluorophenyl)pyridin-2-yl)-5-(trifluoromethyl)-1H-pyrazole-4-carboxylic acid |
| 184 | 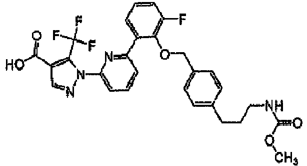 | 1-(6-(3-fluoro-2-((4-(3-((methoxycarbonyl)amino)propyl) benzyl)oxy)phenyl)pyridin-2-yl)-5-(trifluoromethyl)-1H-pyrazole-4-carboxylic acid |
| 185 | 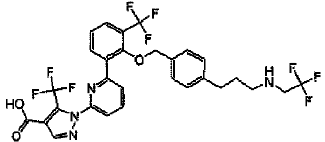 | 1-(6-(2-((4-(3-((2,2,2-trifluoroethyl)amino)propyl)benzyl )oxy)-3-(trifluoromethyl)phenyl)pyridin-2-yl)-5-(trifluoromethyl)-1H-pyrazole-4-carboxylic acid |
| 186 | 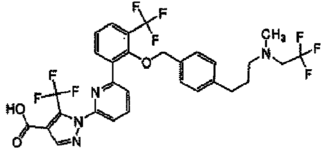 | 1-(6-(2-((4-(3-(methyl(2,2,2-trifluoroethyl)amino)propyl)benzyl )oxy)-3-(trifluoromethyl)phenyl)pyridin-2-yl)-5-(trifluoromethyl)-1H-pyrazole-4-carboxylic acid |

### Compound Class V:

Examples 1 to 191 of compound class V are disclosed in WO2009/071504.

### Example 1: 1-[6-(2-(4-phenethyl)benzyloxy)-phenyl)pyridin-2-yl]-5-trifluoromethyl-pyrazole-4-carboxylic acid

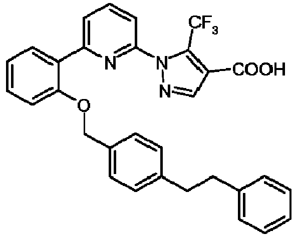

### Example 2: 1-[6-(2-(4-(4-trifluoromethylphenyl)benzyloxy)-phenyl)pyridin-2-yl]-5-trifluoromethyl-pyrazole-4-carboxylic acid

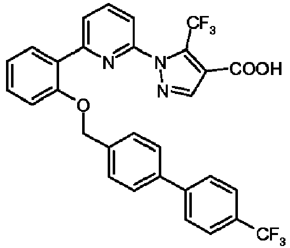

### Example 3: 1-[6-(2-(4-(4-methoxyphenyl)benzyloxy)-phenyl)pyridin-2-yl]-5-trifluoromethyl-pyrazole-4-carboxylic acid

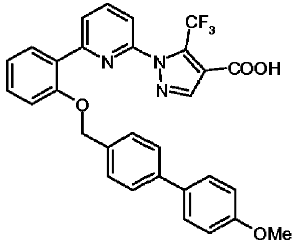

### Example 4: 1-[6-(5-chloro-2-(4-(4-methoxyphenyl)benzyloxy)-phenyl)pyridin-2-yl]-5-trifluoromethyl-pyrazole-4-carboxylic acid

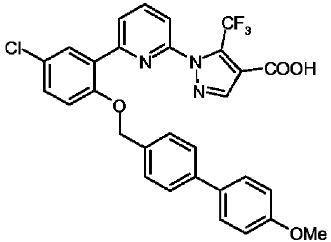

### Example 5: 1-[6-(5-fluoro-2-(4-(4-methoxyphenyl)benzyloxy)-phenyl)pyridin-2-yl]-5-trifluoromethyl-pyrazole-4-carboxylic acid

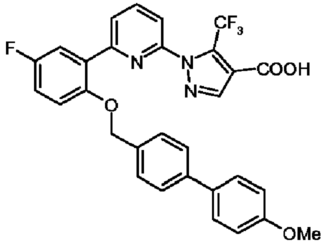

### Example 6: 1-[6-(3,5-difluoro-2-(4-(4-methoxyphenyl)benzyloxy)-phenyl)pyridin-2-yl]-5-trifluoromethyl-pyrazole-4-carboxylic acid

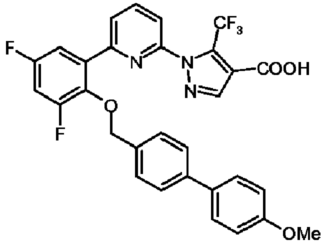

### Example 7: 1-[6-(2-(2-methyl-4-(4-fluorophenyl)benzyloxy)-phenyl)pyridin-2-yl]-5-trifluoromethyl-pyrazole-4-carboxylic acid

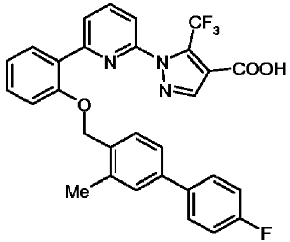

### Example 8: 1-[6-(2-(4-(4-cyanophenoxy)benzyloxy)-phenyl)pyridin-2-yl]-5-trifluoromethyl-pyrazole-4-carboxylic acid

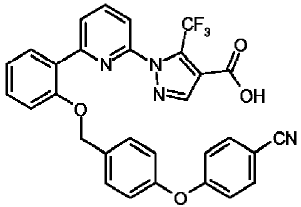

### Example 9: 1-[6-(2-(2-methyl-4-(4-trifluoromethoxyphenyl)benzyloxy)-phenyl)pyridin-2-yl]-5-trifluoromethyl-pyrazole-4-carboxylic acid

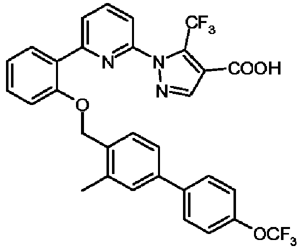

### Example 10: 1-[6-(5-methyl-2-(2-methyl-4-(3-chloro-5-trifluoromethylpyridin-2-yl)benzyloxy)-phenyl)pyridin-2-yl]-5-trifluoromethyl-pyrazole-4-carboxylic acid

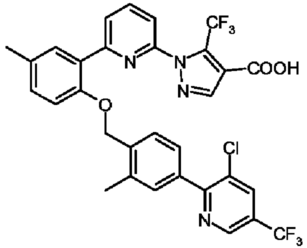

| **Example** | **Name** |
|---|---|
| 11 | 1-[6-(2-(4-(phenyl)phenylmethyloxy)-phenyl)pyridin-2-yl]-5-trifluoromethyl-pyrazole-4-carboxylic acid |
| 12 | 1-[6-(2-(4-(phenethyl)phenylmethyloxy)-phenyl)pyridin-2-yl]-5-methylpyrazole-4-carboxylic acid |
| 13 | 1-[6-(2-(4-(4-azidophenyl)phenylmethyloxy)-phenyl)pyridin-2-yl]-5-trifluoromethyl-pyrazole-4-carboxylic acid |
| 14 | 1-[6-(2-(4-(4-methoxyphenyl)phenylmethyloxy)-phenyl)pyridin-2-yl]-5-methyl-pyrazole-4-carboxylic acid |
| 15 | 1-[6-(2-(4-(4-t-butylphenyl)phenylmethyloxy)-phenyl)pyrid in-2-yl]-5-trifluoromethyl-pyrazole-4-carboxylic acid |
| 16 | 1-[6-(2-(4-(3,4-dichlorophenyl)phenylmethyloxy)-phenyl)pyridin-2-yl]-5-trifluoromethyl-pyrazole-4-carboxylic acid |
| 17 | 1-[6-(2-(4-(4-fluorophenyl)phenylmethyloxy)-phenyl)pyridin-2-yl]-5-trifluoromethyl-pyrazole-4-carboxylic acid |
| 18 | 1-[6-(2-(4-(4-cyanophenyl)phenylmethyloxy)-phenyl)pyridin-2-yl]-5-trifluoromethyl-pyrazole-4-carboxylic acid |
| 19 | 1-[6-(2-(4-(4-chlorophenyl)phenylmethyloxy)-phenyl)pyrid in-2-yl]-5-trifluoromethyl-pyrazole-4-carboxylic acid |
| 20 | 1-[6-(2-(4-(4-trifluoromethoxyphenyl)phenylmethyloxy)-phenyl)pyridin-2-yl]-5-trifluoromethyl-pyrazole-4-carboxylic acid |
| 21 | 1-[6-(2-(4-(4-carboxyphenyl)phenylmethyloxy)-phenyl)pyridin-2-yl]-5-trifluoromethyl-pyrazole-4-carboxylic acid |
| 22 (racemic mixture) | 1-[6-(2-(1-(4-(4-methoxyphenyl)phenyl)ethyloxy)-phenyl)pyridin-2-yl]-5-trifluoromethyl-pyrazole-4-carboxylic acid |
| 23 | 1-[6-(2-(4-(4-fluoro-2-methylphenyl)phenylmethyloxy)-phenyl)pyrid in-2-yl]-5-trifluoromethyl-pyrazole-4-carboxylic acid |
| 24 | 1-[6-(2-(2-methyl-4-(4-trifluoromethylphenyl)phenylmethyloxy)-phenyl)pyridin-2-yl]-5-trifluoromethyl-pyrazole-4-carboxylic acid |
| 25 | 1-[6-(2-(2-methyl-4-(4-methoxyphenyl)phenylmethyloxy)-phenyl)pyridin-2-yl]-5-trifluoromethyl-pyrazole-4-carboxylic acid |
| 26 | 1-[6-(2-(2-methyl-4-(4-cyanophenyl)phenylmethyloxy)-phenyl)pyridin-2-yl]-5-trifluoromethyl-pyrazole-4-carboxylic acid |
| 27 | 1-[6-(2-(2-methyl-4-(4-carboxyphenyl)phenylmethyloxy)-phenyl)pyridin-2-yl]-5-trifluoromethyl-pyrazole-4-carboxylic acid |
| 28 | 1-[6-(2-(2-methyl-4-(4-fluoro-2-methylphenyl)phenylmethyloxy)-phenyl)pyridin-2-yl]-5-trifluoromethyl-pyrazole-4-carboxylic acid |
| 29 | 1-[6-(2-(2-methyl-4-(4-methoxy-2-methylphenyl)phenylmethyloxy)-phenyl)pyridin-2-yl]-5-trifluoromethyl-pyrazole-4-carboxylic acid |
| 30 | 1-[6-(2-(2-methyl-4-(4-methoxy-3-trifluoromethylphenyl)phenylmethyloxy)-phenyl)pyridin-2-yl]-5-trifluoromethyl-pyrazole-4-carboxylic acid |
| 31 | 1-[6-(2-(2-methyl-4-(3-chloro-4-methoxy-phenyl)phenylmethyloxy)-phenyl)pyridin-2-yl]-5-trifluoromethyl-pyrazole-4-carboxylic acid |
| 32 | 1-[6-(2-(2-methyl-4-(3,4-dichloro-phenyl)phenylmethyloxy)-phenyl)pyridin-2-yl]-5-trifluoromethyl-pyrazole-4-carboxylic acid |
| 33 | 1-[6-(2-(2-methyl-4-(4-chloro-2-trifluoromethylphenyl)phenylmethyloxy)-phenyl)pyridin-2-yl]-5-trifluoromethyl-pyrazole-4-carboxylic acid |
| 34 | 1-[6-(2-(2-methyl-4-(2,4-dimethoxy-phenyl)phenylmethyloxy)-phenyl)pyridin-2-yl]-5-trifluoromethyl-pyrazole-4-carboxylic acid |
| 35 | 1-[6-(2-(2-methyl-4-(4-methoxy-2-trifluoromethylphenyl)phenylmethyloxy)-phenyl)pyridin-2-yl]-5-trifluoromethyl-pyrazole-4-carboxylic acid |
| 36 | 1-[6-(2-(2-methyl-4-(3-chloro-4-isopropyloxylphenyl)phenylmethyloxy)-phenyl)pyridin-2-yl]-5-trifluoromethyl-pyrazole-4-carboxylic acid |
| 37 | 1-[6-(2-(2-methyl-4-(4-isopropyloxyl-2-methylphenyl)phenylmethyloxy)-phenyl)pyridin-2-yl]-5-trifluoromethyl-pyrazole-4-carboxylic acid |
| 38 | 1-[6-(2-(2-methyl-4-(4-ethyloxyl-3-trifluoromethylphenyl)phenylmethyloxy)-phenyl)pyridin-2-yl]-5-trifluoromethyl-pyrazole-4-carboxylic acid |
| 39 | 1-[6-(2-(2-methyl-4-(4-chloro-2-methoxyphenyl)phenylmethyloxy)-phenyl)pyridin-2-yl]-5-trifluoromethyl-pyrazole-4-carboxylic acid |
| 40 | 1-[6-(2-(2-methyl-4-(4-ethyloxy-3-methylphenyl)phenylmethyloxy)-phenyl)pyridin-2-yl]-5-trifluoromethyl-pyrazole-4-carboxylic acid |
| 41 | 1-[6-(2-(2-methyl-4-(4-chloro-2-ethyloxyphenyl)phenylmethyloxy)-phenyl)pyridin-2-yl]-5-trifluoromethyl-pyrazole-4-carboxylic acid |
| 42 | 1-[6-(2-(2-methyl-4-(2-chloro-4-ethyloxyphenyl)phenylmethyloxy)-phenyl)pyridin-2-yl]-5-trifluoromethyl-pyrazole-4-carboxylic acid |
| 43 | 1-[6-(2-(2-methyl-4-(2-chloro-4-methoxyphenyl)phenylmethyloxy)-phenyl)pyridin-2-yl]-5-trifluoromethyl-pyrazole-4-carboxylic acid |
| 44 | 1-[6-(2-(2-methoxy-4-(4-fluorophenyl)phenylmethyloxy)-phenyl)pyridin-2-yl]-5-trifluoromethyl-pyrazole-4-carboxylic acid |
| 45 | 1-[6-(2-(2-methoxy-4-(4-trifluoromethylphenyl)phenylmethyloxy)-phenyl)pyridin-2-yl]-5-trifluoromethyl-pyrazole-4-carboxylic acid |
| 46 | 1-[6-(2-(2-methoxy-4-(4-methoxyphenyl)phenylmethyloxy)-phenyl)pyridin-2-yl]-5-trifluoromethyl-pyrazole-4-carboxylic acid |
| 47 | 1-[6-(2-(2-methoxy-4-(4-carboxyphenyl)phenylmethyloxy)-phenyl)pyridin-2-yl]-5-trifluoromethyl-pyrazole-4-carboxylic acid |
| 48 | 1-[6-(2-(2-methoxy-4-(4-methoxy-3-trifluoromethylphenyl)phenylmethyloxy)-phenyl)pyridin-2-yl]-5-trifluoromethyl-pyrazole-4-carboxylic acid |
| 49 | 1-[6-(2-(2-methoxy-4-(2-chloro-4-methoxy-phenyl)phenylmethyloxy)-phenyl)pyridin-2-yl]-5-trifluoromethyl-pyrazole-4-carboxylic acid |
| 50 | 1-[6-(2-(2-methoxy-4-(4-methoxy-2-methylphenyl)phenylmethyloxy)-phenyl)pyridin-2-yl]-5-trifluoromethyl-pyrazole-4-carboxylic acid |
| 51 | 1-[6-(2-(2-fluoro-4-(4-carboxyphenyl)phenylmethyloxy)-phenyl)pyridin-2-yl]-5-trifluoromethyl-pyrazole-4-carboxylic acid |
| 52 | 1-[6-(2-(2-(2-methyl-propyloxy)-4-(4-fluorophenyl)phenylmethyloxy)-phenyl)pyridin-2-yl]-5-trifluoromethyl-pyrazole-4-carboxylic acid |
| 53 | 1-[6-(2-(2-(2-methyl-propyloxy)-4-(4-methoxyphenyl)phenylmethyloxy)-phenyl)pyridin-2-yl]-5-trifluoromethyl-pyrazole-4-carboxylic acid |
| 54 | 1-[6-(2-(2-(2-methyl-propyloxy)-4-(4-cyanophenyl)phenylmethyloxy)-phenyl)pyridin-2-yl]-5-trifluoromethyl-pyrazole-4-carboxylic acid |
| 55 | 1-[6-(2-(2-(methoxyethyloxy)-4-(4-fluorophenyl)phenylmethyloxy)-phenyl)pyridin-2-yl]-5-trifluoromethyl-pyrazole-4-carboxylic acid |
| 56 | 1-[6-(2-(2-(methoxyethyloxy)-4-(4-methoxyphenyl)phenylmethyloxy)-phenyl)pyridin-2-yl]-5-trifluoromethyl-pyrazole-4-carboxylic acid |
| 57 | 1-[6-(2-(2-(methoxyethyloxy)-4-(4-cyanophenyl)phenylmethyloxy)-phenyl)pyridin-2-yl]-5-trifluoromethyl-pyrazole-4-carboxylic acid |
| 58 | 1-[6-(2-(2-propyloxy-4-(4-trifluoromethylphenyl)phenylmethyloxy)-phenyl)pyridin-2-yl]-5-trifluoromethyl-pyrazole-4-carboxylic acid |
| 59 | 1-[6-(2-(2-propyloxy-4-(4-cyanophenyl)phenylmethyloxy)-phenyl)pyridin-2-yl]-5-trifluoromethyl-pyrazole-4-carboxylic acid |
| 60 | 1-[6-(2-(4-(5-trifluoromethylpyridin-2-yl)phenylmethyloxy)-phenyl)pyridin-2-yl]-5-trifluoromethyl-pyrazole-4-carboxylic acid |
| 61 | 1-[6-(2-(4-(5-trifluoromethylpyridin-2-yl)phenylmethyloxy)-phenyl)pyridin-2-yl]-5-methyl-pyrazole-4-carboxylic acid |
| 62 | 1-[6-(2-(4-(5-cyanopyridin-2-yl)phenylmethyloxy)-phenyl)pyridin-2-yl]-5-trifluoromethyl-pyrazole-4-carboxylic acid |
| 63 | 1-[6-(2-(4-(5-methoxypyridin-2-yl)phenylmethyloxy)-phenyl)pyridin-2-yl]-5-trifluoromethyl-pyrazole-4-carboxylic acid |
| 64 | 1-[6-(2-(4-(5-methoxypyridin-2-yl)phenylmethyloxy)-phenyl)pyridin-2-yl]-5-methyl-pyrazole-4-carboxylic acid |
| 65 | 1-[6-(2-(2-methyl-4-(5-trifluoromethylpyridin-2-yl)phenylmethyloxy)-phenyl)pyridin-2-yl]-5-trifluoromethyl-pyrazole-4-carboxylic acid |
| 66 | 1-[6-(2-(2-methyl-4-(5-chloropyridin-2-yl)phenylmethyloxy)-phenyl)pyridin-2-yl]-5-trifluoromethyl-pyrazole-4-carboxylic acid |
| 67 | 1-[6-(2-(2-methyl-4-(5-methoxypyridin-2-yl)phenylmethyloxy)-phenyl)pyridin-2-yl]-5-trifluoromethyl-pyrazole-4-carboxylic acid |
| 68 | 1-[6-(2-(2-methyl-4-(3-chloro-5-trifluoromethylpyridin-2-yl)phenylmethyloxy)-phenyl)pyridin-2-yl]-5-trifluoromethyl-pyrazole-4-carboxylic acid |
| 69 | 1-[6-(2-(2-methyl-4-(6-methoxypyridin-3-yl)phenylmethyloxy)-phenyl)pyridin-2-yl]-6-trifluoromethyl-pyrazole-4-carboxylic acid |
| 70 | 1-[6-(2-((6-(4-methoxyphenyl)pyridin-3-yl)methyloxy)-phenyl)pyridin-2-yl]-5-trifluoromethyl-pyrazole-4-carboxylic acid |
| 71 | 1-[6-(2-(4-(4-methylthiazol-2-yl)phenylmethyloxy)-phenyl)pyrid in-2-yl]-5-methyl-pyrazole-4-carboxylic acid |
| 72 | 1-[6-(2-(4-(phenoxy)phenylmethyloxy)-phenyl)pyridin-2-yl]-5-trifluoromethyl-pyrazole-4-carboxylic acid |
| 73 | 1-[6-(2-(4-(4-carboxyphenyloxy)phenylmethyloxy)-phenyl)pyridin-2-yl]-5-trifluoromethyl-pyrazole-4-carboxylic acid |
| 74 | 1-[6-(2-(4-(5-trifluoromethylpyridin-2-yloxy)phenylmethyloxy)-phenyl)pyridin-2-yl]-5-trifluoromethyl-pyrazole-4-carboxylic acid |
| 75 | 1-[6-(2-(4-(5-trifluoromethylpyridin-2-yloxy)phenylmethyloxy)-phenyl)pyridin-2-yl]-5-methyl-pyrazole-4-carboxylic acid |
| 76 | 1-[6-(2-(4-(5-chloropyridin-2-yloxy)phenylmethyloxy)-phenyl)pyridin-2-yl]-5-trifluoromethyl-pyrazole-4-carboxylic acid |
| 77 | 1-[6-(2-(4-(3-chloro-5-trifluoromethylpyridin-2-yloxy)phenylmethyloxy)-phenyl)pyridin-2-yl]-5-trifluoromethyl-pyrazole-4-carboxylic acid |
| 78 | 1-[6-(2-(2-methoxy-4-(3-chloro-5-trifluoromethylpyridin-2-yloxy)phenylmethyloxy)-phenyl)pyridin-2-yl]-5-trifluoromethyl-pyrazole-4-carboxylic acid |
| 79 | 1-[6-(2-(2-methyl-4-(3-chloro-5-trifluoromethylpyridin-2-yloxy)phenylmethyloxy)-phenyl)pyridin-2-yl]-5-trifluoromethyl-pyrazole-4-carboxylic acid |
| 80 | 1-[6-(2-((6-(3,4-dichlorophenoxy)pyridin-3-yl)methyloxy)-phenyl)pyridin-2-yl]-5-trifluoromethyl-pyrazole-4-carboxylic acid |
| 81 | 1-[6-(2-((6-(4-chlorophenoxy)pyridin-3-yl)methyloxy)-phenyl)pyridin-2-yl]-5-trifluoromethyl-pyrazole-4-carboxylic acid |
| 82 | 1-[6-(2-((6-(4-chlorophenoxy)pyridin-3-yl)methyloxy)-phenyl)pyridin-2-yl]-5-methyl-pyrazole-4-carboxylic acid |
| 83 | 1-[6-(2-((6-(4-methoxyphenoxy)pyridin-3-yl)methyloxy)-phenyl)pyridin-2-yl]-5-trifluoromethyl-pyrazole-4-carboxylic acid |
| 84 | 1-[6-(2-((6-(4-methoxyphenoxy)pyridin-3-yl)methyloxy)-phenyl)pyridin-2-yl]-5-methyl-pyrazole-4-carboxylic acid |
| 85 | 1-(6-{2-[({6-[(4-carboxyphenyl)oxy]-3-pyridinyl}methyl)oxy]phenyl}-pyridin-2-yl)-5-(trifluoromethyl)-1*H*-pyrazole-4-carboxylic acid |
| 86 | 1-[6-(2-(4-(4-(t-butyl)phenylmethyloxy)phenylmethyloxy)-phenyl)pyridin-2-yl]-5-trifluoromethyl-pyrazole-4-carboxylic acid |
| 87 | 1-[6-(3,5-difluoro-2-(4-(4-cyanophenyl)phenylmethyloxy)-phenyl)pyridin-2-yl]-5-trifluoromethyl-pyrazole-4-carboxylic acid |
| 88 | 1-[6-(3,5-difluoro-2-(4-(4-carboxyphenyl)phenylmethyloxy)-phenyl)pyridin-2-yl]-5-trifluoromethyl-pyrazole-4-carboxylic acid |
| 89 (racemic mixture) | 1-[6-(3,5-difluoro-2-(1-(4-(4-methoxyphenyl)phenyl)ethyloxy)-phenyl)pyridin-2-yl]-5-trifluoromethyl-pyrazole-4-carboxylic acid |
| 90 (racemic mixture) | 1-[6-(3,5-difluoro-2-(1-(4-(4-cyanophenyl)phenyl)ethyloxy)-phenyl)pyridin-2-yl]-5-trifluoromethyl-pyrazole-4-carboxylic acid |
| 91 (racemic mixture) | 1-[6-(3,5-difluoro-2-(1-(4-(4-trifluoromethylphenyl)phenyl)ethyloxy)-phenyl)pyridin-2-yl]-5-trifluoromethyl-pyrazole-4-carboxylic acid |
| 92 | 1-[6-(3,5-difluoro-2-(4-(4-cyanophenoxy)phenylmethyloxy)-phenyl)pyridin-2-yl]-5-trifluoromethyl-pyrazole-4-carboxylic acid |
| 93 | 1-[6-(5-chloro-2-(4-(4-cyanophenyl)phenylmethyloxy)-phenyl)pyridin-2-yl]-5-trifluoromethyl-pyrazole-4-carboxylic acid |
| 94 | 1-[6-(5-chloro-2-(2-methyl-4-(4-cyanophenyl)phenylmethyloxy)-phenyl)pyridin-2-yl]-5-trifluoromethyl-pyrazole-4-carboxylic acid |
| 95 | 1-[6-(5-chloro-2-(2-methyl-4-(4-fluorophenyl)phenylmethyloxy)-phenyl)pyridin-2-yl]-5-trifluoromethyl-pyrazole-4-carboxylic acid |
| 96 | 1-[6-(5-chloro-2-(2-methyl-4-(4-methoxyphenyl)phenylmethyloxy)-phenyl)pyridin-2-yl]-5-trifluoromethyl-pyrazole-4-carboxylic acid |
| 97 | 1-[6-(5-chloro-2-(2-methyl-4-(4-carboxyphenyl)phenylmethyloxy)-phenyl)pyridin-2-yl]-5-trifluoromethyl-pyrazole-4-carboxylic acid |
| 98 | 1-[6-(5-chloro-2-(4-(4-cyanophenyloxy)phenylmethyloxy)-phenyl)pyridin-2-yl]-5-trifluoromethyl-pyrazole-4-carboxylic acid |
| 99 | 1-[6-(5-chloro-2-(4-(5-trifluoromethylpyridin-2-yloxy)phenylmethyloxy)-phenyl)pyridin-2-yl]-5-trifluoromethyl-pyrazole-4-carboxylic acid |
| 100 | 1-[6-(5-chloro-2-(2-methyl-4-(5-trifluoromethylpyridin-2-yloxy)phenylmethyloxy)-phenyl)pyridin-2-yl]-5-trifluoromethyl-pyrazole-4-carboxylic acid |
| 101 | 1-[6-(5-fluoro-2-(4-(4-trifluoromethylphenyl)phenylmethyloxy)-phenyl)pyridin-2-yl]-5-trifluoromethyl-pyrazole-4-carboxylic acid |
| 102 | 1-[6-(5-fluoro-2-(4-(4-methoxy-2-methylphenyl)phenylmethyloxy)-phenyl)pyridin-2-yl]-5-trifluoromethyl-pyrazole-4-carboxylic acid |
| 103 | 1-[6-(5-fluoro-2-(4-(4-fluoro-2-methylphenyl)phenylmethyloxy)-phenyl)pyridin-2-yl]-5-trifluoromethyl-pyrazole-4-carboxylic acid |
| 104 | 1-[6-(5-fluoro-2-(2-methyl-4-(4-methoxyphenyl)phenylmethyloxy)-phenyl)pyridin-2-yl]-5-trifluoromethyl-pyrazole-4-carboxylic acid |
| 105 | 1-[6-(5-fluoro-2-(2-methyl-4-(4-cyanophenyl)phenylmethyloxy)-phenyl)pyridin-2-yl]-5-trifluoromethyl-pyrazole-4-carboxylic acid |
| 106 | 1-[6-(5-fluoro-2-(2-methyl-4-(4-fluorophenyl)phenylmethyloxy)-phenyl)pyridin-2-yl]-5-trifluoromethyl-pyrazole-4-carboxylic acid |
| 107 | 1-[6-(5-fluoro-2-(2-methoxy-4-(4-carboxyphenyl)phenylmethyloxy)-phenyl)pyridin-2-yl]-5-trifluoromethyl-pyrazole-4-carboxylic acid |
| 108 | 1-[6-(5-fluoro-2-(2-fluoro-4-(4-fluorophenyl)phenylmethyloxy)-phenyl)pyridin-2-yl]-5-trifluoromethyl-pyrazole-4-carboxylic acid |
| 109 | 1-[6-(5-fluoro-2-(2-flu oro-4-(4-cyanophenyl)phenylmethyloxy)-phenyl)pyridin-2-yl]-5-trifluoromethyl-pyrazole-4-carboxylic acid |
| 110 | 1-[6-(5-fluoro-2-(2-flu oro-4-(4-methoxyphenyl)ph enylmethyloxy)-phenyl)pyridin-2-yl]-5-trifluoromethyl-pyrazole-4-carboxylic acid |
| 111 | 1-[6-(5-fluoro-2-(2-fluoro-4-(4-carboxyphenyl)phenylmethyloxy)-phenyl)pyridin-2-yl]-5-trifluoromethyl-pyrazole-4-carboxylic acid |
| 112 | 1-[6-(5-fluoro-2-(4-(5-trifluoromethylpyridin-2-yloxy)phenylmethyloxy)-phenyl)pyridin-2-yl]-5-trifluoromethyl-pyrazole-4-carboxylic acid |
| 113 | 1-[6-(5-fluoro-2-((6-(3,4-dichlorophenoxy)pyridin-3-yl)methyloxy)-phenyl)pyridin-2-yl]-5-trifluoromethyl-pyrazole-4-carboxylic acid |
| 114 | 1-[6-(3-(propen-2-yl)-2-(4-(4-trifluoromethylphenyl)phenylmethyloxy)-phenyl)pyridin-2-yl]-5-trifluoromethyl-pyrazole-4-carboxylic acid |
| 115 | 1-[6-(3-propyl-2-(4-(4-trifluoromethylphenyl)phenylmethyloxy)-phenyl)pyridin-2-yl]-5-trifluoromethyl-pyrazole-4-carboxylic acid |
| 116 | 1-[6-(3-(propen-2-yl)-2-(2-methyl-4-(4-trifluoromethylphenyl)phenylmethyloxy)-phenyl)pyridin-2-yl]-5-trifluoromethyl-pyrazale-4-carboxylic acid |
| 117 | 1-[6-(3-propyl-2-(2-methyl-4-(4-trifluoromethylphenyl)phenylmethyloxy)-phenyl)pyridin-2-yl]-5-trifluoromethyl-pyrazole-4-carboxylic acid |
| 118 | 1-[6-(3-(propen-2-yl)-2-(4-(4-methoxyphenyl)phenylmethyloxy)-phenyl)pyridin-2-yl]-5-trifluoromethyl-pyrazole-4-carboxylic acid |
| 119 | 1-[6-(3-propyl-2-(4-(4-methoxyphenyl)phenylmethyloxy)-phenyl)pyridin-2-yl]-5-trifluoromethyl-pyrazole-4-carboxylic acid |
| 120 | 1-[6-(5-methyl- 2-(4-(4-cyanophenyl)phenylmethyloxy)-phenyl)pyridin-2-yl]-5-trifluoromethyl-pyrazole-4-carboxylic acid |
| 121 | 1-[6-(5-methyl-2-(2-methyl-4-(4-cyanophenyl)phenylmethyloxy)-phenyl)pyridin-2-yl]-5-trifluoromethyl-pyrazole-4-carboxylic acid |
| 122 | 1-[6-(5-methyl-2-(2-methyl-4-(4-trifluoromethoxyphenyl)phenylmethyloxy)-phenyl)pyridin-2-yl]-5-trifluoromethyl-pyrazole-4-carboxylic acid |
| 123 | 4-[6-(2-(4-(4-cyanophenyl)phenylmethyloxy)-phenyl)pyridin-2-yl]-benzoic acid |
| 124 | 4-[6-(2-(4-(4-trifluoromethylphenyl)phenylmethyloxy)-phenyl)pyridin-2-yl]-benzoic acid |
| 125 | 1-[6-(2-(4-(phenethyl)phenylmethyloxy)-phenyl)pyridin-2-yl]-2-methyl-pyrrole-3-carboxylic acid |
| 126 | 1-[6-(2-(4-(4-methoxyphenyl)phenylmethyloxy)-phenyl)pyridin-2-yl]-2-methyl-pyrrole-3-carboxylic acid |
| 127 | 1-[6-(2-(2-methyl-4-(4-methoxyphenyl)phenylmethyloxy)-phenyl)pyridin-2-yl]-2-methyl-pyrrole-3-carboxylic acid |
| 128 | 1-[6-(5-fluoro-2-(4-(4-methoxyphenyl)phenylmethyloxy)-phenyl)pyridin-2-yl]-2-methyl-pyrrole-3-carboxylic acid |
| 129 | 1-[6-(2-(4-(4-methoxyphenoxy)phenylmethyloxy)-phenyl)pyridin-2-yl]-2-methyl-pyrrole-3-carboxylic acid |
| 130 | 1-[6-(2-(4-(phenethyl)phenylmethyloxy)-phenyl)pyridin-2-yl]-piperidine-4-carboxylic acid |
| 131 | 1-[6-(2-(4-(4-chlorophenyl)phenylmethyloxy)-phenyl)pyridin-2-yi]-piperidine-4-carboxylic acid |
| 132 | 1-[6-(2-(4-(4-cyanophenyl)phenylmethyloxy)-phenyl)pyridi n-2-yl]-piperidine-4-carboxylic acid |
| 133 | 1-[6-(2-(2-methyl-4-(4-trifluoromethylphenyl)phenylmethyloxy)-phenyl)pyridin-2-yl]-piperidine-4-carboxylic acid |
| 134 | 1-[6-(2-(2-propyloxy-4-(4-trifluoromethylphenyl)phenylmethyloxy)-phenyl)pyridin-2-yl]-piperidine-4-carboxylic acid |
| 135 | 1-[6-(2-(4-(5-trifluoromethylpyrid in-2-yloxy)phenyl methyloxy)-phenyl)pyridin-2-yl]-piperidine-4-carboxylic acid |
| 136 | 1-[6-(5-chloro-2-(2-methyl-4-(4-methoxyphenyl)phenylmethyloxy)-phenyl)pyridin-2-yl]-piperidine-4-carboxylic acid |
| 137 | 1-[6-(5-chloro-2-(2-methyl-4-(4-cyanophenyl)phenylmethyloxy)-phenyl)pyridin-2-yl]-piperidine-4-carboxylic acid |
| 138 | 1-[6-(5-chloro-2-(4-(4-trifluoromethylphenoxy)phenylmethyloxy)-phenyl)pyridin-2-yl]-piperidine-4-carboxylic acid |
| 139 | 1-[6-(5-chloro-2-(4-(5-trifluoromethylpyridin-2-yloxy)phenylmethyloxy)-phenyl)pyridin-2-yl]-piperidine-4-carboxylic acid |
| 140 | 1-[6-(5-chloro-2-(4-(4-cyanophenoxy)phenylmethyloxy)-phenyl)pyridin-2-yl]-piperidine-4-carboxylic acid |
| 141 | 1-[6-(3,5-difluoro-2-(2-methyl-4-(4-cyanophenyl)phenylm ethyloxy)-phenyl)pyridin-2-yl]-piperidine-4-carboxylic acid |
| 142 | 1-[6-(3,5-difluoro-2-(4-(4-trifluoromethylphenoxy)phenylmethyloxy)-phenyl)pyridin-2-yl]-piperidine-4-carboxylic acid |
| 143 | 1-[6-(3,5-difluoro-2-(4-(4-cyanophenoxy)phenylmethyloxy)-phenyl)pyridin-2-yl]-piperidine-4-carboxylic acid |
| 144 | 1-[6-(3,5-difluoro-2-(4-(5-trifluoromethylpyridin-2-yloxy)phenylmethyloxy)-phenyl)pyridin-2-yl]-piperidine-4-carboxylic acid |
| 145 | 1-[6-(3,5-difluoro-2-(4-(5-cyanopyridin-2-yloxy)phenylmethyloxy)-phenyl)pyridin-2-yl]-piperidine-4-carboxylic acid |
| 146 | 1-[6-(5-fluoro-2-(2-methyl-4-(4-methoxyphenyl)phenylmethyloxy)-phenyl)pyridin-2-yl]-piperidine-4-carboxylic acid |
| 147 | 1-[6-(5-fluoro-2-(2-methyl-4-(4-cyanophenyl)phenylmethyloxy)-phenyl)pyridin-2-yl]-piperidine-4-carboxylic acid |
| 148 | 1-[6-(5-fluoro-2-(2-methyl-4-(4-fluorophenyl)phenylmethyloxy)-phenyl)pyridin-2-yl]-piperidine-4-carboxylic acid |
| 149 | 1-[6-(5-fluoro-2-(4-(4-methoxyphenyloxy)phenylmethyloxy)-phenyl)pyridin-2-yl]-piperidine-4-carboxylic acid |
| 150 | 1-[6-(5-fluoro-2-(4-(4-cyanophenyloxy)phenylmethyloxy)-phenyl)pyridin-2-yl]-piperidine-4-carboxylic acid |
| 151 | 1-[6-(5-fluoro-2-(4-(4-fluorophenyloxy)phenylmethyloxy)-phenyl)pyridin-2-yl]-piperidine-4-carboxylic acid |
| 152 | 1-[6-(5-fluoro-2-(4-(4-trifluoromethylphenyloxy)phenylmethyloxy)-phenyl)pyridin-2-yl]-piperidine-4-carboxylic acid |
| 153 | 1-[6-(5-methyl-2-(2-methyl-4-(4-cyanophenyl)phenylmethyloxy)-phenyl)pyridin-2-yl]-piperidine-4-carboxylic acid |
| 154 | 1-[6-(5-methyl-2-(2-methyl-4-(4-trifluoromethylphenyl)phenylmethyloxy)-phenyl)pyridin-2-yl]-piperidine-4-carboxylic acid |
| 155 | 1-[6-(5-methyl-2-(2-methyl-4-(4-trifluoromethoxyphenyl)phenylmethyloxy)-phenyl)pyridin-2-yl]-piperidine-4-carboxylic acid |
| 156 | 1-[6-(5-methyl-2-(2-methyl-4-(4-cyano-2-methylphenyl)phenylmethyloxy)-phenyl)pyridin-2-yl]-piperidine-4-carboxylic acid |
| 157 | 1-[6-(5-methyl-2-(2-methyl-4-(4-methoxy-2-trifluoromethylphenyl)phenylmethyloxy)-phenyl)pyridin-2-yl]-piperidine-4-carboxylic acid |
| 158 | 1-[6-(5-methyl-2-(2-methyl-4-(4-ch loro-2-trifluoromethylphenyl)phenylmethyloxy)-phenyl)pyridin-2-yl]-piperidine-4-carboxylic acid |
| 159 | 1-[6-(5-methyl-2-(2-propyloxy-4-(4-cyanophenyl)phenylmethyloxy)-phenyl)pyridin-2-yl]-piperidine-4-carboxylic acid |
| 160 | 1-[6-(5-methyl-2-(2-methyl-4-(3-chloro-5-trifluoromethylpyridin-2-yloxy)phenylmethyloxy)-phenyl)pyridin-2-yl]-piperidine-4-carboxylic acid |
| 161 | 1-[6-(5-trifluoromethyl-2-(2-methyl-4-(4-cyanophenyl)phenylmethyloxy)-phenyl)pyridin-2-yl]-piperidine-4-carboxylic acid |
| 162 | 1-[6-(5-trifluoromethyl-2-(2-methyl-4-(3-ch loro-5-trifluoromethylpyridin-2-yl)phenylmethyloxy)-phenyl)pyridin-2-yl]-piperidine-4-carboxylic acid |
| 163 | 1-[6-(5-trifluoromethyl-2-(2-propyloxy-4-(4-chloro-2-trifluoromethylphenyl)phenylmethyloxy)-phenyl)pyridin-2-yl]-1-piperidine-4-carboxylic acid |
| 164 | 1-[6-(5-trifluoromethyl-2-(2-propyloxy-4-(2-m ethyl-4-trifluoromethylphenyl)phenylmethyloxy)-phenyl)pyridin-2-yl]-1-piperidine-4-carboxylic acid |
| 165 | 1-[6-(5-trifluoromethyl-2-(2-propyloxy-4-(4-cyano-2-methylphenyl)phenylmethyloxy)-phenyl)pyridin-2-yl]-1-piperidine-4-carboxylic acid |
| 166 | 1-[6-(5-trifluoromethyl-2-(2-propyloxy-4-(2-ch loro-4-methoxyphenyl)phenylmethyloxy)-phenyl)pyridin-2-yl]-1-piperidine-4-carboxylic acid |

### Example 167: 1-[6-(2-(4-(4-cyanophenyl)benzyloxy)-phenyl)pyridin-2-yl]-5-methylpyrazole-4-carboxylic acid

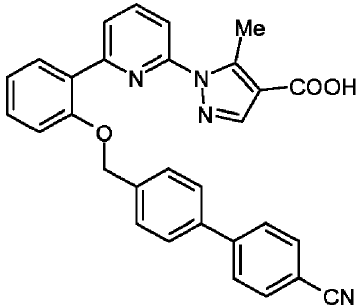

### Example 168: 1-[6-( 2-(4-(4-carboxamidophenyl)benzyloxy)-phenyl)pyridin-2-yl]-5-methyl-pyrazole-4-carboxylic acid

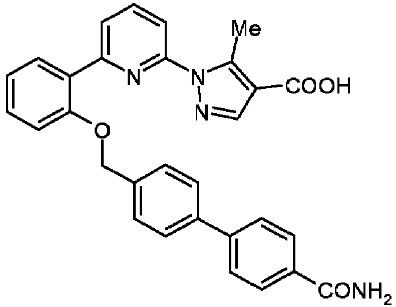

### Example 169: 1-[6-( 2-(4-(4-carboxamidophenoxy)benzyloxy)-phenyl)pyridin-2-yl]-5-trifluoromethyl-pyrazole-4-carboxylic acid

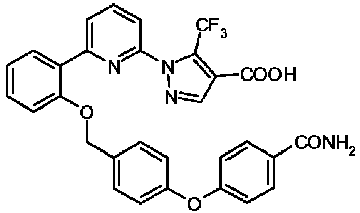

### Example 170: 1-[6-(2-(2-methyl-4-(5-carboxamidopyridin-2-yl)benzyloxy)-phenyl)pyridin-2-yl]-5-trifluoromethyl-pyrazole-4-carboxylic acid

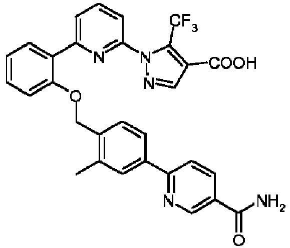

### Example 171: 1-[6-(5-methyl-2-(2-methyl-4-(5-cyanopyridin-2-yl)benzyloxy)-phenyl)pyridin-2-yl]-piperidine-4-carboxylic acid

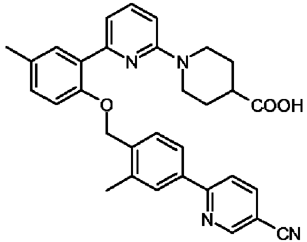

### Example 172: 1-[6-(5-trifluoromethyl-2-(2-methyl-4-(5-cyanopyridin-2-yl)benzyloxy)-phenyl)pyridin-2-yl]-piperidine-4-carboxylic acid

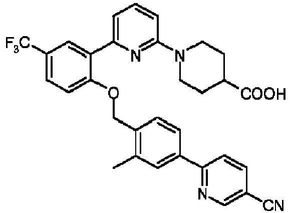

### Example 173: 1-[6-( 5-methyl-2-(2-methyl-4-(3-chloro-5-trifluoromethylpyridin-2-yl)benzyloxy)-phenyl)pyridin-2-yl]-piperidine-4-carboxylic acid

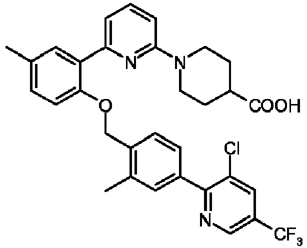

### Example 174: 1-[6-(2-(4-(4-cyanophenyl)benzyloxy)-phenyl)pyridin-2-yl]-2-methyl-pyrrole-3-carboxylic acid

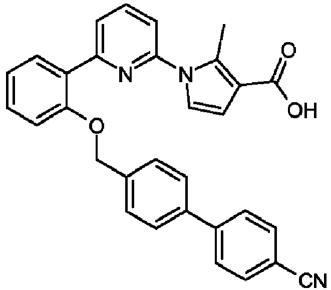

### Example 175: 1-[6-( 2-(2-methyl-4-(4-fluorophenyl)benzyloxy)-phenyl)pyridin-2-yl]-2-methyl-pyrrole-3-carboxylic acid

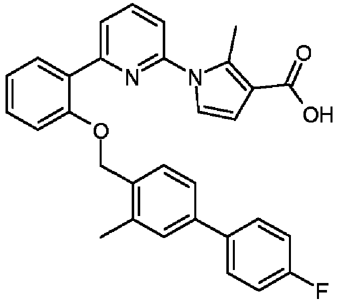

| **Example** | **Name** |
|---|---|
| 176 | 1-[6-(5-fluoro-2-(2-methyl-4-(4-cyanophenyl)phenylmethyloxy)-phenyl)pyridin-2-yl]-2-methyl-pyrrole-3-carboxylic acid |
| 177 | 1-[6-(5-fluoro-2-(2-methyl-4-(4-fluorophenyl)phenylmethyloxy)-phenyl)pyridin-2-yl]-2-methyl-pyrrole-3-carboxylic acid |

### Example 178: 1-[6-( 2-(4-(4-cyanophenoxy)benzyloxy)-phenyl)pyridin-2-yl]-2-methyl-pyrrole-3-carboxylic acid

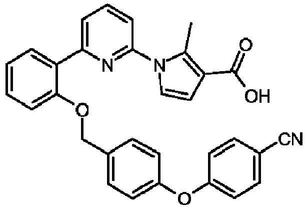

### Example 179: 1-[6-(3,5-difluoro-2-(2-methyl-4-(4-trifluoromethylphenyl)benzyloxy)-phenyl)pyridin-2-yl]-piperidine-4-carboxylic acid

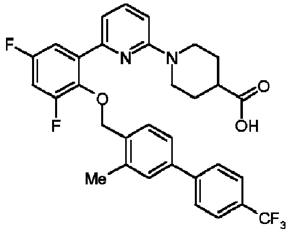

### Example 180: 1-[6-(5-fluoro-2-(2-methyl-4-(4-trifluoromethylphenyl)benzyloxy)-phenyl)pyridin-2-yl]-5-trifluoromethyl-pyrrazole-4-carboxylic acid

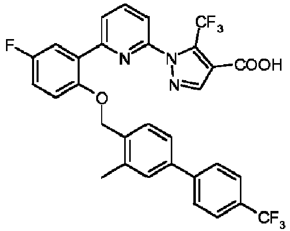

### Example 181: 1-[6-(5-chloro-2-(2-methyl-4-(4-fluorophenyl)benzyloxy)-phenyl)pyridin-2-yl]-piperidine-4-carboxylic acid

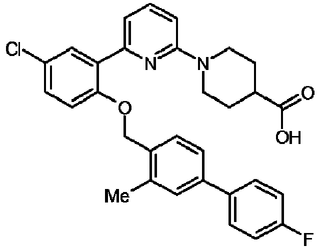

### Example 182: 1-[6-(2-(2-methyl-4-(3,4-dimethoxyphenyl)benzyloxy)-phenyl)pyridin-2-yl]-5-trifluoromethyl-pyrrazole-4-carboxylic acid

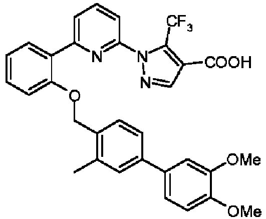

| **Example** | **Name** |
|---|---|
| 183 | 1-[6-(4-chloro-2-(2-methyl-4-(4-trifluoromethoxyphenyl)phenylmethyloxy)-phenyl)pyridin-2-yl]-5-trifluoromethyl-pyrazole-4-carboxylic acid |
| 184 | 1-[6-(6-chloro-2-(2-methyl-4-(4-trifluoromethoxyphenyl)phenylmethyloxy)-phenyl)pyridin-2-yl]-5-trifluoromethyl-pyrazole-4-carboxylic acid |
| 185 | 1-[6-(4-fluoro-2-(2-methyl-4-(4-trifluoromethoxyphenyl)phenylmethyloxy)-phenyl)pyridin-2-yl]-5-trifluoromethyl-pyrazole-4-carboxylic acid |
| 186 | 1-[6-(6-fluoro-2-(2-methyl-4-(4-trifluoromethoxyphenyl)phenylmethyloxy)-phenyl)pyridin-2-yl]-5-trifluoromethyl-pyrazole-4-carboxylic acid |

### Example 187: 1-[6-(2-(4-(4-methoxy-2-methyl-phenyl)benzyloxy)-phenyl)pyridin-2-yl]-5-trifluoromethyl-pyrazole-4-carboxylic acid

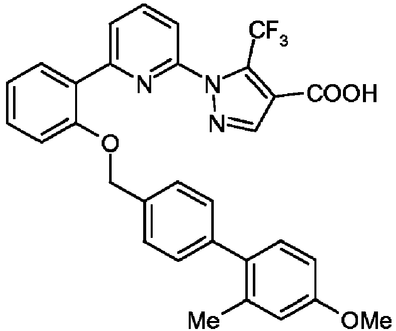

### Example 188: 1-[6-(5-fluoro-2-(2-methyl-4-(4-methoxy-2-methyl-phenyl)benzyloxy)-phenyl)pyridin-2-yl]-5-trifluoromethyl-pyrazole-4-carboxylic acid

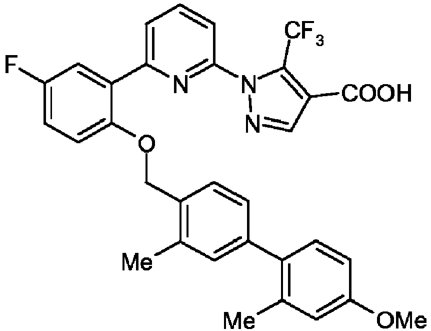

| **Example No.** | **Structure** | **Name** |
|---|---|---|
| **189** | 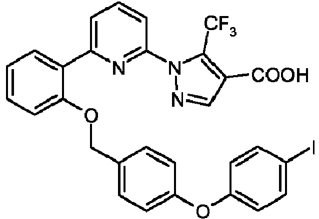 | 1-[6-(2-(4-(4-iodo-phenyloxy)benzyloxy)-phenyl)pyridin-2-yl]-5-trifluoromethyl-pyrazole-4-carboxylic acid |
| **190** | 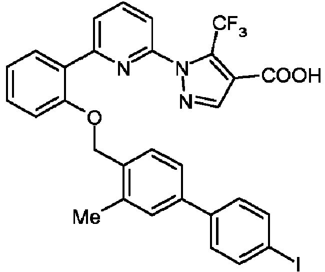 | 1-[6-(2-(2-methyl-4-(4-iodophenyl)benzyloxy)-phenyl)pyridin-2-yl]-5-trifluoromethyl-pyrazole-4-carboxylic acid |
| | | |
| **191** | 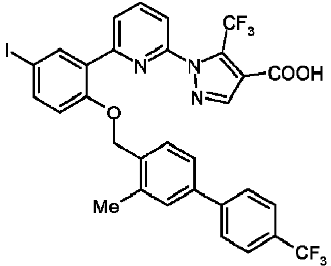 | 1-[6-(5-iodo-2-(2-methyl-4-(4-trifluoromethylphenyl)benzyloxy)-phenyl)pyridin-2-yl]-5-trifluoromethyl-pyrazole-4-carboxylic acid |

### Compound Class VI:

Examples 1 to of 18 compound class VI are disclosed in WO2009/068652.

### Example 1: 1-[6-(3,4-dichlorophenyl)-2-pyridinyl]-5-(trifluoromethyl)-1H-pyrazole-4-carboxylic acid

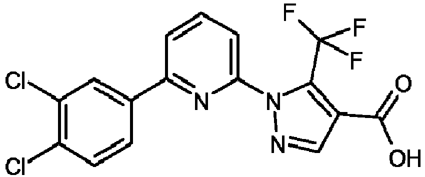

### Example 2: 1-{6-[4-(trifluoromethyl)phenyl]-2-pyridinyl}-5-(trifluoromethyl)-1H-pyrazole-4-carboxylic acid

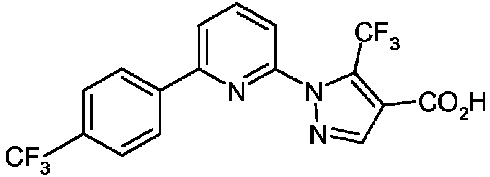

### Example 3: 1-[6-(2,3-dichlorophenyl)-2-pyridinyl]-5-(trifluoromethyl)-1H-pyrazole-4-carboxylic acid

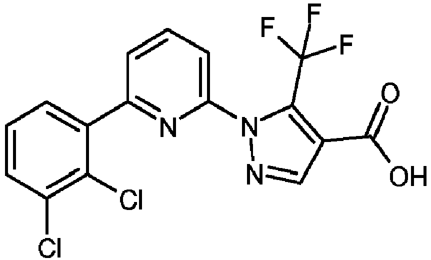

### Example 4: 1-{6-[3-(trifluoromethyl)phenyl]-2-pyridinyl}-5-(trifluoromethyl)-1H-pyrazole-4-carboxylic acid

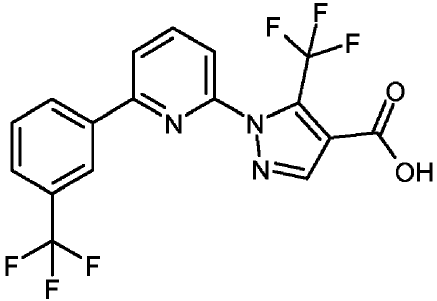

### Example 5: 1-[6-(3,4-difluorophenyl)-2-pyridinyl]-5-(trifluoromethyl)-1H-pyrazole-4-carboxylic acid

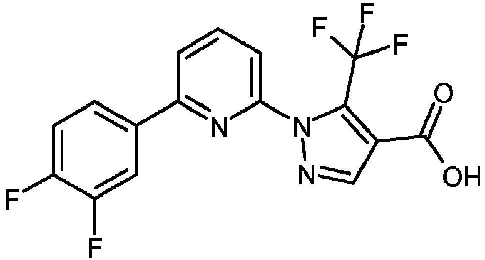

### Example 6: 1-{6-[3,5-bis(trifluoromethyl)phenyl]-2-pyridinyl}-5-(trifluoromethyl)-1H-pyrazole-4-carboxylic acid

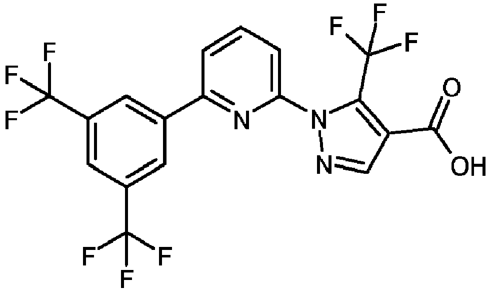

### Example 7: 1-[6-(3-chloro-4-fluorophenyl)-2-pyridinyl]-5-(trifluoromethyl)-1H-pyrazole-4-carboxylic acid

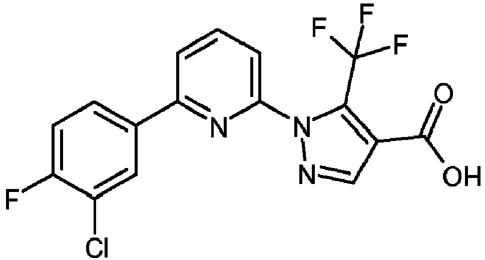

### Example 8: 1-[6-(4-bromo-3-fluorophenyl)-2-pyridinyl]-5-(trifluoromethyl)-1H-pyrazole-4-carboxylic acid

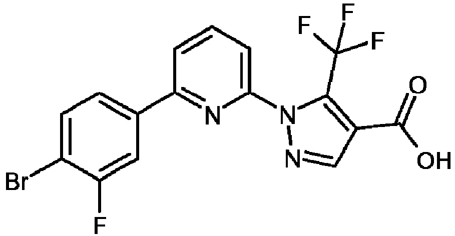

### Example 9: 1-[6-(3,5-dichlorophenyl)-2-pyridinyl]-5-(trifluoromethyl)-1H-pyrazole-4-carboxylic acid

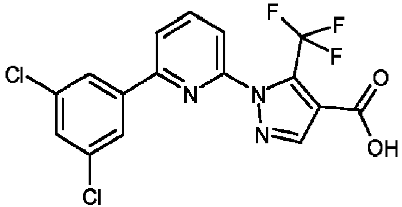

### Example 10: 1-[6-(3,4-dichlorophenyl)-4-methyl-2-pyridinyl]-5-(trifluoromethyl)-1H-pyrazole-4-carboxylic acid

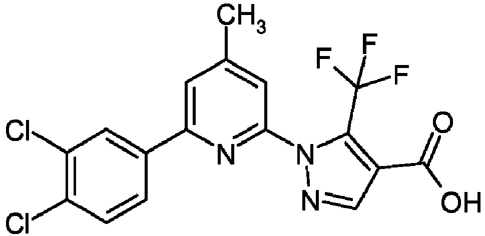

### Example 11: 1-(6-(3,4-dichlorophenyl)-pyridin-2-yl)-pyrrole-3-carboxylic acid

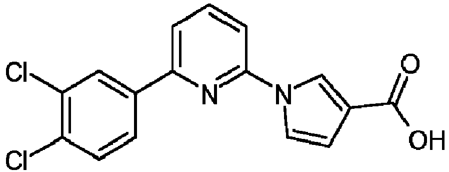

### Example 12: 1-(6-(3,4-dichlorophenyl)-pyridin-2-yl)-2-methyl-pyrrole-3-carboxylic acid

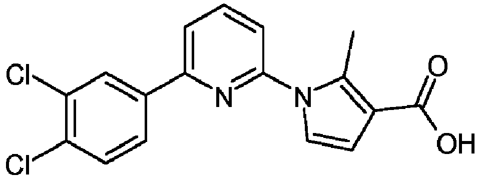

### Example 13 : 1-(6-(3,4-dichlorophenyl)-pyridin-2-yl)-piperidine-4-carboxylic acid

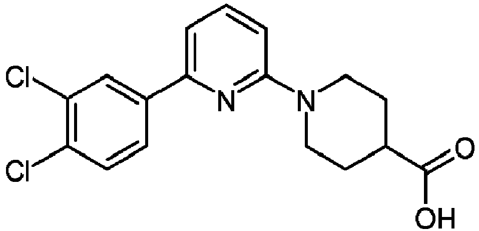

### Example 14: 4-(6-(3-trifluoromethylphenyl)-pyridin-2-yloxy)-benzoic acid

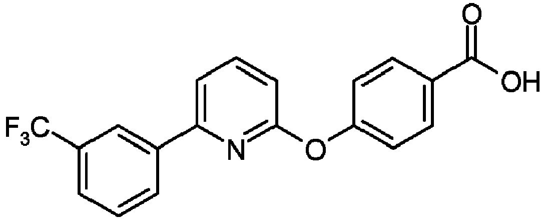

### Example 15: 5-(6-(3-trifluoromethylphenyl)-pyridin-2-yl)-thiophene-2-carboxylic acid

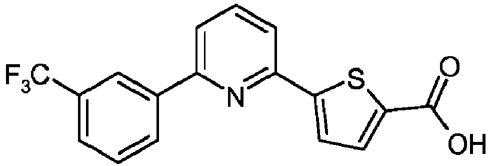

### Example 16: 1-[2-(3,4-dichlorophenyl)-4-pyrimidinyl]-5-(trifluoromethyl)-1H-pyrazole-4-carboxylic acid

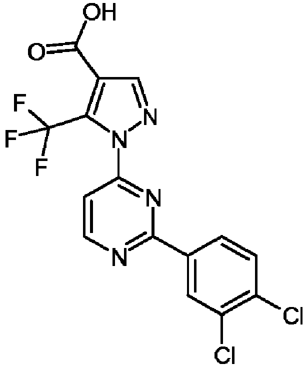

### Example 17: 5-(trifluoromethyl)-1-{2-[3-(trifluoromethyl)phenyl]-4-pyrimidinyl}-1H-pyrazole-4-carboxylic acid

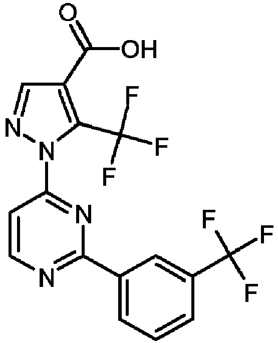

### Example 18: 5-(2-(3-trifluoromethylphenyl)-pyrimidin-4-yl)-thiophene-2-carboxylic acid

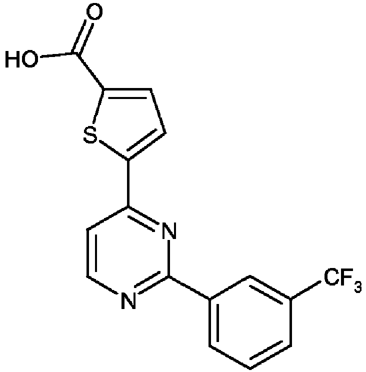

### Compound Class VII:

Examples 1 to 90 of compound class VII are disclosed in WO2010/015652.

### Example 1: 1-[4-(2-{[(4'-cyano-2',3-dimethyl-4-biphenylyl)methyl]oxy}-5-methylphenyl)-1,3-thiazol-2-yl]-4-piperidinecarboxylic acid.

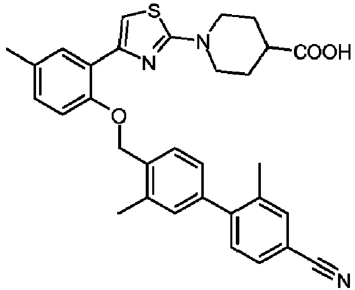

| **Example** | **Name** |
|---|---|
| **2** | 1-[4-(2-{[(4'-cyano-2',3-dimethyl-4-biphenylyl))methyl]oxy}-5-ethylphenyl)-1,3-thiazol-2-yl]-4-piperidinecarboxylic acid |
| **3** | 1-{4-[2-{[(4'-cyano-2',3-dimethy-4-biphenylyl))methyl]oxy}-5-(trifluoromethyl)phenyl]-1,3-thiazol-2-yl}-4-piperidinecarboxylic acid |
| **4** | 1-{4-[2-({[4'-cyano-2'-methyl-3-(propyloxy)-4-biphenylyl]methyl}oxy)-5-methylphenyl]-1,3-thiazol-2-yl}-4-piperidinecarboxylic acid |

### Example 5: 1-{4-[2-({[2'-chloro-3-methyl-4'-(trifluoromethyl)-4-biphenylyl]methyl}oxy)-5-methylphenyl]-1,3-thiazol-2-yl}-4-piperidinecarboxylic acid

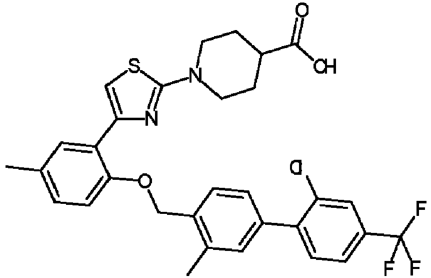

### Example 6: 1-{4-[2-({[4-(2-phenylethyl)phenyl]methyl}oxy)phenyl]-1,3-thiazol-2-yl}-4-piperidinecarboxylic acid

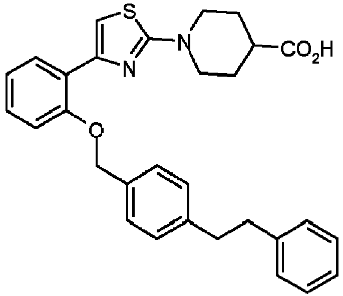

| **Example** | **Name** |
|---|---|
| **7** | 1-{4-[2-({[3'-(methyloxy)-4-biphenylyl]methyl}oxy)phenyl]-1,3-thiazol-2-yl}-4-piperidinecarboxylic acid |
| **8** | 1-[4-(2-{[(4'-fluoro-4-biphenylyl)methyl]oxy}phenyl)-1,3-thiazol-2-yl]-4-piperidinecarboxylic acid |
| **9** | 1-[4-(2-{[(4'-chloro-4-biphenylyl)methyl]oxy}phenyl)-1,3-thiazol-2-yl]-4-piperidinecarboxylic acid |
| **10** | 1-{4-[2-({[4'-(methyloxy)-4-biphenylyl]methyl}oxy)phenyl]-1,3-thiazol-2-yl}-4-piperidinecarboxylic acid |
| **11** | 1-{4-[2-({[4'-(trifluoromethyl)-4-biphenylyl]methyl}oxy)phenyl]-1,3-thiazol-2-yl}-4-piperidinecarboxylic acid |
| **12** | 1-[4-(2-{[(4'-cyano-4-biphenylyl)methyl]oxy}phenyl)-1,3-thiazol-2-yl]-4-piperidinecarboxylic acid |
| **13** | 1-{4-[2-({[3-methyl-4'-(trifluoromethyl)-4-biphenylyl]methyl}oxy)phenyl]-1,3-thiazol-2-yl}-4-piperidinecarboxylic acid |
| **14** | 1-[4-(2-{[(4'-chloro-3-methyl-4-biphenylyl)methyl]oxy}phenyl)-1,3-thiazol-2-yl]-4-piperidinecarboxylic acid |
| **15** | 1-{4-[2-({[3-methyl-4'-(methyloxy)-4-biphenylyl]methyl}oxy)phenyl]-1,3-thiazol-2-yl}-4-piperidinecarboxylic acid |
| **16** | 1-{4-[2-({[3-fluoro-4'-(methyloxy)-4-biphenylyl]methyl}oxy)phenyl]-1,3-thiazol-2-yl}-4-piperidinecarboxylic acid |
| **17** | 1-[4-(2-{[(4'-chloro-3-fluoro-4-biphenylyl)methyl]oxy}phenyl)-1,3-thiazol-2-yl]-4-piperidinecarboxylic acid |
| **18** | 1-(4-{2-[({4-[(4-chlorophenyl)oxy]phenyl}methyl)oxy]phenyl}-1,3-thiazol-2-yl)-4-piperidinecarboxylic acid |
| **19** | 1-[4-(2-{[(4-{[4-(trifluoromethyl)phenyl]oxy}phenyl)methyl]oxy}phenyl)-1,3-thiazol-2-yl]-4-piperidinecarboxylic acid |
| **20** | 1-(4-{2-[({4-[(4-fluorophenyl)oxy]phenyl}methyl)oxy]phenyl}-1,3-thiazol-2-yl)-4-piperidinecarboxylic acid |
| **21** | 1-[4-(2-{[(4'-carboxy-3-fluoro-4-biphenylyl)methyl]oxy}phenyl)-1,3-thiazol-2-yl]-4-piperidinecarboxylic acid |
| **22** | 1-(4-{2-[({4-[(4-cyanophenyl)oxy]phenyl}methyl)oxy]phenyl}-1,3-thiazol-2-yl)-4-piperidinecarboxylic acid |
| **23** | 1-{4-[2-({[3,4'-bis(methyloxy)-4-biphenylyl]methyl}oxy)phenyl]-1,3-thiazol-2-yl}-4-piperidinecarboxylic acid |
| **24** | 1-[4-(2-{[(4-{[4-(methyloxy)phenyl]oxy}phenyl)methyl]oxy}phenyl)-1,3-thiazol-2-yl]-4-piperidinecarboxylic acid |
| **25** | 1-{4-[2-({[4'-cyano-3-(methyloxy)-4-biphenylyl]methyl}oxy)phenyl]-1,3-thiazol-2-yl}-4-piperidinecarboxylic acid |
| **26** | 1-{4-[2-({[3-chloro-4'-(trifluoromethyl)-4-biphenylyl]methyl}oxy)phenyl]-1,3-thiazol-2-yl}-4-piperidinecarboxylic acid |
| **27** | 1-{4-[2-({[3-chloro-4'-(methyloxy)-4-biphenylyl]methyl}oxy)phenyl]-1,3-thiazol-2-yl}-4-piperidinecarboxylic acid |
| **28** | 1-[4-(2-{[(3-chloro-4'-fluoro-4-biphenylyl)methyl]oxy}phenyl)-1,3-thiazol-2-yl]-4-piperidinecarboxylic acid |
| **29** | 1-{4-[2-({[3-(methyloxy)-4'-(trifluoromethyl)-4-biphenylyl]methyl}oxy)phenyl]-1,3-thiazol-2-yl}-4-piperidinecarboxylic acid |
| **30** | 1-[4-(2-{[(4'-cyano-4-biphenylyl)methyl]oxy}-3,5-difluorophenyl)-1,3-thiazol-2-yl]-4-piperidinecarboxylic acid |
| **31** | 1-(4-{2-[({4-[(4-cyanophenyl)oxy]phenyl}methyl)oxy]-3,5-difluorophenyl}-1,3-thiazol-2-yl)-4-piperidinecarboxylic acid |
| **32** | 1-{4-[3,5-difluoro-2-({[4'-(methyloxy)-4-biphenylyl]methyl}oxy)phenyl]-1,3-thiazol-2-yl}-4-piperidinecarboxylic acid |
| **33** | 1-(4-{2-[({2-methyl-4-[6-(methyloxy)-3-pyridinyl]phenyl}methyl)oxy]phenyl}-1,3-thiazol-2-yl)-4-piperidinecarbaxylic acid |
| **34** | 1-{4-[2-({[4'-(hydroxymethyl)-3-methyl-4-biphenylyl]methyl}oxy)phenyl]-1,3-thiazol-2-yl}-4-piperidinecarboxylic acid |
| **35** | 1-[4-(2-{[(4'-fluoro-3-methyl-4-biphenylyl)methyl]oxy}phenyl)-1,3-thiazol-2-yl]-4-piperidinecarboxylic acid |
| **37** | 1-[4-(2-{[(4'-cyano-3-methyl-4-biphenylyl)methyl]oxy}phenyl)-1,3-thiazol-2-yl]-4-piperidinecarboxylic acid |
| | |
| **Example 36** | 1-{4-[4-chloro-2-({[3-methyl-4'-(trifluoromethyl)-4-biphenylyl]methyl}oxy)phenyl]-1,3-thiazol-2-yl}-4-piperidinecarboxylic acid |

### Example 38: 1-[5-chloro-4-(2-{[(4'-cyano-4-biphenylyl)methyl]oxy}phenyl)-1,3-thiazol-2-yl]-4-piperidinecarboxylic acid

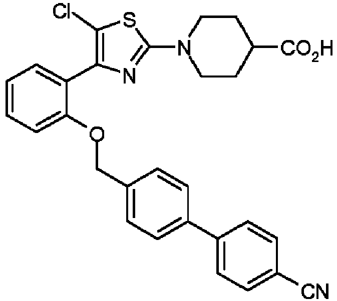

| **Example** | **Name** |
|---|---|
| **39** | 1-{5-chloro-4-[2-({[4-(2-phenylethyl)phenyl]methyl}oxy)phenyl]-1,3-thiazol-2-yl}-4-piperidinecarboxylic acid |
| **40** | 1-(5-chloro-4-{2-[({4-[(4-cyanophenyl)oxy]phenyl}methyl)oxy]phenyl}-1,3-thiazol-2-yl)-4-piperidinecarboxylic acid |

### Example 41: 1-{5-methyl-4-[2-({[4'-(methyloxy)-4-biphenylyl]methyl}oxy)phenyl]-1,3-thiazol-2-yl}-4-piperidinecarboxylic acid

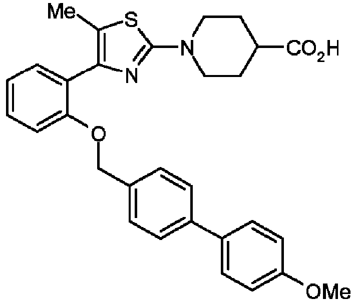

| | |
|---|---|
| **Example 42** | 1-{4-[3,5-difluoro-2-({[3-methyl-4'-(trifluoromethyl)-4-biphenylyl]methyl}oxy)phenyl]-1,3-thiazol-2-yl}-4-piperidinecarboxylic acid |
| | |

| **Example** | Name |
|---|---|
| **43** | 1-[4-(2-{[(4'-cyano-4-biphenylyl)methyl]oxy}phenyl)-5-methyl-1,3-thiazol-2-yl]-4-piperidinecarboxylic acid |
| **45** | 1-(4-{2-[({4-[(4-cyanophenyl)oxy]phenyl}methyl)oxy]phenyl}-5-methyl-1,3-thiazol-2-yl)-4-piperidinecarboxylic acid |
| | |
| **Example 44** | 1-{4-[2-fluoro-6-({[3-methyl-4'-(trifluoromethyl)-4-biphenylyl]methyl}oxy)phenyl]-1,3-thiazol-2-yl}-4-piperidinecarboxylic acid |
| **46** | 1-[4-(2-{[(4'-cyano-3-methy)-4-biphenylyl)methyl]oxy}-3,5-difluorophenyl)-1,3-thiazol-2-yl]-4-piperidinecarboxylic acid |
| **47** | 1-{4-[2-({[4'-(aminocarbonyl)-3-methyl-4-biphenylyl]methyl}oxy)-3,5-difluorophenyl]-1,3-thiazol-2-yl}-4-piperidinecarboxylic acid |
| **48** | 1-[4-(2-{[(4'-cyano-4-biphenylyl)methyl]oxy}-5-fluorophenyl)-1,3-thiazol-2-yl]-4-piperidinecarboxylic acid |
| **49** | 1-[4-(2-{[(4'-cyano-3-methyl-4-biphenylyl)methyl]oxy}-5-fluorophenyl)-1,3-thiazol-2-yl]-4-piperidinecarboxylic acid |
| **50** | 1-{4-[2-({[2',3-dimethyl-4'-(trifluoromethyl)-4-biphenylyl]methyl}oxy)-3,5-difluorophenyl]-1,3-thiazol-2-yl}-4-piperidinecarboxylic acid |
| **51** | 1-{4-[5-chloro-2-({[2',3-dimethyl-4'-(trifluoromethyl)-4-biphenylyl]methyl}oxy)phenyl]-1,3-thiazol-2-yl}-4-piperidinecarboxylic acid |
| **52** | 1-[4-(5-chloro-2-{[(4'-cyano-3-methyl-4-biphenylyl)methyl]oxy}phenyl)-1,3-thiazol-2-yl]-4-piperidinecarboxylic acid |
| **Example 53** | 1-[4-(2-{[(4'-cyano-2',3-dimethyl-4-biphenylyl)methyl]oxy}-3,5-difluorophenyl)-1,3-thiazol-2-yl]-4-piperidinecarboxylic acid |

### Example 54: 1-{4-[5-methyl-2-({[3-methyl-4'-(trifluoromethyl)-4-biphenylyl]methyl}oxy)phenyl]-1,3-thiazol-2-yl}-4-piperidinecarboxylic acid

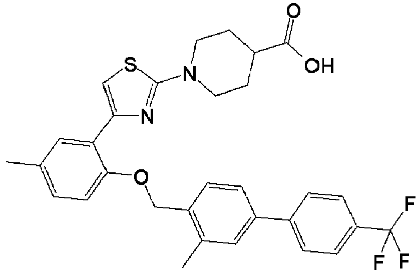

### Example 55: 1-{4-[5-methyl-2-({[3-methyl-4'-(trifluoromethyl)-4-biphenylyl]methyl}oxy)phenyl]-1,3-thiazol-2-yl}-4-piperidinecarboxylic acid-2-aminoethanol (1:1)

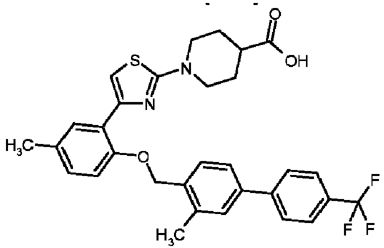

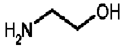

### Example 56: sodium 1-{4-[5-methyl-2-({[3-methyl-4'-(trifluoromethyl)-4-biphenylyl]methyl}oxy)phenyl]-1,3-thiazol-2-yl}-4-piperidinecarboxylate

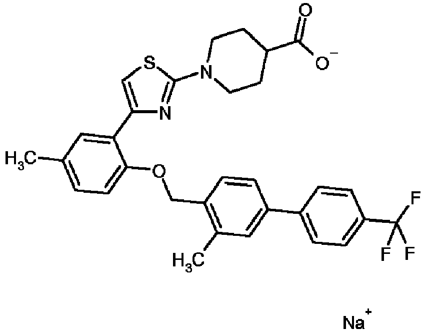

| | |
|---|---|
| **Example 57** | 1-[4-(2-{[(4'-cyano-3-methyl-4-biphenylyl)methyl]oxy}-5-methylphenyl)-1,3-thiazol-2-yl]-4-piperidinecarboxylic acid |
| | |
| **Example 58** | 1-[4-(2-{[(4'-cyano-3-methyl-4-biphenylyl)methyl]oxy}-5-methylphenyl)-1,3-thiazol-2-yl]-4-piperidinecarboxylic acid 2-aminoethanol (1:1) |
| | |
| **Example 59** | 1-(4-{2-{[(4'-cyano-3-methyl-4-biphenylyl)methyl]oxy}-5-[(trifluoromethyl)oxy]phenyl}-1,3-thiazol-2-yl)-4-piperidinecarboxylic acid |
| | |

| **Example** | **Name** |
|---|---|
| **60** | 1-(4-{5-methyl-2-[({3-methyl-4'-[(trifluoromethyl)oxy]-4-biphenylyl}methyl)oxy]phenyl}-1,3-thiazol-2-yl)-4-piperidinecarboxylic acid |
| **61** | 1-(4-{2-[({4'-[(dimethylamino)carbonyl]-3-methyl-4-biphenylyl}methyl)oxy]-5-methylphenyl}-1,3-thiazol-2-yl)-4-piperidinecarboxylic acid |
| | |
| **Example 62** | 1-(4-{5-chloro-2-[({3-methyl-4'-[(trifluoromethyl)oxy]-4-biphenylyl}methyl)oxy]phenyl}-1,3-thiazol-2-yl)-4-piperidinecarboxylic acid |
| | |
| **Example 63** | 1-{4-[2-{[(4'-cyano-3-methyl-4-biphenylyl)methyl]oxy}-5-(trifluoromethyl)phenyl]-1,3-thiazol-2-yl}-4-piperidinecarboxylic acid |
| **64** | 1-{4-[5-(methyloxy)-2-({[3-methyl-4'-(trifluoromethyl)-4-biphenylyl]methyl}oxy)phenyl]-1,3-thiazol-2-yl}-4-piperidinecarboxylic acid |
| **65** | 1-(4-{5-(methyloxy)-2-[({3-methyl-4'-[(trifluoromethyl)oxy]-4-biphenylyl}methyl)oxy]phenyl}-1,3-thiazol-2-yl)-4-piperidinecarboxylic acid |
| **66** | 1-{4-[2-{[(4'-cyano-3-methyl-4-biphenylyl)methyl]oxy}-5-(methyloxy)phenyl]-1,3-thiazol-2-yl}-4-piperidinecarboxylic acid |
| **67** | 1-{4-[2-{[(4'-cyano-3-methyl-4-biphenylyl)methyl]oxy}-5-(1-methylethyl)phenyl]-1,3-thiazol-2-yl}-4-piperidinecarboxylic acid |
| **68** | 1-[4-(2-{[(4'-cyano-3-methyl-4-biphenylyl)methyl]oxy}-5-ethylphenyl)-1,3-thiazol-2-yl]-4-piperidinecarboxylic acid |
| **69** | 1-{4-[5-(1-methylethyl)-2-({[3-methyl-4'-(trifluoromethyl)-4-biphenylyl]methyl}oxy)phenyl]-1,3-thiazol-2-yl}-4-piperidinecarboxylic acid |
| **70** | 1-{4-[2-[({4-[3-chloro-5-(trifluoromethyl)-2-pyridinyl]-2-methylphenyl}methyl)oxy]-3-(trifluoromethyl)phenyl]-1,3-thiazol-2-yl}-4-piperidinecarboxylic acid |
| **71** | 1-(4-[3-methyl-2-({[3-methyl-4'-(trifluoromethyl)-4-biphenylyl]methyl}oxy)phenyl]-1,3-thiazol-2-yl}-4-piperidinecarboxylic acid |
| | |
| **72** | 1-(4-{2-[({4-[3-chloro-5-(trifluoromethyl)-2-pyridinyl]-2-methylphenyl}methyl)oxy]-5-methylphenyl}-1,3-thiazol-2-yl)-4-piperidinecarboxylic acid |
| **73** | 1-(4-{2-[({4-[3-chloro-5-(trifluoromethyl)-2-pyridinyl]-2-methylphenyl}methyl)oxy]-5-ethylphenyl}-1,3-thiazol-2-yl)-4-piperidinecarboxylic acid |
| **74** | 1-{4-[5-ethyl-2-({[3-methyl-4'-(trifluoromethyl)-4-biphenylyl]methyl}oxy)phenyl]-1,3-thiazol-2-yl}-4-piperidinecarboxylic acid |
| **75** | 1-(4-{5-ethyl-2-[({3-methyl-4'-[(trifluoromethyl)oxy]-4-biphenylyl}methyl)oxy]phenyl}-1,3-thiazol-2-yl)-4-piperidinecarboxylic acid |
| **76** | 1-{4-[2-({[3-methyl-4'-(trifluoromethyl)-4-biphenylyl]methyl}oxy)-3-(trifluoromethyl)phenyl]-1,3-thiazol-2-yl}-4-piperidinecarboxylic acid |
| **77** | 1-{4-[2-{[(4'-cyano-3-methyl-4-biphenylyl)methyl]oxy}-3-(trifluoromethyl)phenyl]-1,3-thiazol-2-yl}-4-piperidinecarboxylic acid |
| **78** | 1-[4-(2-{[(4'-cyano-3-methyl-4-biphenylyl)methyl]oxy}-3-methylphenyl)-1,3-thiazol-2-yl]-4-piperidinecarboxylic acid |
| **79** | 1-{4-[2-[({4-[3-chloro-5-(trifluoromethyl)-2-pyridinyl]-2-methylphenyl}methyl)oxy]-5-(trifluoromethyl)phenyl]-1,3-thiazol-2-yl}-4-piperidinecarboxylic acid |
| **80** | 1-{4-[2-{[(4'-cyano-2',3-dimethyl-4-biphenylyl)methyl]oxy}-3-(trifluoromethyl)phenyl]-1,3-thiazol-2-yl}-4-piperidinecarboxylic acid |

### Example 81: GSK2236881A 1-{4-[2-({[4-(5-cyano-2-pyridinyl)-2-methylphenyl]methyl}oxy)-5-methylphenyl]-1,3-thiazol-2-yl}-4-piperidinecarboxylic acid (N7106-76-1)

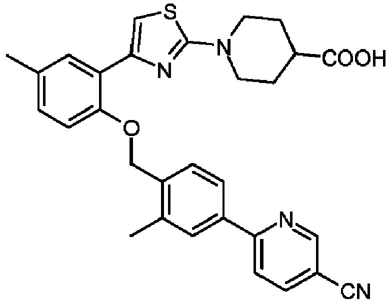

| **Example** | **Name** |
|---|---|
| **82** | 1-{4-[5-methyl-2-({[3-methyl-4'-(methyloxy)-2'-(trifluoromethyl)-4-biphenylyl]methyl}oxy)phenyl]-1,3-thiazol-2-yl}-4-piperidinecarboxylic acid |
| **83** | 1-{4-[5-ethyl-2-({[3-methyl-2',4'-bis(trifluoromethyl)-4-biphenylyl]methyl}oxy)phenyl]-1,3-thiazol-2-yl}-4-piperidinecarboxylic acid |
| **84** | 1-{4-[5-methyl-2-({[3-(propyloxy)-4'-(trifluoromethyl)-4-biphenylyl]methyl}oxy)phenyl]-1,3-thiazol-2-yl}-4-piperidinecarboxylic acid |
| **85** | 1-{4-[5-methyl-2-({[3-methyl-2',4'-bis(trifluoromethyl)-4-biphenylyl]methyl}oxy)phenyl]-1,3-thiazol-2-yl}-4-piperidinecarboxylic acid |
| **86** | 1-(4-[2-({[4-(5-cyano-2-pyridinyl)-2-methylphenyl]methyl}oxy)-5-(trifluoromethyl)phenyl]-1,3-thiazol-2-yl}4-piperidinecarboxylic acid |
| **87** | 1-{4-[2-({[2'-chloro-3-methyl-4'-(trifluoromethyl)-4-biphenylyl]methyl}oxy)-3-(trifluoromethyl)phenyl]-1,3-thiazol-2-yl}-4-piperidinecarboxylic acid |
| **88** | 1-{4-[2-({[4-(5-cyano-2-pyridinyl)-2-methylphenyl]methyl}oxy)-3,5-dim ethyl phenyl]-1,3-thiazol-2-yl}-4-piperidinecarboxylic acid |
| | |
| **89** | 1-[4-(2-{[(4'-cyano-3-methyl-4-biphenylyl)methyl]oxy}-3,5-dimethylphenyl)-1,3-thiazol-2-yl]-4-piperidinecarboxylic acid |
| | |
| **Example 90** | 1-{4-[4-methyl-2-({[3-methyl-4'-(trifluoromethyl)-4-biphenylyl]methyl}oxy)phenyl]-1,3-thiazol-2-yl}-4-piperidinecarboxylic acid |

### Compound Class VIII:

Examples 1 to 47 of compound class VIII are disclosed in WO2010/015653.

### Example 1: 1-(4-(5-methyl-2-(2-methyl-4-(4-cyano-2-methylphenyl)benzyloxy)-phenyl)pyrimidin-2-yl)-piperidine-4-carboxylic acid

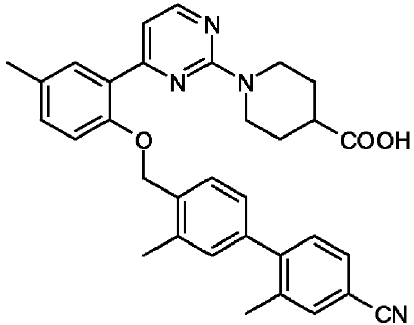

### Example 2: 1-(4-(5-methyl-2-(2-methyl-4-(4-methoxy-2-trifluoromethylphenyl)benzyloxy)-phenyl)pyrimidin-2-yl)-piperidine-4-carboxylic acid

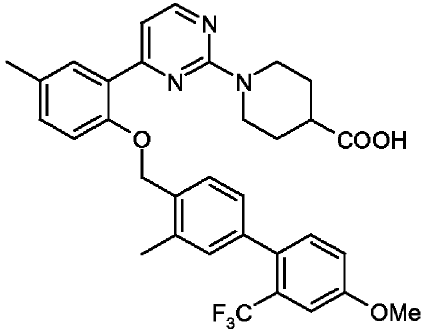

| **Example No.** | **Name** |
|---|---|
| 3 | 1-[4-(2-(4-(4-methoxyphenyl)phenylmethyloxy)-phenyl)pyrimidin-2-yl]-5-trifluoromethyl-pyrazole-4-carboxylic acid |
| 4 | 1-[4-(5-chloro-2-(4-(4-methoxyphenyl)phenylmethyloxy)-phenyl)pyrimidin-2-yl]-5-trifluoromethyl-pyrazole-4-carboxylic acid |
| 5 | 1-[4-(2-(4-(4-cyanophenyl)phenylmethyloxy)-phenyl)pyrimidin-2-yl]-5-trifluoromethyl-pyrazole-4-carboxylic acid |
| 6 | 1-[4-(2-(4-(4-trifluoromethylphenyl)phenylmethyloxy)-phenyl)pyrimidin-2-yl]-5-trifluoromethyl-pyrazole-4-carboxylic acid |
| 7 | 1-[4-(2-(2-methyl-4-(4-cyanophenyl)phenylmethyloxy)-phenyl)pyrimidin-2-yl]-5-trifluoromethyl-pyrazole-4-carboxylic acid |
| 8 | 1-[4-(2-(2-methyl-4-(4-methoxyphenyl)phenylmethyloxy)-phenyl)pyrimidin-2-yl]-5-trifluoromethyl-pyrazole-4-carboxylic acid |
| 9 | 1-[4-(2-(2-methyl-4-(4-trifluoromethylphenyl)phenylmethyloxy)-phenyl)pyrimidin-2-yl]-5-trifluoromethyl-pyrazole-4-carboxylic acid |
| 10 | 1-[4-(5-chloro-2-(2-methyl-4-(4-methoxyphenyl)phenylmethyloxy)-phenyl)pyrimidin-2-yl]-5-trifluoromethyl-pyrazole-4-carboxylic acid |
| 11 | 1-[4-(2-(4-(4-cyanophenyloxy)phenylmethyloxy)-phenyl)pyrimidin-2-yl]-5-trifluoromethyl-pyrazole-4-carboxylic acid |
| 12 | 1-[4-(5-chloro-2-(4-(4-cyanophenyloxy)phenylmethyloxy)-phenyl)pyrimidin-2-yl]-5-trifluoromethyl-pyrazole-4-carboxylic acid |
| 13 | 1-[4-(5-methyl-2-(4-(3-chloro-5-trifluoromethylpyridin-2-yloxy)phenylmethyloxy)phenyl)pyrimidin-2-yl]-5-trifluoromethyl-pyrazole-4-carboxylic acid |
| 14 | 1-[4-(5-methyl-2-(2-methoxy-4-(3-chloro-5-trifluoromethylpyridin-2-yloxy)phenylmethyloxy)phenyl)pyrimidin-2-yl]-5-trifluoromethyl-pyrazole-4-carboxylic acid |
| 15 | 1-[4-(5-methyl-2-(2-methyl-4-(3-chloro-5-trifluoromethylpyridin-2-yloxy)phenylmethyloxy)phenyl)pyrimidin-2-yl]-5-trifluoromethyl-pyrazole-4-carboxylic acid |
| 16 | 1-[4-(2-(2-methyl-4-(4-methoxyphenyl)phenylmethyloxy)-phenyl)pyrimidin-2-yl]-piperidine-4-carboxylic acid |
| 17 | 1-[4-(2-(2-methyl-4-(2,4-dimethoxy-phenyl)phenylmethyloxy)-phenyl)pyrimidin-2-yl]-piperidine-4-carboxylic acid |
| 18 | 1-[4-(2-(2-methyl-4-(4-methoxy-2-methyl-phenyl)phenylmethyloxy)-phenyl)pyrimidin-2-yl]-piperidine-4-carboxylic acid |
| 19 | 1-[4-(2-(2-methyl-4-(4-trifluoromethylphenyl)phenylmethyloxy)-phenyl)pyrimidin-2-yl]-piperidine-4-carboxylic acid |
| 20 | 1-[4-(2-(2-methoxy-4-(4-trifluoromethylphenyl)phenylmethyloxy)-phenyl)pyrimidin-2-yl]-piperidine-4-carboxylic acid |
| 21 | 1-[4-(2-(2-methyl-4-(4-cyanophenyl)phenylmethyloxy)-phenyl)pyrimidin-2-yl]-piperidine-4-carboxylic acid |
| 22 | 1-[4-(5-fluoro-2-(2-methyl-4-(4-methoxyphenyl)phenylmethyloxy)-phenyl)pvrimidin-2-yl]-piperidine-4-carboxylic acid |
| 23 | 1-[4-(5-fluoro-2-(2-methyl-4-(4-fluorophenyl)phenylmethyloxy)-phenyl)pyrimidin-2-yl]-piperidine-4-carboxylic acid |
| 24 | 1-[4-(5-fluoro-2-(2-methyl-4-(4-cyanophenyl)phenylmethyloxy)-phenyl)pyrimidin-2-yl]-piperidine-4-carboxylic acid |
| 25 | 1-[4-(5-fluoro-2-(4-(4-cyanophenyloxy)phenylmethyloxy)-phenyl)pyrimidin-2-yl]-piperidine-4-carboxylic acid |
| 26 | 1-[4-(5-fluoro-2-(4-(5-trifluoromethylpyridin-2-yloxy)phenylmethyloxy)-phenyl)pyrimidin-2-yl]-piperidine-4-carboxylic acid |
| 27 | 1-[4-(5-methyl-2-(2-methyl-4-(4-trifluoromethoxyphenyl)phenylmethyloxy)-phenyl)pyrimidin-2-yl]-piperidine-4-carboxylic acid |
| 28 | 1-[4-(5-methyl-2-(2-methyl-4-(4-methoxyphenyl)phenylmethyloxy)-phenyl)pyrimid-in-2-yl]-piperidine-4-carboxylic acid |
| 29 | 1-[4-(5-methyl-2-(2-methyl-4-(4-cyanophenyl)phenylmethyloxy)-phenyl)pyrimidin-2-yl]-piperidine-4-carboxylic acid |
| 30 | 1-[4-(5-methyl-2-(2-methyl-4-(2-methyl-4-trifluoromethylphenyl)phenylmethyloxy)-phenyl)pyrimidin-2-yl]-piperidine-4-carboxylic acid |
| 31 | 1-[4-(5-methyl-2-(2-methyl-4-(2-ch loro-4-methoxyphenyl)phenylmethyloxy)-phenyl)pyrimidin-2-yl]-piperidine-4-carboxylic acid |
| 32 | 1-[4-(5-methyl-2-(2-propyloxy-4-(4-trifluoromethylphenyl)phenylmethyloxy)-phenyl)pyrimidin-2-yl]-piperidine-4-carboxylic acid |
| 33 | 1-[4-(5-methyl-2-(2-propyloxy-4-(4-cyanophenyl)phenylmethyloxy)-phenyl)pyrimidin-2-yl]-piperidine-4-carboxylic acid |
| 34 | 1-[4-(5-methyl-2-(2-propyloxy-4-(2-chloro-4-methoxyphenyl)phenylmethyloxy)-phenyl)pyrimidin-2-yl]-piperidine-4-carboxylic acid |
| 35 | 1-[4-(5-methyl-2-(2-propyloxy-4-(4-methoxy-2-trifluoromethylphenyl)phenylmethyloxy)-phenyl)pyrimidin-2-yl]-piperidine-4-carboxylic acid |
| 36 | 1-[4-(5-methyl-2-(2-propyloxy-4-(4-chloro-2-trifluoromethylphenyl)phenylmethyloxy)-phenyl)pyrimidin-2-yl]-piperidine-4-carboxylic acid |
| 37 | 1-[4-(5-methyl-2-(2-methyl-4-(6-methoxypyridin-3-yl)phenylmethyloxy)-phenyl)pyrimidin-2-yl]-piperidine-4-carboxylic acid |
| 38 | 1-[4-(5-methyl-2-(2-methyl-4-(5-trifluoromethylpyridin-2-yl)phenylmethyloxy)-phenyl)pyrimidin-2-yl]-piperidine-4-carboxylic acid |
| 39 | 1-[4-(5-methyl-2-(2-methyl-4-(3-ch loro-5-trifluoromethylpyrid in-2-yl)phenylmethyloxy)-phenyl)pyrimidin-2-yl]-piperidine-4-carboxylic acid |
| 40 | 1-[4-(5-methyl-2-(2-methyl-4-(3-ch loro-5-trifluoromethylpyrid in-2-yloxy)phenylmethyloxy)-phenyl)pyrimidin-2-yl]-piperidine-4-carboxylic acid |
| 41 | 1-[4-(5-methyl-2-(2-methyl-4-(2-ch loro-4-cyanophenoxy)phenylmethyloxy)-phenyl)pyrimidin-2-yl]-piperidine-4-carboxylic acid |
| 42 | 1-[4-(5-trifluoromethyl-2-(2-methyl-4-(4-ch loro-2-trifluoromethylphenyl)phenylmethyloxy)-phenyl)pyrimidin-2-yl]-piperidine-4-carboxylic acid |
| 43 | 1-[4-(5-trifluoromethyl-2-(2-methyl-4-(3-chloro-5-trifluoromethylpyridin-2-yl)phenylmethyloxy)-phenyl)pyrimidin-2-yl]-piperidine-4-carboxylic acid |
| 44 | 1-[2-(2-(4-(4-cyanophenyl)phenylmethyloxy)-phenyl)pyrimidin-4-yl]-5-trifluoromethyl-pyrazole-4-carboxylic acid |
| 45 | 1-[2-(2-(4-(4-trifluoromethylphenyl)phenylmethyloxy)-phenyl)pyrimidin-4-yl]-5-trifluoromethyl-pyrazole-4-carboxylic acid |
| 46 | 1-[2-(2-(4-(4-methoxyphenyl)phenylmethyloxy)-phenyl)pyrimidin-4-yl]-5-trifluoromethyl-pyrazole-4-carboxylic acid |

### Example 47: 1-(4-(5-trifluoromethyl-2-(2-methyl-4-(4-cyanophenyl)benzyloxy)-phenyl)pyrimidin-2-yl)-piperidine-4-carboxylic acid

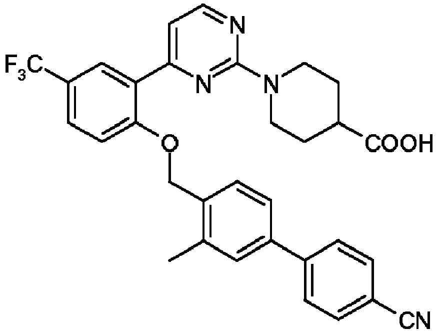

### Compound class IX:

Examples 1 to 63 of compound class IX are disclosed in WO 2011/115804.

### Example 1

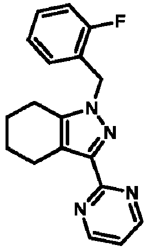

### Example 2

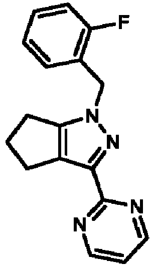

### Example 3

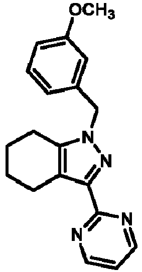

### Example 4

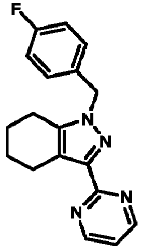

### Example 5

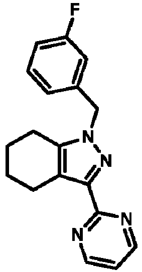

### Example 6

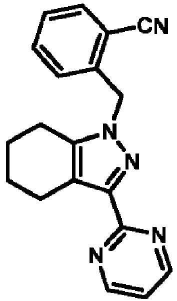

### Example 7

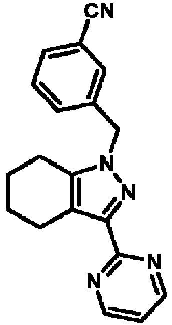

### Example 8

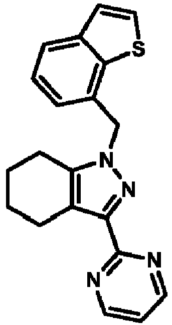

### Example 9

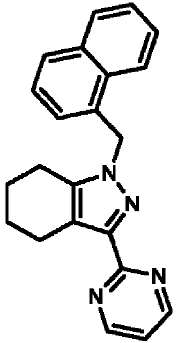

### Example 10

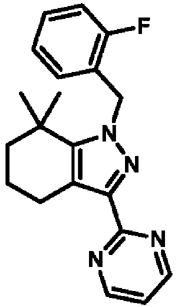

### Example 11

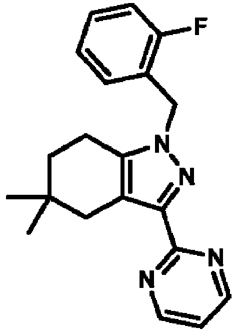

### Example 12

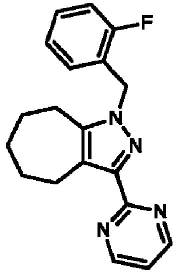

### Example 13

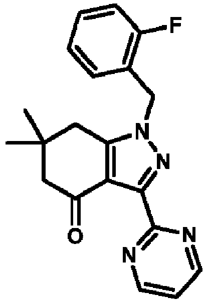

### Example 14

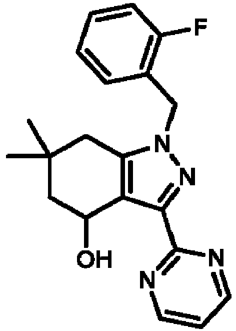

### Example 15

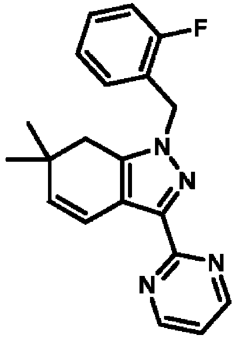

### Example 16

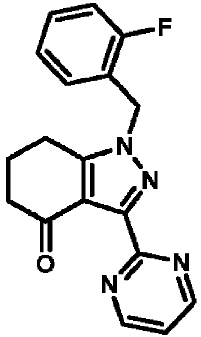

### Example 17

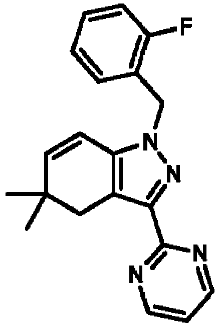

### Example 18

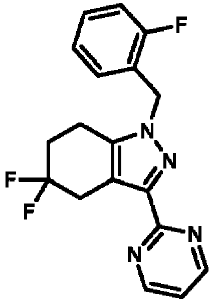

### Example 19

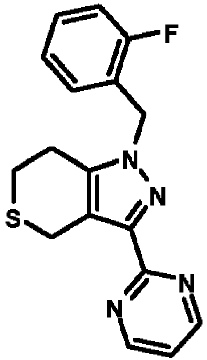

### Example 20

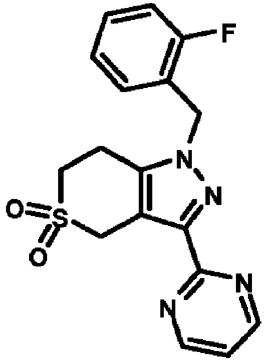

### Example 21

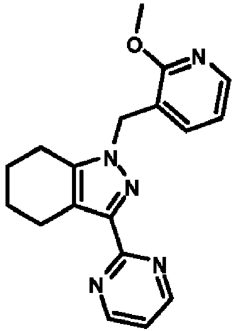

### Example 22

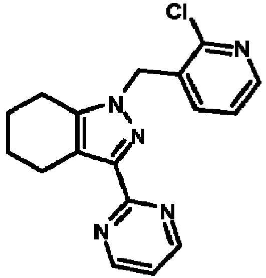

### Example 23

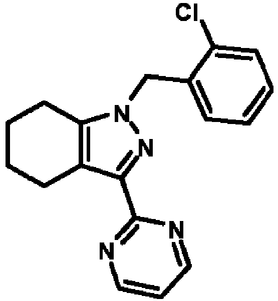

### Example 24

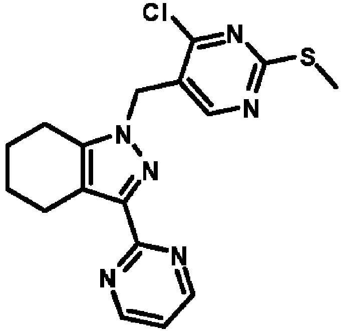

### Example 25

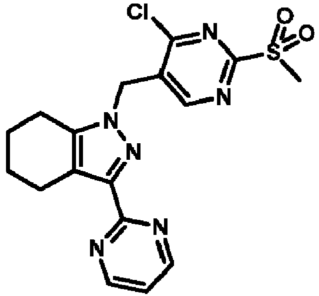

### Example 26

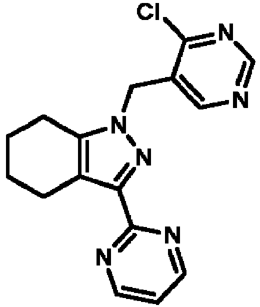

### Example 27

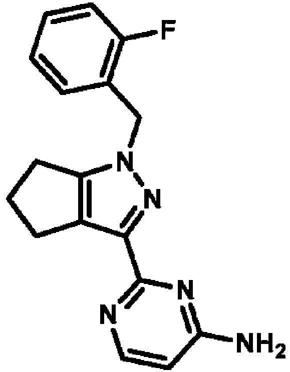

### Example 28

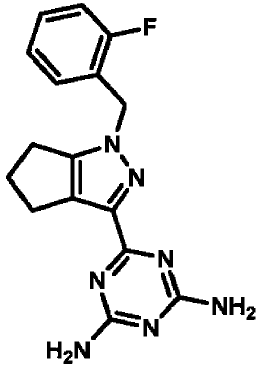

### Example 29

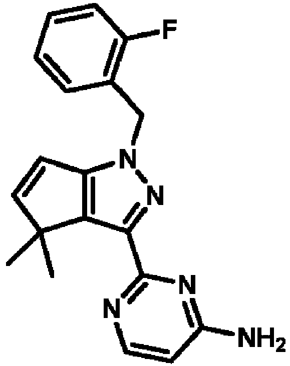

### Example 30

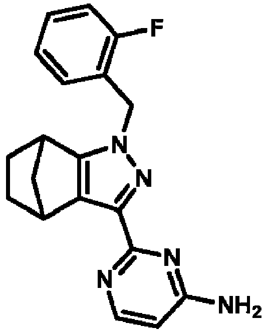

### Example 31

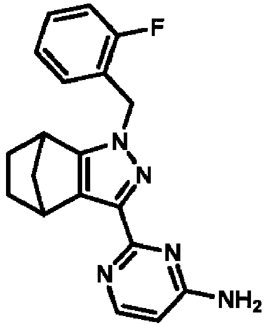

### Example 32

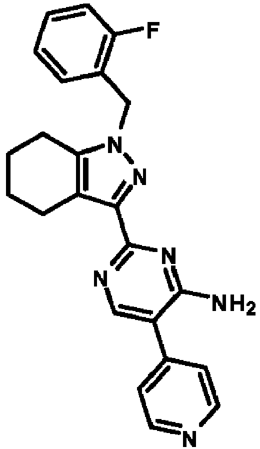

### Example 33

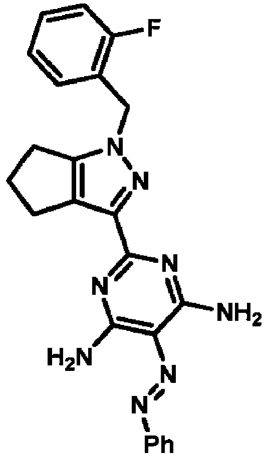

### Example 34

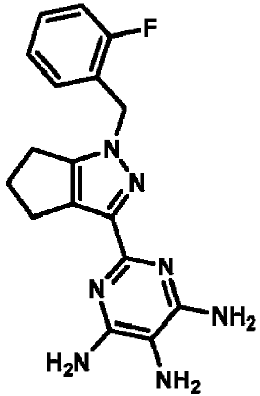

### Example 35

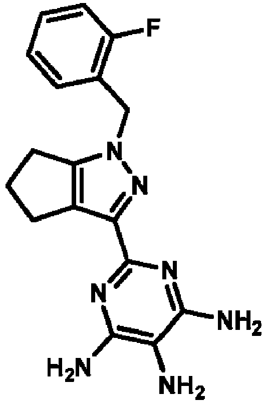

### Example 36

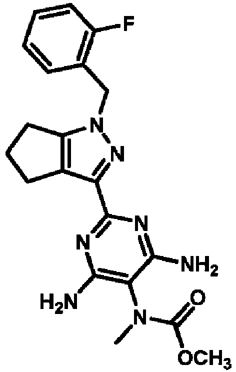

### Example 37

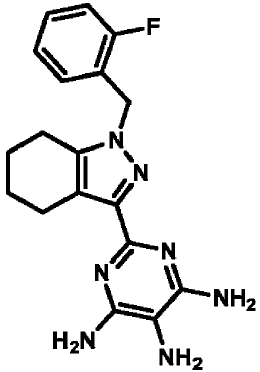

### Example 38

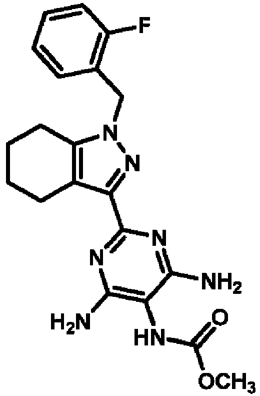

### Example 39

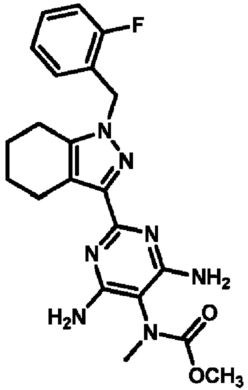

### Example 40

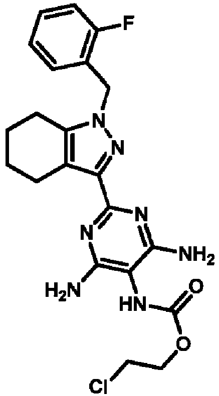

### Example 41

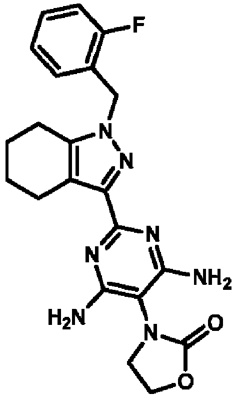

### Example 42

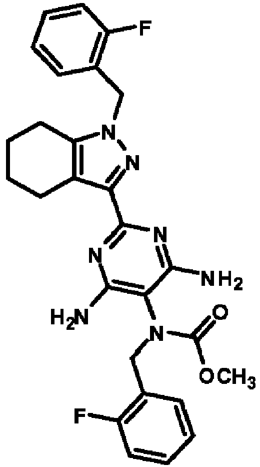

### Example 43

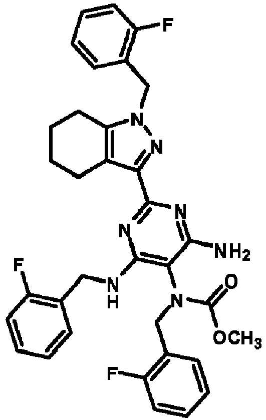

### Example 44

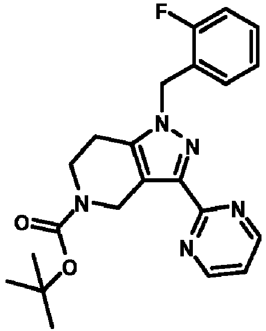

### Example 45

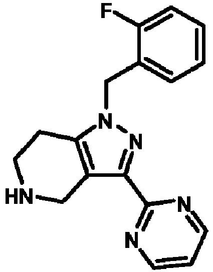

### Example 46

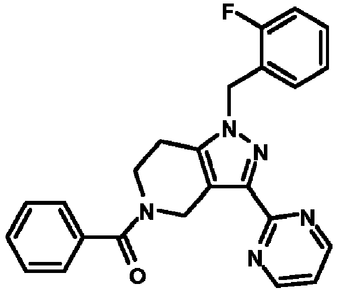

### Example 47

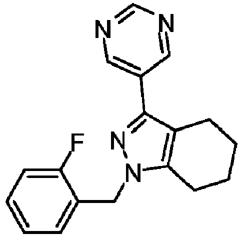

### Example 48

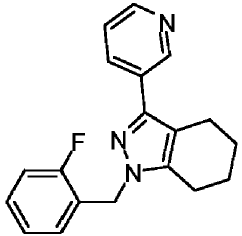

### Example 49

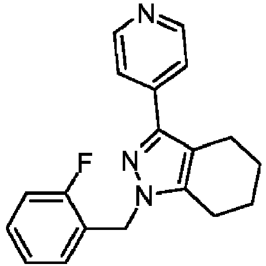

### Example 50

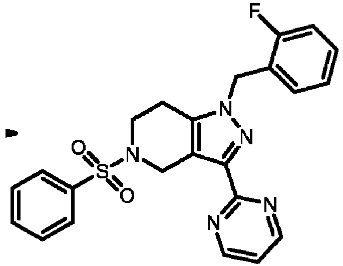

### Example 51

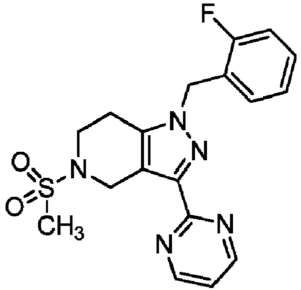

### Example 52

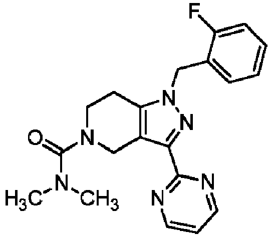

### Example 53

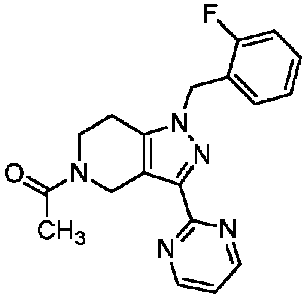

### Example 54

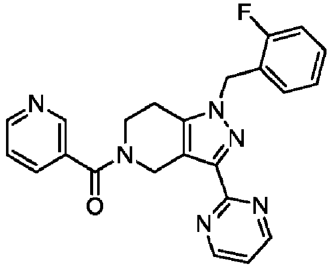

### Example 55

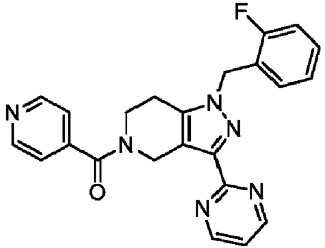

### Example 56

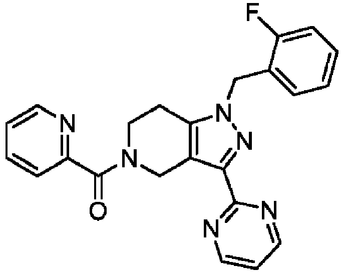

### Example 57

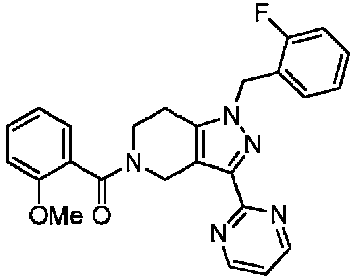

### Example 58

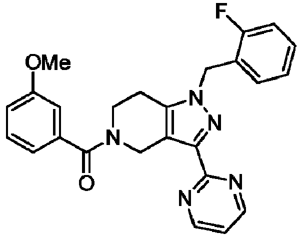

### Example 59

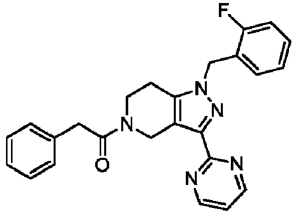

### Example 60

### 1-(2-fluorobenzyl)-5-phenyl-3-(pyrimidin-5-yl)-4,5,6,7-tetrahydro-1H-pyrazolo[4,3-c]pyridine

### Example 61

### 1-(2-fluorobenzyl)-5-(pyridine-3-yl)-3-(pyrimidin-5-yl)-4,5,6,7-tetrahydro-1H-pyrazolo[4,3-c]pyridine

### Example 62

### 1-(2-fluorobenzyl)-5-(pyridine-4-yl)-3-(pyrimidin-5-yl)-4,5,6,7-tetrahydro-1H-pyrazolo[4,3-c]pyridine

### Example 63

### 1-(2-fluorobenzyl)-5-(pyridine-2-yl)-3-(pyrimidin-5-yl)-4,5,6,7-tetrahydro-1H-pyrazolo[4,3-c]pyridine

### Compound class X:

Examples 1 to 227 of compound class X are disclosed in WO 2011/119518.

| EXAMPLE | IUPAC NAME |
|---|---|
| 2 | 2-[7-(2,3,6-trifluorobenzyl)imidazo[1,5-b]pyridazin-5-yl]-5-(1,3,5-trimethyl-1H-pyrazol-4-yl)pyrimidin-4-amine |
| 3 | 5-[1,5-dimethyl-3-(trifluoromethyl)-1*H*-pyrazol-4-yl]-2-[7-(2,3,6-trifluorobenzyl)imidazo[1,5-*b*]pyridazin-5-yl]pyrimidin-4-amine |
| 4 | 5-(2-methylpyridin-4-yl)-2-[7-(2,3,6-trifluorobenzyl)imidazo[1,5-*b*]pyridazin-5-yl]pyrimidin-4-amine |
| 5 | methyl {4,6-diamino-2-[7-(2,3,6-trifluorobenzyl)imidazo[1,5-*b*]pyridazin-5-yl]pyrimidin-5-yl}carbamate |
| 6 | methyl {4,6-diamino-2-[7-(2,3,6-trifluorobenzyl)imidazo[1,5-*b*]pyridazin-5-yl]pyrimidin-5-yl}methylcarbamate |
| 7 | 5-quinolin-5-yl-2-[7-(2,3,6-trifluorobenzyl)imidazo[1,5*-b*]pyridazin-5-yl]pyrimidin-4-amine |
| 8 | 5-pyridin-4-yl-2-[7-(2,3,6-trifluorobenzyl)imidazo[1,5-*b*]pyridazin-5-yl]pyrimidin-4-amine |
| 9 | 5-pyridin-3-yl-2-[7-(2,3,6-trifluorobenzyl)imidazo[1,5-*b*]pyridazin-5-yl]pyrimidin-4-amine |
| 10 | 2-[7-(2,3,6-trifluorobenzyl)imidazo[1,5-*b*]pyridazin-5-yl]-5-[5-(trifluoromethyl)pyridin-3-yl]pyrimidin-4-amine |
| 11 | 5-(5-fluoropyridin-3-yl)-2-[7-(2,3,6-trifluorobenzyl)imidazo[1,5-*b*]pyridazin-5-yl]pyrimidin-4-amine |
| 12 | 5-(2-methoxypyridin-3-yl)-2-[7-(2,3,6-tri-fluorobenzyl)imidazo[1,5-*b*]pyridazin-5-yl]pyrimidin-4-amine |
| 13 | 5-(4-methylpyridin-3-yl)-2-[7-(2,3,6-trifluorobenzyl)imidazo[1,5-*b*]pyridazin-5-yl]pyrimidin-4-amine |
| 14 | 5-(4-methoxypyridin-3-yl)-2-[7-(2,3,6-trifluorobenzyl)imidazo[1,5-*b*]pyridazin-5-yl]pyrimidin-4-amine |
| 15 | 5-(1-methyl-1*H*-pyrazol-4-yl)-2-[7-(2,3,6-trifluorobenzyl)imidazo[1,5-*b*]pyridazin-5-yl]pyrimidin-4-amine |
| 16 | 5-(1-methyl-1*H*-pyrazol-5-yl)-2-[7-(2,3,6-trifluorobenzyl)imidazo[1,5-*b*]pyridazin-5-yl]pyrimidin-4-amine |
| 17 | 5-(7-fluoroquinolin-4-yl)-2-[7-(2,3,6-trifluorobenzyl)imidazo[1,5-*b*]pyridazin-5-yl]pyrimidin-4-amine |
| 18 | 2-[7-(2,3,6-trifluorobenzyl)imidazo[1,5-*b*]pyridazin-5-yl]-5-[2-(trifluoromethyl)quinolin-4-yl]pyrimidin-4-amine |
| 19 | 5-(3-fluoropyridin-4-yl)-2-[7-(2,3.6-trifluorobenzyl)imidazo[1,5-*b*]pyridazin-5-yl]pyrimidin-4-amine |
| 20 | 2-[7-(2,3,6-trifluorobenzyl)imidazo[1,5-*b*]pyridazin-5-yl]-5-[2-(trifluoromethyl)pyridin-4-yl]pyrimidin-4-amine |
| 21 | 2-[7-(2,3,6-trifluorobenzyl)imidazo[1,5-*b*]pyridazin-5-yl]-5-[2-(trifluoromethyl)quinolin-4-yl]pyrimidin-4-amine |
| 22 | 5-[1-methyl-3-(trifluoromethyl)-1*H*-pyrazol-4-yl]-2-[7-(2,3,6-trifluorobenzyl)imidazo[1,5-*b*]pyridazin-5-yl]pyrimidin-4-amine |
| 23 | 5-pyridin.4-yl-2-[7-(2,3,5-trifluorobenzyl)imidazo[1,5*-b*]pyridazin-5-yl]pyrimidin-4-amine |
| 24 | 5-pyridin-3-yl-2-[7-(2,3,5-trifluorobenzyl)imidazo[1,5-*b*]pyridazin-5-yl]pyrimidin-4-amine |
| 25 | 5-(4-methoxypyridin-3-yl)-2-[7-(2,3,5-trifluorobenzyl)imidazo[1,5-*b*]pyridazin-5-yl]pyrimidin-4-amine |
| 26 | 5-(4-methylpyridin-3-yl)-2-[7-(2,3,5-trifluorobenzyl)imidazo[1,5-*b*]pyridazin-5-yl]pyrimidin-4-amine |
| 27 | 5-quinolin-5-yl-2-[7-(2,3,5-trifluorobenzyl)imidazo[1,5-*b*]pyridazin-5-yl]pyrimidin-4-amine |
| 28 | 5-(2-methylpyridin-4-yl)-2-[7-(2,3,5-trifluorobenzyl)imidazo[1,5-*b*]pyridazin-5-yl]pyrimidin-4-amine |
| 29 | 2-[7-(2-chloro-3,6-difluorobenzyl)imidazo[1,5-*b*]pyridazin-5-yl]-5-pyridin-4-ylpyrimidin-4-amine |
| 30 | 2-[7-(2-chloro-3,6-difluorobenzyl)imidazo[1,5-*b*)pyridazin-5-yl]-5-(1,3,5-trimethyl-1*H*-pyrazol-4-yl)pyrimidin-4-amine |
| 31 | 2-[7-(2-chloro-3,6-difluorobenzyl)imidazo[1,5-*b*]pyridazin-5-yl]-5-(2-methylpyridin-4-yl)pyrimidin-4-amine |
| 32 | 2-[7-(2-chloro-3,6-difluorobenzyl)imidazo[1,5-*b*]pyridazin-5-yl]-5-quinolin-5-ylpyrimidin-4-amine |
| 33 | 2-[7-(2-chloro-3,6-difluorobenzyl)imidazo[1,5-*b*]pyridazin-5-yl]-5-[2-(trifluoromethyl)pyridin-4-yl]pyrimidin-4-amine |
| 34 | 2-[7-(2-chloro-3,6-difluorobenzyl)imidazo[1,5*-b*]pyridazin-5-yl]-5-pyridin-3-ylpyrimidin-4-amine |
| 35 | 2-[7-(2-chloro-3,6-difluorobenzyl)imidazo[1,5-*b*]pyridazin-5-yl]-5-(4-methoxypyridin-3-yl)pyrimidin-4-amine |
| 36 | 2-[7-(2-chloro-3,6-difluorobenzyl)imidazo[1,5-*b*]pyridazin-5-yl]-5-[5-(trifluoromethyl)pyridin-3-yl]pyrimidin-4-amine |
| 37 | 2-[7-(2-chloro-3,6-difluorobenzyl)imidazo[1,5-*b*]pyridazin-5-yl]-5-(4-methylpyridin-3-yl)pyrimidin-4-amine |
| 38 | 2-[7-(2-chloro-3,6-difluorobenzyl)imidazo[1,5-*b*]pyridazin-5-yl]-5-(1-methyl-1*H*-pyrazol-4-yl)pyrimidin-4-amine |
| 39 | 2-[7-(2-chloro-3,6-difluorobenzyl)imidazo[1,5-*b*]pyridazin-5-yl]-5-[1,5-dimethyl-3-(trifluoromethyl)-1*H*-pyrazol-4-yl]pyrimidin-4-amine |
| 40 | 2-[7-(2,3,5-trifluorobenzyl)imidazo[1,5-*b*]pyridazin-5-yl]-5-(1,3,5-trimethyl-1*H*-pyrazol-4-yl)pyrimidin-4-amine |
| 41 | 5-[1,5-dimethyl-3-(trifluoromethyl)-1*H*-pyrazol-4-yl]-2-[7-(2,3,5-trifluorobenzyl)imidazo[1,5-*b*]pyridazin-5-yl]pyrimidin-4-amine |
| 42 | 2-[7-(3-chloro-2,6-difluorobenzyl)imidazo[1,5-*b*]pyridazin-5-yl]-5-(1,3,5-trimethyl-1*H*-pyrazol-4-yl)pyrimidin-4-amine |
| 43 | 2-[7-(2,3,5-trifluorobenzyl)imidazo[1,5-*b*]pyridazin-5-yl]-5-[2-(trifluoromethyl)pyridin-4-yl]pyrimidin-4-amine |
| 44 | 2-[7-(2,3,5-trifluorobenzyl)imidazo[1,5*-b*]pyridazin-5-yl]-5-[5-(trifluoromethyl)pyridin-3-yl]pyrimidin-4-amine |
| 45 | 5-[3-(methylsulfonyl)phenyl]-2-[7-(2,3,5-trifluorobenzyl)imidazo[1,5-*b*]pyridazin-5-yl]pyrimidin-4-amine |
| 46 | 2-[7-(2-chloro-3,5-difluorobenzyl)imidazo[1,5*-b*]pyridazin-5-yl]-5-[3-(methylsulfonyl)phenyl]pyrimidin-4-amine |
| 47 | 5-[2-(methylsulfonyl)phenyl]-2-[7-(2,3,5-trifluorobenzyl)imidazo[1,5-*b*]pyridazin-5-yl]pyrimidin-4-amine |
| 48 | 5-[4-(methylsulfonyl)phenyl]-2-[7-(2,3,5-trifluorobenzyl)imidazo[1,5-*b*]pyridazin-5-yl]pyrimidin-4-amine |
| 49 | 5-(3-methoxyphenyl)-2-[7-(2,3,5-trifluorobenzyl)imidazo[1,5-*b*]pyridazin-5-yl]pyrimidin-4-amine |
| 50 | -(2-methoxypyridin-3-yl)-2-[7-(2,3,4-trifluorobenzyl)imidazo[1,5-*b*]pyridazin-5-yl]pyrimidin-4-amine |
| 51 | 5-(4-methoxypyridin-3-yl)-2-[7-(2,3,4-trifluorobenzyl)imidazo[1,5-*b*]pyridazin-5-yl]pyrimidin-4-amine |
| 52 | 2-[7-(2,3,4-trifluorobenzyl)imidazo[1,5*-b*]pyridazin-5-yl]-5-[5-(trifluoromethyl)pyridin-3-yl]pyrimidin-4-amine |
| 53 | 5-pyridin-4-yl-2-[7-(2,3,4-trifluorobenzyl)imidazo[1,5-*b*]pyridazin-5-yl]pyrimidin-4-amine |
| 54 | 5-(1-methyl-1*H*-pyrazol-5-yl)-2-[7-(2,3,6-trifluorobenzyl)imidazo[1,5-*b*]pyridazin-5-yl]pyrimidin-4-amine |
| 55 | 5-[3-(methylsulfonyl)phenyl]-2-[7-(2,3,4-trifluorobenzyl)imidazo[1,5-*b*]pyridazin-5-yl]pyrimidin-4-amine |
| 56 | 2-[6-fluoro-3-(2,3,6-trifluorobenzyl)imidazo[1,5-*a*]pyridin-1-yl]-5-pyridin-3-ylpyrimidin-4-amine |
| 57 | 2-[6-fluoro-3-(2,3,6-trifluorobenzyl)imidazo[1,5-*a*]pyridin-1-yl]-5-quinolin-5-ylpyrimidin-4-amine |
| 58 | 2-[6-fluoro-3-(2,3,6-trifluorobenzyl)imidazo[1,5-*a*]pyridin-1-yl]-5-pyridin-4-ylpyrimidin-4-amine |
| 59 | 2-[6-fluoro-3-(2,3,6-trifluorobenzyl)imidazo[1,5-*a*]pyridin-1-yl]-5-(1,3,5-trimethyl-1*H*-pyrazol-4-yl)pyrimidin-4-amine |
| 60 | 2-[6-fluoro-3-(2,3,6-trifluorobenzyl)imidazo[1,5-*a*]pyridin-1-yl]-5-[5-(trifluoromethyl)pyridin-3-yl]pyrimidin-4-amine |
| 61 | 5-(5-fluoropyridin-3-yl)-2-[6-fluoro-3-(2,3,6-trifluorobenzyl)imidazo[1,5-*a*]pyndin-1-yl]pyrimidin-4-amine |
| 62 | 2-[6-fluoro-3-(2,3,6-trifluorobenzyl)imidazo[1,5-*a*]pyridin-1-yl]-5-(2-methoxypyridin-3-yl)pyrimidin-4-amine |
| 63 | 2-[6-fluoro-3-(2,3,6-trifluorobenzyl)imidazo[1,5-*a*]pyridin-1-yl]-5-(4-methylpyridin-3-yl)pyrimidin-4-amine |
| 64 | 2-[6-fluoro-3-(2,3,6-trifluorobenzyl)imidazo[1,5-*a*]pyridin-1-yl]-5-(2-methylpyridin-4-yl)pyrimidin-4-amine |
| 65 | 2-[6-fluoro-3-(2,3,6-trifluorobenzyl)imidazo[1,5-*a*]pyridin-1-yl]-5-(4-methoxypyridin-3-yl)pyrimidin-4-amine |
| 66 | 2-[6-fluoro-3-(2,3,6-trifluorobenzyl)imidazo[1,5-*a*]pyridin-1-yl]-5,5'-bipyrimidin-4-amine |
| 67 | 2-[6-fluoro-3-(2,3,6-trifluorobenzyl)imidazo[1,5-*a*]pyridin-1-yl]-5-(1-methyl-1*H*-pyrazol-4-yl)pyrimidin-4-amine |
| 68 | 2-[6-fluoro-3-(2,3,6-trifluorobenzyl)imidazo[1,5-*a*]pyridin-1-yl]-5-(1-methyl-1*H*-pyrazol-5-yl)pyrimidin-4-amine |
| 69 | 5-[1,5-dimethyl-3-(trifluoromethyl)-1*H*-pyrazol-4-yl]-2-[6-fluoro-3-(2,3,6-trifluorobenzyl)imidazo[1,5-*a*]pyridin-1-yl]pyrimidin-4-amine |
| 70 | -2-[6-fluoro-3-(2,3,6-trifluorobenzyl)imidazo[1,5-*a*]pyridin-1-yl]-5-[2-(trifluoromethyl)pyridin-4-yl]pyrimidin-4-amine |
| 71 | 5-(3-fluoropyridin-4-yl)-2-[6-fluoro-3-(2,3,6-trifluorobenzyl)imidazo[1,5*-a*]pyridin-1-yl]pyrimidin-4-amine |
| 72 | 2-[6-fluoro-3-(2,3,6-trifluorobenzyl)imidazo[1,5-*a*]pyridin-1-yl]-5-[2-(trifluoromethyl)quinolin-4-yl]pyrimidin-4-amine |
| 73 | 5-(6-fluoroquinolin-4-yl)-2-[6-fluoro-3-(2,3,6-trifluorobenzyl)imidazo[1,5-*a*]pyridin-1-yl]pyrimidin-4-amine |
| 74 | 2-[6-fluoro-3-(2,3,6-trifluorobenzyl)imidazo[1,5-*a*]pyridin-1-yl]-5-quinolin-4-ylpyrimidin-4-amine |
| 75 | 2-[6-fluoro-3-(2,3,6-trifluorobenzyl)imidazo[1,5-*a*]pyridin-1-yl]-5-[1-methyl-3-(trifluoromethyl)-1*H*-pyrazol-4-yl]pyrimidin-4-amine |
| 76 | 5-[5-(methoxymethyl)-1,3-dimethyl-1*H*-pyrazol-4-yl]-2-[7-(2,3,6-trifluorobenzyl)imidazo[1,5-*b*]pyridazin-5-yl]pyrimidin-4-amine |
| 77 | 2-[6-fluoro-3-(2,3,6-trifluorobenzyl)imidazo[1,5-*a*]pyridin-1-yl]-5-(1-methyl-1*H*-pyrrolo[2,3*-b*]pyridin-4-yl)pyrimidin-4-amine |
| 78 | 2-[6-fluoro-3-(2,3,6-trifluorobenzyl)imidazo[1,5-*a*]pyridin-1-yl]-5-(3-methylpyridin-4-yl)pyrimidin-4-amine |
| 79 | 2-[6-fluoro-3-(2,3,6-trifluorobenzyl)imidazo[1,5-*a*]pyridin-1-yl]-5-(2-methoxypyridin-4-yl)pyrimidin-4-amine |
| 80 | 5-(3,5-dimethylisoxazol-4-yl)-2-[6-fluoro-3-(2,3,6-trifluorobenzyl)imidazo[1,5-*a*]pyridin-1-yl]pyrimidin-4-amine |
| 81 | 2-[6-fluoro-3-(2,3,6-trifluorobenzyl)imidazo[1,5-*a*]pyridin-1-yl]-5-(1-methyl-1*H*-imidazol-5-yl)pyrimidin-4-amine |
| 82 | 2-[6-fluoro-3-(2,3,6-trifluorobenzyl)imidazo[1,5-*a*]pyridin-1-yl]-5-[3-(methylsulfonyl)phenyl]pyrimidin-4-amine |
| 83 | 2-[5-chloro-3-(2,3,6-trifluorobenzyl)-1*H*-indazol-1-yl]-5-(1-methyl-1*H-*pyrazol-4-yl)pyrimidine-4,6-diamine |
| 84 | 2-[5-chloro-3-(2,3,6-trifluorobenzyl)-1*H*-indazol-1-yl]-2'-methyl-5,5'-bipyrimidine-4,6-diamine |
| 85 | 2-[5-chloro-3-(2,3.6-trifluorobenzyl)-1*H*-indazol-1-yl]-5-pyridin-4-ylpyrimidine-4,6-diamine |
| 86 | 2-[5-chloro-3-(2,3,6-trifluorobenzyl)-1*H*-indazo-1-yl]-5-(1-isobutyl-1*H-*pyrazol-4-yl)pyrimidine-4,6-diamine |
| 87 | 2-[5-chloro-3-(2,3,6-trifluorobenzyl)-1*H*-indazol-1-yl]-5-[1-(3-methylbutyl)-1*H*-pyrazol-4-yl]pyrimidine-4,6-diamine |
| 88 | 5-(1-*tert*-butyl-3-methyl-1*H*-pyrazol-4-yl)-2-[5-chloro-3-(2,3,6-trifluorobenzyl)-1*H*-indazol-1-yl]pyrimidine-4,6-diamine |
| 89 | 2-[5-chloro-3-(2,3,6-trifluorobenzyl)-1*H*-indazol-1-yl]-5-[1-(cyclopropylmethyl)-3-methyl-1*H*-pyrazol-4-yl]pyrimidine-4,6-diamine |
| 90 | 2-[5-chloro-3-(2,3,6-trifluorobenzyl)-1*H*-indazol-1-yl]-5-[5-(methoxymethyl)-1,3-dimethyl-1*H-*pyrazol-4-yl]pyrimidine-4,6-diamine |
| 91 | 2-[5-chloro-3-(2,3,6-trifluorobenzyl)-1*H*-indazol-1-yl]-5-(1,3,5-trimethyl-1*H*-pyrazol-4-yl)pyrimidine-4,6-diamine |
| 92 | 2-[5-chloro-3-(2,3,6-trifluorobenzyl)-1*H*-indazol-1-yl]-5-pyridin-3-ylpyrimidine-4,6-diamine |
| 93 | 2-[5-chloro-3-(2,3,6-trifluorobenzyl)-1*H*-indazol-1-yl]-5-(6-fluoropyridin-3-yl)pyrimidine-4,6-diamine |
| 94 | 2-[5-chloro-3-(2,3,6-trifluorobenzyl)-1*H*-indazol-1-yl]-5-[3-(methylsulfonyl)phenyl]pyrimidine-4,6-diamine |
| 95 | 2-[5-chloro-3-(2,3,6-trifluorobenzyl)-1*H*-indazol-1-yl]-5-(2-methylpyridin-4-yl)pyrimidine-4,6-diamine |
| 96 | 2-[5-chloro-3-(2,3,6-trifluorobenzyl)-1*H*-indazol-1-yl]-5-(5-fluoropyridin-3-yl)pyrimidine-4,6-diamine |
| 97 | 2-[5-chloro-3-(2,3,6-trifluorobenzyl)-1*H*-indazol-1-yl]-5-[1-methyl-5-(trifluoromethyl)-1*H*-pyrazol-4-yl]pyrimidine-4,6-diamine |
| 98 | 2-[5-chloro-3-(2,3,6-trifluorobenzyl)-1*H*-indazol-1-yl]-5-(5-methoxypyridin-3-yl)pyrimidine-4,6-diamine |
| 99 | 2-[5-chloro-3-(2,3,6-trifluorobenzyl)-1*H*-indazol-1-yl]-5-(3,5-dimethylisoxazol-4-yl)pyrimidine-4,6-diamine |
| 100 | 2-[5-chloro-3-(2,3,6-trifluorobenzyl)-1*H*-indazol-1-yl]-5-(2-methylpyridin-3-yl)pyrimidine-4,6-diamine |
| 101 | 2-[5-chloro-3-(2,3,6-trifluorobenzyl)-1*H*-indazol-1-yl]-5-(6-methylpyridin-3-yl)pyrimidine-4,6-diamine |
| 102 | 2-[5-chloro-3-(2,3,6-trifluorobenzyl)-1*H*-indazol-1-yl]-5-(5-methylpyridin-3-yl)pyrimidine-4,6-diamine |
| 103 | 2-[5-chloro-3-(2,3,6-trifluorobenzyl)-1*H*-indazol-1-yl]-5-(1-methyl-1*H-*pyrazol-5-yl)pyrimidine-4,6-diamine |
| 104 | 2-[5-chloro-3-(2,3,6-trifluorobenzyl)-1*H*-indazol-1-yl]-5-(6-methoxypyridin-3-yl)pyrimidine-4,6-diamine |
| 105 | 2-[5-chloro-3-(2,3,6-trifluorobenzyl)-1*H*-indazol-1-yl]-5-(2-methoxypyridin-4-yl)pyrimidine-4,6-diamine |
| 106 | 5-(2-aminopyridin-4-yl)-2-[5-chloro-3-(2,3,6-trifluorobenzyl)-1*H-*indazol-1-yl]pyrimidine-4,6-diamine |
| 107 | 2-[5-chloro-3-(2,3,6-trifluorobenzyl)-1*H-*indazol-1-yl]-5-(3,5-dimethyl-1*H*-pyrazol-4-yl)pyrimidine-4,6-diamine |
| 108 | methyl (4-{4,6-diamino-2-[5-chloro-3-(2,3,6-trifluorobenzyl)-1*H-*indazol-1-yl]pyrimidin-5-yl}pyridin-2-yl)carbamate |
| 109 | 2-[5-chloro-3-(2,3,6-trifluorobenzyl)-1*H*-indazol-1-yl]-5-(6-methoxypyridin-2-yl)pyrimidine-4,6-diamine |
| 110 | methyl (5-{4,6-diamino-2-[5-chloro-3-(2,3,6-trifluorobenzyl)-1*H-*indazol-1-yl]pyrimidin-5-yl}pyridin-2-yl)carbamate |
| 111 | 2-[5-chloro-3-(2,3,6-trifluorobenzyl)-1*H*-indazol-1-yl]-5-(2-methoxypyridin-3-yl)pyrimidine-4,6-diamine |
| 112 | 2-[5-chloro-3-(2,3,6-trifluorobenzyl)-1*H*-indazol-1-yl]-5-(3-fluoro-2-morpholin-4-ylpyridin-4-yl)pyrimidine-4,6-diamine |
| 113 | 2-[5-chloro-3-(2,3,6-trifluorobenzyl)-1*H*-indazol-1-yl]-5-pyrazin-2-ylpyrimidine-4,6-diamine |
| 114 | *N*-(5-{4,6-diamino-2-[5-chloro-3-(2,3,6-trifluorobenzyl)-1*H*-indazol-1-yl]pyrimidin-5-yl}pyridin-2-yl)acetamide |
| 115 | 2-[5-fluoro-3-(2,3,6-trifluorobenzyl)-1*H*-indazol-1-yl]-5-(2-methoxypyridin-4-yl)pyrimidine-4,6-diamine |
| 116 | 2-[5-fluoro-3-(2,3,6-trifluorobenzyl)-1*H*-indazol-1-yl]-5-[5-(trifluoromethyl)pyridin-3-yl]pyrimidine-4,6-diamine |
| 117 | 2-[5-fluoro-3-(2,3,6-trifluorobenzyl)-1*H*-indazol-1-yl]-5-pyridin-3-ylpyrimidine-4,6-diamine |
| 118 | 5-(6-fluoropyridin-3-yl)-2-[5-fluoro-3-(2,3,6-trifluorobenzy])-1*H-*indazol-1-yl]pyrimidine-4,6-diamine |
| 119 | 5-(6-aminopyridin-3-yl)-2-[5-fluoro-3-(2,3,6-trifluorobenzyl)-1*H-*indazol-1-yl]pyrimidine-4,6-diamine |
| 120 | 2-[5-fluoro-3-(2,3,6-trifluorobenzyl)-1*H*-indazol-1-yl]-5-[3-(methylsulfonyl)phenyl]pyrimidine-4,6-diamine |
| 121 | 2-[5-fluoro-3-(2,3,6-trifluorobenzyl)-1*H*-indazol-1-yl]-5-quinolin-5-ylpyrimidine-4,6-diamine |
| 122 | 2-[5-fluoro-3-(2.3,6-trifluorobenzyl)-1*H*-indazol-1-yl]-5-(2-methylpyridin-4-yl)pyrimidine-4,6-diamine |
| 123 | 3-{4,6-diamino-2-[5-fluoro-3-(2,3,6-trifluorobenzyl)-1*H*-indazol-1-yl]pyrimidin-5-yl}quinolin-2-ol |
| 124 | 5-(5-fluoropyridin-3-yl)-2-[5-fluoro-3-(2,3,6-trifluorobenzyl)-1*H-*indazol-1-yl]pyrimidine-4,6-diamine |
| 125 | 5-(2-fluoroquinolin-3-yl)-2-[5-fluoro-3-(2,3,6-trifluorobenzyl)-1*H-*indazol-1-y]pyrimidine-4,6-diamine |
| 126 | 2-[5-fluoro-3-(2,3,6-trifluorobenzyl)-1*H*-indazol-1-yl]-5-pyridin-4-ylpyrimidine-4,6-diamine |
| 127 | 2-[5-fluoro-3-(2,3,6-trifluorobenzyl)-1*H*-indazol-1-yl]-5-(1,3,5-trimethyl-1*H*-pyrazol-4-yl)pyrimidine-4,6-diamine |
| 128 | 2-[5-fluoro-3-(2,3,6-trifluorobenzyl)-1*H*-indazol-1-yl]-5-[1-methyl-5-(trifluoromethyl)-1H-pyrazol-4-yl]pyrimidine-4,6-diamine |
| 129 | 2-[5-fluoro-3-(2,3,6-trifluorobenzyl)-1*H*-indazol-1-yl]-5-[1-methyl-3-(trifluoromethyl)-1*H*-pyrazol-5-yl]pyrimidine-4.6-diamine |
| 130 | 5-(3,5-dimethylisoxazol-4-yl)-2-[5-fluoro-3-(2,3,6-trifluorobenzyl)-1*H-*indazol-1-yl]pyrimidine-4,6-diamine |
| 131 | 2-[5-fluoro-3-(2,3,6-trifluorobenzyl)-1*H*-indazol-1-yl]-5-(2-methoxypyridin-4-yl)pyrimidine-4,6-diamíne |
| 132 | 2-[5-fluoro-3-(2,3,6-trifluorobenzyl)-1*H*-indazol-1-yl]-2',4'-dimethoxy-5,5'-bipyrimidine-4,6-diamine |
| 133 | 2-[5-fluoro-3-(2,3,6-trifluorobenzyl)-1*H*-indazol-1-yl]-5-(2-methoxypyridin-3-yl)pyrimidine-4,6-diamine |
| 134 | 2-[5-fluoro-3-(2,3,6-trifiuorobenzyl)-1*H*-indazol-1-yl]-5-(5-methoxypyridin-3-yl)pyrimidine-4,6-diamine |
| 135 | 2-[5-fluoro-3-(2,3,6-trifluorobenzyl)-1*H*-indazol-1-yl]-2'-methyl-5,5'-bipyrimidine-4,6-diamine |
| 136 | 2-[5-fluoro-3-(2,3,6-trifluorobenzyl)-1*H*-indazol-1-yl]-2'-methoxy-5,5'-bipyrimidine-4,6-diamine |
| 137 | 2-[5-fluoro-3-(2,3,6-trifluorobenzyl)-1*H*-indazol-1-yl]-5-(6-methoxypyridin-2-yl)pyrimidine-4,6-diamine |
| 138 | 5-(2,6-dimethoxypyridin-3-yl)-2-[5-fluoro-3-(2,3,6-trifluorobenzyl)-1*H-*indazol-1-yl]pyrimidine-4,6-diamine |
| 139 | 2-[5-fluoro-3-(2,3,6-trifluorobenzyl)-1*H*-indazol-1-yl]-5-(4-methox_ypyridin-3-yl)pyrimidine-4,6-diamine |
| 140 | 2-[5-fluoro-3-(2,3,6-trifluorobenzyl)-1H-indazol-1-yl]-5,5'-bipyrimidine-4,6-diamine |
| 141 | 5-(4-fluoropyridin-3-yl)-2-[5-fluoro-3-(2,3,6-trifluorobenzyl)-1H-indazol-1-y!J-Qyrimidine-4,6-diamine |
| 142 | 2-[5-fluoro-3-(2,3,6-trifluorobenzyl)-1H-indazol-1-yl]-5-(1,3-thiazol-2-yl)pyrimidine-4,6-diamine |
| 143 | 2-[5-fluoro-3-(2,3,6-trifluorobenzyl)-1H-indazol-1-yl]-5-(4-methyl-2-phenyl-1,3-thiazol-5.,rl)_pyrimidine-4,6-diamine |
| 144 | *N*-{4,6-diamino-2-[5-fluoro-3-(2,3,6-trifluorobenzyl)-1*H*-indazol-1-yl]pyrimidin-5-yl}-2-methylpropanamide |
| 145 | 2-(5-fluoro-3-(2,3,6-trifluorobenzyl)-1H-indazol-1-yl]-5-(1,3-oxazol-2-yl)_Nrimidine-4,6-diamine |
| 146 | 5-(2-methylpyridin-4-yl)-2-[3-(2,3,6-trifluorobenzyl)-1H-indazol-1-yl]pyrimidine-4,6-diamine |
| 147 | 5-(6-fluoropyridin-3-yl)-2-[3-(2,3,6-trifluorobenzyl)-1H-indazol-1-y!!]QYrimidine-4,6-diamine |
| 148 | 2-[3-(2,3,6-trifluorobenzyl)-1H-indazol-1-yl]-5-(1,3,5-trimethyl-1H-pyrazol-4-yl):pyrimidine-4,6-diamine |
| 149 | 5-(5-fluoropyridin-3-yl)-2-[3-(2,3,6-trifluorobenzyl)-1H-indazol-1-yl]pyrimidine-4,6-diamine |
| 150 | 5-(1-methyl-1H-pyrazol-4-yl)-2-[3-(2,3,6-trifluorobenzyl)-1H-indazol-1-_yl]_Qyrimidine-4,6-diamine |
| 151 | 5-(2-methylpyridin-4-yl)-2-[3-(2,3,6-trifluorobenzyl)-1H-indazol-1-yl]pyrimidine-4,6-diamine |
| 152 | 5-pyridin-4-yl-2-[3-(2,3,6-trifluorobenzyl)-1H-indazol-1-yl]pyrimidme-4,6-diamine |
| 153 | 5-pyridin-3-yl-2-[3-(2,3,6-trifluorobenzyl)-1H-indazol-1-yl]pyrimidine-4,6-diamine |
| 154 | 5-(1-methyl-5-(trifluoromethyl)-1H-pyrazol-4-yl]-2-[3-(2,3,6- trifluorobenzyl)-1H-indazol-1-yl]pyrimidine-4,6- |
| 155 | 5-(2-methoxypyridin-4-yl)-2-[3-(2,3,6-trifluorobenzyl)-1H-indazol-1-yl]pyrimidine-4,6-diamine |
| 156 | 5-(3,5-dimethylisoxazol-4-yl)-2-[3-(2,3,6-trifluorobenzyl)-1H-indazol-1-yl]pyrimidine-4,6-diamine |
| 157 | 5-(2-methylpyridin-3-yl)-2-[3-(2,3,6-trifluorobenzyl)-1H-indazol-1-yl]pyrimidine-4,6-diamine |
| 158 | 5-(6-methylpyridin-3-yl)-2-[3-(2,3,6-trifluorobenzyl)-1*H*-indazol-1-yl]pyrimidine-4,6-diamine |
| 159 | 2'-methyl-2-[3-(2,3,6-trifluorobenzyl)-1*H*-indazol-1-yl]-5,5'-bipyrimidine-4,6-diamine |
| 160 | 5-(3-fluoropyridin-4-yl)-2-[3-(2,3,6-trifluorobenzyl)-1*H*-indazol-1-yl]pyrimidine-4,6-diamine |
| 161 | 5-[6-(dimethylamino)pyridin-3-yl]-2-[3-(2,3,6-trifluorobenzyl)-1*H-*indazol-1-yl]pyrimidine-4,6-diamine |
| 162 | 5-(2-methoxypyridin-3-yl)-2-[3-(2,3,6-trifluorobenzyl)-1*H*-indazol-1-yl]pyrimidine-4,6-diamine |
| 163 | 5-(6-methoxypyridin-3-yl)-2-[3-(2,3,6-trifluorobenzyl)-1*H*-indazol-1-yl]pyrimidine-4,6-diamine |
| 164 | 5-(4-methyl-2-phenyl-1,3-thiazol-5-yl)-2-[3-(2,3,6-trifluorobenzyl)-1*H-*indazol-1-yl]pyrimidine-4.6-diamine |
| 165 | 5-pyrazin-2-yl-2-[3-(2,3,6-trifluorobenzyl)-1*H*-indazol-1-yl]pyrimidine-4,6-diamine |
| 166 | 5-(4-methoxypyridin-3-yl)-2-[3-(2,3,6-trifluorobenzyl)-1*H*-indazol-1-yl]pyrimidine-4,6-diamine |
| 167 | 5-(2,6-dimethoxypyridin-3-yl)-2-[3-(2,3,6-trifluorobenzyl)-1*H*-indazol-1-yl]pyrimidine-4,6-diamine |
| 168 | 5-(6-methoxypyridin-2-yl)-2-[3-(2,3,6-trifluorobenzyl)-1*H*-indazol-1-yl]pyrimidine-4,6-diamine |
| 169 | 5-pyridin-2-yl-2-[3-(2,3,6-trifluorobenzyl)-1*H*-indazol-1-yl]pyrimidine-4,6-diamine |
| 170 | 2-[5-methoxy-3-(2,3,6-trifluorobenzyl)-1*H*-indazol-1-yl]-5-(1-methyl-1*H*-pyrazol-4-yl)pyrimidine-4,6-diamine |
| 171 | 2-[5-methoxy-3-(2,3,6-trifluorobenzyl)-1*H*-indazol-1-yl]-5-pyridin-3-ylpyrimidine-4,6-diamine |
| 172 | 2-[5-methoxy-3-(2,3,6-trifluorobenzyl)-1*H*-indazol-1-yl]-5-[5-(trifluoromethyl)pyridin-3-yl]pyrimidine-4,6-diamine |
| 173 | 2-[5-methoxy-3-(2,3,6-trifluorobenzyl)-1*H*-indazol-1-yl]-5-(1,3,5-trimethyl-1*H*-pyrazol-4-yl)pyrimidine-4,6-diamine |
| 174 | 5-pyridin-4-yl-2-[3-(2,3,6-trifluorobenzyl)-1*H*-pyrazolo[4,3-*b*]pyridin-1-yl]pyrimidine-4,6-diamine |
| 175 | 5-(1-methyl-1*H*-pyrazol-4-yl)-2-[3-(2,3,6-trifluorobenzyl)-1*H-*pyrazolo[4,3-*b*]pyridin-1-yl]pyrimidine-4,6-diamine |
| 176 | methyl {4,6-diamino-2-[5-chloro-3-(2,3,6-trifluorobenzyl)-1*H*-indazol-1-yl]pyrimidin-5-yl}carbamate |
| 177 | methyl {4,6-diamino-2-[5-chloro-3-(2,3,6-trifluorobenzyl)-1*H-*indazol-1-yl]pyrimidin-5-yl}methylcarbamate |
| 178 | *N-*{4,6-diamino-2-[5-chloro-3-(2,3.6-trifluorobenzyl)-1*H*-indazol-1-yl]pyrimidin-5-yl}cyclopropanecarboxamide |
| 179 | *S*-methyl {4,6-diamino-2-[5-chloro-3-(2,3,6-trifluorobenzyl)-1*H*-indazol-1-yl]pyrimidin-5-yl}thiocarbamate |
| 180 | *S*-methyl {4,6-diamino-2-[5-fluoro-3-(2,3,6-trifluorobenzyl)-1*H*-indazol-1-yl]pyrimidin-5-yl}thiocarbamate |
| 181 | methyl {4,6-diamino-2-[5-fluoro-3-(2,3,6-trifluorobenzyl)-1*H*-indazol-1-yl]pyrimidin-5-yl}methylcarbamate |
| 182 | methyl {4,6-diamino-2-[3-(2,3,6-trifluorobenzyl)-1*H*-indazol-1-yl]pyrimidin-5-yl}carbamate |
| 183 | *S*-methyl {4,6-diamino-2-[3-(2,3,6-trifluorobenzyl)-1*H*-indazol-1-yl]pyrimidin-5-yl}thiocarbamate |
| 184 | methyl {4,6-diamino-2-[3-(2,3,6-trifluorobenzyl)-1*H*-indazol-1-yl]pyrimidin-5-yl}methylcarbamate |
| 185 | ethyl {4,6-diamino-2-[3-(2,3,6-trifluorobenzyl)-1*H*-indazol-1-yl]pyrimidin-5-yl}carbamate |
| 186 | methyl {4,6-diamino-2-[5-chloro-3-(2,3,5-trifluorobenzyl)-1*H*-indazol-1-yl]pyrimidin-5-yl}carbamate |
| 187 | *S*-methyl {4,6-diamino-2-[5-chloro-3-(2,3,5-trifluorobenzyl)-1*H*-indazol-1-yl]pyrimidin-5-yl}thiocarbamate |
| 188 | methyl {4.6-diamino-2-[5-chloro-3-(2,3,5-trifluorobenzyl)-1*H*-indazol-1-yl]pyrimidin-5-yl}methylcarbamate |
| 189 | methyl {4,6-diamino-2-[3-(2,3,5-trifluorobenzyl)-1*H*-indazol-1-yl]pyrimidin-5-yl} carbamate |
| 190 | methyl {4,6-diamino-2-[3-(2,3,5-trifluorobenzyl)-1*H*-indazol-1-yl]pyrimidin-5-yl}methylcarbamate |
| 191 | *S*-methyl {4,6-diamino-2-[3-(2,3,5-trifluorobenzyl)-1H-indazol-1-yl]pyrimidin-5-yl}methylthiocarbamate |
| 192 | ethyl {4,6-diamino-2-[3-(2,3,5-trifluorobenzyl)-1*H*-indazol-1-yl]pyrimidin-5-yl}carbamate |
| 193 | ethyl {4,6-diamino-2-[3-(2,3,5-trifluorobenzyl)-1*H*-indazol-1-yl]pyrimidin-5-yl}methylcarbamate |
| 194 | methyl {4,6-diamino-2-[5-phenyl-3-(2,3,6-trifluorobenzyl)-1*H*-indazol-1-yl]pyrimidin-5-yl}methylcarbamate |
| 195 | methyl {4,6-diamino-2-[5-fluoro-3-(2,3,5-trifluorobenzyl)-1*H*-indazol-1-yl]pyrimidin-5-yl}carbamate |
| 196 | methyl {4,6-diamino-2-[5-fluoro-3-(2,3,5-trifluorobenzyl)-1*H*-indazol-1-yl]pyrimidin-5-yl}methylcarbamate |
| 197 | ethyl {4,6-diamino-2-[5-chloro-3-(2,3,6-trifluorobenzyl)-1*H*-indazol-1-yl]pyrimidin-5-yl}carbamate |
| 198 | ethyl {4,6-diamino-2-[5-chloro-3-(2,3,5-trifluorobenzyl)-1*H*-indazol-1-yl]pyrimidin-5-yl}carbamate |
| 199 | methyl {4,6-diamino-2-[5-chloro-3-(2,3,6-trifluorobenzyl)-1*H-*pyrazolo[4,3*-b*]pyridin-1-yl]pyrimidin-5-yl}carbamate |
| 200 | methyl {4,6-diamino-2-[5-chloro-3-(2,3,6-trifluorobenzyl)-1*H-*pyrazolo[4,3-*b*]pyridin-1-yl]pyrimidin-5-yl}methylcarbamate |
| 201 | methyl {4,6-diamino-2-[3-(2,3,6-trifluorobenzyl)-1H-pyrazolo[4,3-b]pyridin-1-yl]pyrimidin-5-yl}carbamate |
| 202 | methyl {4,6-diamino-2-[3-(3-chloro-2,5-difluorobenzyl)-5-fluoro-1*H-*indazol-1-yl]pyrimidin-5-yl}carbamate |
| 203 | methyl {4,6-diamino-2-[3-(2-chloro-3,6-difluorobenzyl)-5-fluoro-1*H-*indazol-1-yl]pyrimidin-5-yl}carbamate |
| 204 | methyl {4,6-diamino-2-[3-(2,6-difluoro-3-methylbenzyl)-5-fluoro-1*H-*indazol-1-yl]pyrimidin-5-yl}carbamate |
| 205 | *S*-methyl {4,6-diamino-2-[5-fluoro-3-(2,3,6-trichlorobenzyl)-1*H*-indazol-1-yl]pyrimidin-5-yl}thiocarbamate |
| 206 | *N*-{4,6-diamino-2-[5-fluoro-3-(2,3,6-trifluorobenzyl)-1*H*-indazol-1-yl]pyrimidin-5-yl}cyclopropanecarboxamide |
| 207 | *N*-{4,6-diamino-2-[3-(2,3,6-trifluorobenzyl)-1*H*-indazol-1-yl]pyrimidin-5-yl}cyclopropanecarboxamide |
| 208 | 2-[3-(2-cyclopentylethyl)-1*H*-indazol-1-yl]-5-pyridin-3-ylpyrimidine-4,6-diamine |
| 209 | 2-[3-(2-cyclopentylethyl)-1*H*-indazol-1-yl]-5-(1-methyl-1*H*-pyrazol-4-yl)pyrimidine-4,6-diamine |
| 210 | 2-[3-(2-cyclopentylethyl)-1*H-*indazol-1-yl]-5-(2-methylpyridin-4-yl)pyrimidine-4,6-diamine |
| 211 | 2-[3-(2-cyclopentylethyl)-1*H*-indazol-1-yl]-5-pyridin-4-ylpyrimidine-4,6-diamine |
| 212 | 2-[3-(2-cyclopentylethyl)-1*H-in*dazol-1-yl]-5-(1,3,5-trimethyl-1*H-*pyrazol-4-yl)pyrimidine-4,6-diamine |
| 213 | 2-[3-(2-cyclopentylethyl)-1*H*-indazol-1-yl]-5-[5-(trifluoromethyl)pyridin-3-yl]pyrimidine-4,6-diamine |
| 214 | 2-[3-(2-cyclopentylethyl)-1*H*-indazol-1-yl]-5-[1-methyl-3-(trifluoromethyl)-1*H*-pyrazol-4-yl]pyrimidine-4,6-diamine |
| 215 | 2-[5-chloro-3-(2-cyclopentylethyl)-1*H*-indazol-1-yl]-5-pyridin-3-ylpyrimidine-4,6-diamine |
| 216 | 2-[5-chloro-3-(2-cyclopentylethyl)-1*H*-indazol-1-yl]-5-(2-methylpyridin-4-yl)pyrimidine-4,6-diamine |
| 217 | 2-[5-chloro-3-(2-cyclopentylethyl)-1*H*-indazol-1-yl]-5-(1-methyl-1*H-*pyrazol-4-yl)pyrimidine-4,6-diamine |
| 218 | 2-[5-chloro-3-(2-cyclopentylethyl)-1*H*-indazol-1-yl]-5-(1,3,5-trimethyl-1*H*-pyrazol-4-yl)pyrimidine-4,6-diamine |
| 219 | 2-{3-(2-cyclopentylethyl)-5-[5-(trifluoromethyl)pyridin-3-yl]-1*H*-indazol-1-yl}-5-[5-(trifluoromethyl)pyridin-3-yl]pyrimidine-4,6-diamine |
| 220 | 2-[3-(2-cyclopentylethyl)-5-fluoro-1*H*-indazol-1-yl]-5-pyridin-3-ylpyrimidine-4,6-diamine |
| 221 | 2-[3-(2-cyclopentylethyl)-5-fluoro-1*H*-indazol-1-yl]-5-(2-methylpyridin-4-yl)pyrimidine-4,6-diamine |
| 222 | 2-[3-(2-cyclopentylethyl)-5-fluoro-1*H*-indazol-1-yl]-5-(1-methyl-1*H-*pyrazol-4-yl)pyrimidine-4,6-diamine |
| 223 | 2-[3-(2-cyclopentylethyl)-5-fluoro-1*H*-indazol-1-yl]-5-(1,3,5-trimethyl-1*H*-pyrazol-4-yl)pyrimidine-4,6-diamine |
| 224 | 2-(5-chloro-3-pentyl-1*H*-indazol-1-yl)-5-(1-methyl-1*H*-pyrazol-4-yl)pyrimidine-4,6-diamine |
| 225 | 2-(5-chloro-3-pentyl-1*H*-indazol-1-yl)-5-(2-methylpyridin-4-yl)pyrimidine-4,6-diamine |
| 226 | 2-(5-chloro-3-pentyl-1*H*-indazol-1-yl)-5-(1,3,5-trimethyl-1*H*-pyrazol-4-yl)pyrimidine-4,6-diamine |
| 227 | 2-(3-pentyl-1*H*-indazol-1-yl)-5-(1,3,5-trimethyl-1*H*-pyrazol-4-yl)pyrimidine-4,6-diamine |

3-(4-Amino-5-cyclopropylpyrimidin-2-yl)-1-(2-fluorobenzyl)-1*H*-pyrazolo[3,4-*b*]pyridine (1A) known as BAY 41-2272 disclosed as example 1 in WO 00/06568.

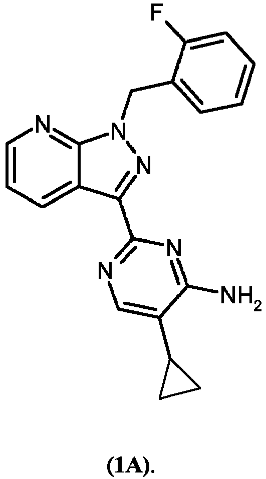

A further embodiment of the invention is the combination of stimulators and/or activators of the soluble guanylate cyclase according to compounds classes I to X with PDE5 inhibitors for the prevention and treatment of fibrotic diseases, such as Systemic Sclerosis, scleroderma, and the concomitant fibrosis of internal organs. The following PDE 5 inhibitors are preferred for the combination with sGC stimulators and/or activators:

*Tadalafil* ((6R,12aR) -2,3,6,7,12,12a - Hexahydro - 2 - methyl - 6 - (3,4-methylene -dioxyphenyl) pyrazino(1',2':1,6) pyrido(3,4-b)indole-1,4-dione), *Vardenafil* (2-(2-Ethoxy-5-(4-ethylpiperazin-1-yl-1-sulfonyl)phenyl)-5-methyl-7-propyl-3H-imidazo (5,1-f) (1,2,4)triazin-4-one), *Sildenafil* (3-[2-ethoxy-5-(4-methylpiperazin-1-yl)sulfonyl-phenyl]- 7- methy 1- 9- propy 1-2,4,7,8- tetrazabicyclo [4.3.0]nona -3,8,10-trien-5-one), *Udenafil* 5-[2-propyloxy-5-(1-methyl-2-pyrrolidinylethylamidosulfonyl)phenyl]-methyl-3-propyl-1,6-dihydro-7H-pyrazolo(4,3-d)pyrimidine-7-one, *Dasantafil* 7-(3-Bromo-4-methoxybenzyl)-1-ethyl-8-[[(1,2)-2-hydroxycyclopentyl]amino]-3-(2-hydroxyethyl)-3,7-dihydro-1-purine-2,6-dione, *Avanafil* 4-{[(3-chloro-4-methoxyphenyl)methyl]amino}-2-[(2S)-2-(hydroxymethyl)pyrrolidin-1-yl]-N-(pyrimidin-2-ylmethyl)pyrimidine-5-carboxamide, Mirodenafil, Lodenafil, UK 369.003, UK 371.800, *SLx 2101* of Surface Logix, *LAS* 34179Triazolo[1,2-]xanthine,6-methyl-4-propyl-2-[2-propoxy-5-(4-methylpiperazino)sulfonyl]phenyl or salts, hydrates or hydrates of the salts.

Especially preferred are combinations of compounds according to examples of compound classes I to X with vardenafil and/or sildenafil.

Especially preferred are combinations of compounds according to examples of compound classes I to X with vardenafil and/or sildenafil for use in the prevention and/or treatment of Systemic Sclerosis (SSc).

Especially preferred are compounds according to examples of compound classes I to X for use in the prevention and/or treatment of Systemic Sclerosis SSc.

Especially preferred are compounds according to examples of compound classes I to X for use in the prevention and/or treatment of Systemic Sclerosis SSc.

Especially preferred is at least one compound according to examples of compound classes I to X in combination with vardenafil or sildenafil for use in the prevention and/or treatment of scleroderma.

A further embodiment of the invention is the combination of stimulators and/or activators of the soluble guanylate cyclase with imunosupressant therapy (i.e. cyclophosphamide CYP, methotrexate MTX,), with kinase inhibitors, (i.e. sorafenib, regorafenib, imatinib, dasatinib), with glucocorticoids (i.e. prednisolon, methylprednisoln), with Anti-CD20 antibodies, with P144 beta-glycan, with abatacept

A further embodiement of the invention is the combination of stimulators and/or activators of the soluble guanylate cyclase with ACE-inhibitors (i.e. captopril, enalapril), calcium channel blockers (i.e. nifedipine), prostanoids (i.e. iloprost), endothelin antagonists (i.e. bosentan).

Another preferred embodiment of the invention are compounds and/or combinations indicated above for use in the prevention and/or treatment of Systemic Sclerosis (SSc), diffuse Systemic Sclerosis (dSSc), limited Systemic Sclerosis (ISSc), overlap type of Systemic Sclerosis, undifferentiated type of Systemic Sclerosis, Systemic Sclerosis sine scleroderma, skin fibrosis, scleroderma, nephrogenic fibrosing dermopathy (NFD), keloid formation.

Another preferred embodiment of the invention are compounds and/or combinations indicated above for use in the prevention and/or treatment of scleroderma.

Another preferred embodiment of the invention are compounds and/or combinations indicated above for use in the prevention and/or treatment of Systemic Sclerosis SSc concomitant fibrosis of internal organs, comprising the gut, the lung, the kidney and the blood vessels.

Another preferred embodiment of the invention is the use for the production of a medicament for prevention and/or treatment of Systemic Sclerosis (SSc) comprising an effective amount of a compound and/or a combination as indicated above.

Another preferred embodiment of the invention is the use for the production of a medicament for prevention and/or treatment of scleroderma comprising an effective amount of a compound and/or a combination as indicated above.

Another preferred embodiment of the invention is the use for the production of a medicament for prevention and/or treatment of Systemic Sclerosis (SSc), diffuse Systemic Sclerosis (dSSc), limited Systemic Sclerosis (ISSc), overlap type of Systemic Sclerosis, undifferentiated type of Systemic Sclerosis, Systemic Sclerosis sine scleroderma, skin fibrosis, scleroderma, nephrogenic fibrosing dermopathy (NFD), keloid formation comprising an effective amount of a compound and/or a combination as indicated above.

Another preferred embodiment of the invention is the use for the production of a medicament for prevention and/or treatment of Systemic Sclerosis SSc concomitant fibrosis of internal organs, comprising the gut, the lung, the kidney and the blood vessels comprising an effective amount of a compound and/or a combination as indicated above.

Another preferred embodiment of the invention is the pharmaceutical formulation comprising at least one compound or one combination as indicated above for the use in the prevention and/or treatment of Systemic Sclerosis (SSc).

Another preferred embodiment of the invention is the pharmaceutical formulation comprising at least one compound or one combination as indicated above for the use in the prevention and/or treatment of Systemic Sclerosis (SSc), diffuse Systemic Sclerosis (dSSc), limited Systemic Sclerosis (ISSc), overlap type of Systemic Sclerosis, undifferentiated type of Systemic Sclerosis, Systemic Sclerosis sine scleroderma, skin fibrosis, scleroderma, nephrogenic fibrosing dermopathy (NFD), keloid formation.

Another preferred embodiment of the invention is the pharmaceutical formulation comprising at least one compound or one combination as indicated above for the use in the prevention and/or treatment of Systemic Sclerosis (SSc).

Another preferred embodiment of the invention is the pharmaceutical formulation comprising at least one compound or one combination as indicated above for the use in the prevention and/or treatment of Systemic Sclerosis SSc concomitant fibrosis of internal organs, comprising the gut, the lung, the kidney and the blood vessels.

Another preferred embodiment of the invention is a kit comprising at least one sGC stimulator and/or activator as indicated above or a combination as indicated above for the use in the prevention and/or treatment of Systemic Sclerosis (SSc).

Another preferred embodiment of the invention is a kit as indicated above for the use in the prevention and/or treatment of Systemic Sclerosis (SSc), diffuse Systemic Sclerosis (dSSc), limited Systemic Sclerosis (ISSc), overlap type of Systemic Sclerosis, undifferentiated type of Systemic Sclerosis, Systemic Sclerosis sine scleroderma, skin fibrosis, scleroderma, nephrogenic fibrosing dermopathy (NFD), keloid formation.

Another preferred embodiment of the invention is a kit comprising at least one sGC stimulator and/or activator as indicated above or a combination as indicated above for the use in the prevention and/or treatment of scleroderma.

Another preferred embodiment of the invention is a kit as indicated above for the use in the prevention and/or treatment of Systemic Sclerosis SSc concomitant fibrosis of internal organs, comprising the gut, the lung, the kidney and the blood vessels.

A pharmaceutical composition of the invention is formulated to be compatible with its intended route of administration. Examples of routes of administration include parenteral e.g., intravenous, intradermal, subcutaneous' oral (e.g.' inhalation)' transdermal (topical) transmucosal and rectal administration. Pharmaceutical compositions suitable for injectable use include sterile aqueous solutions (where water soluble) or dispersions and sterile powders for the extemporaneous preparation of sterile injectable solutions or dispersions. The carrier can be a solvent or dispersion medium containing, for example, water, ethanol, a pharmaceutically acceptable polyol like glycerol, propylene glycol, liquid polyetheylene glycol, and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents for example, parabens, chlorobutanol, phenol, ascorbic acid, thimerosal, and the like. In many cases, it will be preferable to include isotonic agents, for example, sugars, polyalcohols such as maitol sorbitol sodium chloride in the composition.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed.

Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or con1 starch; a lubricant such as magnesium stearate or sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from a pressurized container or dispenser which contains a suitable propellant, e.g.' a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Bio degradable, biocompatible polymers can be used, such as ethylene vinyl acetate, polyanhydrides, polyglycolic acid, collagen, polyorthoesters, and polylactic acid.

Drawings:
Figure 1: Effects of example 1A on mean arterial blood pressure (left) and heart rate (right).

### Experimental Part

### Example A

### Bleomycin-induced skin fibrosis

Local skin fibrosis was induced in 6-week-old, pathogen-free, female DBA/2 mice (Charles River, Sulzfeld, Germany) by repeated (every other day) subcutaneous injections of bleomycin (0,5 mg/ml in saline) in a defined area of the upper back. Control mice were injected in the same manner with saline only and served as reference. For all groups the injection volume was 100 µl. Concomitant to bleomycin treatment, the mice were treated orally with test drug or vehicle. Mice were treated a) with vehicle b) with 1 mg/kg example 1A, and c) with 3 mg/kg example 1A, twice a day via gavage for 21 days. After this 3 weeks treatment period, the animals were sacrificed and skin samples were obtained for analysis.

### Histological analysis

The injected skin areas were fixed in 4% formalin and embedded in paraffin. Histological sections were stained with hematoxylin and eosin for the determination of dermal thickness. The dermal thickness was determined by measuring the largest distance between the epidermal-dermal junction and the dermal-subcutaneous fat junction. The measurements were performed by an examiner blinded to the treatment of the mice.

### Hydroxyproline assay

To analyze the collagen content in skin samples, hydroxyproline assay was performed. After digestion of punch biopsies (Ø 3mm) in 6M HCl for three hours at 120°C, chloramine T (0.06 M) was added and samples were mixed and incubated for 20 min at room temperature. 3.15 M perchloric acid and 20 % p-dimethylaminobenzaldehyde were added and samples were incubated for additional 20 min at 60 °C. The absorbance was determined at 557 nm.

### Immunohistochemistry for α-smooth muscle actin

The expression of α-smooth muscle actin (αSMA) was analyzed in paraffin embedded sections. After deparaffinization, samples were incubated with 3% bovine serum albumin followed by incubation with 3% H₂O₂. αSMA positive cells in mouse sections were detected by incubation with monoclonal anti-αSMA antibodies (clone 1A4, Sigma-Aldrich, Steinheim, Germany). Irrelevant isotype antibodies in the same concentration were used for control (Santa Cruz Biotechnology, Santa Cruz, CA, USA). Antibodies labeled with horseradish peroxidase (Dako, Hamburg, Germany) were used as secondary antibodies. The expression of the NICD and αSMA was visualized with DAB peroxidase substrate solution (Sigma-Aldrich). The number of myofibroblasts was counted from 4 different sections of lesional skin for each mouse by an examiner blinded to the treatment of the mice.

**Table 1: Effects of example 1A on development of Bleomycin-induced skin fibrosis.**

| | | a) Bleomycin + | b) Bleomycin + | c) Bleomycin + |
|---|---|---|---|---|
| | | vehicle | 1 mg/kg example 1A | 3mg/kg example 1A |
| Dermal thickness | | 1.70 | 1.37 | 1.19 |
| Collagen content | | 1.31 | 1.19 | 1.11 |
| Myofibroblast count | | 3.72 | 3.23 | 1.90 |

Fibrosis parameters expressed as x-fold change with respect to vehicle-treated control

### Example B

### Bleomycin-induced skin fibrosis

Local skin fibrosis was induced in 6-week-old, pathogen-free, female DBA/2 mice (Charles River, Sulzfeld, Germany) by repeated (every other day) subcutaneous injections of bleomycin (0,5 mg/ml in saline) in a defined area of the upper back. Control mice were injected in the same manner with saline only. For all groups the injection volume was 100 µl. The study comprises 4 arms with
a) mice receiving saline injection for 6 weeks (serving as reference)
b) mice receiving Bleomycin injection for 6 weeks
c) mice receiving Bleomycin injection for 6 weeks and additional treatment with example 1A (3 mg/kg) twice a day via gavage for the last 3 weeks
d) mice receiving the first 3 weeks bleomycin injections and the second 3 weeks saline injection.

After 6 weeks the animals were sacrificed and skin samples were obtained for analysis.

Histological analysis, hydroxyproline assay and immunhistochemistry for α-smooth muscel actin were performed as described in the Example 1A section.

**Table 3: Effects of example 1A (3mg/kg p.o.) on established Bleomycin-induced skin fibrosis Fibrosis parameters expressed as x-fold change with respect to vehicle-treated control (group a)**

| | | b) Bleomycin | c) Bleomycin 6 weeks + | d) Bleomycin 3 weeks + |
|---|---|---|---|---|
| | | 6 weeks | 3 weeks example 1A | 3 weeks NaCl |
| Dermal thickness | | 1.57 | 1.26 | 1.40 |
| | | | | |
| Myofibroblast count | | 3.87 | 1.68 | 3.50 |

### Example C

### Tight skin mouse model

In addition to the mouse model of bleomycin-induced dermal fibrosis, the tight-skin (Tsk-1) mouse model of systemic sclerosis was used to evaluate the anti-fibrotic potential of test drugs. Due to a dominant mutation in fibrillin-1, the phenotype of Tsk-1 is characterized by an increased hypodermal thickness. Genotyping of Tsk-1 mice was performed by PCR with the following primers: mutated fibrillin-1/ Tsk-1 forward primer: 5' - GTTGGCAACTATACCTGCAT - 3', reverse primer: 5' - CCTTTCCTGGTAACATAGGA - 3'. Tsk-1 mice were treated daily with test drug or vehicle, respectively, by oral gavage. In addition, a group of corresponding wild type(pa/pa) mice was treated with vehicle. The treatment was started at an age of five weeks. After five weeks of treatment, mice were sacrificed by cervical dislocation and skin samples were obtained for analysis.

Histological analysis, hydroxyproline assay and immunhistochemistry for α-smooth muscel actin were performed as described in the Example 1A section.

**Table 4: Effects of example 1A on established skin fibrosis in Tsk-mice**

| | | Tsk-1 + | Tsk-1 + | Tsk-1 + |
|---|---|---|---|---|
| | | vehicle | 1 mg/kg example 1A | 3mg/kg example 1A |
| hypodermal thickness | | 5.03 | 3.46 | 2.88 |
| Collagen content | | 2.46 | 1.61 | 1.67 |
| Myofibroblast count | | 2.64 | 2.12 | 1.70 |

Fibrosis parameters expressed as x-fold change with respect to vehicle-treated wild type mice

### Example D

The haemodynamic effects of i.e. example (1A) were analyzed in conscious mice. Telemetric implants (DSI®) were used. Signals were received with RMC1-DSI® receiver plates, compiled and analyzied with PONEMAH® physiology platform software.

The mice received either placebo (tylose), 0.3 mg/kg example 1A, 1mg/kg example 1A, 3mg/kg example 1A (Figure 1). The blood pressure and heart rate was monitored before and after application of placebo or the compounds. Figure 1 shows effects of example 1A on blood pressure (left) and heart rate (right).

### Example E

The effects of example 1A and vardenafil as standalone and in combination were analyzed in vitro in human dermal fibroblasts in vitro. Example 1A, vardenafil and combinations thereof significantly blocked the TGFbeta-induced Collagen gene expression and Hydroxyproline (HP) deposition.

### References:

Evgenov OV, Pacher P, Schmidt PM et al. (2006): NO-independent stimulators and activators of soluble guanylate cyclase: discovery and therapeutic potential. Nat. Rev. Drug. Discov. 5(9):755-68
Ferrini MG, Kovanecz I, Nolazco G (2006): Effects of long-term vardenafil treatment on the development of fibrotic plaques in a rat model of Peyronie's disease. B. J. U. 97:625-633.
Harris ED, et al. (2005): Kelley's Textbook of Rhematology 7th edition. Elsevier Saunders, Philadelphia PA
Kaplan SA, Gonzalez RR (2007): Phosphodiesterase type 5 inhibitors for the treatment of male lower urinary tract symptoms. Rev. Urol. 9(2):73-77
Khanna D and Denton CP (2010) Evidence-based management of rapidly progressing systemic sclerosis. Best. Pract. Res. Clin. Rheumatol. 24:387-400
Knorr A, Hirth-Dietrich C, Alonso-Alija C. et al. (2008): Nitric oxide-independent activation of soluble guanylate cyclase by BAY 60-2770 in experimental liver fibrosis. Arzneimittelforschung 58:71-80.
MVary K K. T. McVary, W. Monnig, J. L. Camps, Jr., J. M. Young, L. J. Tseng and G. van den Ende (2007): Sildenafil citrate improves erectile function and ur inary symptoms in men with erectile dysfunction and lower urinary tract symptoms associated with benign prostatic hyperplasia: a randomized, double-blind trial. J. Urol. 177: 1071-1077.
McVary KT, Roehrborn CG, Kaminetsky JC, Auerbach SM, Wachs B, Young JM, Esler A, Sides GD, Denes BS. (2007): Tadalafil relieves lower urinary tract symptoms secondary to benign prostatic hyperplasia. J Urol. 177:1401-1407.
Ong VH and Denton CP (2010): Innovative therapies for systemic sclerosis Curr. Opin. Rheumatol. 22:264-272.
Porst H, Sandner P, Ulbrich E. (2008): Vardenafil in the treatment of lower urinary tract symptoms secondary to benign prostatic hyperplasia. Curr. Urol. Rep. 9:295-301.
Sandner P, Neuser D, Bischoff E (2009): Erectile dysfunction and lower urinary tract. Handb. Exp. Pharmacol. 191:507-531.
Spiera R, Gordon J, Mersten J, Magro C, Mehta M, Wildmann H, Kloiber S, Kirou K, Lyman S, Crow M (2011): Imatinib mesylate (Gleevec) in the treatment of diffuse cutaneous systemic sclerosis: results of a 1year, phse IIa, single-arm open-label clinical trial. Ann. Rheum. Dis. Epub Mar 11, 2011

## Claims

1. Compounds according to examples of compound classes I to X
compound class I
| Example | IUPAC NAME |
|---|---|
| 1 | 4-amino-2-[5-chloro-3-(3,3,3-trifluoropropyl)-1*H-*indazol-1-yl]-5,5-dimethyl-5,7-dihydro-6*H*-pyrrolo[2,3-*d*]pyrimidin-6-one |
| 2 | 4-amino-5,5-dimethyl-2-[3-(3,3,3-trifluoropropyl)-1*H*-indazol-1-yl]-5,7-dihydro-6*H-*pyrrolo[2,3-*d*]pyrimidin-6-one |
| 3 | 4-amino-2-[5-chloro-3-(2,3,6-trifluorobenzyl)-1*H*-indazol-1-yl]-5,5-dimethyl-5,7-dihydro-6*H*-pyrrolo[2,3-*d*]pyrimidin-6-one |
| 4 | 4-amino-5,5-dimethyl-2-[3-(2,3,6-trifluorobenzyl)-1*H*-indazol-1-yl]-5,7-dihydro-6*H-*pyrrolo[2,3-*d*]pyrimidin-6-one |
| 5 | 4-amino-2-(5-fluoro-3-hexyl-1*H-*indazol-1-yl)-5,5-dimethyl-5,7-dihydro-6*H-*pyrrolo[2-*d*]pyrimidin-6-one |
| 6 | 4-amino-2-[5-bromo-3-(2,3,6-trifluorobenzyl)-1*H-*indazol-1-yl]-5,5-dimethyl-5,7-dihydro-6*H*-pyrrolo[2,3-*d*]pyrimidin-6-one |
| 7 | 4-amino-5,5-dimethyl-2-[5-pyridin-4-yl-3-(2,3,6-trifluorobenzyl)-1*H*-indazol-1-yl]-5,7-dihydro-6*H*-pyrrolo[2,3-*d*]pyrimidin-6-one |
| 8 | 4-amino-5,5-dimethyl-2-[3-(4,4,4-trifluorobutyl)-1*H*-thieno[3,4-*c*]pyrazol-1-yl]-5,7-dihydro-6*H*-pyrrolo[2,3-*d*]pyrimidin-6-one |
| 9 | 4-amino-5,5-dimethyl-2-[3-(2,3,6-trifluorobenzyl)-1*H*-thieno[3,4-*c*]pyrazol-1-yl]-5,7-dihydro-6*H*-pyrrolo[2,3-*d*]pyrimidin-6-one |
| 10 | 4-amino-5,5-dimethyl-2-[3-(2,3,6-trifluorobenzyl)-4,6-dihydro-1*H*-thieno[3,4-*c*]pyrazol-1-yl]-5,7-dihydro-6*H*-pyrrolo[2,3-*d*]pyrimidin-6-one |
| 11 | 4-amino-2-[3-(2-cyclopentylethyl)-1*H*-indazol-1-yl]-5,5-dimethyl-5-7-dihydro-6*H-*pyrrolo[2,3-*d*]pyrimidin-6-one |
| 12 | 4-amino-2-[3-(2-fluorobenzyl)-1*H*-indazol-1-yl]-5,5-dimethyl-5-7-dihydro-6*H-*pyrrolo[2,3-*d*]pyrimidin-6-one |
| 13 | 4-amino-2-[5-chloro-3-(2-fluorobenzyl)-1*H*-indazol-1-yl]-5,5-dimethyl-5-7-dihydro-6*H-*pyrrolo[2,3-*d*]pyrimidin-6-one |
| 14 | 4-amino-2-[5-fluoro-3-(2-fluorobenzyl)-1*H*-indazol-1-yl]-5,5-dimethyl-5-7-dihydro-6*H-*pyrrolo[2,3-*d*]pyrimidin-6-one |
| 15 | 4-amino-2-[5-chloro-3-(2,3-difluorobenzyl)-1*H*-indazol-1-yl]-5,5-dimethyl-5-7-dihydro-6*H*-pyrrolo[2,3-*d*]pyrimidin-6-one |
| 16 | 4-amino-2-[3-(2,3-difluorobenzyl)-5-fluoro-1*H*-indazol-1-yl]-5,5-dimethyl-5-7-dihydro-6*H*-pyrrolo[2,3-*d*]pyrimidin-6-one |
| 17 | 4-amino-2-[3-(2,3-difluorobenzyl)-1*H*-indazol-1-yl]-5,5-dimethyl-5-7-dihydro-6*H-*pyrrolo[2,3-*d*]pyrimidin-6-one |
| 18 | 4-amino-2-[3-(2-fluorobenzyl)-5-phenyl-1*H*-indazol-1-yl]-5,5-dimethyl-5-7-dihydro-6*H-*pyrrolo[2,3-*d*]pyrimidin-6-one |
| 19 | 4-amino-2-[5-fluoro-3-(2,3,6-trifluorobenzyl)-1*H*-indazol-1-yl]-5,5-dimethyl-5-7-dihydro-6*H-*pyrrolo[2,3-*d*]pyrimidin-6-one |
| 20 | 4-amino-5-5-dimethyl-2-[5-pyridin-3-yl-3-(2,3,6-trifluorobenzyl)-1*H*-indazol-1-yl]-5-7-dihydro-6*H-*pyrrolo[2,3-*d*]pyrimidin-6-one |
| 21 | 4-amino-5-5-dimethyl-2-[5-(1-methyl-1*H*-pyrazol-4-yl)-3-(2,3,6-trifluorobenzyl)-1*H-*indazol-1-yl]-5-7-dihydro-6*H-*pyrrolo[2,3-*d*]pyrimidin-6-one |
| 22 | 4-amino-2-[5-(3-5-dimethyl-1*H*-pyrazol-4-yl)-3-(2,3,6-trifluorobenzyl)-1*H*-indazol-1-yl]-5,5-dimethyl-5-7-dihydro-6*H-*pyrrolo[2,3-*d*]pyrimidin-6-one |
| 23 | 4-amino-2-[5-(3-furyl)-3-(2,3,6-trifluorobenzyl)-1*H*-indazol-1-yl]-5,5-dimethyl-5-7-dihydro-6*H-*pyrrolo[2,3-*d*]pyrimidin-6-one |
| 24 | 4-amino-5,5-dimethyl-2-[5-(4-methyl-3-thienyl)-3-(2,3,6-trifluorobenzyl)-1*H*-indazol-1-yl]-5-7-dihydro-6*H-*pyrrolo[2,3-*d*]pyrimidin-6-one |
| 25 | 4-amino-2-[5-cyclopropyl-3-(2,3,6-trifluorobenzyl)-1*H*-indazol-1-yl]-5,5-dimethyl-5-7-dihydro-6*H-*pyrrolo[2,3-*d*]pyrimidin-6-one |
| 26 | 4-amino-5,5-dimethyl-2-[5-pyridin-4-yl-3-(2,3,6-trifluorobenzyl)-1*H*-indazol-1-yl]-5-7-dihydro-6*H-*pyrrolo[2,3-*d*]pyrimidin-6-one |
| 27 | 4-amino-5,5-dimethyl-2-[5-phenyl-3-(2,3,6-trifluorobenzyl)-1*H*-indazol-1-yl]-5-7-dihydro-6*H-*pyrrolo[2,3-*d*]pyrimidin-6-one |
| 28 | 4-amino-2-[5-chloro-3-(pyrimidin-5-ylmethyl)-1*H*-indazol-1-yl]-5,5-dimethyl-5-7-dihydro-6*H-*pyrrolo[2,3-*d*]pyrimidin-6-one |
| 29 | 4-amino-5,5-dimethyl-2[5-(3-thienyl)-3-(2,3,6-trifluorobenzyl)-1*H*-indazol-1-yl]-5-7-dihydro-6*H-*pyrrolo[2,3-*d*]pyrimidin-6-one |
| 30 | 4-amino-2-[5-(5-fluoropyridin-3-yl)-3-(2,3,6-trifluorobenzyl)-1*H*-indazol-1-yl]-5,5-dimethyl-5,7-dihydro-6*H-*pyrrolo[2,3-*d*]pyrimidin-6-one |
| 31 | 4-amino-2-[5-(6-fluoropyridin-3-yl)-3-(2,3,6-trifluorobenzyl)-1*H*-indazol-1-yl]-5,5-dimethyl-5,7-dihydro-6*H-*pyrrolo[2,3-*d*]pyrimidin-6-one |
| 32 | 4-amino-5,5-dimethyl-2-[3-(2,3,6-trifluorobenzyl)-5-[5-(trifluoromethyl)pyridin-3-yl]-1*H*-indazol-1-yl]-5,7-dihydro-6*H-*pyrrolo[2,3-*d*]pyrimidin-6-one |
| 33 | 4-amino-2-[3-(6-bromo-2,3-difluorobenzyl)-5-chloro-1*H*-indazol-1-yl]-5,5-dimethyl-5,7-dihydro-6*H-*pyrrolo[2,3-*d*]pyrimidin-6-one |
| 34 | 4-amino-2-[3-(2-cyclopentylethyl)-1*H*-indazol-1-yl]-5,5-dimethyl-5,7-dihydro-6*H-*pyrrolo[2,3-*d*]pyrimidin-6-one |
| 35 | 4-amino-2-(5-fluoro-3-pentyl-1*H*-indazol-1-yl)-5,5-dimethyl-5,7-dihydro-6*H-*pyrrolo[2,3-*d*]pyrimidin-6-one |
| 36 | 4-amino-2-[5-fluoro-3-(3,3,3-trifluoropropyl)-1*H*-indazol-1-yl]-5,5-dimethyl-5,7-dihydro-6*H-*pyrrolo[2,3-*d*]pyrimidin-6-one |
| 37 | 4-amino-2-[3-(2-cyclopentylethyl)-5-fluoro-1*H*-indazol-1-yl]-5,5-dimethyl-5,7-dihydro-6*H-*pyrrolo[2,3-*d*]pyrimidin-6-one |
| 38 | 4-amino-2-[3-(2-cyclopentylethyl)-5-fluoro-1*H*-indazol-1-yl)-5,5-dimethyl-5,7-dihydro-6*H-*pyrrolo[2,3-*d*]pyrimidin-6-one |
| 39 | 4-amino-2-[5-fluoro-3-(4,4,4-trifluorobutyl)-1*H*-indazol-1-yl]-5,5-dimethyl-5,7-dihydro-6*H-*pyrrolo[2,3-*d*]pyrimidin-6-one |
| 40 | 4-amino-2-(5-chloro-3-pentyl-1*H*-indazol-1-yl)-5,5-dimethyl-5,7-dihydro-6*H-*pyrrolo[2,3-*d*]pyrimidin-6-one |
| 41 | 4-amino-2-(3-butyl-5-chloro-1*H*-indazol-1-yl)-5,5-dimethyl-5,7-dihydro-6*H*-pyrrolo[2 *d*]pyrimidin-6-one |
| 42 | 4-amino-2-[5-chloro-3-(4,4,4-trifluorobutyl)-1*H*-indazol-1-yl]-5,5-dimethyl-5,7-dihydro-6*H*-pyrrolo[2,3-*d*]pyrimidin-6-one |
| 43 | 4-amino-2-(5-chloro-3-pent-4-on-1-yl-1*H*-indazol-1-yl)-5,5-dimethyl-5,7-dihydro-6*H-*pyrrolo[2,3-*d*]pyrimidin-6-one |
| 44 | 4-amino-2-(3-but-3-en-1yl-5-chloro-1*H*-indazol-1-yl)-5,5-dimethyl-5,7-dihydro-6*H-*pyrrolo[2,3-*d*]pyrimidin-6-one |
| 45 | 4-amino-2-(5-chloro-3-propyl-1*H*-indazol-1-yl)-5,5-dimethyl-5,7-dihydro-6*H-*pyrrolo[2,3-*d*]pyrimidin-6-one |
| 46 | ethyl 3-[1-(4-amino-5,5-dimethyl-6-oxo-6,7-dihydro-5*H*-pyrrolo[2,3-*d*]pyrimidin-2-yl) 5-chloro-1*H*-indazol-3-yl]propanoate |
| 47 | 4-amino-2-[5-chloro-3-(3,3-dimethylbutyl)-1*H*-indazol-1-yl]-5,5-dimethyl-5,7-dihydro 6*H-*pyrrolo[2,3-*d*]pyrimidin-6-one |
| 48 | 4-amino-2-[3-(2,3-difluorobenzyl)-1*H-*thieno[3,4-*c*]pyrazol-1-yl]-5,5-dimethyl-5,7-dihydro-6*H-*pyrrolo[2,3-*d*]pirimidin-6-one |
| 49 | 4-amino-2-[6-chloro-3-(2,3-difluorobenzyl)-1*H*-thieno[3,4-*c*]pyrazol-1-yl]-5,5-dimethy 5,7-dihydro-6*H-*pyrrolo[2,3-*d*]pyrimidin-6-one |
| 50 | 4-amino-2-[5-chloro-3-(2,3-difluorobenzyl)-1*H*-thieno[2,3-*c*]pyrazol-1-yl]-5,5-dimethyl-5,7-dihydro-6*H*-pyrrolo[2,3-*d*]pyrimidin-6-one |
| 51 | 4-amino-2-(3-(2,3-difluorobenzyl)-1*H*-thieno[3,2-*c*]pyrazol-1-yl]-5,5-dimethyl-5,7-dihydro-6*H*-pyrrolo[2,3-*d*]pyrimidin-6-one |
| 52 | 4-amino-[5-chloro-3-(2,3,6-trifluorobenzyl)-1*H*-thieno[2,3-c]pyrazol-1-yl]-5,5-dimethyl-5,7-dihydro-6*H*-pyrrolo[2,3-*d*]pyrimidin-6-one |
| 53 | 4-amino-5,5-dimethyl-2-[3-(2,3,6-trifluorobenzyl)-1*H*-thieno[3,2-*c*]pyrazol-1-yl]-5,7-dihydro-6*H*-pyrrolo[2,3-*d*]pyrimidin-6-one |
| 54 | 4-amino-5,5-dimethyl-2-[5-methyl-3-(2,3,6-trifluorobenzyl)pyrazolo[4,3-*c*]pyrazol-1(5*H*)-yl]-5,7-dihydro-6*H*-pyrrolo[2,3-*d*]pyrimidin-6-one |
| 55 | 4-amino-5,5-dimethyl-2-[3-(2,3,6-trifluorobenzyl)-1*H*-thieno[2,3-*c*]pyrazol-1-yl]-5,7-dihydro-6*H*-pyrrolo[2,3-*d*]pyrimidin-6-one |
| 56 | 4-amino-5,5-dimethyl-2-[6-methyl-3-(2,3,6-trifluorobenzyl)pyrazolo[3,4-*c*]pyrazol-1(6*H*)-yl]-5,7-dihydro-6*H*-pyrrolo[2,3-*d*]pyrimidin-6-one |
| 57 | 4-amino-5,5-dimethyl-2-[3-(2,3,6-trifluorobenzyl)-1*H*-pyrazolo[4,3-*c*]pyridin-1-yl]-5,7-dihydro-6*H*-pyrrolo[2,3-*d*]pyrimidin-6-one |
| 58 | 4-amino-5,5-dimethyl-2-[7-(2,3,6-trifluorobenzyl)imidazo[1,5-*b*]pyridazin-5-yl]-5,7-dihydro-6*H*-pyrrolo[2,3-*d*]pyrimidin-6-one |
| 59 | 4-amino-2-[6-chloro-3-(2,3,6-trifluorobenzyl)imidazo[1,5-*a*]pyridin-1-yl]-5,5-dimethyl-5,7-dihydro-6*H*-pyrrolo[2,3-*d*]pyrimidin-6-one |
| 60 | 4-amino-2-[6-fluoro-3-(2,3,6-trifluorobenzyl)imidazo[1,5-*a*]pyridin-1-yl]-5,5-dimethyl-5,7-dihydro-6*H*-pyrrolo[2,3-*d*]pyrimidin-6-one |
| 61 | 4-amino-5,5-dimethyl-2-[5-(2,3,6-trifluorobenzyl)imidazo[5,1-*b*][1,3]thiazol-7-yl]-5,7-dihydro-6*H*-pyrrolo[2,3-*d*]pyrimidin-6-one |
| 62 | 4-amino-5,5-dimethyl-2-[1-(2,3,6-trifluorobenzyl)imidazo[1,5-*a*]pyridin-3-yl]-5,7-dihydro-6*H*-pyrrolo[2,3-*d*]pyrimidin-6-one |
| 63 | 4-amino-2-[3-(2,3,-difluorobenzyl)imidazo[1,5-*a*]pyridin-1-yl]-5,5-dimethyl-5,7-dihydro-6*H*-pyrrolo[2,3-*d*]pyrimidin-6-one |
| 64 | 4-amino-2-[7-(2,3-difluorobenzyl)imidazo[1,5-*b*]pyridazin-5-yl]-5,5-dimethyl-5,7-dihydro-6*H*-pyrrolo[2,3-*d*]pyrimidin-6-one |
| 65 | 4-amino-2-[3-(2,3-difluorobenzyl)-6-fluoroimidazo[1,5-*a*]pyridin-1-yl]-5,5-dimethyl-5,7-dihydro-6*H*-pyrrolo[2,3-*d*]pyrimidin-6-one |
| 66 | 4-amino-2-[7-(2-fluorobenzyl)imidazo[1,5-*b*]pyridazin-5-yl]-5,5-dimethyl-5,7-dihydro-6*H*-pyrrolo[2,3-*d*]pyrimidin-6-one |
| 67 | 4-amino-2-[3-(2-fluorobenzyl)imidazo[1,5-*a*]pyridin-1-yl]-5,5-dimethyl-5,7-dihydro-6*H-*pyrrolo[2,3-*d*]pyrimidin-6-one |
| 68 | 4-amino-2-[6-fluoro-3-(2-fluorobenzyl)imidazo[1,5-*a*]pyridin-1-yl]-5,5-dimethyl-5,7-dihydro-6*H*-pyrrolo[2,3-*d*]pyrimidin-6-one |
| 69 | 4-amino-2-[6-chloro-3-(2-fluorobenzyl)imidazo[1,5-*a*]pyridin-1-yl]-5,5-dimethyl-5,7-dihydro-6*H*-pyrrolo[2,3-*d*]pyrimidin-6-one |
| 70 | 4-amino-2-[7-(2,3-difluorobenzyl)-2-methylimidazo[1,5-*b*]pyridazin-5-yl]-5,5-dimethyl-5,7-dihydro-6*H*-pyrrolo[2,3-*d*]pyrimidin-6-one |
| 71 | 4-amino-2-[6-chloro-3-(2,3-difluorobenzyl)imidazo[1,5-*a*]pyridin-1-yl]-5,5-dimethyl-5,7-dihydro-6*H*-pyrrolo[2,3-*d*]pyrimidin-6-one |
| 72 | 4-amino-2-(3-benzylimidazo[1,5-*a*]pyridin-1-yl)-5,5-dimethyl-5,7-dihydro-6*H-*pyrrolo[2,3-*d*]pyrimidin-6-one |
| 73 | 4-amino-5,5-dimethyl-2-[3-(2,3,6-trifluorobenzyl)imidazo[1,5-*a*]pyridin-1-yl]-5,7-dihydro-6*H*-pyrrolo[2,3-*d*]pyrimidin-6-one |
| 74 | 4-amino-5,5-dimethyl-2-[6-phenyl-3-(2,3,6-trifluorobenzyl)imidazo[1,5-*a*]pyridin-1-yl 5,7-dihydro-6*H*-pyrrolo[2,3-*d*]pyrimidin-6-one |
| 75 | 4-amino-2-[6-(2-fluorophenyl)-3-(2,3,6-trifluorobenzyl)imidazo[1,5-*a*]pyridin-1-yl]-5, dimethyl-5,7-dihydro-6*H*-pyrrolo[2,3-*d*]pyrimidin-6-one |
| 76 | 4-amino-2-[6-(3-fluorophenyl)-3-(2,3,6-trifluorobenzyl)imidazo[1,5-*a*]pyridin-1-yl]-5, dimethyl-5,7-dihydro-6*H*-pyrrolo[2,3-*d*]pyrimidin-6-one |
| 77 | 4-amino-2-[6-(4-fluorophenyl)-3-(2,3,6-trifluorobenzyl)imidazo[1,5-*a*]pyridin-1-yl]-5, dimethyl-5,7-dihydro-6*H*-pyrrolo[2,3-*d*]pyrimidin-6-one |
| 78 | 4-amino-2-[6-(3-chlorophenyl)-3-(2,3,6-trifluorobenzyl)imidazo[1,5-*a*]pyridin-1-yl]-5, dimethyl-5,7-dihydro-6*H*-pyrrolo[2,3-*d*]pyrimidin-6-one |
| 79 | 4-amino-5,5-dimethyl-2-[6-(3-thienyl)-3-(2,3,6-trifluorobenzyl)imidazo[1,5-*a*]pyridin-yl]-5,7-dihydro-6*H*-pyrrolo[2,3-*d*]pyrimidin-6-one |
| 80 | 4-amino-2-[6-cyclopropyl-3-(2,3,6-trifluorobenzyl)imidazo[1,5-*a*]pyridin-1-yl]-5,5-dimethyl-5,7-dihydro-6*H*-pyrrolo[2,3-*d*]pyrimidin-6-one |
| 81 | 4-amino-5,5-dimethyl-2-[7-(3,3,3-trifluoropropyl)imidazo[1,5-*b*]pyridazin-5-yl]-5,7-dihydro-6*H*-pyrrolo[2,3-*d*]pyrimidin-6-one |
| 82 | 4-amino-5,5-dimethyl-2-[3-(2,4,6-trifluorobenzyl)imidazo[1,5-*a*]pyridin-1-yl]-5,7-dihydro-6*H*-pyrrolo[2,3-*d*]pyrimidin-6-one |
| 83 | 4-amino-2-[3-(2-chloro-6-fluoro-3-methylbenzyl)imidazo[1,5-*a*]pyridin-1-yl]-5,5-dimethyl-5,7-dihydro-6*H*-pyrrolo[2,3-*d*]pyrimidin-6-one |
| 84 | 4-amino-2-[3-(2-cyclopentylethyl)imidazo[1,5-*a*]pyridin-1-yl]-5,5-dimethyl-5,7-dihydro-6*H*-pyrrolo[2,3-*d*]pyrimidin-6-one |
| 85 | 4-amino-5,5-dimethyl-2-[7-(4,4,4-trifluorobutyl)imidazo[1,5-*b*]pyridin-5-yl]-5,7-dihydro-6*H*-pyrrolo[2,3-*d*]pyrimidin-6-one |
| 86 | 4-amino-5,5-dimethyl-2-[3-(4,4,4-trifluorobutyl)imidazo[1,5-*a*]pyridin-1-yl]-5,7-dihydro-6*H*-pyrrolo[2,3-*d*]pyrimidin-6-one |
| 87 | 4-amino-5,5-dimethyl-2-{3-[2-(2-thienyl)ethyl]imidazo[1,5-*a*]pyridin-1-yl}-5,7-dihydro-6*H*-pyrrolo[2,3-*d*]pyrimidin-6-one |
| 88 | 4-amino-2-[3-(2-cyclopropylethyl)imidazo[1,5-*a*]pyridin-1-yl]-5,5-dimethyl-5,7-dihydro-6*H*-pyrrolo[2,3-*d*]pyrimidin-6-one |
| 89 | 4-amino-5,5-dimethyl-2-(3-pentylimidazo[1,5-*a*]pyridin-1-yl)-5,7-dihydro-6*H-*pyrrolo[2,3-*d*]pyrimidin-6-one |
| 90 | 4-amino-5,5-dimethyl-2-(7-pentylimidazo[1,5-*b*]pyridin-5-yl)-5,7-dihydro-6*H-*pyrrolo[2,3-*d*]pyrimidin-6-one |
| 91 | 4-amino-5,5-dimethyl-2-[3-(3-methylbutyl)imidazo[1,5-*a*]pyridin-1-yl]-5,7-dihydro-6*H-*pyrrolo[2,3-*d*]pyrimidin-6-one |
| 92 | 4-amino-5,5-dimethyl-2-[3-methyl-5-(2,3,6-trifluorobenzyl)imidazo[5,1-*b*][1,3]thiazol 7-yl]-5,7-dihydro-6*H*-pyrrolo[2,3-*d*]pyrimidin-6-one |
| 93 | 4-amino-5,5-dimethyl-2-[2-methyl-5-(2,3,6-trifluorobenzyl)imidazo[5,1-*b*][1,3]thiazol 7-yl]-5,7-dihydro-6*H*-pyrrolo[2,3-*d*]pyrimidin-6-one |
| 94 | 4-amino-2-[5-(2-fluorobenzyl)imidazo[5,1-*b*][1,3]thiazol-7-yl)-5,5-dimethyl-5,7-dihydro-6*H*-pyrrolo[2,3-*d*]pyrimidin-6-one |
| 95 | 4-amino-5,5-dimethyl-2-[1-(3,3,3-trifluoropropyl)-1*H*-indazol-3-yl]-5,7-dimethyl-6*H-*pyrrolo[2,3-*d*]pyrimidin-6-one |
| 96 | 4-amino-2-[5-chloro-3-(2,3,6-trifluorobenzyl)-1*H*-indazol-1-yl]-5,5-dimethyl-5,8-dihydropyrido[2,3-*d*]pyrimidin-7(6*H*)-one |
| 97 | 4-amino-5,5-dimethyl-2-[3-(2,3,6-trifluorobenzyl)-1*H*-indazol-1-yl]-5,8-dihydropyrido[2,3-*d*]pyrimidin-7(6*H*)-one |
| 98 | 4-amino-5,5-dimethyl-2-[3-(2,3,6-trifluorobenzyl)-1*H*-thieno[3,4-*c*]pyrazol-1-yl]-5,8-dihydropyrido[2,3-*d*]pyrimidin-7(6*H*)-one |
| 99 | 4-amino-5,5-dimethyl-2-[3-(2,3,6-trifluorobenzyl)-4,6-dihydro-1*H*-thieno[3,4-*c*]pyrazol-1-yl]-5,8-dihydropyrido[2,3-*d*]pyrimidin-7(6*H*)-one |
| 100 | 4-amino-2-[5-chloro-3-(3,3,3-trifluoropropyl)-1*H*-indazol-1-yl]-5-ethyl-5-methyl-5,7-dihydro-6*H-*pyrrolo[2,3-*d*]pyrimidin-6-one |
| 101 | 4-amino-2-[5-chloro-3-(3,3,3-trifluoropropyl)-1*H*-indazol-1-yl]-5-methyl-5-propyl-5,7-dihydro-6*H-*pyrrolo[2,3-*d*]pyrimidin-6-one |
| 102 | 4-amino-2-[5-chloro-3-(3,3-dimethylbutyl)-1*H*-indazol-1-yl]-5-ethyl-5-methyl-5,7-dihydro-6*H-*pyrrolo[2,3-*d*]pyrimidin-6-one |
| 103 | 4-amino-5-ethyl-2-[3-(2-fluorobenzyl)imidazo[1,5-*a*]pyridin-1-yl]-5-methyl-5,7-dihydro-6*H-*pyrrolo[2,3-*d*]pyrimidin-6-one |
| 104 | 4-amino-5,5-dimethyl-2-[3-(3,3,4,4,4-pentafluorobutyl)-1*H*-indazol-1-yl]-5,7-dihydro-6*H-*pyrrolo[2,3-*d*]pyrimidin-6-one |
| 105 | 4-amino-2-[5-fluoro-3-(3,3,4,4,4-pentafluorobutyl)-1*H*-indazol-1-yl]-5,5-dimethyl-5,7-dihydro-6*H-*pyrrolo[2,3-*d*]pyrimidin-6-one |
| 106 | 4-amino-2-[5-chloro-3-(3,3,4,4,4-pentafluorobutyl)-1*H*-indazol-1-yl]-5,5-dimethyl-5,7 dihydro-6*H-*pyrrolo[2,3-*d*]pyrimidin-6-one |
| 107 | 4-amino-5,5-dimethyl-2-[3-(3,3,4,4,4-pentafluorobutyl)-1*H*-pyrazolo[4,3-*b*]pyridin-1-yl]-5,7-dihydro-6*H-*pyrrolo[2,3-*d*]pyrimidin-6-one |
| | |
| 108 | 4-amino-5,5-dimethyl-2-[3-(3,3,4,4,4-pentafluorobutyl)imidazo[1,5-*a*]pyridin-1-yl]-5,7 dihydro-6*H-*pyrrolo[2,3-*d*]pyrimidin-6-one |
| 109 | 4-amino-2-[6-fluoro-3-(3,3,4,4,4-pentafluorobutyl)imidazo[1,5-*a*]pyridin-1-yl]-5,5-dimetyl-5,7-dihydro-6*H-*pyrrolo[2,3-*d*]pyrimidin-6-one |
| 110 | 4-amino-2-[6-chloro-3-(3,3,4,4,4-pentafluorobutyl)imidazo[1,5-*a*]pyridin-1-yl]-5,5-dimethyl-5,7-dihydro-6*H-*pyrrolo[2,3-*d*]pyrimidin-6-one |
| 111 | 4-amino-2-[6-chloro-1-(3,3,4,4,4-pentafluorobutyl)1*H*-indazol-3-yl]-5,5-dimethyl-5,7 dihydro-6*H-*pyrrolo[2,3-*d*]pyrimidin-6-one |
compound class II
Example 1: 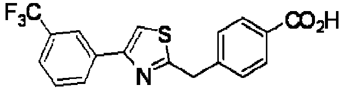 Example 2: 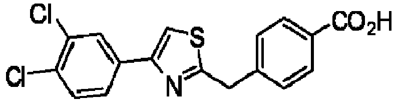 Example 3: 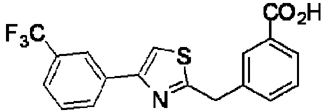 Example 4: 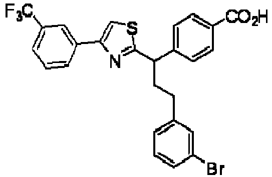 Example 5: 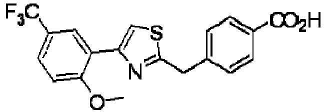 Example 6: 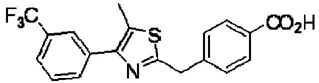 Example 7: 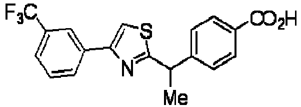 Example 8: 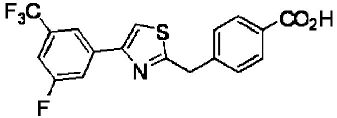 Example 9: 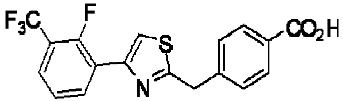 Example 10: 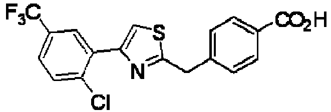 Example 11: 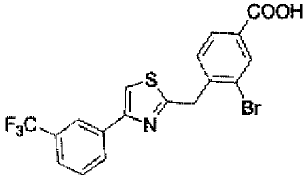 Example 12: 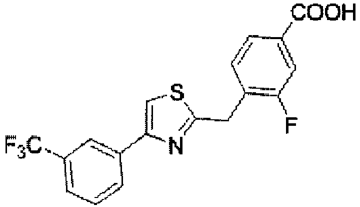 Example 13: 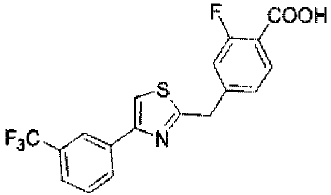 Example 14: 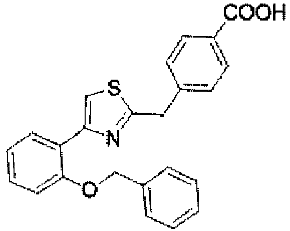 Example 15: 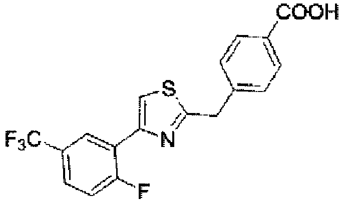 Example 16: 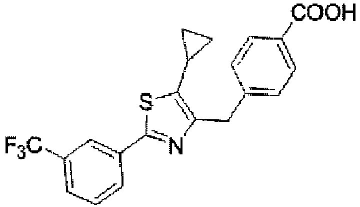 Example 17: 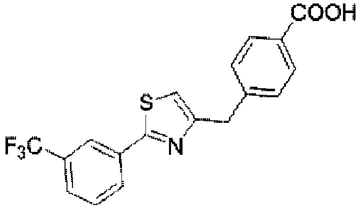 Example 18: 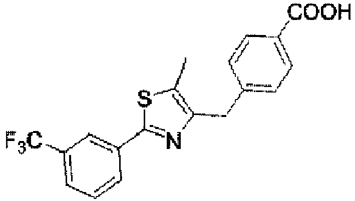 Example 19: 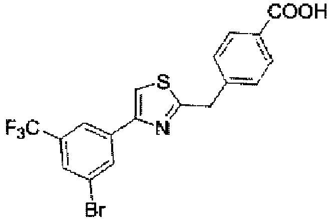 Example 20: 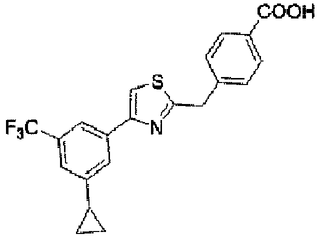 Example 21: 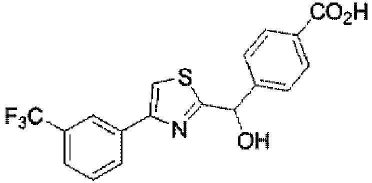 Example 22: 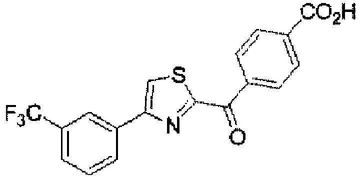 Example 23: 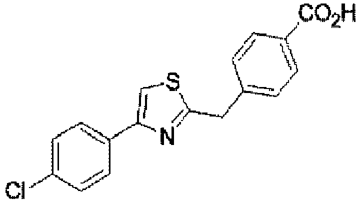 Example 24: 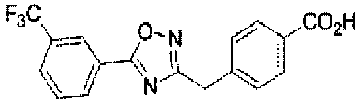 Example 25: 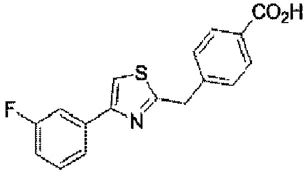 Example 26: 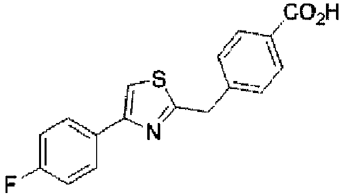 Example 27: 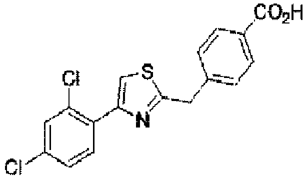 Example 28: 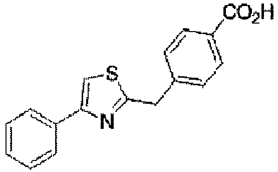 Example 29: 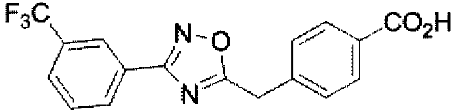 Example 30: 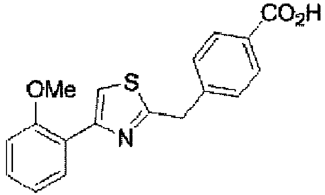 Example 31: 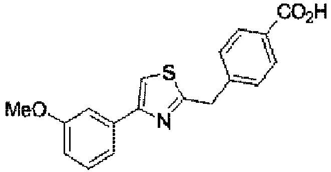 Example 32: 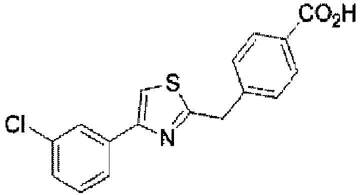 Example 33: 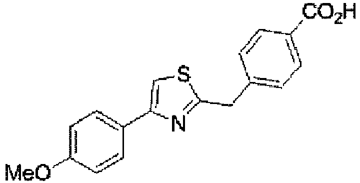 Example 34: 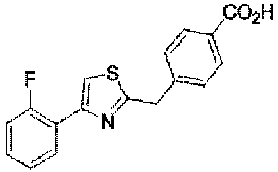 Example 35: 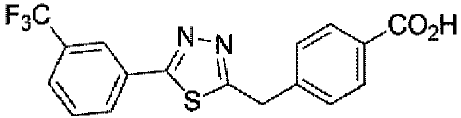 Example 36: 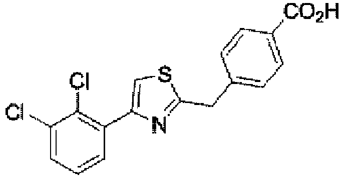 Example 37: 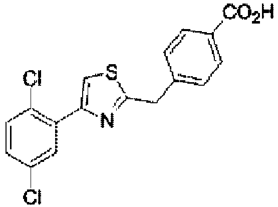 Example 38: 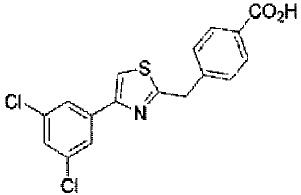 Example 39: 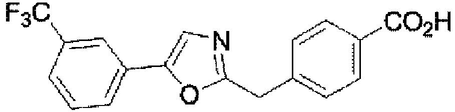 Example 40: 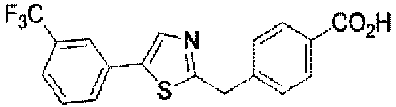 Example 41: 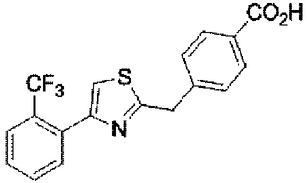 Example 42: 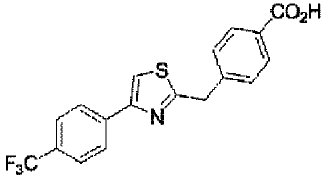 Example 43: 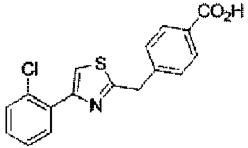 Example 44: 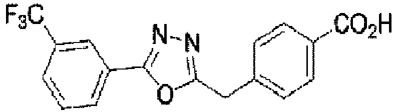 Example 45: 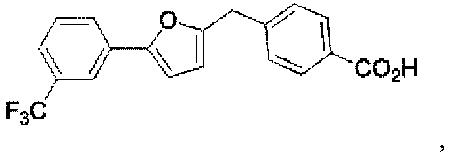 Compound class III
Example 1 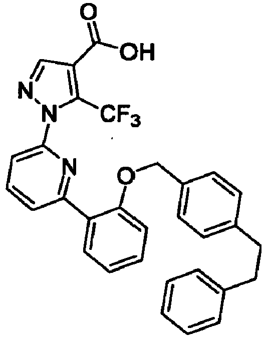 Example 2 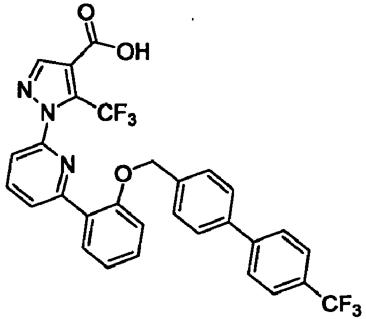 Example 3 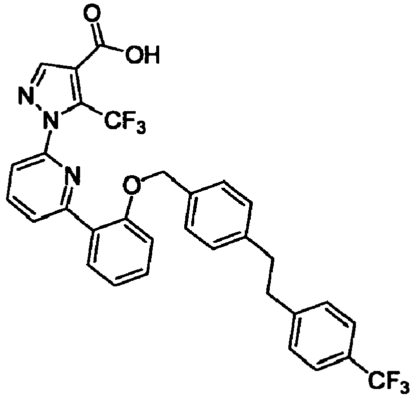 **Example 4** 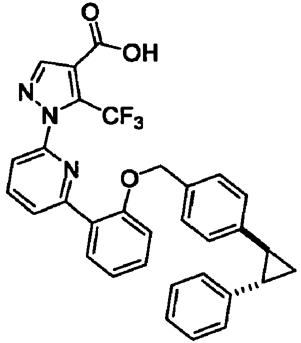 **Example 5** 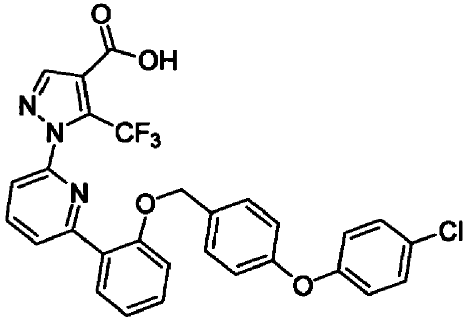 **Example 6** 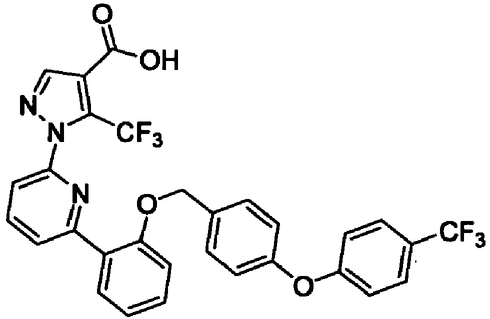 **Example 7** 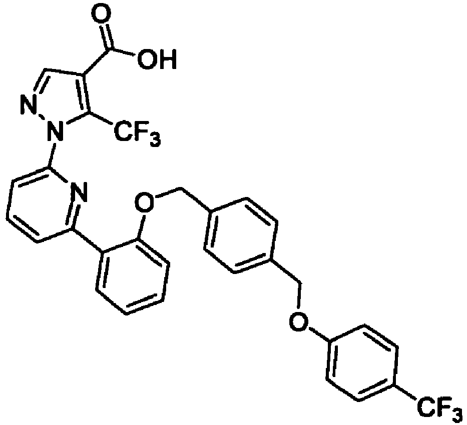 **Example 8** 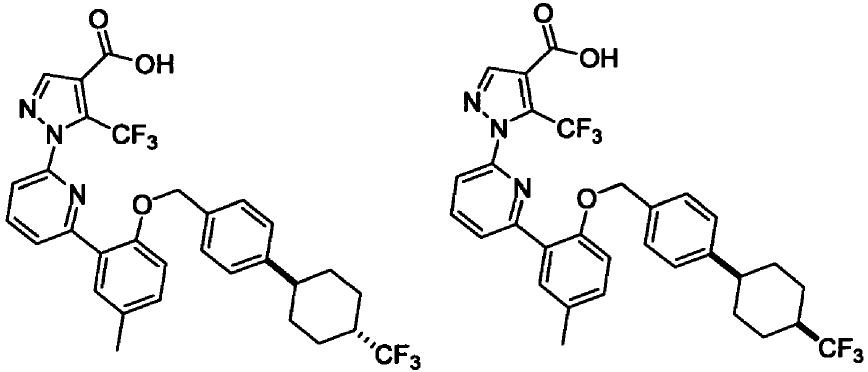 **Example 9** 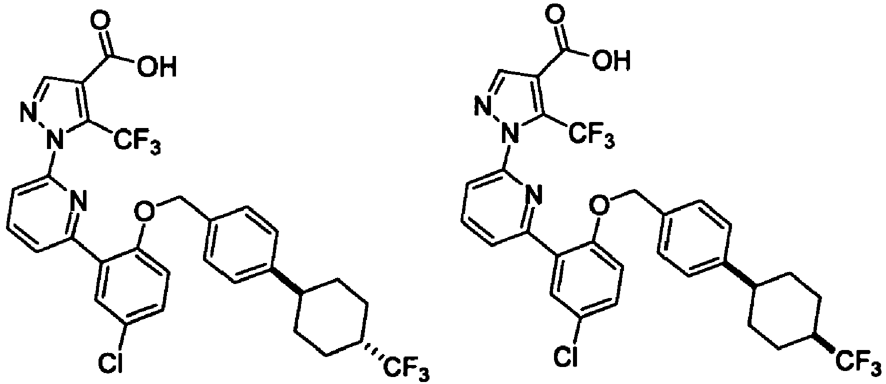 **Example 10** 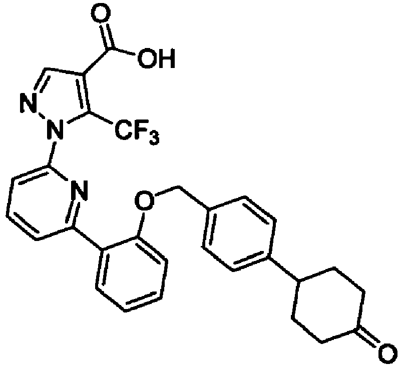 **Example 11** 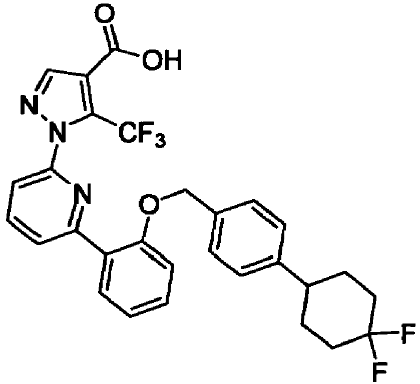 **Example 12** 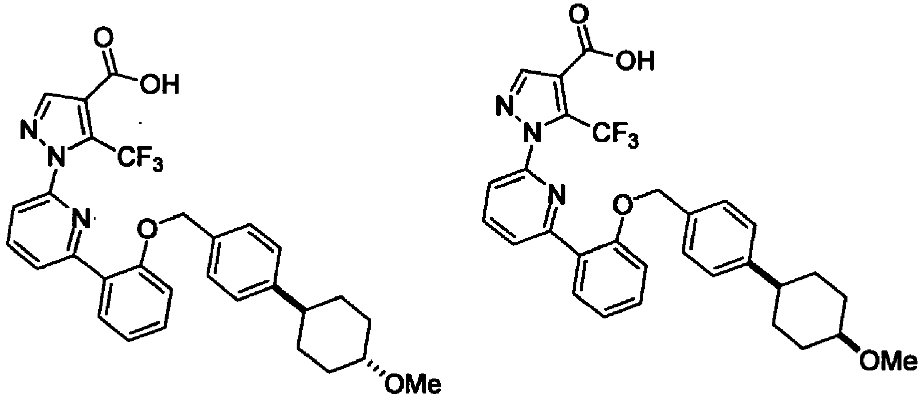 **Example 13** 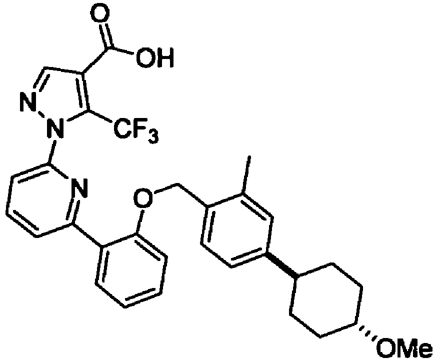 **Example 14** 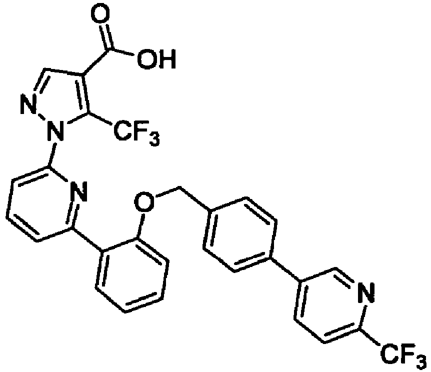 **Example 15** 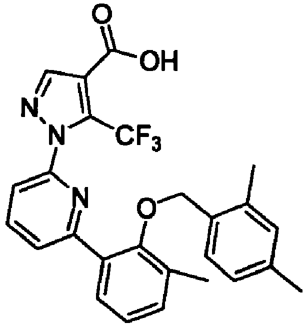 **Example 16** 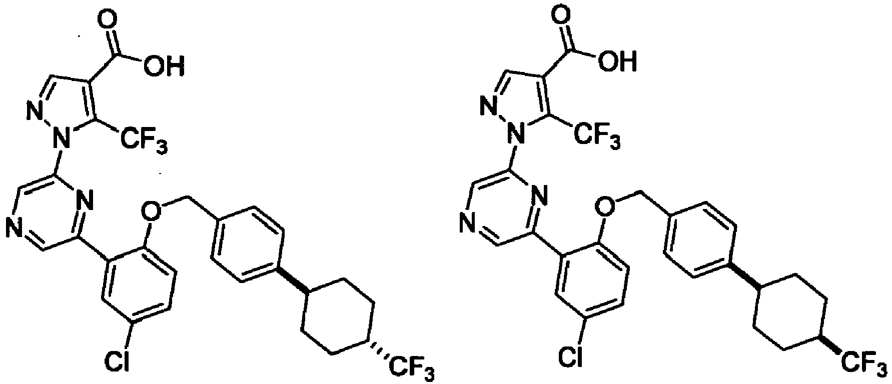 **Example 17** 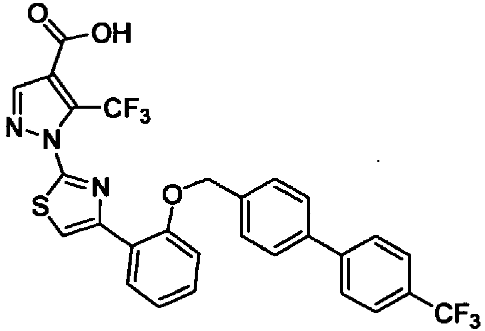 **Example 18** 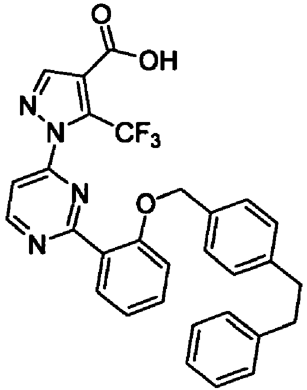 **Example 19** 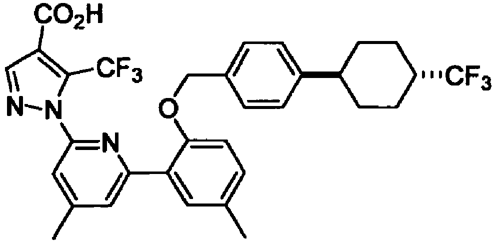 The compounds in **TABLE 1** were prepared using the chemistry described in **Examples 1-19.**
**TABLE 1**
| | | | | | |
|---|---|---|---|---|---|
| | | | | 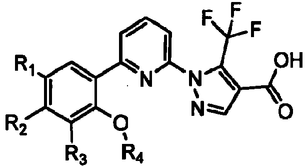 | |
| Entry | R1 | R2 | R3 | R4 | MS |
|---|---|---|---|---|---|
| 20 | -CF₃ | H | H | 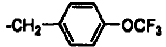 | 592.1 [M+H]⁺ |
| 21 | H | H | H | 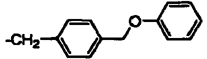 | 546.2 [M+H]⁺ |
| 22 | H | H | H | 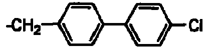 | 572.1 [M+Na]⁺ |
| 23 | H | H | H | 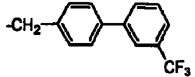 | 584.1 [M+H]⁺ |
| 24 | Me | H | H | 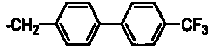 | 598.7 [M+H]⁺ |
| 25 | Me | H | H | 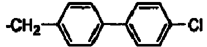 | 564.6 [M+H]⁺ |
| 26 | Me | H | H | 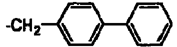 | 528.4 [M-H]⁻ |
| 27 | Br | H | H | 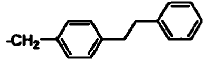 | 624.5 [M+H]⁺ |
| 29 | cPr | H | H | 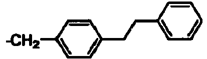 | 584.5 [M+H]⁺ |
| 30 | CF₃ | H | H | 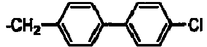 | 618.2 [M+H]⁺ |
| 31 | CF₃ | H | H | 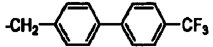 | 652.3 [M+H]⁺ |
| 32 | CF₃ | H | H | 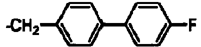 | 602.2 [M+H]⁺ |
| 33 | H | H | H | 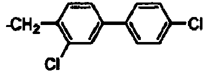 | 584.4 [M+H]⁺ |
| 34 | H | H | H | 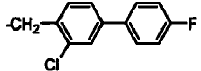 | 568.1 [M+H]⁺ |
| 35 | H | H | H | 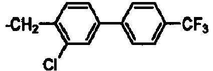 | 618.0 [M+H]⁺ |
| 36 | H | H | H | 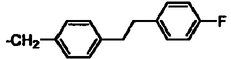 | 562.5 [M+H]⁺ |
| 37 | H | H | H | 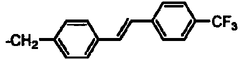 | 610.5 [M+H]⁺ |
| 38 | Me | H | H | 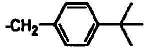 | 510.6 [M+H]⁺ |
| 39 | Me | H | H | 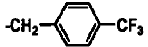 | 522.5 [M+H]⁻ |
| 40 | H | H | H | 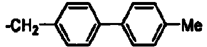 | 552.2 [M+Na]⁺ |
| 41 | H | H | H | 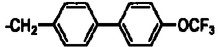 | 600.2 [M+H]⁺ |
| 42 | Me | H | H | 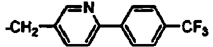 | 599.6 [M+H]⁺ |
| 43 | H | H | H | 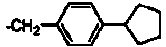 | 508.6 [M+H]⁺ |
| 44 | H | H | H | 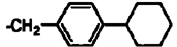 | 522.6 [M+H]⁺ |
| 45 | H | H | H | 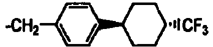 | 590.5 [M+H]⁺ |
| 46 | H | H | H | 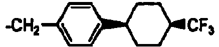 | 590.5 [M+H]⁺ |
| 47 | Me | H | I | 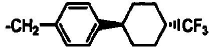 | 730.6 [M+H]⁺ |
| 48 | Cl | H | I | 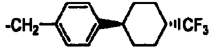 | 750.6 [M+H]⁺ |
| 49 | H | H | I | 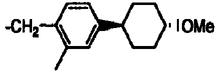 | 692.9 [M+H]⁺ |
| 50 | cPr | H | H | 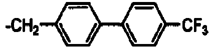 | 624.2 [M+H]⁺ |
| 51 | H | H | H | 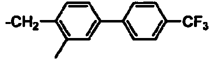 | 598.2 [M+H]⁺ |
| 52 | H | H | H | 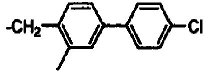 | 586.2 [M+Na]⁺ |
| 53 | H | H | H | 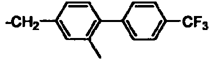 | 598.2 [M+H]⁺ |
| 54 | H | H | H | 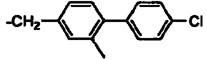 | 564.2 [M+H]⁺ |
| 55 | H | H | H | 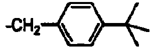 | 496.6 [M+H]⁺ |
| 56 | H | H | H | 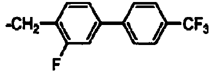 | 602.4 [M+H]⁺ |
| 57 | H | H | H | 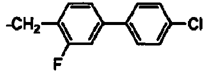 | 568.5 [M+H]⁺ |
| 58 | H | H | H | 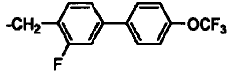 | 618.4 [M+H]⁺ |
| 59 | H | H | H | 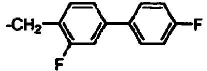 | 552.4 [M+H]⁺ |
| 60 | H | H | H | 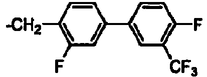 | 620.4 [M+H]⁺ |
| 61 | H | H | H | 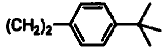 | 510.5 [M+H]⁺ |
| 62 | F | H | H | 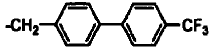 | 602.3 [M+H]⁺ |
| 63 | F | H | H | 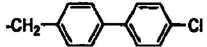 | 566.3 [M-H]⁻ |
| 64 | F | H | H | 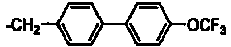 | 618.3 [M+H]⁺ |
| 65 | F | H | H | 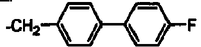 | 552.4 [M+H]⁺ |
| 66 | F | H | H | 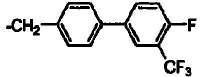 | 620.4 [M+H]⁺ |
| 67 | F | H | H | 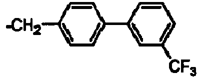 | 602.4 [M+H]⁺ |
| 68 | H | H | H | 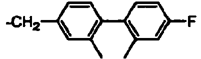 | 562.6 [M+H]⁺ |
| 69 | H | H | H | 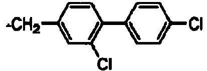 | 584.0 [M+H]⁺ |
| 70 | H | H | H | 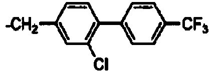 | 618.0 [M+H]⁺ |
| 71 | Me | H | H | 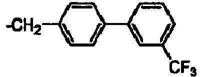 | 598.2 [M+H]⁺ |
| 72 | H | H | H | 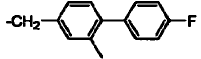 | 548.2 [M+H]⁺ |
| 73 | H | H | H | 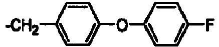 | 548.5 [M-H]⁻ |
| 74 | H | H | H | 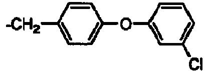 | 564.4 [M-H]⁻ |
| 75 | H | H | H | 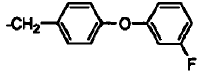 | 548.5 [M-H]⁻ |
| 76 | H | H | H | 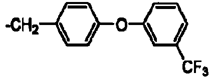 | 598.4 [M-H]⁻ |
| 77 | H | H | H | 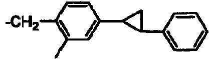 | 568.5 [M-H]⁻ |
| 78 | H | H | H | 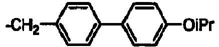 | 596.2 [M+Na]⁺ |
| 79 | H | H | H | 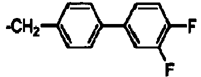 | 552.2 [M+H]⁺ |
| 80 | H | H | H | 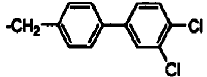 | 584.0 [M+H]⁺ |
| 81 | H | H | H | 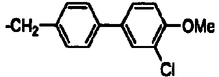 | 602.1 [M+Na]⁺ |
| 82 | H | H | H | 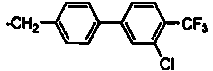 | 617.8 [M+H]⁺ |
| 83 | H | H | H | 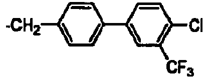 | 617.8 [M+H]⁺ |
| 84 | Me | H | H | 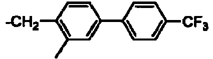 | 612.2 [M+H]⁺ |
| 85 | Me | H | H | 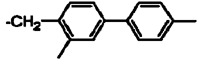 | 580.1 [M+Na]⁺ |
| 86 | Me | H | H | 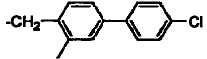 | 600.0 [M+Na]⁺ |
| 87 | Me | H | H | 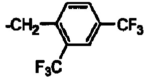 | 590.0 [M+H]⁺ |
| 88 | Me | H | H | 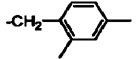 | 482.1 [M+H]⁺ |
| 89 | H | H | H | 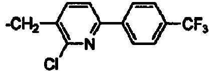 | 619.1 [M+H]⁺ |
| 90 | H | H | H | 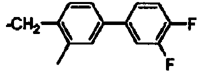 | 566.1 [M+H]⁺ |
| 91 | H | H | H | 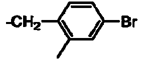 | 534.0 [M+H]⁺ |
| 92 | H | H | H | 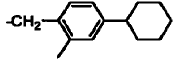 | 558.1 [M+Na]⁺ |
| 93 | H | H | H | 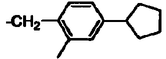 | 544.1 [M+Na]⁺ |
| 94 | H | H | H | 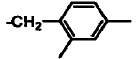 | 468.0 [M+H]⁺ |
| 95 | Me | H | H | 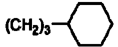 | 488.0 [M+H]⁺ |
| 96 | Me | H | H | 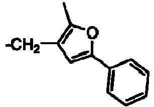 | 556.1 [M+Na]⁺ |
| 97 | Me | H | H | 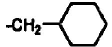 | 460.0 [M+H]⁺ |
| 98 | Me | H | H | 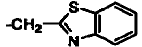 | 511.1 [M+H]⁺ |
| 99 | H | H | H | 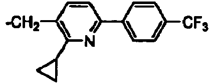 | 625.4 [M+H]⁺ |
| 100 | H | H | H | 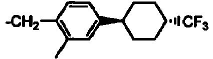 | 604.4 [M+H]⁺ |
| 101 | H | H | H | 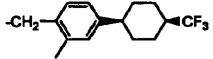 | 604.5 [M+H]⁺ |
| 102 | Me | H | H | 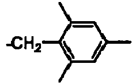 | 518.0 [M+Na]⁺ |
| 103 | CF3 | H | H | 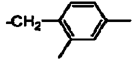 | 536.6 [M+H]⁺ |
| 104 | CF3 | H | H | 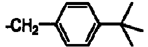 | 564.6 [M+H]⁺ |
| 105 | Me | H | H | 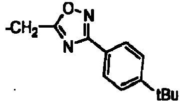 | 578.6 [M+H]⁺ |
| 106 | Me | H | H | 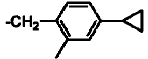 | 508.6 [M+H]⁺ |
| 107 | CF3 | H | H | 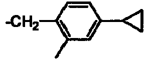 | 584.5 [M+H]⁺ |
| 108 | H | H | H | 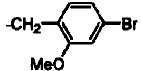 | 548.0 [M+H]⁺ |
| 109 | H | H | H | 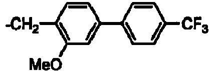 | 614.1 [M+H]⁺ |
| 110 | H | H | H | 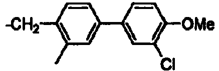 | 616.2 [M+Na]⁺ |
| 111 | H | H | H | 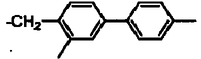 | 566.2 [M+Na]⁺ |
| 112 | H | H | H | 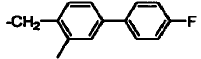 | 570.0 [M+Na]⁺ |
| 113 | H | H | H | 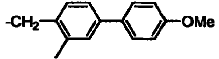 | 582.0 [M+Na]⁺ |
| 114 | H | H | H | 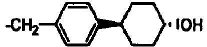 | 538.8 [M+H]⁺ |
| 115 | H | H | H | 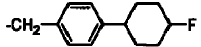 | 540.9 [M+H]⁺ |
| 116 | H | H | H | 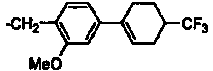 | 618.1 [M+H]⁺ |
| 117 | H | H | H | 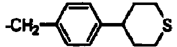 | 540.5 [M+H]⁺ |
| 118 | H | H | H | 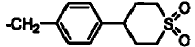 | 572.5 [M+H]⁺ |
| 119 | H | H | H | 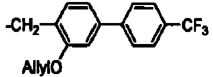 | 640.2 [M+H]⁺ |
| 120 | Me | H | H | 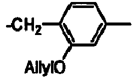 | 524.6 [M+H]⁺ |
| 121 | H | H | H | 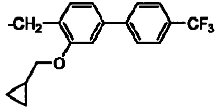 | 564.2 [M+H]⁺ |
| 122 | H | H | H | 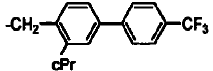 | 624.2 [M+H]⁺ |
| 123 | H | H | H | 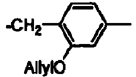 | 510.6 [M+H]⁺ |
| 124 | Me | H | H | 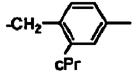 | 530.7 [M+H]⁺ |
| 125 | H | H | H | 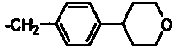 | 546.6 [M+Na]⁺ |
| 126 | Cl | H | H | 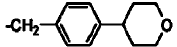 | 580.6 [M+Na]⁺ |
| 127 | H | H | H | 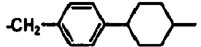 | 536.7 [M+H]⁺ |
| 128 | H | H | H | 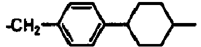 | 536.9 [M+H]⁺ |
| 129 | Cl | H | H | 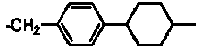 | 568.7 [M-H]⁻ |
| 130 | Cl | H | H | 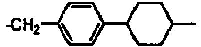 | 568.8 [M-H]⁻ |
| 131 | H | H | H | 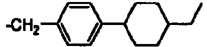 | 548.8 [M-H]⁻ |
| 132 | H | H | H | 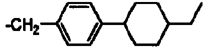 | 548.8 [M-H]⁻ |
| 133 | Cl | H | H | 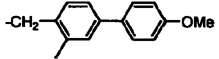 | 616.6 [M+Na]⁺ |
| 134 | Cl | H | H | 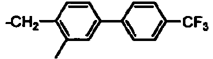 | 630.8 [M-H]⁻ |
| 135 | Cl | H | H | 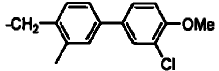 | 626.7 [M-H]⁻ |
| 136 | Me | H | H | 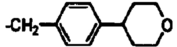 | 538.8 [M+H]⁺ |
| 137 | Me | H | H | 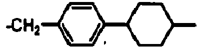 | 550.9 [M+H]⁺ |
| 138 | Me | H | H | 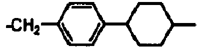 | 550.9 [M+H]⁺ |
| 139 | H | H | H | 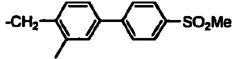 | 608.8 [M+H]⁺ |
| 140 | Me | H | H | 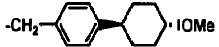 | 566.6 [M+H]⁺ |
| 141 | Me | H | H | 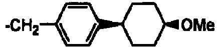 | 586.6 [M+H]⁺ |
| 142 | H | H | H | 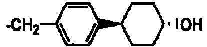 | 550.9 [M-H]⁻ |
| 143 | cPr | H | H | 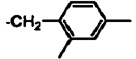 | 508.8 [M+H]⁺ |
| 144 | Cl | H | H | 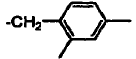 | 500.7 [M-H]⁻ |
| 145 | H | H | H | 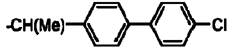 | 562.4 [M-H]⁻ |
| 146 | H | Cl | H | 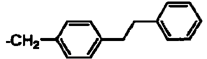 | 578.5 [M+H]⁺ |
| 147 | H | F | H | 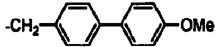 | 586.5 [M+Na]⁺ |
| 148 | H | F | H | 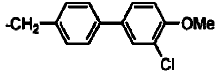 | 620.5 [M+Na]⁺ |
| 149 | H | F | H | 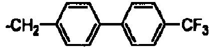 | 602.6 [M+H]⁺ |
| 150 | H | H | cPr | 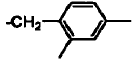 | 508.8 [M+H]⁺ |
| 151 | H | H | Cl | 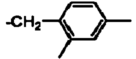 | 500.7 [M-H]⁻ |
| 152 | Me | H | Cl | 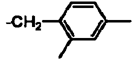 | 516.7 [M+H]⁺ |
| 153 | I | H | H | 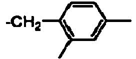 | 592.7 [M-H]⁻ |
| 154 | H | H | I | 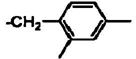 | 592.7 [M-H]⁻ |
| 155 | Me | H | Cl | 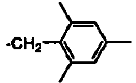 | 530.7 [M+H]⁺ |
| 156 | Me | H | Cl | 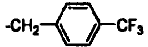 | 556.7 [M+H]⁺ |
| 157 | Me | H | Cl | 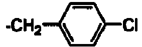 | 522.6 [M+H]⁺ |
| 158 | H | H | H | 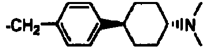 | 580.0 [M+H]⁺ |
| 159 | H | H | Me | 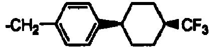 | 604.9 [M+H]⁺ |
| 160 | H | H | Me | 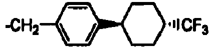 | 604.9 [M+H]⁺ |
| 161 | -CF₃ | H | H | 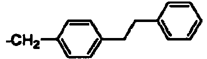 | 612.6 [M+H]⁺ |
| 162 | Cl | H | H | 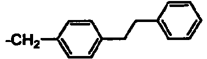 | 558.5 [M+H]⁺ |
| 163 | H | H | H | 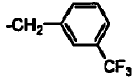 | 508.6 [M+H]⁺ |
| 164 | H | H | H | 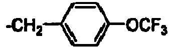 | 524.5 [M+H]⁺ |
| 165 | H | H | H | 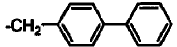 | 516.6 [M+H]⁺ |
| 166 | Cl | H | H | 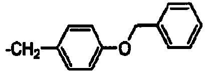 | 578.4 [M-H]⁻ |
| 167 | Cl | H | H | 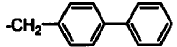 | 550.2 [M+H]⁺ |
| 168 | Cl | H | H | 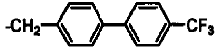 | 618.2 [M+H]⁺ |
| 169 | Cl | H | H | 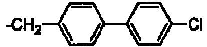 | 584.2 [M+H]⁺ |
| 170 | Cl | H | H | 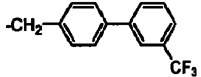 | 618.2 [M+H]⁺ |
| 171 | H | H | H | 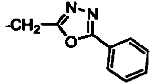 | 508.5 [M+H]⁺ |
| 172 | H | H | H | 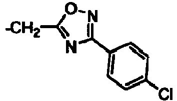 | 542.4 [M+H]⁺ |
| 173 | H | H | H | 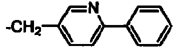 | 517.5 [M+H]⁺ |
| 174 | H | H | H | 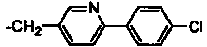 | 551.4 [M+H]⁺ |
| 175 | H | H | H | 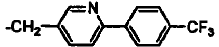 | 585.4 [M+H]⁺ |
| 176 | Me | H | H | 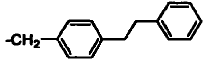 | 558.5 [M+H]⁺ |
| 177 | F | H | H | 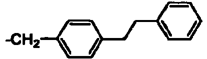 | 562.5 [M+H]⁺ |
The compounds in **TABLE 2** were prepared using the chemistry described in **Examples 1-9.**
**TABLE 2**
| | | | | | |
|---|---|---|---|---|---|
| | | | | 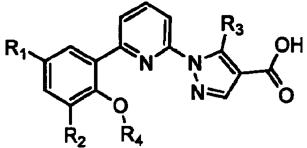 | |
| Entry | R1 | R2 | R3 | R4 | MS |
|---|---|---|---|---|---|
| 178 | H | H | Me | 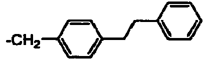 | 490.2 [M+H]⁺ |
| 179 | Cl | H | Me | 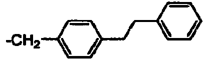 | 524.1 [M+H]⁺ |
| 180 | H | H | Me | 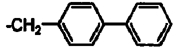 | 462.2 [M+H]⁺ |
| 181 | Cl | H | Me | 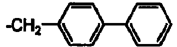 | 496.1 [M+H]⁺ |
| 182 | H | H | Me | 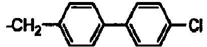 | 496.1 [M+H]⁺ |
| 183 | H | H | Me | 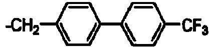 | 529.9 [M+H]⁺ |
| 184 | H | H | CF2H | 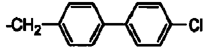 | 532.0 [M+H]⁺ |
| 185 | H | H | CF2H | 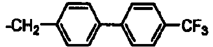 | 566.2 [M+H]⁺ |
| 186 | H | H | CF2H | 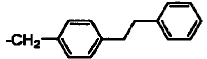 | 526.2 [M+H]⁺ |
| 187 | Cl | H | CF2H | 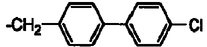 | 566.0 [M+H]⁺ |
| 188 | Cl | H | CF2H | 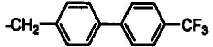 | 600.0 [M+H]⁺ |
| 189 | H | H | CF2H | 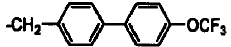 | 582.0 [M+H]⁺ |
| 190 | H | H | CF2H | 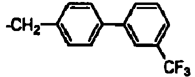 | 566.1 [M+H]⁺ |
| 191 | Me | H | CF2H | 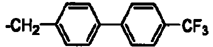 | 580.5 [M+H]⁺ |
| 192 | Me | H | CF2H | 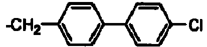 | 546.5 [M+H]⁺ |
| 193 | H | H | CF2H | 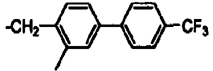 | 580.5 [M+H]⁺ |
| 194 | H | H | CF2H | 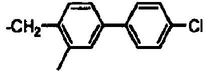 | 546.5 [M+H]⁺ |
| 195 | H | H | CF2H | 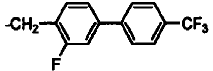 | 584.4 [M+H]⁺ |
| 196 | H | H | CF2H | 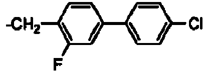 | 550.4 [M+H]⁺ |
| 197 | H | H | Et | 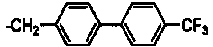 | 544.5 [M+H]⁺ |
| 198 | H | H | iPr | 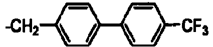 | 558.5 [M+H]⁺ |
| 199 | H | H | cPr | 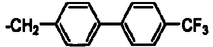 | 556.5 [M+H]⁺ |
| 200 | H | H | -CH₂OMe | 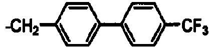 | 560.5 [M+H]⁺ |
| 201 | H | H | Et | 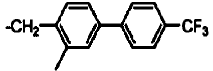 | 558.5 [M+H]⁺ |
| 202 | H | H | iPr | 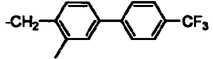 | 572.5 [M+H]⁺ |
| 203 | H | H | cPr | 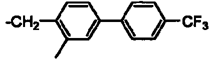 | 570.5 [M+H]⁺ |
| 204 | H | H | -CH₂OMe | 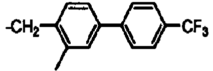 | 574.5 [M+H]⁺ |
| 205 | H | H | CF2H | 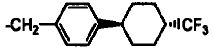 | 572.6 [M+H]⁺ |
| 206 | H | H | CF2H | 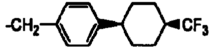 | 572.6 [M+H]⁺ |
| 207 | H | H | Pr | 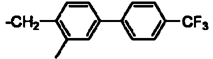 | 572.6 [M+H]⁺ |
| 208 | H | H | Ph | 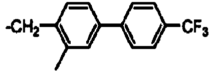 | 606.6 [M+H]⁺ |
| 209 | Cl | Cl | CF2H | 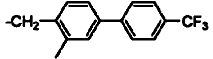 | 648.4 [M+H]⁺ |
| 210 | Cl | H | CF2H | 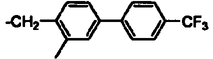 | 614.5 [M+H]⁺ |
| 211 | Cl | H | CF2H | 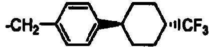 | 606.5 [M+H]⁺ |
| 212 | Cl | H | CF2H | 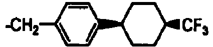 | 606.5 [M+H]⁺ |
| 213 | Cl | Cl | CF2H | 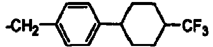 | 640.5 [M+H]⁺ |
| 214 | H | H | CF2H | 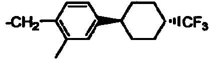 | 587.0 [M+H]⁺ |
| 215 | H | H | CF2H | 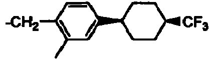 | 587.0 [M+H]⁺ |
The compounds in **TABLE 3** were synthesized using chemistry described in **Examples 1-19.**
**TABLE 3**
| Entry | R | MS |
|---|---|---|
| 216 | 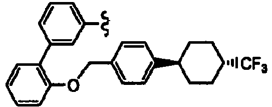 | 589.5 [M+H]⁺ |
| 217 | 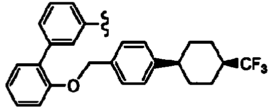 | 5 89.5 [M+H]⁺ |
| 218 | 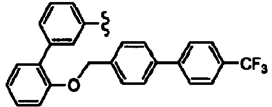 | 5 83.5 [M+H]⁺ |
| 219 | 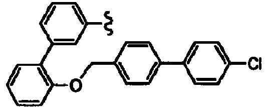 | 549.5 [M+H]⁺ |
| 220 | 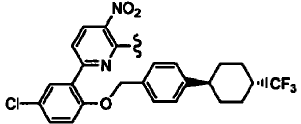 | 669.9 [M+H]⁺ |
| 221 | 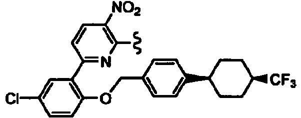 | 669.9 [M+H]⁺ |
| 222 | 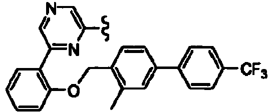 | 599.5 [M+H]⁺ |
| 223 | 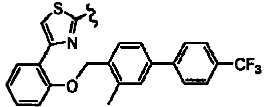 | 604.5 [M+H]⁺ |
| 224 | 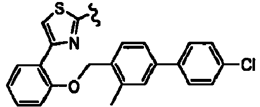 | 570.4 [M+H]⁺ |
| 225 | 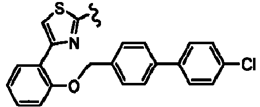 | 556.5 [M+H]⁺ |
| 226 | 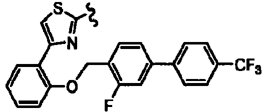 | 608.5 [M+H]⁺ |
| 227 | 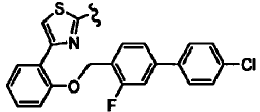 | 574.4 [M+H]⁺ |
| 228 | 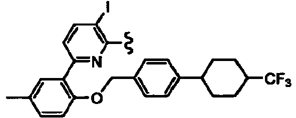 | 730.8 [M+H]⁺ |
| 229 | 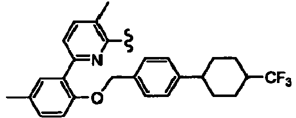 | 618.8 [M+H]⁺ |
| 230 | 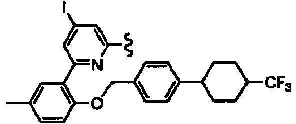 | 730.8 [M+H]⁺ |
| 231 | 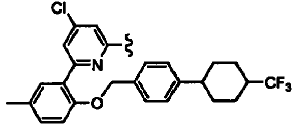 | 638.8 [M+H]⁺ |
| 232 | 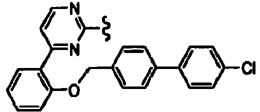 | 551.1 [M+H]⁺ |
| 233 | 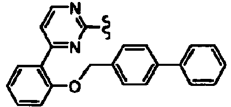 | 517.2 [M+H]⁺ |
| 234 | 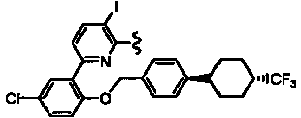 | 750.9 [M+H]⁺ |
| 235 | 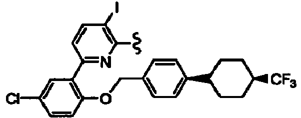 | 750.9 [M+H]⁺ |
**Example 236** 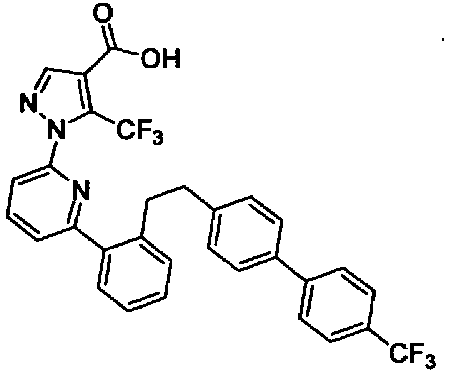 **Example 237** 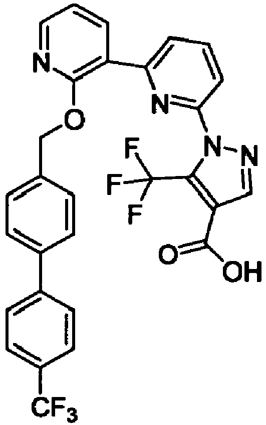 **Example 238** 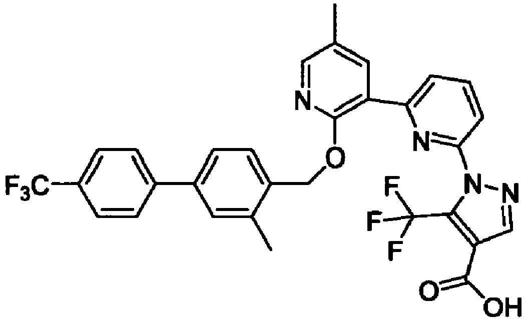 **Example 239** 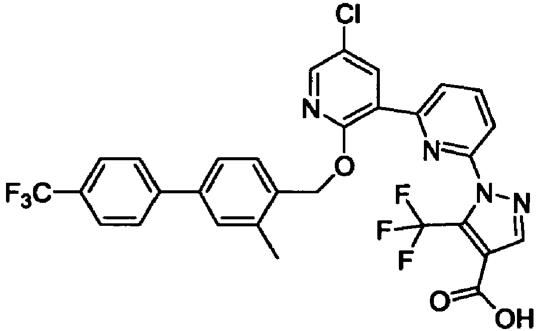 **Example 240** 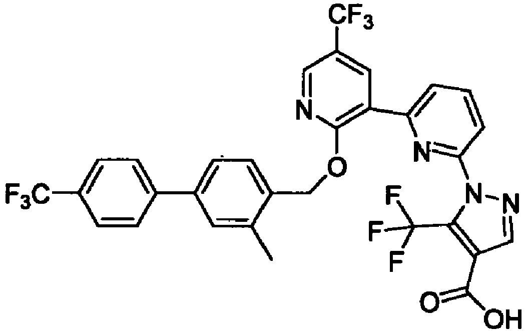 **Example 241** 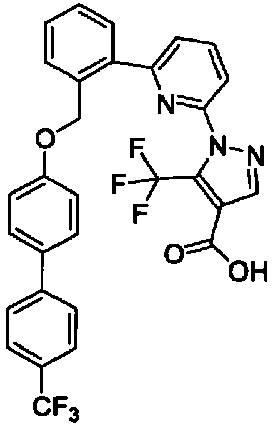 **Example 242** 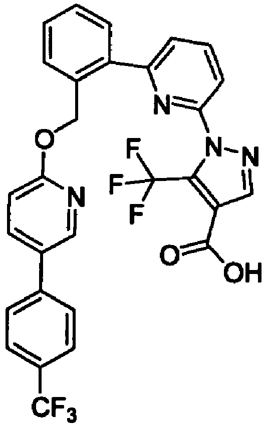 **Example 243** 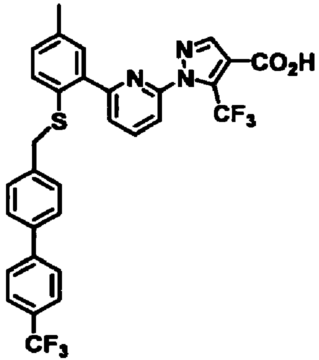 The compounds listed in **TABLE 4** were prepared using chemistry described in **Examples 236-243.**
**TABLE 4**
| | | | | |
|---|---|---|---|---|
| | | | 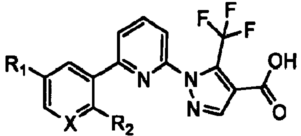 | |
| Entry | X | R1 | R2 | MS ([M+H]⁺) |
|---|---|---|---|---|
| 244 | CH | H | 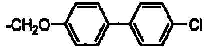 | 549.9 |
| 245 | CH | H | 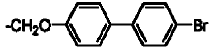 | 595.8 |
| 246 | CH | Cl | 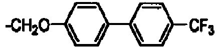 | 617.8 |
| 247 | CH | H | 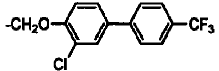 | 617.8 |
| 248 | CH | H | 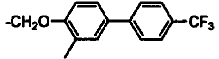 | 597.9 |
| 249 | CH | Me | 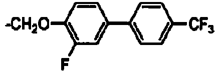 | 615.8 |
| 250 | CH | Me | 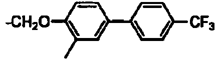 | 611.8 |
| 251 | CH | H | 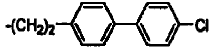 | 548.4 |
| 252 | N | H | 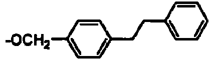 | 544.9 |
| 253 | N | H | 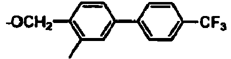 | 599.0 |
| 254 | N | Cl | 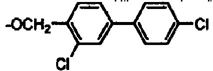 | 620.8 |
| 255 | N | Cl | 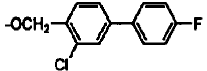 | 602.8 |
| 256 | N | Me | 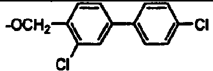 | 598.8 |
| 257 | N | Me | 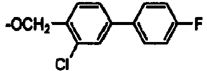 | 582.9 |
| 258 | N | Cl | 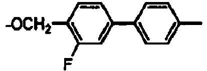 | 582.9 |
| 259 | N | Cl | 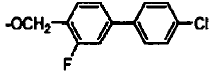 | 602.8 |
| 260 | N | Cl | 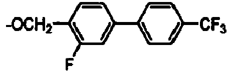 | 636.7 |
| 261 | N | Cl | 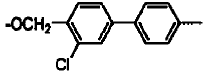 | 598.8 |
| 262 | N | Cl | 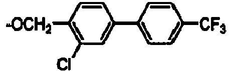 | 652.8 |
| 263 | N | Cl | 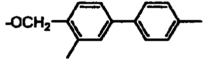 | 578.9 |
| 264 | N | Cl | 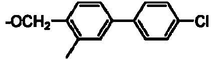 | 598.8 |
| 265 | N | Cl | 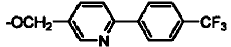 | 619.9 |
| 266 | N | Me | 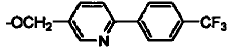 | 600.0 |
| 267 | N | CF₃ | 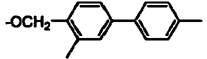 | 613.0 |
| 268 | CH | H | 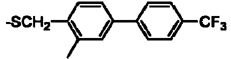 | 614.9 |
| 269 | CH | H | 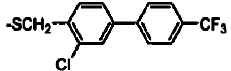 | 634.9 |
| 270 | CH | H | 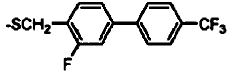 | 618.9 |
| 271 | CH | H | 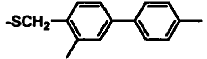 | 560.9 |
| 272 | CH | H | 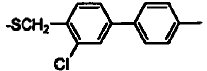 | 580.9 |
| 273 | CH | H | 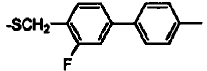 | 564.9 |
| 274 | CH | H | 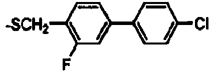 | 584.8 |
| 275 | CH | Me | 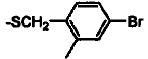 | 562.7 |
| 276 | CH | Me | 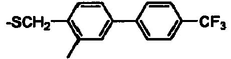 | 628.9 |
| 277 | CH | Me | 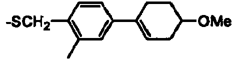 | 595.0 |
| 278 | CH | Me | 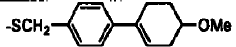 | 580.9 |
| 279 | CH | Me | 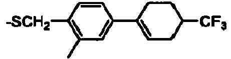 | 632.9 |
| 280 | CH | Me | 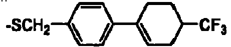 | 618.9 |
| 281 | CH | Me | 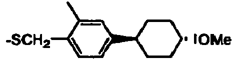 | 596.8 |
| 282 | CH | Me | 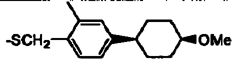 | 596.8 |
| 283 (isom er A) | CH | Me | 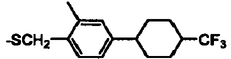 | 635.0 |
| 284 (isom er B) | CH | Me | 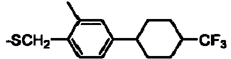 | 635.0 |
| 285 | CH | Me | 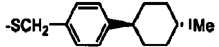 | 566.9 |
| 286 | CH | Me | 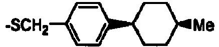 | 566.9 |
| 287 | CH | Me | 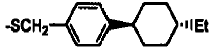 | 580.9 |
| 288 | CH | Me | 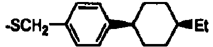 | 580.9 |
| 289 | CH | Me | 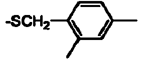 | 498.7 |
| 290 | CH | Me | 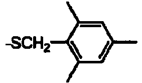 | 512.7 |
| 291 | CH | Cl | 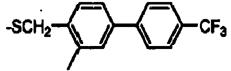 | 648.7 |
| 292 | CH | Cl | 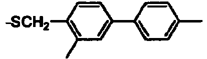 | 594.7 |
| 293 (isom er A) | CH | Cl | 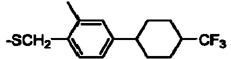 | 654.8 |
| 294 (isom er B) | CH | Cl | 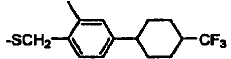 | 654.7 |
| 295 | CH | Cl | 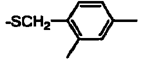 | 518.7 |
| 296 | CH | Cl | 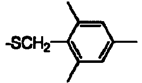 | 532.8 |
| 297 | CH | Cl | 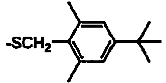 | 574.9 |
| 298 | CH | Cl | 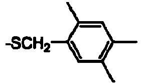 | 532.8 |
| **299** | CH | Cl | 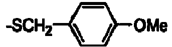 | 520.7 |
**Example 300** 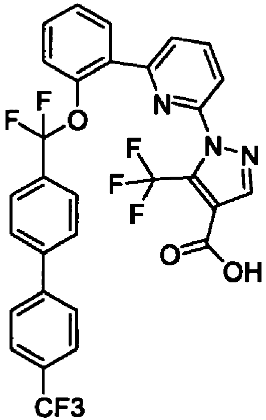 **Example 301** 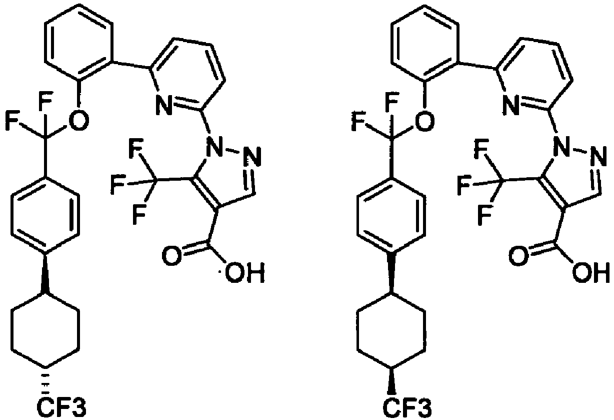 **Example 302** 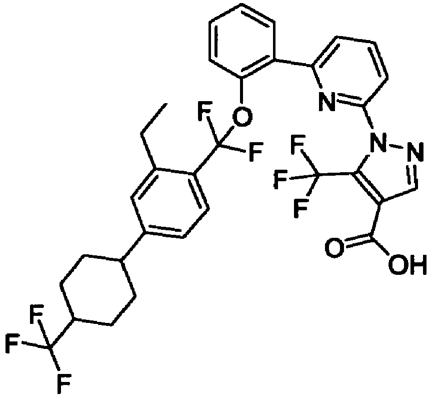 **Example 303** 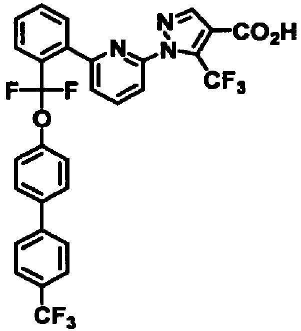 The compounds in **TABLE 5** were prepared using chemistry described in **Examples** 3**00-303.**
**TABLE 5**
| | | | | |
|---|---|---|---|---|
| | | | 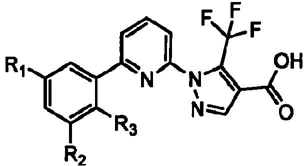 | |
| Entry | R1 | R2 | R3 | MS ([M-F]⁺) |
|---|---|---|---|---|
| 304 | Me | H | 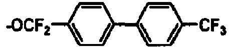 | 613.9 |
| 305 | H | H | 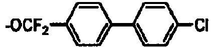 | 565.9 |
| 306 | H | H | 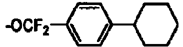 | 538.0 |
| 307 | H | H | 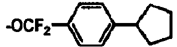 | 524.0 |
| 308 | H | H | 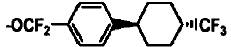 | 605.8 |
| 309 | H | H | 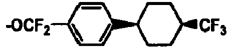 | 605.8 |
| 310 | H | H | 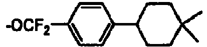 | 566.0 |
| 311 | Me | H | 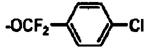 | 504.0 |
| 312 | Me | H | 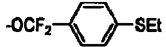 | 530.0 |
| 313 | Me | H | 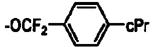 | 510.1 |
| 314 | Me | H | 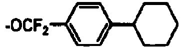 | 552.0 |
| 315 | H | H | 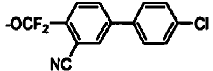 | 590.8 |
| 316 | H | H | 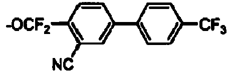 | 624.8 |
| 317 | Cl | H | 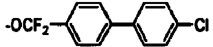 | 599.8 |
| 318 | H | H | 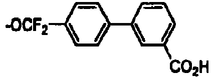 | 575.9 |
| 319 | Cl | H | 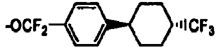 | 639.9 |
| 320 | Cl | H | 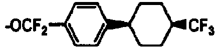 | 639.9 |
| 321 | Cl | H | 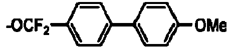 | 595.9 |
| 322 | H | H | 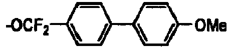 | 561.9 |
| 323 | Me | H | 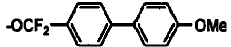 | 576.0 |
| 324 | Cl | H | 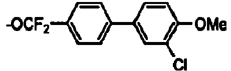 | 629.9 |
| 325 | H | H | 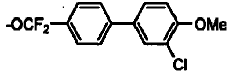 | 595.9 |
| 326 | Me | H | 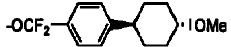 | 582.6 |
| 327 | Me | Br | 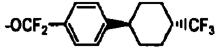 | 698.5 |
| 328 | H | H | 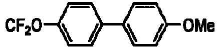 | 562.9 |
| 329 | H | H | 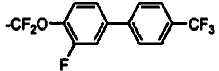 | 618.9 |
**Example 330** 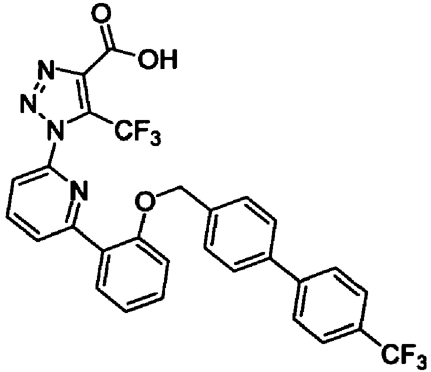 The compounds in **TABLE 6** were prepared using chemistry described in **Examples 1 and 330.**
**TABLE 6**
| | | | |
|---|---|---|---|
| | | 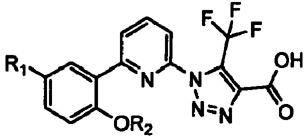 | |
| Entry | R1 | R2 | MS |
|---|---|---|---|
| 331 | H | 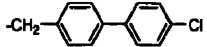 | 551.0 [M+H]⁺ |
| 332 | Me | 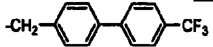 | 599.5 [M+H]⁺ |
| | | | |
| 333 | Me | 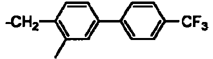 | 613.5 [M+H]⁺ |
| 334 | Me | 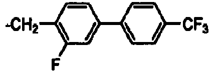 | 617.4 [M+H]⁺ |
Compound Class IV
**Example 1** 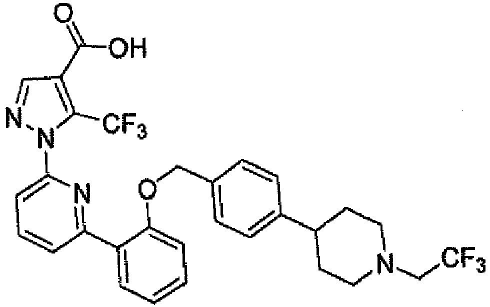 **Example 2** 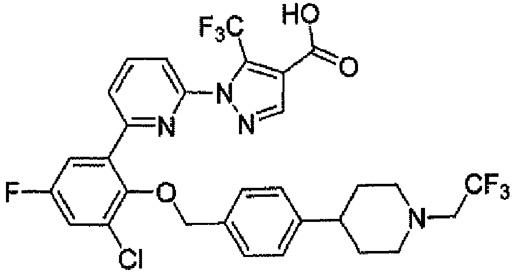 **Example 3** 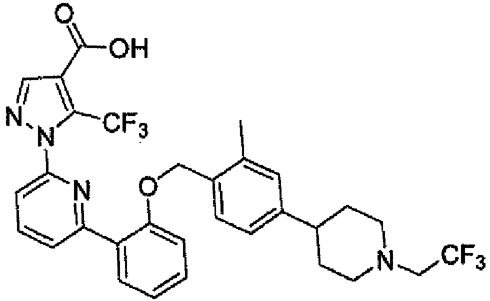 **Example 4** 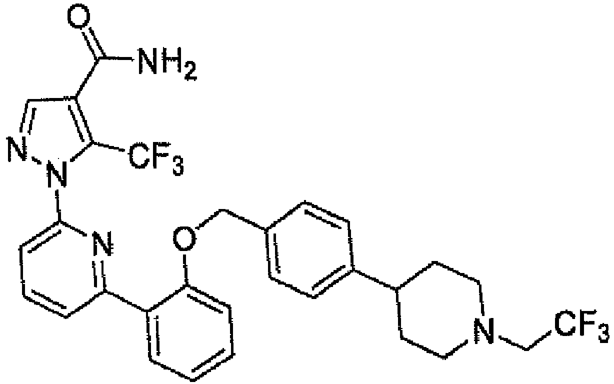 **Example 5** 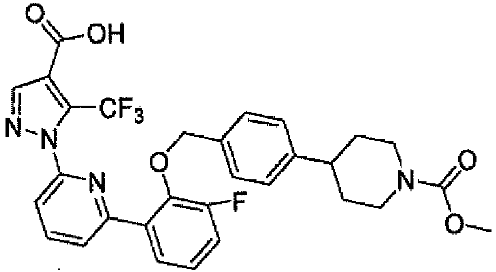 **Example 6** 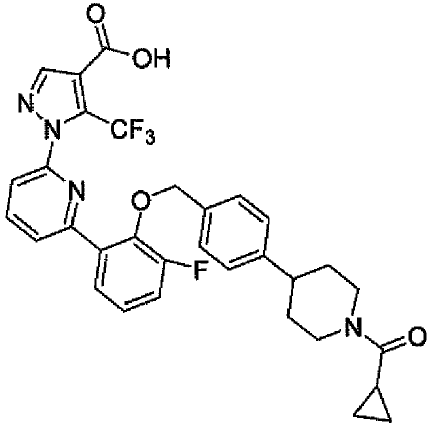 **Example 7** 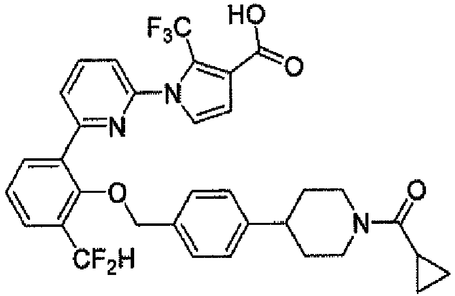 **Example 8** 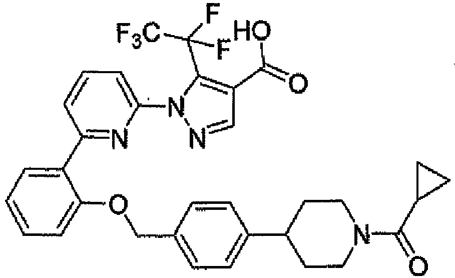 **Example 9** 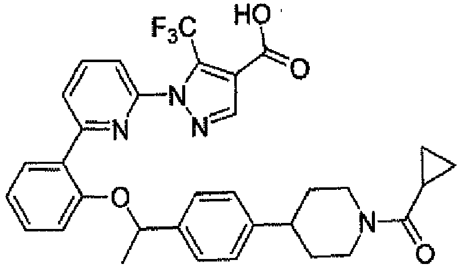 **Example 10** 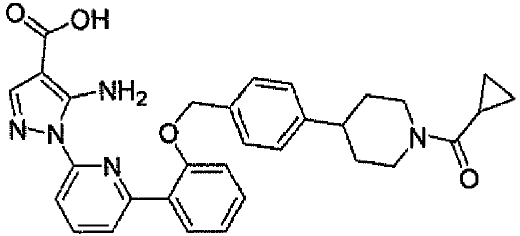 **Example 11** 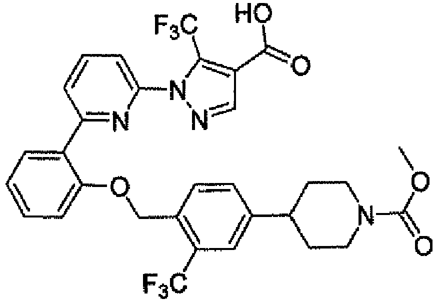 **Example 12** 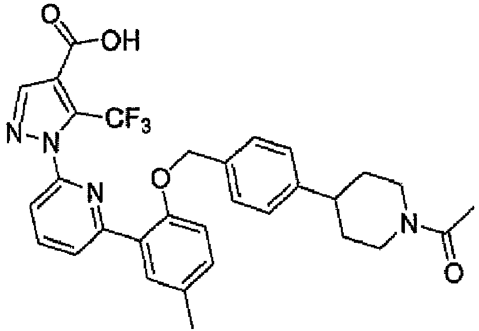 **Example 13** 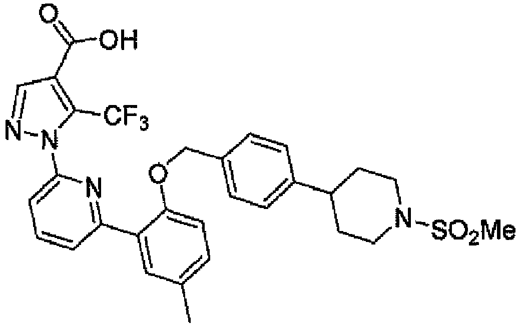 **Example 14** 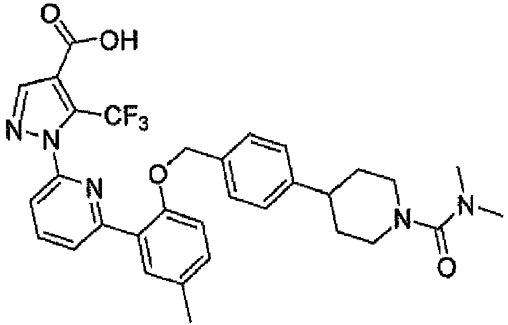 The compounds in **Table** 1 were prepared using chemistry described in **Examples 1-14.**
**Table 1.**
| Example | Structure | IUPAC name |
|---|---|---|
| 15 | 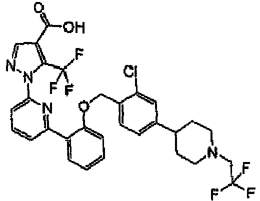 | 1-(6-(2-((2-chloro-4-(1-(2,2,2-trifluoroethyl)piperidin-4-yl)benzyl)oxy)phenyl)pyridin-2-yl)-5-(trifluoromethyl)-1H-pyrazole-4-carboxylic acid |
| 16 | 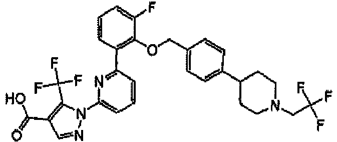 | 1-(6-(3-fluoro-2-((4-(1-(2,2,2-trifluoroethyl)piperidin-4-yl)benzyl)oxy)phenyl)pyridin-2-yl)-5-(trifluoromethyl)-1H-pyrazole-4-carboxylic acid |
| 17 | 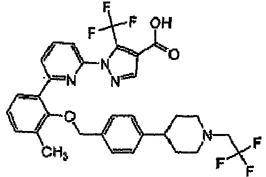 | 1-(6-(3-methyl-2-((4-(1-(2,2,2-trifluoroethyl)piperidin-4-yl)benzyl)oxy)phenyl)pyridin-2-yl)-5-(trifluoromethyl)-1H-pyrazole-4-carboxylic acid |
| 18 | 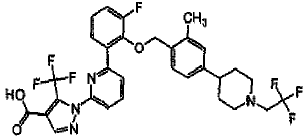 | 1-(6-(3-fluoro-2-((2-methyl-4-(1-(2,2,2-trifluoroethyl)piperidin-4-yl)benzyl)oxy)phenyl)pyridin-2-yl)-5-(trifluoromethyl)-1H-pyrazole-4-carboxylic acid |
| 19 | 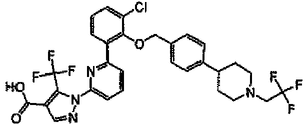 | 1-(6-(3-chloro-2-((4-(1-(2,2,2-trifluoroethyl)piperidin-4-yl)benzyl)oxy)phenyl)pyridin-2-yl)-5-(trifluoromethyl)-1H-pyrazole-4-carboxylic acid |
| 20 | 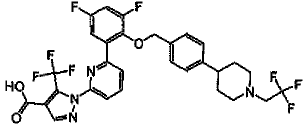 | 1-(6-(3,5-difluoro-2-((4-(1-(2,2,2-trifluoroethyl)piperidin-4-yl)benzyl)oxy)phenyl)pyridin-2-yl)-5-(trifluoromethyl)-1H-pyrazole-4-carboxylic acid |
| 21 | 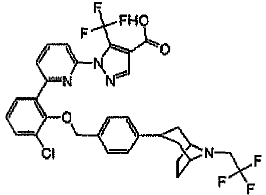 | 1-(6-(3-chloro-2-((4-(8-(2,2,2-trifluoroethyl)-8-azabicyclo[3.2.1]oct-3-yl)benzyl)oxy)phenyl)pyridin-2-yl)-5-(trifluoromethyl)-1H-pyrazole-4-carboxylic acid |
| 22 | 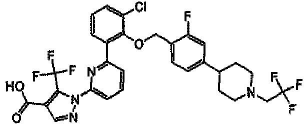 | 1-(6-(3-chloro-2-((2-fluoro-4-(1-(2,2,2-trifluoroethyl)piperidin-4-yl)benzyl)oxy)phenyl)pyridin-2-yl)-5-(trifluoromethyl)-1H-pyrazole-4-carboxylic acid |
| 23 | 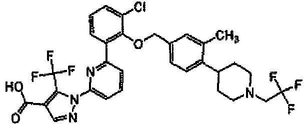 | 1-(6-(3-chloro-2-((3-methyl-4-(1-(2,2,2-trifluoroethyl)piperidin-4-yl)benzyl)oxy)phenyl)pyridin-2-yl)-5-(trifluoromethyl)-1H-pyrazole-4-carboxylic acid |
| 24 | 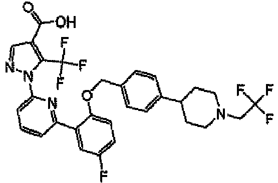 | 1-(6-(5-fluoro-2-((4-(1-(2,2,2-trifluoroethyl)piperidin-4-yl)benzyl)oxy)phenyl)pyridin-2-yl)-5-(trifluoromethyl)-1H-pyrazole-4-carboxylic acid |
| 25 | 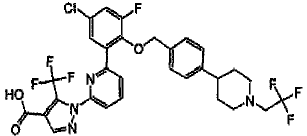 | 1-(6-(5-chloro-3-fluoro-2-((4-(1-(2,2,2-trifluoroethyl)piperidin-4-yl)benzyl)oxy)phenyl)pyridin-2-yl)-5-(trifluoromethyl)-1H-pyrazole-4-carboxylic acid |
| 26 | 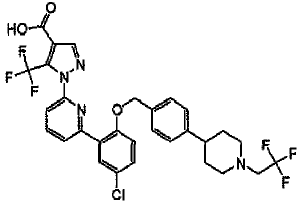 | 1-(6-(5-chloro-2-((4-(1-(2,2,2-trifluoroethyl)piperidin-4-yl)benzyl)oxy)phenyl)pyridin-2-yl)-5-(trifluoromethyl)-1H-pyrazole-4-carboxylic acid |
| 27 | 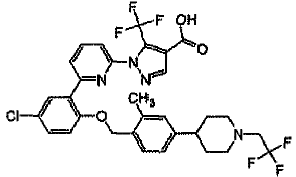 | 1-(6-(5-chloro-2-((2-methyl-4-(1-(2,2,2-trifluoroethyl)piperidin-4-yl)benzyl)oxy)phenyl)pyridin-2-yl)-5-(trifluoromethyl)-1H-pyrazole-4-carboxylic acid |
| 28 | 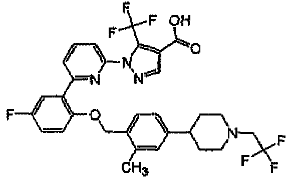 | 1-(6-(5-fluoro-2-((2-methyl-4-(1-(2,2,2-trifluoroethyl)piperidin-4-yl)benzyl)oxy)phenyl)pyridin-2-yl)-5-(trifluoromethyl)-1H-pyrazole-4-carboxylic acid |
| 29 | 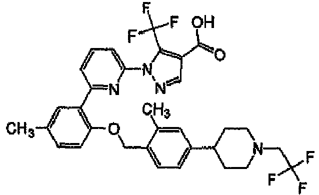 | 1-(6-(5-methyl-2-((2-methyl-4-(1-(2,2,2-trifluoroethyl)piperidin-4-yl)benzyl)oxy)phenyl)pyridin-2-yl)-5-(trifluoromethyl)-1H-pyrazole-4-carboxylic acid |
| 30 | 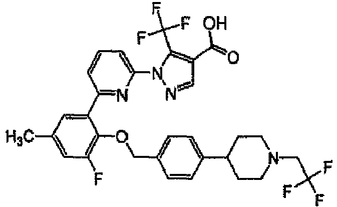 | 1-(6-(3-fluoro-5-methyl-2-((4-(1-(2,2,2-trifluoroethyl)piperidin-4-yl)benzyl)oxy)phenyl)pyridin-2-yl)-5-(trifluoromethyl)-1H-pyrazole-4-carboxylic acid |
| 31 | 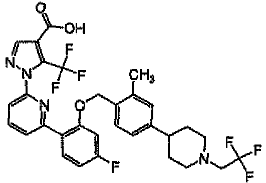 | 1-(6-(4-fluoro-2-((2-methyl-4-(1-(2,2,2-trifluoroethyl)piperidin-4-yl)benzyl)oxy)phenyl)pyridin-2-yl)-5-(trifluoromethyl)-1H-pyrazole-4-carboxylic acid |
| 32 | 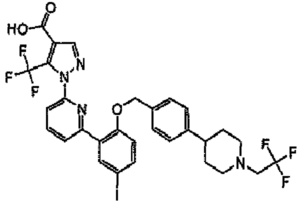 | 1-(6-(5-iodo-2-((4-(1-(2,2,2-trifluoroethyl)piperidin-4-yl)benzyl)oxy)phenyl)pyridin-2-yl)-5-(trifluoromethyl)-1H-pyrazole-4-carboxylic acid |
| 33 | 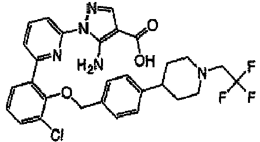 | 5-amino-1-(6-(3-chloro-2-((4-(1-(2,2,2-trifluoroethyl)piperidin-4-yl)benzyl)oxy)phenyl)pyridin-2-yl)-1H-pyrazole-4-carboxylic acid |
| 34 | 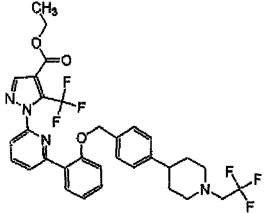 | ethyl 1-(6-(2-((4-(1-(2,2,2-trifluoroethyl)piperidin-4-yl)benzyl)oxy)phenyl)pyridin-2-yl)-5-(trifluoromethyl)-1H-pyrazole-4-carboxylate |
| 35 | 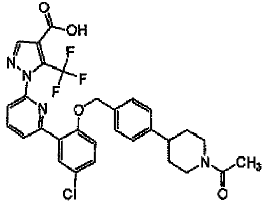 | 1-(6-(2-((4-(1-acetylpiperidin-4-yl)benzyl)oxy)-5-chlorophenyl)pyridin-2-yl)-5-(trifluoromethyl)-1H-pyrazole-4-carboxylic acid |
| 36 | 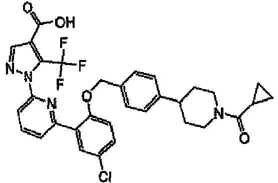 | 1-(6-(5-chloro-2-((4-(1-(cyclopropylcarbonyl)piperidin-4-yl)benzyl)oxy)phenyl)pyridin-2-yl)-5-(trifluoromethyl)-1H-pyrazole-4-carboxylic acid |
| 37 | 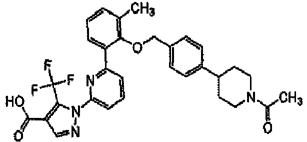 | 1-(6-(2-((4-(1-acetylpiperidin-4-yl)benzyl)oxy)-3-methylphenyl)pyridin-2-yl)-5-(trifluoromethyl)-1H-pyrazole-4-carboxylic acid |
| 38 | 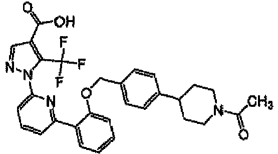 | 1-(6-(2-((4-(1-acetylpiperidin-4-yl)benzyl)oxy)phenyl)pyridin-2-yl)-5-(trifluoromethyl)-1H-pyrazole-4-carboxylic acid |
| 39 | 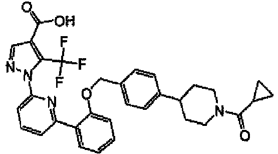 | 1-(6-(2-((4-(1-(cyclopropylcarbonyl)piperidin-4-yl)benzyl)oxy)phenyl)pyridin-2-yl)-5-(trifluoromethyl)-1H-pyrazole-4-carboxylic acid |
| 40 | 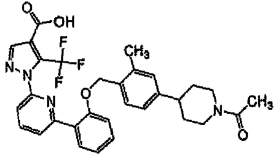 | 1-(6-(2-((4-(1-acetylpiperidin-4-yl)-2-methylbenzyl)oxy)phenyl)pyridi n-2-yl)-5-(trifluoromethyl)-1H-pyrazole-4-carboxylic acid |
| 41 | 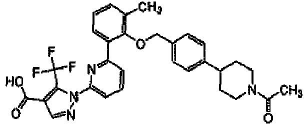 | 1-(6-(2-((4-(1-acetylpiperidin-4-yl)benzyl)oxy)-3-methylphenyl)pyridin-2-yl)-5-(trifluoromethyl)-1H-pyrazole-4-carboxylic acid |
| 42 | 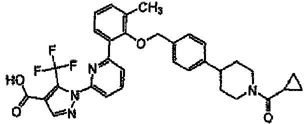 | 1-(6-(2-((4-(1-(cyclopropylcarbonyl)piperidin-4-yl)benzyl)oxy)-3-methylphenyl)pyridin-2-yl)-5-(trifluoromethyl)-1H-pyrazole-4-carboxylic acid |
| 43 | 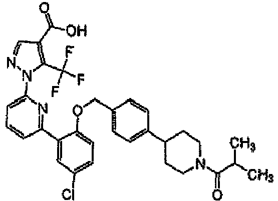 | 1-(6-(5-chloro-2-((4-(1-isobutyrylpiperidin-4-yl)benzyl)oxy)phenyl)pyridin-2-yl)-5-(trifluoromethyl)-1H-pyrazole-4-carboxylic acid |
| 44 | 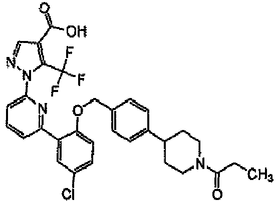 | 1-(6-(5-chloro-2-((4-(1-propionylpiperidin-4-yl)benzyl)oxy)phenyl)pyridin-2-yl)-5-(trifluoromethyl)-1H-pyrazole-4-carboxylic acid |
| 45 | 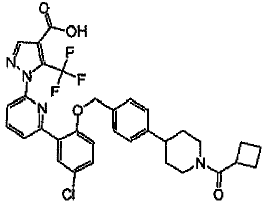 | 1-(6-(5-chloro-2-((4-(1-(cyclobutylcarbonyl)piperidin-4-yl)benzyl)oxy)phenyl)pyridin-2-yl)-5-(trifluoromethyl)-1H-pyrazole-4-carboxylic acid |
| 46 | 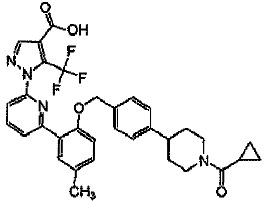 | 1-(6-(2-((4-(1-(cyclopropylcarbonyl)piperidin-4-yl)benzyl)oxy)-5-methylphenyl)pyridin-2-yl)-5-(trifluoromethyl)-1H-pyrazole-4-carboxylic acid |
| 47 | 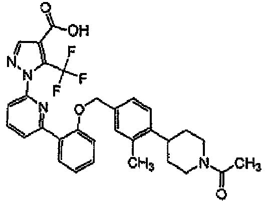 | 1-(6-(2-((4-(1-acetylpiperidin-4-yl)-3-methylbenzyl)oxy)phenyl)pyridi n-2-yl)-5-(trifluoromethyl)-1H-pyrazole-4-carboxylic acid |
| 48 | 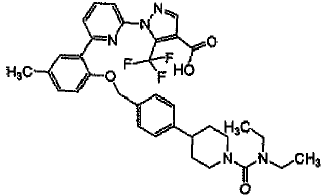 | 1-(6-(2-((4-(1-((diethylamino)carbonyl)piperid in-4-yl)benzyl)oxy)-5-methylphenyl)pyridin-2-yl)-5-(trifluoromethyl)-1H-pyrazole-4-carboxylic acid |
| 49 | 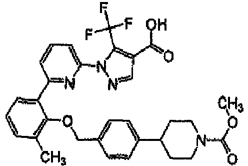 | 1-(6-(2-((4-(1-(methoxycarbonyl)piperidin-4-yl)benzyl)oxy)-3-methylphenyl)pyridin-2-yl)-5-(trifluoromethyl)-1H-pyrazole-4-carboxylic acid |
| 50 | 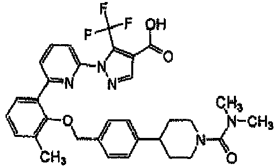 | 1-(6-(2-((4-(1-((dimethylamino)carbonyl)piper idin-4-yl)benzyl)oxy)-3-methylphenyl)pyridin-2-yl)-5-(trifluoromethyl)-1H-pyrazole-4-carboxylic acid |
| 51 | 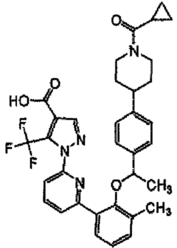 | 1-(6-(2-(1-(4-(1-(cyclopropylcarbonyl)piperidin-4-yl)phenyl)ethoxy)-3-methylphenyl)pyridin-2-yl)-5-(trifluoromethyl)-1H-pyrazole-4-carboxylic acid |
| 52 | 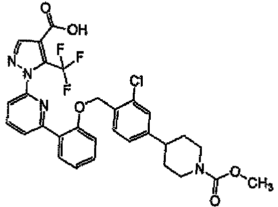 | 1-(6-(2-((2-chloro-4-(1-(methoxycarbonyl)piperidin-4-yl)benzyl)oxy)phenyl)pyridin-2-yl)-5-(trifluoromethyl)-1H-pyrazole-4-carboxylic acid |
| 53 | 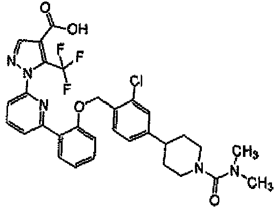 | 1-(6-(2-((2-chloro-4-(1-((dimethylamino)carbonyl)piper idin-4-yl)benzyl)oxy)phenyl)pyridin-2-yl)-5-(trifluoromethyl)-1H-pyrazole-4-carboxylic acid |
| 54 | 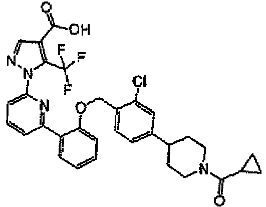 | 1-(6-(2-((2-chloro-4-(1-(cyclopropylcarbonyl)piperidin-4-yl)benzyl)oxy)phenyl)pyridin-2-yl)-5-(trifluoromethyl)-1H-pyrazole-4-carboxylic acid |
| 55 | 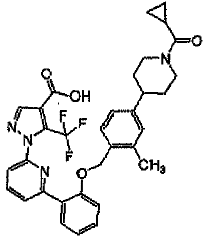 | 1-(6-(2-((4-(1-(cyclopropylcarbonyl)piperidin-4-yl)-2-methylbenzyl)oxy)phenyl)pyridi n-2-yl)-5-(trifluoromethyl)-1H-pyrazole-4-carboxylic acid |
| 56 | 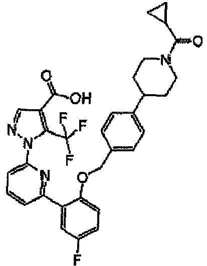 | 1-(6-(2-((4-(1-(cyclopropylcarbonyl)piperidin-4-yl)benzyl)oxy)-5-fluorophenyl)pyridin-2-yl)-5-(trifluoromethyl)-1H-pyrazole-4-carboxylic acid |
| 57 | 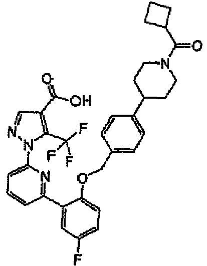 | 1-(6-(2-((4-(1-(cyclobutylcarbonyl)piperidin-4-yl)benzyl)oxy)-5-fluorophenyl)pyridin-2-yl)-5-(trifluoromethyl)-1H-pyrazole-4-carboxylic acid |
| 58 | 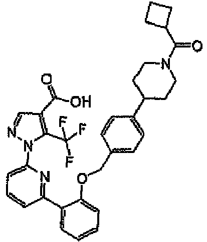 | 1-(6-(2-((4-(1-(cyclobutylcarbonyl)piperidin-4-yl)benzyl)oxy)phenyl)pyridin-2-yl)-5-(trifluoromethyl)-1H-pyrazole-4-carboxylic acid |
| 59 | 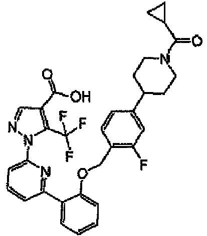 | 1-(6-(2-((4-(1-(cyclopropylcarbonyl)piperidin-4-yl)-2-fluorobenzyl)oxy)phenyl)pyridin -2-yl)-5-(trifluoromethyl)-1H-pyrazole-4-carboxylic acid |
| 60 | 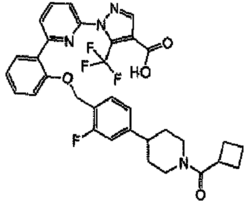 | 1-(6-(2-((4-(1-(cyclobutylcabonyl)piperidin-4-yl)-2-fluorobenzyl)oxy)phenyl)pyridin -2-yl)-5-(trifluoromethyl)-1H-pyrazole-4-carboxylic acid |
| 61 | 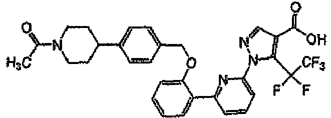 | 1-(6-(2-((4-(1-acetylpiperidin-4-yl)benzyl)oxy)phenyl)pyridin-2-yl)-5-(pentafluoroethyl)-1H-pyrazole-4-carboxylic acid |
| 62 | 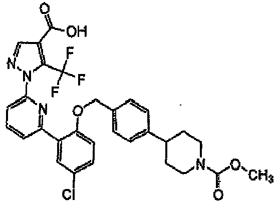 | 1-(6-(5-chloro-2-((4-(1-(methoxycarbonyl)piperidin-4-yl)benzyl)oxy)phenyl)pyridin-2-yl)-5-(trifluoromethyl)-1H-pyrazole-4-carboxylic acid |
| 63 | 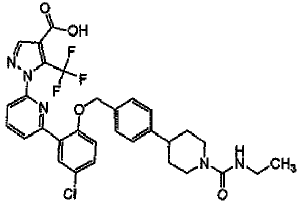 | 1-(6-(5-chloro-2-((4-(1-((ethylamino)carbonyl)piperidin -4-yl)benzyl)oxy)phenyl)pyridin-2-yl)-5-(trifluoromethyl)-1H pyrazole-4-carboxylic acid |
| 64 | 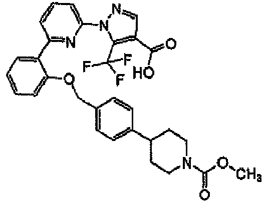 | 1-(6-(2-((4-(1-(methoxycarbonyl)piperidin-4-yl)benzyl)oxy)phenyl)pyridin-2-yl)-5-(trifluoromethyl)-1H-pyrazole-4-carboxylic acid |
| 65 | 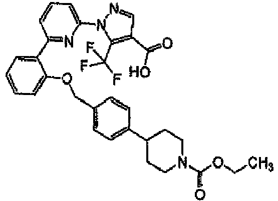 | 1-(6-(2-((4-(1-(ethoxycarbonyl)piperidin-4-yl)benzyl)oxy)phenyl)pyridin-2-yl)-5-(trifluoromethyl)-1H-pyrazole-4-carboxylic acid |
| 66 | 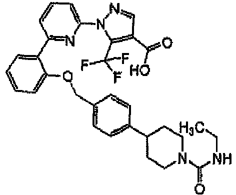 | 1-(6-(2-((4-(1-((ethylamino)carbonyl)piperidin -4-yl)benzyl)oxy)phenyl)pyridin-2-yl)-5-(trifluoromethyl)-1H-pyrazole-4-carboxylic acid |
| 67 | 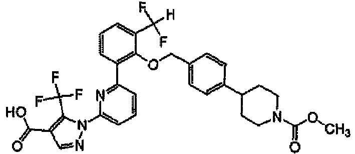 | 1-(6-(3-(difluoromethyl)-2-((4-(1-(methoxycarbonyl)piperidin-4-yl)benzyl)oxy)phenyl)pyridin-2-yl)-5-(trifluoromethyl)-1H-pyrazole-4-carboxylic acid |
| 68 | 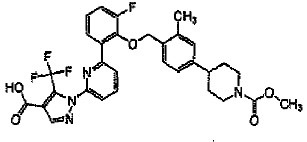 | 1-(6-(3-fluoro-2-((4-(1-(methoxycarbonyl)piperidin-4-yl)-2-methylbenzyl)oxy)phenyl)pyridi n-2-yl)-5-(trifluoromethyl)-1H-pyrazole-4-carboxylic acid |
| 69 | 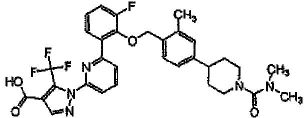 | 1-(6-(2-((4-(1-((dimethylamino)carbonyl)piper idin-4-yl)-2-methylbenzyl)oxy)-3-fluorophenyl)pyridin-2-yl)-5-(trifluoromethyl)-1H-pyrazole-4-carboxylic acid |
| 70 | 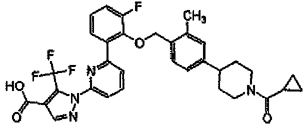 | 1-(6-(2-((4-(1-(cyclopropylcarbonyl)piperidin-4-yl)-2-methylbenzyl)oxy)-3-fluorophenyl)pyridin-2-yl)-5-(trifluoromethyl)-1H-pyrazole-4-carboxylic acid |
| 71 | 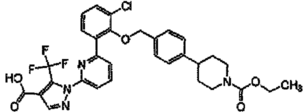 | 1-(6-(3-chloro-2-((4-(1-(ethoxycarbonyl)piperidin-4-yl)benzyl)oxy)phenyl)pyridin-2-yl)-5-(trifluoromethyl)-1H-pyrazole-4-carboxylic acid |
| 72 | 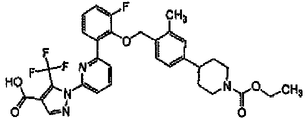 | 1-(6-(2-((4-(1-(ethoxycarbonyl)piperidin-4-yl)-2-methylbenzyl)oxy)-3-fluorophenyl)pyridin-2-yl)-5-(trifluoromethyl)-1H-pyrazole-4-carboxylic acid |
| 73 | 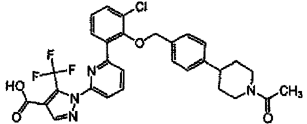 | 1-(6-(2-((4-(1-acetylpiperidin-4-yl)benzyl)oxy)-3-chlorophenyl)pyridin-2-yl)-5-(trifluoromethyl)-1H-pyrazole-4-carboxylic acid |
| 74 | 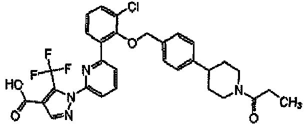 | 1-(6-(3-chloro-2-((4-(1-propionylpiperidin-4-yl)benzyl)oxy)phenyl)pyridin-2-yl)-5-(trifluoromethyl)-1H-pyrazole-4-carboxylic acid |
| 75 | 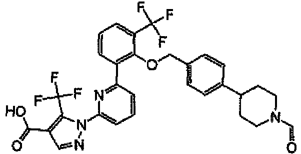 | 1-(6-(2-((4-(1-formylpiperidin-4-yl)benzyl)oxy)-3-(trifluoromethyl)phenyl)pyridin-2-yl)-5-(trifluoromethyl)-1H-pyrazole-4-carboxylic acid |
| 76 | 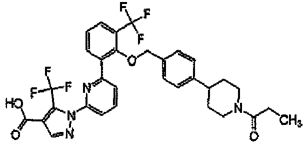 | 1-(6-(2-((4-(1-propionylpiperidin-4-yl)benzyl)oxy)-3-(trifluoromethyl)phenyl)pyridin-2-yl)-5-(trifluoromethyl)-1H-pyrazole-4-carboxylic acid |
| 77 | 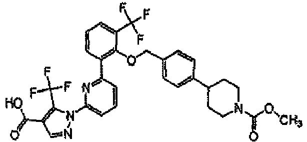 | 1-(6-(2-((4-(1-(methoxycarbonyl)piperidin-4-yl)benzyl)oxy)-3-(trifluoromethyl)phenyl)pyridin-2-yl)-5-(trifluoromethyl)-1H-pyrazole-4-carboxylic acid |
| 78 | 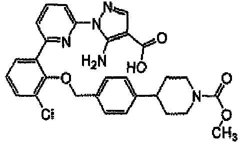 | 5-amino-1-(6-(3-chloro-2-((4-(1-(methoxycarbonyl)piperidin-4-yl)benzyl)oxy)phenyl)pyridin-2-yl)-1H-pyrazole-4-carboxylic acid |
| 79 | 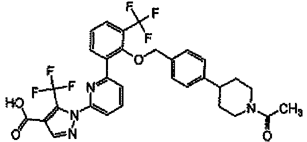 | 1-(6-(2-((4-(1-acetylpiperidin-4-yl)benzyl)oxy)-3-(trifluoromethyl)phenyl)pyridin-2-yl)-5-(trifluoromethyl)-1H-pyrazole-4-carboxylic acid |
| 80 | 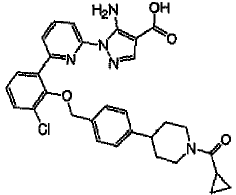 | 5-amino-1-(6-(3-chloro-2-((4-(1-(cyclopropylcarbonyl)piperidin-4-yl)benzyl)oxy)phenyl)pyridin-2-yl)-1H-pyrazole-4-carboxylic acid |
| 81 | 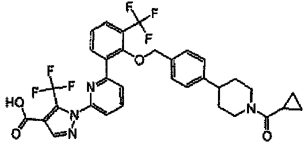 | 1-(6-(2-((4-(1-(cyclopropylcarbonyl)piperidin-4-yl)benzyl)oxy)-3-(trifluoromethyl)phenyl)pyridin-2-yl)-5-(trifluoromethyl)-1H-pyrazole-4-carboxylic acid |
| 82 | 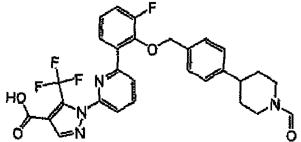 | 1-(6-(3-fluoro-2-((4-(1-formylpiperidin-4-yl)benzyl)oxy)phenyl)pyridin-2-yl)-5-(trifluoromethyl)-1H-pyrazole-4-carboxylic acid |
| 83 | 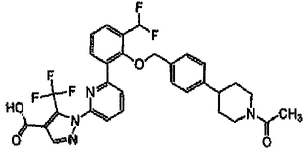 | 1-(6-(2-((4-(1-acetylpiperidin-4-yl)benzyl)oxy)-3-(difluoromethyl)phenyl)pyridin-2-yl)-5-(trifluoromethyl)-1H-pyrazole-4-carboxylic acid |
| 84 | 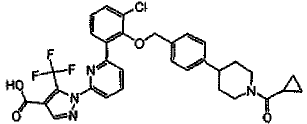 | 1-(6-(3-chloro-2-((4-(1-(cyclopropylcarbonyl)piperidin-4-yl)benzyl)oxy)phenyl)pyridin-2-yl)-5-(trifluoromethyl)-1H-pyrazole-4-carboxylic acid |
| 85 | 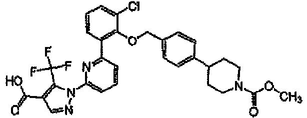 | 1-(6-(3-chloro-2-((4-(1-(methoxycarbonyl)piperidin-4-yl)benzyl)oxy)phenyl)pyridin-2-yl)-5-(trifluoromethyl)-1H-pyrazole-4-carboxylic acid |
| 86 | 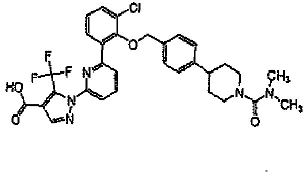 | 1-(6-(3-chloro-2-((4-(1-((dimethylamino)carbonyl)piper idin-4-yl)benzyl)oxy)phenyl)pyridin-2-yl)-5-(trifluoromethyl)-1H-pyrazole-4-carboxylic acid |
| 87 | 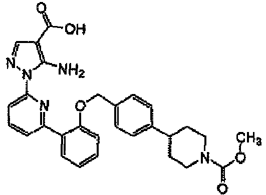 | 5-amino-1-(6-(2-((4-(1-(methoxycarbonyl)piperidin-4-yl)benzyl)oxy)phenyl)pyridin-2-yl)-1H-pyraxole-4-carboxylic acid |
| 88 | 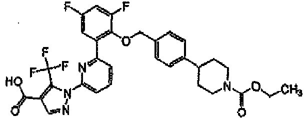 | 1-(6-(2-((4-(1-(ethoxycarbonyl)piperidin-4-yl)benzyl)oxy)-3,5-difluorophenyl)pyridin-2-yl)-5-(trifluoromethyl)-1H-pyrazole-4-carboxylic acid |
| 89 | 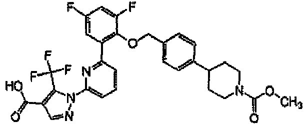 | 1-(6-(2-((4-(1-(methoxycarbonyl)piperidin-4-yl)benzyl)oxy)-3,5-difluorophenyl)pyridin-2-yl)-5-(trifluoromethyl)-1H-pyrazole-4-carboxylic acid |
| 90 | 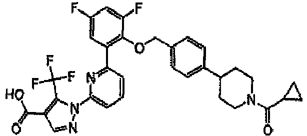 | 1-(6-(2-((4-(1-(cyclopropylcarbonyl)piperidin-4-yl)benzyl)oxy)-3,5-difluorophenyl)pyridin-2-yl)-5-(trifluoromethyl)-1H-pyrazole-4-carboxylic acid |
| 91 | 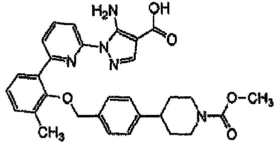 | 5-amino-1-(6-(2-((4-(1-(methoxycarbonyl)piperidin-4-yl)benzyl)oxy)-3-methylphenyl)pyridin-2-yl)-1H-pyrazole-4-carboxylic acid |
| 92 | 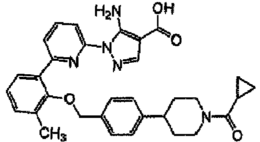 | 5-amino-1-(6-(2-((4-(1-(cyclopropylcarbonyl)piperidin-4-yl)benzyl)oxy)-3-methylphenyl)pyridin-2-yl)-1H-pyrazole-4-carboxylic acid |
| 93 | 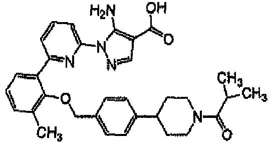 | 5-amino-1-(6-(2-((4-(1-isobutyrylpiperidin-4-yl)benzyl)oxy)-3-methylphenyl)pyridin-2-yl)-1H-pyrazole-4-carboxylic acid |
| 94 | 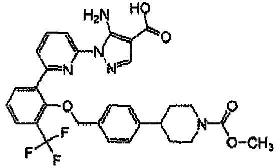 | 5-amino-1-(6-(2-((4-(1-(methoxycarbonyl)piperidin-4-yl)benzyl)oxy)-3-(trifluoromethyl)phenyl)pyridin-2-yl)-1H-pyrazole-4-carboxylic acid |
| 95 | 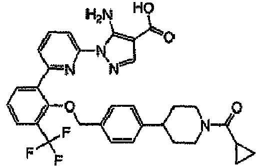 | 5-amino-1-(6-(2-((4-(1-(cyclopropylcarbonyl)piperidin-4-yl)benzyl)oxy)-3-(trifluoromethyl)phenyl)pyridin-2-yl)-1H-pyrazole-4-carboxylic acid |
| 96 | 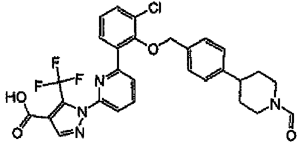 | 1-(6-(3-chloro-2-((4-(1-formylpiperidin-4-yl)benzyl)oxy)phenyl)pyridin-2-yl)-5-(trifluoromethyl)-1H-pyrazolo-4-carboxylic acid |
| 97 | 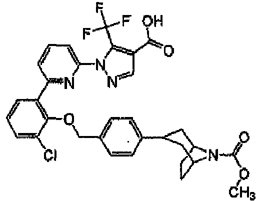 | 1-(6-(3-chloro-2-((4-(8-(methoxycarbonyl)-8-azabicyclo[3.2.1]oct-3-yl)benzyl)oxy)phenyl)pyridin-2-yl)-5-(trifluoromethyl)-1H-pyrazole-4-carboxylic acid |
| 98 | 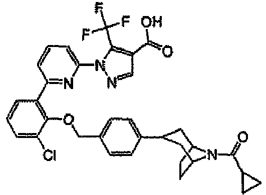 | 1-(6-(3-chloro-2-((4-(8-(cyclopropylcarbonyl)-8-azabicyclo[3.2.1]oct-3-yl)benzyl)oxy)phenyl)pyridin-2-yl)-5-(trifluoromethyl)-1H-pyrazole-4-carboxylic acid |
| 99 | 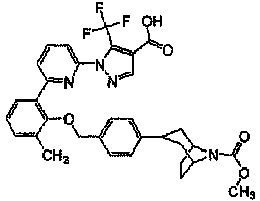 | 1-(6-(2-((4-(8-(methoxycarbonyl)-8-azabicyclo[3.2.1)oct-3-yl)benzyl)oxy)-3-methylphenyl)pyridin-2-yl)-5-(trifluoromethyl)-1H-pyrazole-4-carboxylic acid |
| 100 | 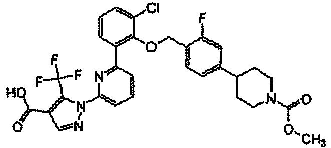 | 1-(6-(3-chloro-2-((2-fluoro-4-(1-(methoxycarbonyl)piperidin-4-yl)benzyl)oxy)phenyl)pyridin-2-yl)-5-(trifluoromethyl)-1H-pyrazole-4-carboxylic acid |
| 101 | 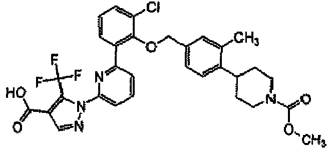 | 1-(6-(3-chloro-2-((4-(1-(methoxycarbonyl)piperidin-4-yl)-3-methylbenzyl)oxy)phenyl)pyridi n-2-yl)-5-(trifluoromethyl)-1H-pyrazole-4-carboxylic acid |
| 102 | 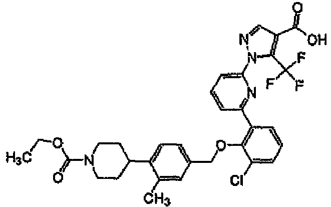 | 1-(6-(3-chloro-2-((4-(1-(ethoxycarbonyl)piperidin-4-yl)-3-methylbenzyl)oxy)phenyl)pyridi n-2-yl)-5-(trifluoromethyl)-1H-pyrazole-4-carboxylic acid |
| 103 | 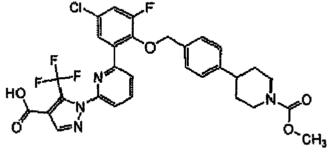 | 1-(6-(5-chloro-3-fluoro-2-((4-(1-(methoxycarbonyl)piperidin-4-yl)benzyl)oxy)phenyl)pyridin-2-yl)-5-(trifluoromethyl)-1H-pyrazole-4-carboxylic acid |
| 104 | 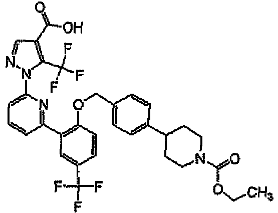 | 1-(6-(2-((4-(1-(ethoxycarbonyl)piperidin-4-yl)benzyl)oxy)-5-(trifluoromethyl)phenyl)pyridin-2-yl)-5-(trifluoromethyl)-1H-pyrazole-4-carboxylic acid |
| 105 | 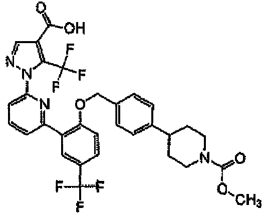 | 1-(6-(2-((4-(1-(methoxycarbonyl)piperidin-4-yl)benzyl)oxy)-5-(trifluoromethyl)phenyl)pyridin-2-yl)-5-(trifluoromethyl)-1H-pyrazole-4-carboxylic acid |
| 106 | 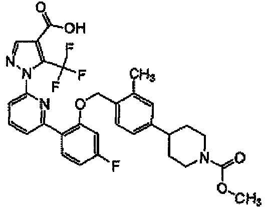 | 1-(6-(4-fluoro-2-((4-(1-(methoxycarbonyl)piperidin-4-yl)-2-methylbenzyl)oxy)phenyl)pyridi n-2-yl)-5-(trifluoromethyl)-1H-pyrazole-4-carboxylic acid |
| 107 | 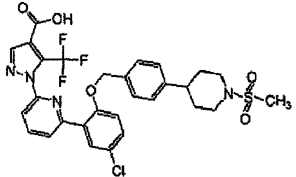 | 1-(6-(5-chloro-2-((4-(1-(methylsulfonyl)piperidin-4-yl)benzyl)oxy)phenyl)pyridin-2-yl)-5-(trifluoromethyl)-1H-pyrazole-4-carboxylic acid |
| 108 | 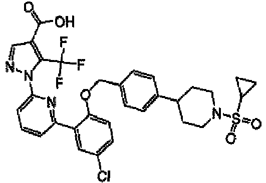 | 1-(6-(5-chloro-2-((4-(1-(cyclopropylsulfonyl)piperidin-4-yl)benzyl)oxy)phenyl)pyridin-2-yl)-5-(trifluoromethyl)-1H-pyrazole-4-carboxylic acid |
| 109 | 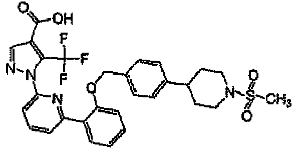 | 1-(6-(2-((4-(1-(methylsulfonyl)piperidin-4-yl)benzyl)oxy)phenyl)pyridin-2-yl)-5-(trifluoromethyl)-1H-pyrazole-4-carboxylic acid |
| 110 | 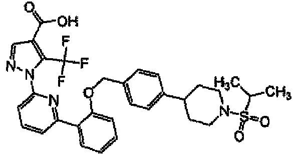 | 1-(6-(2-((4-(1-(isopropylsulfonyl)piperidin-4-yl)benzyl)oxy)phenyl)pyridin-2-yl)-5-(trifluoromethyl)-1H-pyrazole-4-carboxylic acid |
| 111 | 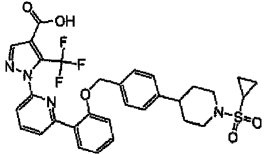 | 1-(6-(2-((4-(1-(cyclopropylsulfonyl)piperidin-4-yl)benzyl)oxy)phenyl)pyridin-2-yl)-5-(trifluoromethyl)-1H-pyrazole-4-carboxylic acid |
| 112 | 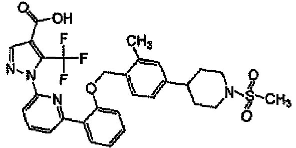 | 1-(6-(2-((2-methyl-4-(1-(methylsulfonyl)piperidin-4-yl)benzyl)oxy)phenyl)pyridin-2-yl)-5-(trifluoromethyl)-1H-pyrazole-4-carboxylic acid |
| 113 | 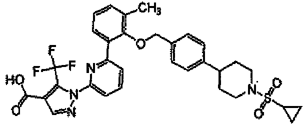 | 1-(6-(2-((4-(1-(cyclopropylsulfonyl)piperidin-4-yl)benzyl)oxy)-3-methylphenyl)pyridin-2-yl)-5-(trifluoromethyl)-1H-pyrazole-4-carboxylic acid |
| 114 | 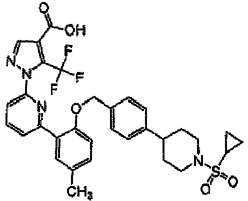 | 1-(6-(2-((4-(1-(cyclopropylsulfonyl)piperidin-4-yl)benzyl)oxy)-5-methylphenyl)pyridin-2-yl)-5-(trifluoromethyl)-1H-pyrazole-4-carboxylic acid |
| 115 | 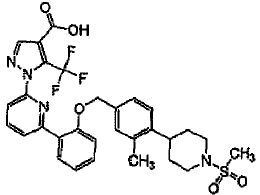 | 1-(6-(2-((3-methyl-4-(1-(methylsulfonyl)piperidin-4-yl)benzyl)oxy)phenyl)pyridin-2-yl)-5-(trifluoromethyl)-1H-pyrazole-4-carboxylic acid |
| 116 | 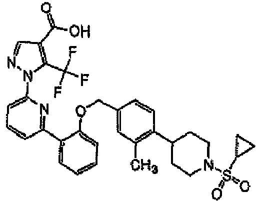 | 1-(6-(2-((4-(1-(cyclopropylsulfonyl)piperidin-4-yl)-3-methylbenzyl)oxy)phenyl)pyridi n-2-yl)-5-(trifluoromethyl)-1H-pyrazole-4-carboxylic acid |
| 117 | 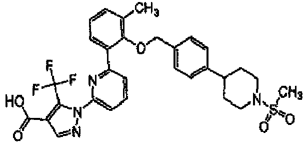 | 1-(6-(3-methyl-2-((4-(1-(methylsulfonyl)piperidin-4-yl)benzyl)oxy)phenyl)pyridin-2-yl)-5-(trifluoromethyl)-1H-pyrazole-4-carboxylic acid |
| 118 | 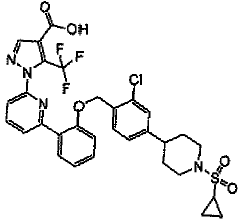 | 1-(6-(2-((2-chloro-4-(1-(cyclopropylsulfonyl)piperidin-4-yl)benzyl)oxy)phenyl)pyridin-2-yl)-5-(trifluoromethyl)-1H-pyrazole-4-carboxylic acid |
| 119 | 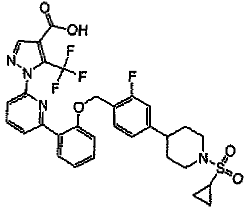 | 1-(6-(2-((4-(1-(cyclopropylsulfonyl)piperidin-4-yl)-2-fluorobenzyl)oxy)phenyl)pyridin -2-yl)-5-(trifluoromethyl)-1H-pyrazole-4-carboxylic acid |
| 120 | 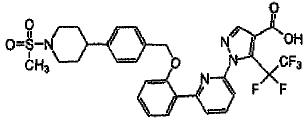 | 1-(6-(2-((4-(1-(methylsulfonyl)piperidin-4-yl)benzyl)oxy)phenyl)pyridin-2-yl)-5-(pentafluoroethyl)-1H-pyrazole-4-carboxylic acid |
| 121 | 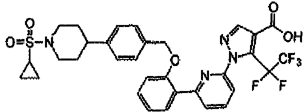 | 1-(6-(2-((4-(1-(cyclopropylsulfonyl)piperidin-4-yl)benzyl)oxy)phenyl)pyridin-2-yl)-5-(pentafluoroethyl)-1H-pyrazole-4-carboxylic acid |
| 122 | 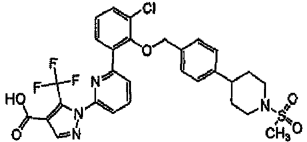 | 1-(6-(3-chloro-2-((4-(1-(methylsulfonyl)piperidin-4-yl)benzyl)oxy)phenyl)pyridin-2-yl)-5-(trifluoromethyl)-1H-pyrazole-4-carboxylic acid |
| 123 | 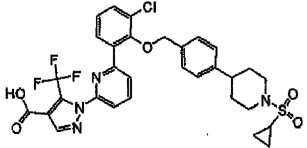 | 1-(6-(3-chloro-2-((4-(1-(cyclopropylsulfonyl)piperidin-4-yl)benzyl)oxy)phenyl)pyridin-2-yl)-5-(trifluoromethyl)-1H-pyrazole-4-carboxylic acid |
| 124 | 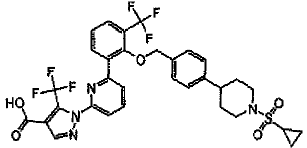 | 1-(6-(2-((4-(1-(cyclopropylsulfonyl)piperidin-4-yl)benzyl)oxy)-3-(trifluoromethyl)phenyl)pyridin-2-yl)-5-(trifluoromethyl)-1H-pyrazole-4-carboxylic acid |
| 125 | 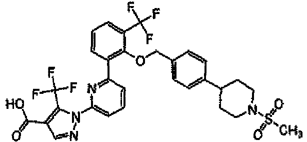 | 1-(6-(2-((4-(1-(methylsulfonyl)piperidin-4-yl)benzyl)oxy)-3-(trifluoromethyl)phenyl)pyridin-2-yl)-5-(trifluoromethyl)-1H-pyrazole-4-carboxylic acid |
| 126 | 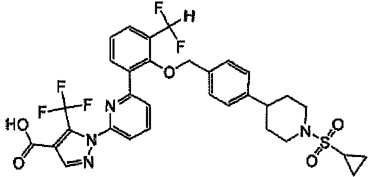 | 1-(6-(2-((4-(1-(cyclopropylsulfonyl)piperidin-4-yl)benzyl)oxy)-3-(difluoromethyl)phenyl)pyridin-2-yl)-5-(trifluoromethyl)-1H-pyrazole-4-carboxylic acid |
| 127 | 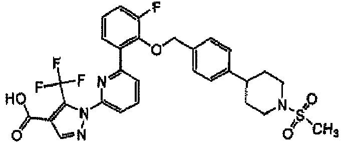 | 1-(6-(3-fluoro-2-((4-(1-(methylsulfonyl)piperidin-4-yl)benzyl)oxy)phenyl)pyridin-2-yl)-5-(trifluoromethyl)-1H-pyrazole-4-carboxylic acid |
| 128 | 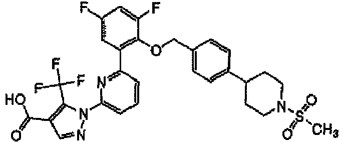 | 1-(6-(3,5-difluoro-2-((4-(1-(methylsulfonyl)piperidin-4-henyl)pyridin-2-yl)-5-(trifluoromethyl)-1H-pyrazole-4-carboxylic acid |
| 129 | 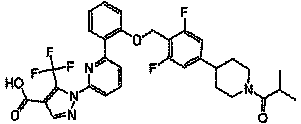 | 1-(6-(2-((2,6-difluoro-4-(1-isobutyrylpiperidin-4-yl)benzyl)oxy)phenyl)pyridin-2-yl)-5-(trifluoromethyl)-1H-pyrazole-4-carboxylic acid |
| 130 | 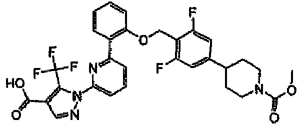 | 1-(6-(2-((2,6-difluoro-4-(1-(methoxycarbonyl)piperidin-4-yl)benzyl)oxy)phenyl)pyridin-2-yl)-5-(trifluoromethyl)-1H-pyrazole-4-carboxylic acid |
| 131 | 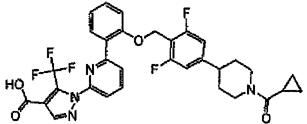 | 1-(6-(2-((4-(1-(cyclopropylcarbonyl)piperidin-4-yl)-2,6-difluorobenzyl)oxy)phenyl)pyrid in-2-yl)-5-(trifluoromethyl)-1H-pyrazole-4-carboxylic acid |
| 132 | 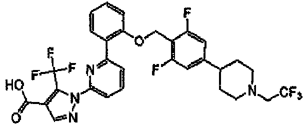 | 1-(6-(2-((2,6-difluoro-4-(1-(2,2,2-trifluoroethyl)piperidin-4-yl)benzyl)oxy)phenyl)pyridin-2-yl)-5-(trifluoromethyl)-1H-pyrazole-4-carboxylic acid |
| 133 | 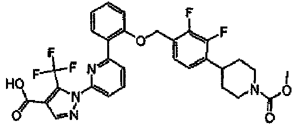 | 1-(6-(2-((2,3-difluoro-4-(1-(methoxycarbonyl)piperidin-4-yl)benzyl)oxy) phenyl)pyridin-2-yl)-5-(trifluoromethyl)-1H-pyrazole-4-carboxylic acid |
| 134 | 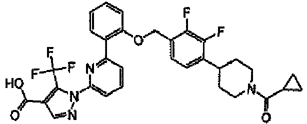 | 1-(6-(2-((4-(1-(cyclopropylcarbonyl)piperidin-4-yl)-2,3-difluorobenzyl) oxy)phenyl)pyridin-2yl)-5-(trifluoromethyl)-1H-pyrazole-4-carboxylic acid |
| 135 | 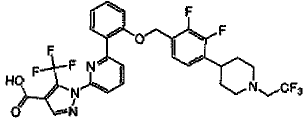 | 1-(6-(2-((2,3-difluoro-4-(1-(2,2,2-trifluoroethyl)piperidin-4-yl)benzyl)oxy)phenyl)pyridin-2-yl)-5-(trifluoromethyl)-1H-pyrazole-4-carboxylic acid |
Example **136** 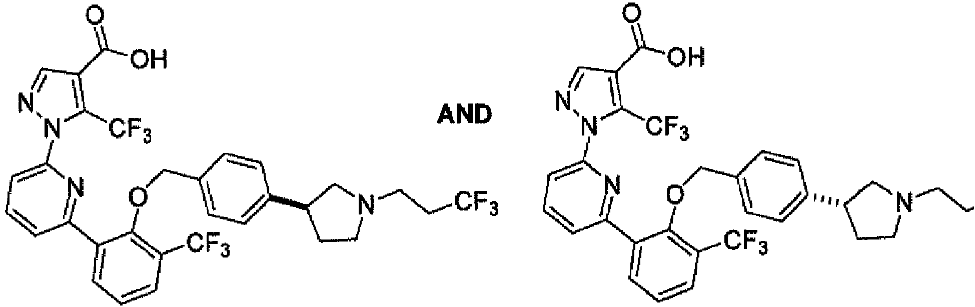 The compounds in Table 2 were prepared using chemistry described in Example 136, or by analogy to chemistry described in Examples **1-14.**
**Table 2**
| Example | Structure | IUPAC |
|---|---|---|
| 137 | 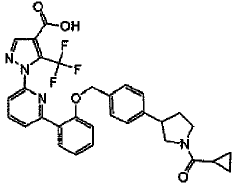 | 1-(6-(2-((4-(1-(cyclopropylcarbonyl)pyrrolidin-3-yl)benzyl)oxy)phenyl)pyridin-2-yl)-5-(trifluoromethyl)-1H-pyrazole-4-carboxylic acid |
| 138, 139 | 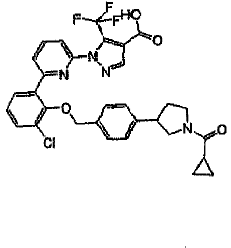 | 1-(6-(3-chloro-2-((4-((3R)-1-(cyclopropylcarbonyl)pyrrolidin-3-yl)benzyl)oxy)phenyl)pyridin-2-yl)-5-(trifluoromethyl)-1H-pyrazole-4-carboxylic acid And 1-(6-(3-chloro-2-((4-((3S)-1-(cyclopropylcarbonyl)pyrrolidin-3-yl)benzyl)oxy)phenyl)pyridin-2-yl)-5-(trifluoromethyl)-1H-pyrazole-4-carboxylic acid |
| 140, 141 | 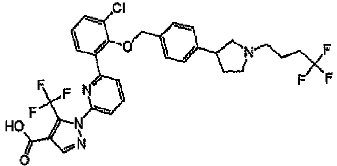 | 1-(6-(3-chloro-2-((4-(3R)-(1-(4,4,4-trifluorobutyl)pyrrolidin-3-yl)benzyl)oxy)phenyl)pyridin-2-yl)-5-(trifluoromethyl)-1H-pyrazole-4-carboxylic acid And 1-(6-(3-chloro-2-((4-(3S)-(1-(4,4,4-trifluorobutyl)pyrrolidin-3-yl)benzyl)oxy)phenyl)pyridin-2-yl)-5-(trifluoromethyl)-1H-pyrazole-4-carboxylic acid |
| 142 | 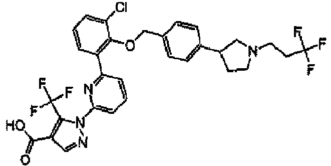 | 1-(6-(3-chloro-2-((4-(1-(3,3,3-trifluoropropyl)pyrrolidin-3-yl)benzyl)oxy)phenyl)pyridin-2-yl)-5-(trifluoromethyl)-1H-pyrazole-4-carboxylic acid |
| 143, 144 | 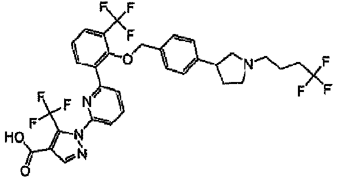 | 1-(6-(2-((4-(3R)-(1-(4,4,4-trifluorobutyl)pyrrolidin-3-yl)benzyl)oxy)-3-(trifluoromethyl)phenyl)pyridin-2-yl)-5-(trifluoromethyl)-1H-pyrazole-4-carboxylic acid AND 1-(6-(2-((4-(3S)-(1-(4,4,4-trifluorobutyl)pyrrolidin-3-yl)benzyl)oxy)-3-(trifluoromethyl)phenyl)pyridin-2-yl)-5-(trifluoromethyl)-1H-pyrazole-4-carboxylic acid |
| 145 | 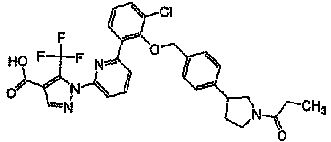 | 1-(6-(3-chloro-2-((4-(1-propionylpyrrolidin-3-yl)benzyl)oxy)phenyl)pyridin-2-yl)-5-(trifluoromethyl)-1H-pyrazole-4-carboxylic acid |
| 146 | 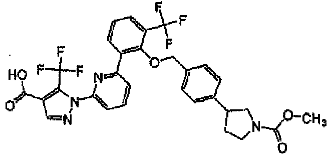 | 1-(6-(2-((4-(1-(methoxycarbonyl)pyrrolidin-3-yl)benzyl)oxy)-3-(trifluoromethyl)phenyl)pyridin-2-yl)-5-(trifluoromethyl)-1H-pyrazole-4-carboxylic acid |
| 147 | 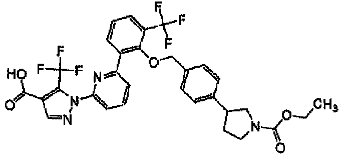 | 1-(6-(2-((4-(1-(ethoxycarbonyl)pyrrolidin-3-yl)benzyl)oxy)-3-(trifluoromethyl)phenyl)pyridin-2-yl)-5-(trifluoromethyl)-1H-pyrazole-4-carboxylic acid |
**Example 148** 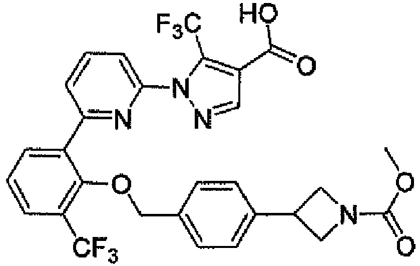 The compounds listed in **Table 3** were prepared using chemistry described in **Example 148,** and/or by analogy to chemistry described in Examples **1-14** and **Example 136.**
**Table 3**
| Examples | Structure | IUPAC |
|---|---|---|
| 149 | 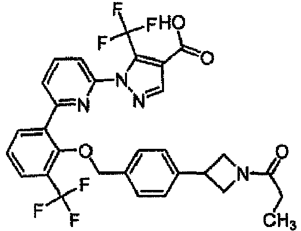 | 1-(6-(2-((4-(1-propionylazetidin-3-yl)benzyl)oxy)-3-(trifluoromethyl)phenyl)pyridin-2-yl)-5-(trifluoromethyl)-1H-pyrazole-4-carboxylic acid |
| 150 | 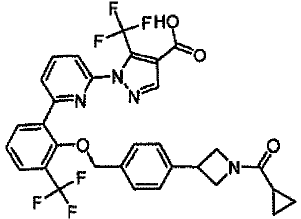 | 1-(6-(2-((4-(1-(cyclopropylcarbonyl)azetidin-3-yl)benzyl)oxy)-3-(trifluoromethyl)phenyl)pyridin-2-yl)-5-(trifluoromethyl)-1H-pyrazole-4-carboxylic acid |
| 151 | 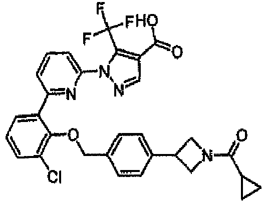 | 1-(6-(3-chloro-2-((4-(1-(cyclopropylcarbonyl)azetidin-3-yl)benzyl)oxy)phenyl)pyridin-2-yl)-5-(trifluoromethyl)-1H-pyrazole-4-carboxylic acid |
| 152 | 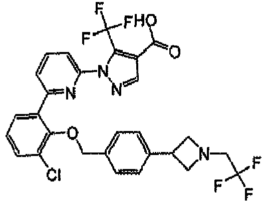 | 1-(6-(3-chloro-2-((4-(1-(2,2,2-trifluoroethyl)azetidin-3-yl)benzyl)oxy)phenyl)pyridin-2-yl)-5-(trifluoromethyl)-1H-pyrazole-4-carboxylic acid |
| 153 | 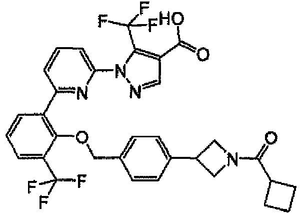 | 1-(6-(2-((4-(1-(cyclobutylcarbonyl)azetidin-3-yl)benzyl)oxy)-3-(trifluoromethyl)phenyl)pyridin-2-yl)-5-(trifluoromethyl)-1H-pyrazole-4-carboxylic acid |
| 154 | 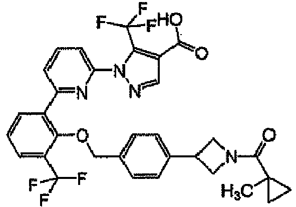 | 1-(6-(2-((4-(1-((1-methylcyclopropyl)carbonyl)aze tidin-3-yl)benzyl)oxy)-3-(trifluoromethyl)phenyl)pyridin-2-yl)-5-(trifluoromethyl)-1H-pyrazole-4-carboxylic acid |
| 155 | 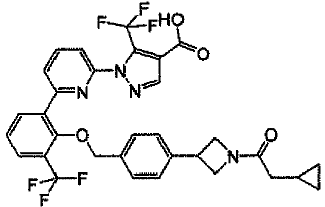 | 1-(6-(2-((4-(1-(cyclopropylacetyl)azetidin-3-yl)benzyl)oxy)-3-(trifluoromethyl)phenyl)pyridin-2-yl)-5-(trifluoromethyl)-1H-pyrazole-4-carboxylic acid |
| 156 | 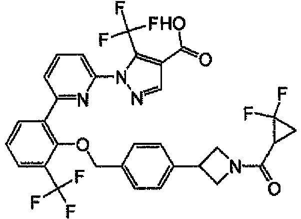 | 1-(6-(2-((4-(1-((2,2-difluorocyclopropyl)carbonyl)az etidin-3-yl)benzyl)oxy)-3-(trifluoromethyl)phenyl)pyridin-2-yl)-5-(trifluoromethyl)-1H-pyrazole-4-carboxylic acid |
| 157 | 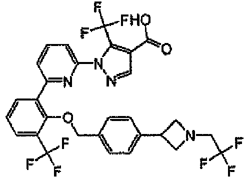 | 1-(6-(2-((4-(1-(2,2,2-trifluoroethyl)azetidin-3-yl)benzyl)oxy)-3-(trifluoromethyl)phenyl)pyridin-2-yl)-5-(trifluoromethyl)-1H-pyrazole-4-carboxylic acid |
| 158 | 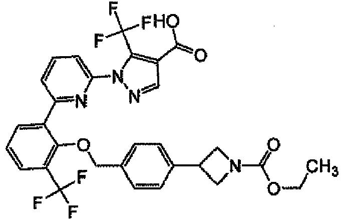 | 1-(6-(2-((4-(1-(ethoxycarbonyl)azetidin-3-yl)benzyl)oxy)-3-(trifluoromethyl)phenyl)pyridin-2-yl)-5-(trifluoromethyl)-1H-pyrazole-4-carboxylic acid |
| 159 | 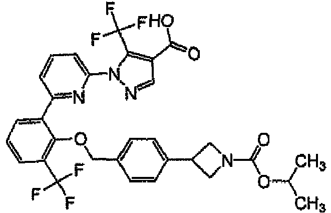 | 1-(6-(2-((4-(1-(isopropoxycarbonyl)azetidin-3-yl)benzyl)oxy)-3-(trifluoromethyl)phenyl)pyridin-2-yl)-5-(trifluoromethyl)-1H-pyrazole-4-carboxylic acid |
| 160 | 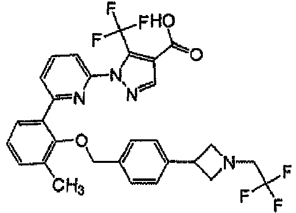 | 1-(6-(3-methyl-2-((4-(1-(2,2,2-trifluoroethyl)azetidin-3-yl)benzyl)oxy)phenyl)pyridin-2-yl)-5-(trifluoromethyl)-1H-pyrazole-4-carboxylic acid |
| 161 | 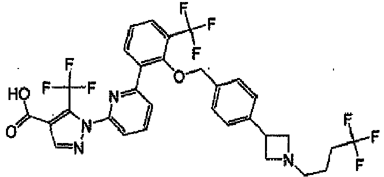 | 1-(6-(2-((4-(1-(4,4,4-trifluorobutyl)azetidin-3-yl)benzyl)oxy)-3-(trifluoromethyl)phenyl)pyridin-2-yl)-5-(trifluoromethyl)-1H-pyrazole-4-carboxylic acid |
**Example 162** 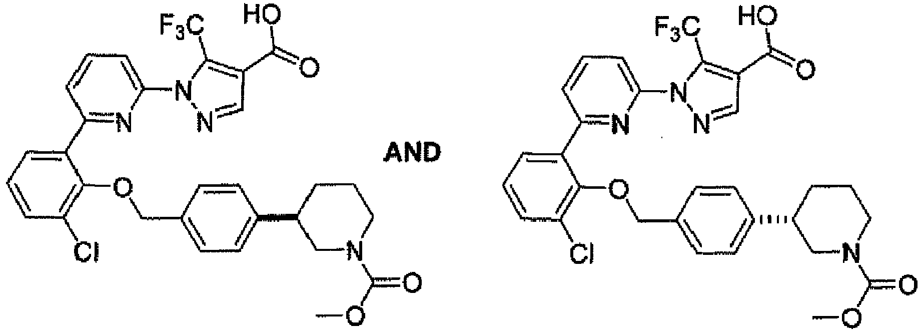 The compounds listed in **Table 4** were prepared using chemistry described in **Example 162,** and/or by analogy to chemistry described in Examples **1-14, Example 136,** and **Example 148.**
**Table 4**
| Example | Structure | IUPAC |
|---|---|---|
| 163, 164 | 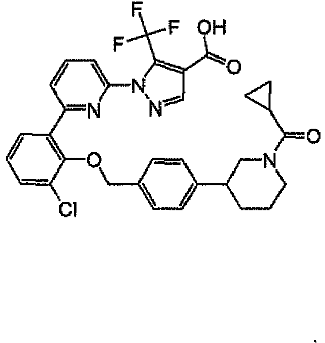 | 1-(6-(3-chloro-2-((4-(3R)-(1-(cyclopropylcarbonyl)piperidin-3-yl)benzyl)oxy)phenyl)pyridin-2-yl)-5-(trifluoromethyl)-1H-pyrazole-4-carboxylic acid AND 1-(6-(3-chloro-2-((4-(3S)-(1-(cyclopropylcarbonyl)piperidin-3-yl)benzyl)oxy)phenyl)pyridin-2-yl)-5-(trifluoromethyl)-1H-pyrazole-4-carboxylic acid |
| 165, 166 | 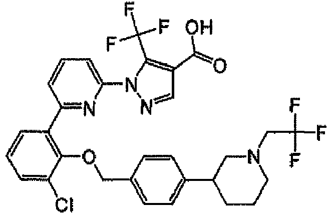 | 1-(6-(3-chloro-2-((4-(3R)-(1-(2,2,2-trifluoroethyl)piperidin-3-yl)benzyl)oxy)phenyl)pyridin-2-yl)-5-(trifluoromethyl)-1H-pyrazole-4-carboxylic acid **AND** 1-(6-(3-chloro-2-((4-(3S)-(1-(2,2,2-trifluoroethyl)piperidin-3-yl)benzyl)oxy)phenyl)pyridin-2-yl)-5-(trifluoromethyl)-1H-pyrazole-4-carboxylic acid |
**Example 167** 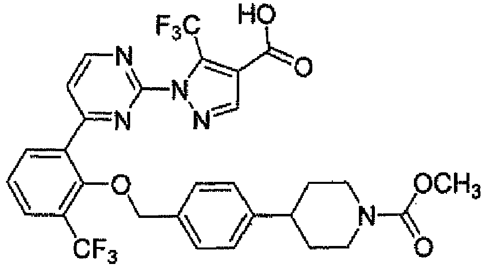 The compounds listed in **Table 5** were prepared using chemistry described in **Example 167,** and/or by analogy to chemistry described in **Examples 1-14, Examples 136, 148,** and **162.**
**Table 5.**
| Example | Structure | IUPAC |
|---|---|---|
| 168 | 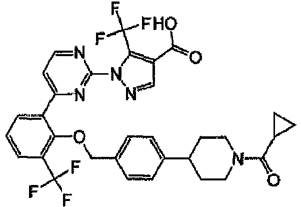 | 1-(4-(2-((4-(1-(cyclopropylcarbonyl)piperidin-4-yl)benzyl)oxy)-3-(trifluoromethyl)phenyl)pyrimidin-2-yl)-5-(trifluoromethyl)-1H-pyrazole-4-carboxylic acid |
| 169 | 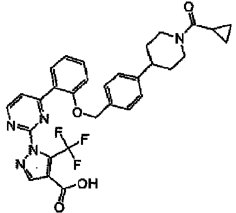 | 1-(4-(2-((4-(1-(cyclopropylcarbonyl)piperidin-4-yl)benzyl)oxy)phenyl)pyrimidin-2-yl)-5-(trifluoromethyl)-1H-pyrazole-4-carboxylic acid |
| 170 | 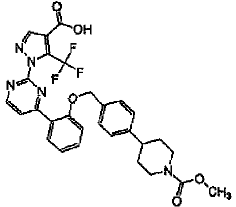 | 1-(4-(2-((4-(1-(methoxycarbonyl)piperidin-4-yl)benzyl)oxy)phenyl)pyrimidin-2-yl)-5-(trifluoromethyl)-1H-pyrazole-4-carboxylic acid |
| 171 | 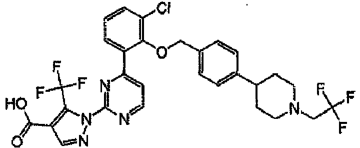 | 1-(4-(3-chloro-2-((4-(1-(2,2,2-trifluoroethyl)piperidin-4-yl)benzyl)oxy)phenyl)pyrimidin-2-yl)-5-(trifluoromethyl)-1H-pyrazole-4-carboxylic acid |
| 172 | 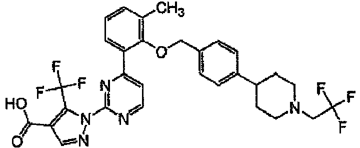 | 1-(4-(3-methyl-2-((4-(1-(2,2,2-trifluoroethyl)piperidin-4-yl)benzyl)oxy)phenyl)pyrimidin-2-yl)-5-(trifluoromethyl)-1H-pyrazole-4-carboxylic acid |
| 173 | 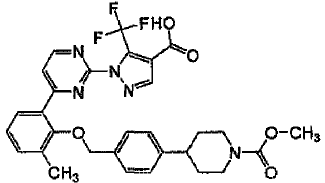 | 1-(4-(2-((4-(1-(methoxycarbonyl)piperidin-4-yl)benzyl)oxy)-3-methylphenyl)pyrimidin-2-yl)-5-(trifluoromethyl)-1H-pyrazole-4-carboxylic acid |
| 174 | 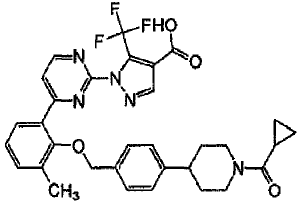 | 1-(4-(2-((4-(1-(cyclopropylcarbonyl)piperidin-4-yl)benzyl)oxy)-3-methylphenyl)pyrimidin-2-yl)-5-(trifluoromethyl)-1H-pyrazole-4-carboxylic acid |
| 175 | 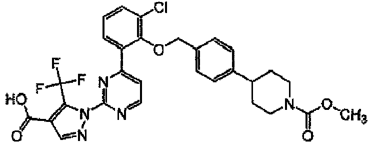 | 1-(4-(3-chloro-2-((4-(1-(methoxycarbonyl)piperidin-4-yl)benzyl)oxy)phenyl)pyrimidin-2-yl)-5-(trifluoromethyl)-1H-pyrazole-4-carboxylic acid |
| 176 | 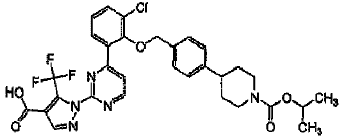 | 1-(4-(3-chloro-2-((4-(1-(isopropoxycarbonyl)piperidin-4-yl)benzyl)oxy)phenyl)pyrimidin-2-yl)-5-(trifluoromethyl)-1H-pyrazole-4-carboxylic acid |
| 177 | 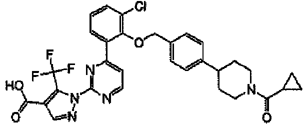 | 1-(4-(3-chloro-2-((4-(1-(cyclopropylcarbonyl)piperidin-4-yl)benzyl)oxy)phenyl)pyrimidin-2-yl)-5-(trifluoromethyl)-1H-pyrazole-4-carboxylic acid |
| 178 | 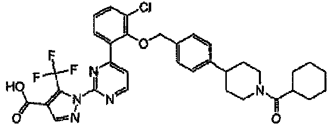 | 1-(4-(3-chloro-2-((4-(1-(cyclohexylcarbonyl)piperidin-4-yl)benzyl)oxy)phenyl)pyrimidin-2-yl)-5-(trifluoromethyl)-1H-pyrazole-4-carboxylic acid |
| 179 | 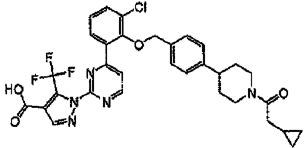 | 1-(4-(3-chloro-2-((4-(1-(cyclopropylacetyl)piperidin-4-yl)benzyl)oxy)phenyl)pyrimidin-2-yl)-5-(trifluoromethyl)-1H-pyrazole-4-carboxylic acid |
**Example 180** 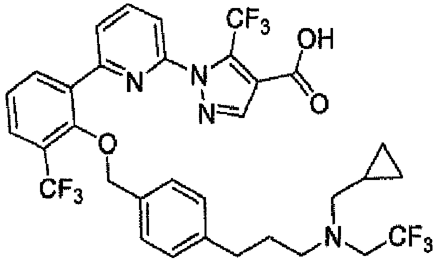 The compounds listed in **Table 6** were prepared using chemistry described in **Example 180,** and/or by analogy to chemistry described in **Examples 1-14, 136, 148, 162**, and **167.**
**Table 6**
| Example | Structure | IUPAC |
|---|---|---|
| 181 | 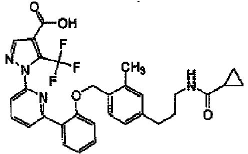 | 1-(6-(2-((4-(3-((cyclopropylcarbonyl)amino)prop yl)-2-methylbenzyl)ox y)phenyl)pyridin-2-yl)-5-(trifluoromethyl)-1H-pyrazole-4-carboxylic acid |
| 182 | 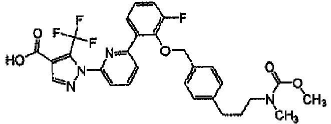 | 1-(6-(3-fluoro-2-((4-(3-((methoxycarbonyl)-(methyl)amino)propyl)benzyl)oxy) -phenyl)pyridin-2-yl)-5-(trifluoromethyl)--pyrazole-4-carboxylic acid |
| 183 | 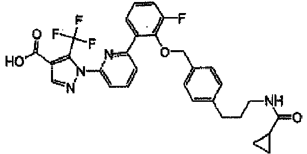 | 1-(6-(2-((4-(3-((cyclopropylcarbonyl)amino)prop yl) benzyl)oxy)-3-fluorophenyl)pyridin-2-yl)-5-(trifluoromethyl)-1H-pyrazole-4-carboxylic acid |
| 184 | 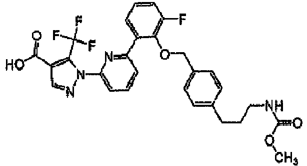 | 1-(6-(3-fluoro-2-((4-(3-((methoxycarbonyl)amino)propyl) benzyl)oxy)phenyl)pyridin-2-yl)-5-(trifluoromethyl)-1H-pyrazole-4-carboxylic acid |
| 185 | 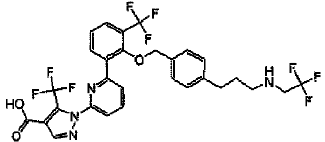 | 1-(6-(2-((4-(3-((2,2,2-trifluoroethyl)amino)propyl)benzyl )oxy)-3-(trifluoromethyl)phenyl)pyridin-2-yl)-5-(trifluoromethyl)-1H-pyrazole-4-carboxylic acid |
| 186 | 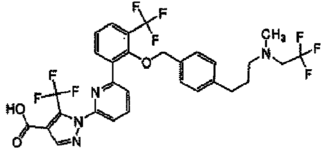 | 1-(6-(2-((4-(3-(methyl(2,2,2-trifluoroethyl)amino)propyl)benzyl )oxy)-3-(trifluoromethyl)phenyl)pyridin-2-yl)-5-(trifluoromethyl)-1H-pyrazole-4-carboxylic acid |
Compound Class V
**Example 1: 1-[6-(2-4-phenethyl)benzyloxy)-phenyl)pyridin-2-yl]-5-trifluoromethyl-pyrazole-4-carboxylic acid** 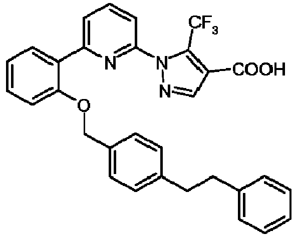 **Example 2: 1-[6-(2-(4-(4-trifluoromethylphenyl)benzyloxy)-phenyl)pyridin-2-yl]-5-trifluoromethyl-pyrazole-4-carboxylic acid** 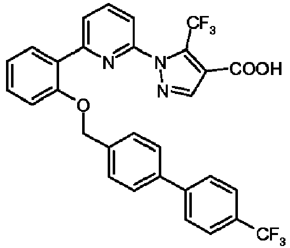 **Example 3: 1-[6-(2-(4-(4-methoxyphenyl)benzyloxy)-phenyl)pyridin-2-yl]-5-trifluoromethyl-pyrazole-4-carboxylic acid** 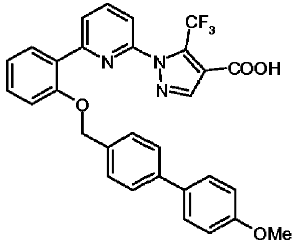 **Example 4: 1-[6-(5-chloro-2-(4-(4-methoxyphenyl)benzyloxy)-phenyl)pyridin-2-yl]-5-trifluoromethyl-pyrazole-4-carboxylic acid** 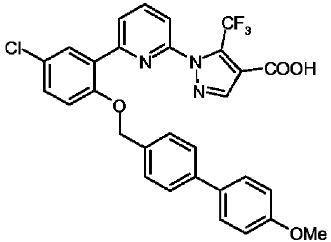 **Example 5: 1-[6-(5-fluoro-2-(4-(4-methoxyphenyl)benzyloxy)-phenyl)pyridin-2-yl]-5-trifluoromethyl-pyrazole-4-carboxylic acid** 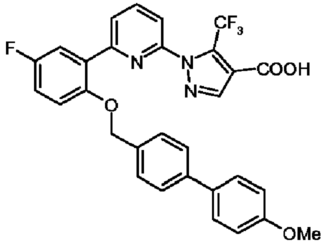 **Example 6: 1-[6-(3,5-difluoro-2-(4-(4-methoxyphenyl)benzyloxy)-phenyl)pyridin-2-yl]-5-trifluoromethyl-pyrazole-4-carboxylic** acid 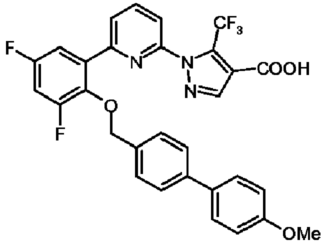 **Example 7: 1-[6-(2-(2-methyl-4-(4-fluorophenyl)benzyloxy)-phenyl)pyridin-2-yl]-5-trifluoromethyl-pyrazole-4-carboxylic acid** 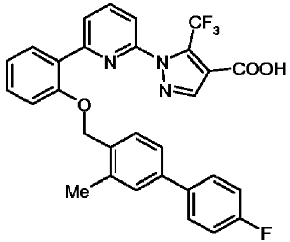 **Example 8**: **1-[6-(2-(4-(4-cyanophenoxy)benzyloxy)-phenyl)pyridin-2-yl]-5-trifluoromethyl-pyrazole-4-carboxylic acid** 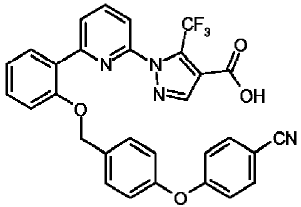 **Example 9: 1-[6-(2-(2-methyl-4-(4-trifluoromethoxyphenyl)benzyloxy)-phenyl)pyridin-2-yl]-5-trifluoromethyl-pyrazole-4-carboxylic** acid 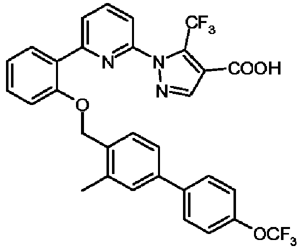 **Example 10: 1-[6-(5-methyl-2-(2-methyl-4-(3-chloro-5-trifluoromethylpyridin-2-yl)benzyloxy)-phenyl)pyridin-2-yl]-5-trifluoromethyl-pyrazole-4-carboxylic acid** 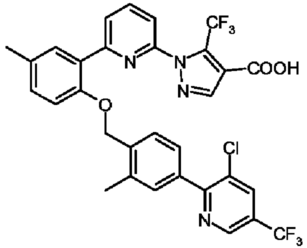
| **Example** | **Name** |
|---|---|
| 11 | 1-[6-(2-(4-(phenyl)phenylmethyloxy)-phenyl)pyridin-2-yl]-5-trifluoromethyl-pyrazole-4-carboxylic acid |
| 12 | 1-[6-(2-(4-(phenethyl)phenylmethyloxy)-phenyl)pyridin-2-yl]-5-methylpyrazole-4-carboxylic acid |
| 13 | 1-[6-(2-(4-(4-azidophenyl)phenylmethyloxy)-phenyl)pyridin-2-yl]-5-trifluoromethyl-pyrazole-4-carboxylic acid |
| 14 | 1-[6-(2-(4-(4-methoxyphenyl)phenylmethyloxy)-phenyl)pyridin-2-yl]-5-methyl-pyrazole-4-carboxylic acid |
| 15 | 1-[6-(2-(4-(4-t-butylphenyl)phenylmethyloxy)-phenyl)pyrid in-2-yl]-5-trifluoromethyl-pyrazole-4-carboxylic acid |
| 16 | 1-[6-(2-(4-(3,4-dichlorophenyl)phenylmethyloxy)-phenyl)pyridin-2-yl]-5-trifluoromethyl-pyrazole-4-carboxylic acid |
| 17 | 1-[6-(2-(4-(4-fluorophenyl)phenylmethyloxy)-phenyl)pyridin-2-yl]-5-trifluoromethyl-pyrazole-4-carboxylic acid |
| 18 | 1-[6-(2-(4-(4-cyanophenyl)phenylmethyloxy)-phenyl)pyridin-2-yl]-5-trifluoromethyl-pyrazole-4-carboxylic acid |
| 19 | 1-[6-(2-(4-(4-chlorophenyl)phenylmethyloxy)-phenyl)pyrid in-2-yl]-5-trifluoromethyl-pyrazole-4-carboxylic acid |
| 20 | 1-[6-(2-(4-(4-trifluoromethoxyphenyl)phenylmethyloxy)-phenyl)pyridin-2-yl]-5-trifluoromethyl-pyrazole-4-carboxylic acid |
| 21 | 1-[6-(2-(4-(4-carboxyphenyl)phenylmethyloxy)-phenyl)pyridin-2-yl]-5-trifluoromethyl-pyrazole-4-carboxylic acid |
| 22 (racemic mixture) | 1-[6-(2-(1-(4-(4-methoxyphenyl)phenyl)ethyloxy)-phenyl)pyridin-2-yl]-5-trifluoromethyl-pyrazole-4-carboxylic acid |
| 23 | 1-[6-(2-(4-(4-fluoro-2-methylphenyl)phenylmethyloxy)-phenyl)pyrid in-2-yl]-5-trifluoromethyl-pyrazole-4-carboxylic acid |
| 24 | 1-[6-(2-(2-methy)-4-(4-trifluoromethylphenyl)phenylmethyloxy)-phenyl)pyridin-2-yl]-5-trifluoromethyl-pyrazole-4-carboxylic acid |
| 25 | 1-[6-(2-(2-methyl-4-(4-methoxyphenyl)phenylmethyloxy)-phenyl)pyridin-2-yl]-5-trifluoromethyl-pyrazole-4-carboxylic acid |
| 26 | 1-[6-(2-(2-methyl-4-(4-cyanophenyl)phenylmethyloxy)-phenyl)pyridin-2-yl]-5-trifluoromethyl-pyrazole-4-carboxylic acid |
| 27 | 1-[6-(2-(2-methyl-4-(4-carboxyphenyl)phenylmethyloxy)-phenyl)pyridin-2-yl]-5-trifluoromethyl-pyrazole-4-carboxylic acid |
| 28 | 1-[6-(2-(2-methyl-4-(4-fluoro-2-methylphenyl)phenylmethyloxy)-phenyl)pyridin-2-yl]-5-trifluoromethyl-pyrazole-4-carboxylic acid |
| 29 | 1-[6-(2-(2-methyl-4-(4-methoxy-2-methylphenyl)phenylmethyloxy)-phenyl)pyridin-2-yl]-5-trifluoromethyl-pyrazole-4-carboxylic acid |
| 30 | 1-[6-(2-(2-methyl-4-(4-methoxy-3-trifluoromethylphenyl)phenylmethyloxy)-phenyl)pyridin-2-yl]-5-trifluoromethyl-pyrazole-4-carboxylic acid |
| 31 | 1-[6-(2-(2-methyl-4-(3-chloro-4-methoxy-phenyl)phenylmethyloxy)-phenyl)pyridin-2-yl]-5-trifluoromethyl-pyrazole-4-carboxylic acid |
| 32 | 1-[6-(2-(2-methyl-4-(3,4-dichloro-phenyl)phenylmethyloxy)-phenyl)pyridin-2-yl]-5-trifluoromethyl-pyrazole-4-carboxylic acid |
| 33 | 1-[6-(2-(2-methyl-4-(4-chloro-2-trifluoromethylphenyl)phenylmethyloxy)-phenyl)pyridin-2-yl]-5-trifluoromethyl-pyrazole-4-carboxylic acid |
| 34 | 1-[6-(2-(2-methyl-4-(2,4-dimethoxy-phenyl)phenylmethyloxy)-phenyl)pyridin-2-yl]-5-trifluoromethyl-pyrazole-4-carboxylic acid |
| 35 | 1-[6-(2-(2-methyl-4-(4-methoxy-2-trifluoromethylphenyl)phenylmethyloxy)-phenyl)pyridin-2-yl]-5-trifluoromethyl-pyrazole-4-carboxylic acid |
| 36 | 1-[6-(2-(2-methyl-4-(3-chloro-4-isopropyloxylphenyl)phenylmethyloxy)-phenyl)pyridin-2-yl]-5-trifluoromethyl-pyrazole-4-carboxylic acid |
| 37 | 1-[6-(2-(2-methyl-4-(4-isopropyloxyl-2-methylphenyl)phenylmethyloxy)-phenyl)pyridin-2-yl]-5-trifluoromethyl-pyrazole-4-carboxylic acid |
| 38 | 1-[6-(2-(2-methyl-4-(4-ethyloxyl-3-trifluoromethylphenyl)phenylmethyloxy)-phenyl)pyridin-2-yl]-5-trifluoromethyl-pyrazole-4-carboxylic acid |
| 39 | 1-[6-(2-(2-methyl-4-(4-chloro-2-methoxyphenyl)phenylmethyloxy)-phenyl)pyridin-2-yl]-5-trifluoromethyl-pyrazole-4-carboxylic acid |
| 40 | 1-[6-(2-(2-methyl-4-(4-ethyloxy-3-methylphenyl)phenylmethyloxy)-phenyl)pyridin-2-yl]-5-trifluoromethyl-pyrazole-4-carboxylic acid |
| 41 | 1-[6-(2-(2-methyl-4-(4-chloro-2-ethyloxyphenyl)phenylmethyloxy)-phenyl)pyridin-2-yl]-5-trifluoromethyl-pyrazole-4-carboxylic acid |
| 42 | 1-[6-(2-(2-methyl-4-(2-chloro-4-ethyloxyphenyl)phenylmethyloxy)-phenyl)pyridin-2-yl]-5-trifluoromethyl-pyrazole-4-carboxylic acid |
| 43 | 1-[6-(2-(2-methyl-4-(2-chloro-4-methoxyphenyl)phenylmethyloxy)-phenyl)pyridin-2-yl]-5-trifluoromethyl-pyrazole-4-carboxylic acid |
| 44 | 1-[6-(2-(2-methoxy-4-(4-fluorophenyl)phenylmethyloxy)-phenyl)pyridin-2-yl]-5-trifluoromethyl-pyrazole-4-carboxylic acid |
| 45 | 1-[6-(2-(2-methoxy-4-(4-trifluoromethylphenyl)phenylmethyloxy)-phenyl)pyridin-2-yl]-5-trifluoromethyl-pyrazole-4-carboxylic acid |
| 46 | 1-[6-(2-(2-methoxy-4-(4-methoxyphenyl)phenylmethyloxy)-phenyl)pyridin-2-yl]-5-trifluoromethyl-pyrazole-4-carboxylic acid |
| 47 | 1-[6-(2-(2-methoxy-4-(4-carboxyphenyl)phenylmethyloxy)-phenyl)pyridin-2-yl]-5-trifluoromethyl-pyrazole-4-carboxylic acid |
| 48 | 1-[6-(2-(2-methoxy-4-(4-methoxy-3-trifluoromethylphenyl)phenylmethyloxy)-phenyl)pyridin-2-yl]-5-trifluoromethyl-pyrazole-4-carboxylic acid |
| 49 | 1-[6-(2-(2-methoxy-4-(2-chloro-4-methoxy-phenyl)phenylmethyloxy)-phenyl)pyridin-2-yl]-5-trifluoromethyl-pyrazole-4-carboxylic acid |
| 50 | 1-[6-(2-(2-methoxy-4-(4-methoxy-2-methylphenyl)phenylmethyloxy)-phenyl)pyridin-2-yl]-5-trifluoromethyl-pyrazole-4-carboxylic acid |
| 51 | 1-[6-(2-(2-fluoro-4-(4-carboxyphenyl)phenylmethyloxy)-phenyl)pyridin-2-yl]-5-trifluoromethyl-pyrazole-4-carboxylic acid |
| 52 | 1-[6-(2-(2-(2-methyl-propyloxy)-4-(4-fluorophenyl)phenylmethyloxy)-phenyl)pyridin-2-yl]-5-trifluoromethyl-pyrazole-4-carboxylic acid |
| 53 | 1-[6-(2-(2-(2-methyl-propyloxy)-4-(4-methoxyphenyl)phenylmethyloxy)-phenyl)pyridin-2-yl]-5-trifluoromethyl-pyrazole-4-carboxylic acid |
| 54 | 1-[6-(2-(2-(2-methyl-propyloxy)-4-(4-cyanophenyl)phenylmethyloxy)-phenyl)pyridin-2-yl]-5-trifluoromethyl-pyrazole-4-carboxylic acid |
| 55 | 1-[6-(2-(2-(methoxyethyloxy)-4-(4-fluorophenyl)phenylmethyloxy)-phenyl)pyridin-2-yl]-5-trifluoromethyl-pyrazole-4-carboxylic acid |
| 56 | 1-[6-(2-(2-(methoxyethyloxy)-4-(4-methoxyphenyl)phenylmethyloxy)-phenyl)pyridin-2-yl]-5-trifluoromethyl-pyrazole-4-carboxylic acid |
| 57 | 1-[6-(2-(2-(methoxyethyloxy)-4-(4-cyanophenyl)phenylmethyloxy)-phenyl)pyridin-2-yl]-5-trifluoromethyl-pyrazole-4-carboxylic acid |
| 58 | 1-[6-(2-(2-propyloxy-4-(4-trifluoromethylphenyl)phenylmethyloxy)-phenyl)pyridin-2-yl]-5-trifluoromethyl-pyrazole-4-carboxylic acid |
| 59 | 1-[6-(2-(2-propyloxy-4-(4-cyanophenyl)phenylmethyloxy)-phenyl)pyridin-2-yl]-5-trifluoromethyl-pyrazole-4-carboxylic acid |
| 60 | 1-[6-(2-(4-(5-trifluoromethylpyridin-2-yl)phenylmethyloxy)-phenyl)pyridin-2-yl]-5-trifluoromethyl-pyrazole-4-carboxylic acid |
| 61 | 1-[6-(2-(4-(5-trifluoromethylpyridin-2-yl)phenylmethyloxy)-phenyl)pyridin-2-yl]-5-methyl-pyrazole-4-carboxylic acid |
| 62 | 1-[6-(2-(4-(5-cyanopyridin-2-yl)phenylmethyloxy)-phenyl)pyridin-2-yl]-5-trifluoromethyl-pyrazole-4-carboxylic acid |
| 63 | 1-[6-(2-(4-(5-methoxypyridin-2-yl)phenylmethyloxy)-phenyl)pyridin-2-yl]-5-trifluoromethyl-pyrazole-4-carboxylic acid |
| 64 | 1-[6-(2-(4-(5-methoxypyridin-2-yl)phenylmethyloxy)-phenyl)pyrid in-2-yl]-5-methyl-pyrazole-4-carboxylic acid |
| 65 | 1-[6-(2-(2-methyl-4-(5-trifluoromethylpyridin-2-yl)phenylmethyloxy)-phenyl)pyridin-2-yl]-5-trifluoromethyl-pyrazole-4-carboxylic acid |
| 66 | 1-[6-(2-(2-methyl-4-(5-chloropyridin-2-yl)phenylmethyloxy)-phenyl)pyridin-2-yl]-5-trifluoromethyl-pyrazole-4-carboxylic acid |
| 67 | 1-[6-(2-(2-methyl-4-(5-methoxypyridin-2-yl)phenylmethyloxy)-phenyl)pyridin-2-yl]-5-trifluoromethyl-pyrazole-4-carboxylic acid |
| 68 | 1-[6-(2-(2-methyl-4-(3-chloro-5-trifluoromethylpyridin-2-yl)phenylmethyloxy)-phenyl)pyridin-2-yl]-5-trifluoromethyl-pyrazole-4-carboxylic acid |
| 69 | 1-[6-(2-(2-methyl-4-(6-methoxypyridin-3-yl)phenylmethyloxy)-phenyl)pyridin-2-yl]-5-trifluoromethyl-pyrazole-4-carboxylic acid |
| 70 | 1-[6-(2-((6-(4-methoxyphenyl)pyridin-3-yl)methyloxy)-phenyl)pyridin-2-yl]-5-trifluoromethyl-pyrazole-4-carboxylic acid |
| 71 | 1-[6-(2-(4-(4-methylthiazol-2-yl)phenylmethyloxy)-phenyl)pyrid in-2-yl]-5-methyl-pyrazole-4-carboxylic acid |
| 72 | 1-[6-(2-(4-(phenoxy)phenylmethyloxy)-phenyl)pyridin-2-yl]-5-trifluoromethyl-pyrazole-4-carboxylic acid |
| 73 | 1-[6-(2-(4-(4-carboxyphenyloxy)phenylmethyloxy)-phenyl)pyridin-2-yl]-5-trifluoromethyl-pyrazole-4-carboxylic acid |
| 74 | 1-[6-(2-(4-(5-trifluoromethylpyridin-2-yloxy)phenylmethyloxy)-phenyl)pyridin-2-yl]-5-trifluoromethyl-pyrazole-4-carboxylic acid |
| 75 | 1-[6-(2-(4-(5-trifluoromethylpyridin-2-yloxy)phenylmethyloxy)-phenyl)pyridin-2-yl]-5-methyl-pyrazole-4-carboxylic acid |
| 76 | 1-[6-(2-(4-(5-chloropyridin-2-yloxy)phenylmethyloxy)-phenyl)pyridin-2-yl]-5-trifluoromethyl-pyrazole-4-carboxylic acid |
| 77 | 1-[6-(2-(4-(3-chloro-5-trifluoromethylpyridin-2-yloxy)phenylmethyloxy)-phenyl)pyridin-2-yl]-5-trifluoromethyl-pyrazole-4-carboxylic acid |
| 78 | 1-[6-(2-(2-methoxy-4-(3-chloro-5-trifluoromethylpyridin-2-yloxy)phenylmethyloxy)-phenyl)pyridin-2-yl]-5-trifluoromethyl-pyrazole-4-carboxylic acid |
| 79 | 1-[6-(2-(2-methyl-4-(3-chloro-5-trifluoromethylpyridin-2-yloxy)phenylmethyloxy)-phenyl)pyridin-2-yl]-5-trifluoromethyl-pyrazole-4-carboxylic acid |
| 80 | 1-[6-(2-((6-(3,4-dichlorophenoxy)pyridin-3-yl)methyloxy)-phenyl)pyridin-2-yl]-5-trifluoromethyl-pyrazole-4-carboxylic acid |
| 81 | 1-[6-(2-((6-(4-chlorophenoxy)pyridin-3-yl)methyloxy)-phenyl)pyridin-2-yl]-5-trifluoromethyl-pyrazole-4-carboxylic acid |
| 82 | 1-[6-(2-((6-(4-chlorophenoxy)pyridin-3-yl)methyloxy)-phenyl)pyridin-2-yl]-5-methyl-pyrazole-4-carboxylic acid |
| 83 | 1-[6-(2-((6-(4-methoxyphenoxy)pyridin-3-yl)methyloxy)-phenyl)pyridin-2-yl]-5-trifluoromethyl-pyrazole-4-carboxylic acid |
| 84 | 1-[6-(2-((6-(4-methoxyphenoxy)pyridin-3-yl)methyloxy)-phenyl)pyridin-2-yl]-5-methyl-pyrazole-4-carboxylic acid |
| 85 | 1-(6-{2-[({6-[(4-carboxyphenyl)oxy]-3-pyridinyl}methyl)oxy]phenyl}-pyridin-2-yl)-5-(trifluoromethyl)-1*H*-pyrazole-4-carboxylic acid |
| 86 | 1-[6-(2-(4-(4-(t-butyl)phenylmethyloxy)phenylmethyloxy)-phenyl)pyridin-2-yl]-5-trifluoromethyl-pyrazole-4-carboxylic acid |
| 87 | 1-[6-(3,5-difluoro-2-(4-(4-cyanophenyl)phenylmethyloxy)-phenyl)pyridin-2-yl]-5-trifluoromethyl-pyrazole-4-carboxylic acid |
| 88 | 1-[6-(3,5-difluoro-2-(4-(4-carboxyphenyl)phenylmethyloxy)-phenyl)pyridin-2-yl]-5-trifluoromethyl-pyrazole-4-carboxylic acid |
| 89 (racemic mixture) | 1-[6-(3,5-difluoro-2-(1-(4-(4-methoxyphenyl)phenyl)ethyloxy)-phenyl)pyridin-2-yl]-5-trifluoromethyl-pyrazole-4-carboxylic acid |
| 90 (racemic mixture) | 1-[6-(3,5-difluoro-2-(1-(4-(4-cyanophenyl)phenyl)ethyloxy)-phenyl)pyridin-2-yl]-5-trifluoromethyl-pyrazole-4-carboxylic acid |
| 91 (racemic mixture) | 1-[6-(3,5-difluoro-2-(1-(4-(4-trifluoromethylphenyl)phenyl)ethyloxy)-phenyl)pyridin-2-yl]-5-trifluoromethyl-pyrazole-4-carboxylic acid |
| 92 | 1-[6-(3,5-difluoro-2-(4-(4-cyanophenoxy)phenylmethyloxy)-phenyl)pyridin-2-yl]-5-trifluoromethyl-pyrazole-4-carboxylic acid |
| 93 | 1-[6-(5-chloro-2-(4-(4-cyanophenyl)phenylmethyloxy)-phenyl)pyridin-2-yl]-5-trifluoromethyl-pyrazole-4-carboxylic acid |
| 94 | 1-[6-(5-chloro-2-(2-methyl-4-(4-cyanophenyl)phenylmethyloxy)-phenyl)pyridin-2-yl]-5-trifluoromethyl-pyrazole-4-carboxylic acid |
| 95 | 1-[6-(5-chloro-2-(2-methyl-4-(4-fluorophenyl)phenylmethyloxy)-phenyl)pyridin-2-yl]-5-trifluoromethyl-pyrazole-4-carboxylic acid |
| 96 | 1-[6-(5-chloro-2-(2-methyl-4-(4-methoxyphenyl)phenylmethyloxy)-phenyl)pyridin-2-yl]-5-trifluoromethyl-pyrazole-4-carboxylic acid |
| 97 | 1-[6-(5-chloro-2-(2-methyl-4-(4-carboxyphenyl)phenylmethyloxy)-phenyl)pyridin-2-yl]-5-trifluoromethyl-pyrazole-4-carboxylic acid |
| 98 | 1-[6-(5-chloro-2-(4-(4-cyanophenyloxy)phenylmethyloxy)-phenyl)pyridin-2-yl]-5-trifluoromethyl-pyrazole-4-carboxylic acid |
| 99 | 1-[6-(5-chloro-2-(4-(5-trifluoromethylpyridin-2-yloxy)phenylmethyloxy)-phenyl)pyridin-2-yl]-5-trifluoromethyl-pyrazole-4-carboxylic acid |
| 100 | 1-[6-(5-chloro-2-(2-methyl-4-(5-trifluoromethylpyridin-2-yloxy)phenylmethyloxy)-phenyl)pyridin-2-yl]-5-trifluoromethyl-pyrazole-4-carboxylic acid |
| 101 | 1-[6-(5-fluoro-2-(4-(4-trifluoromethylphenyl)phenylmethyloxy)-phenyl)pyridin-2-yl]-5-trifluoromethyl-pyrazole-4-carboxylic acid |
| 102 | 1-[6-(5-fluoro-2-(4-(4-methoxy-2-methylphenyl)phenylmethyloxy)-phenyl)pyridin-2-yl]-5-trifluoromethyl-pyrazole-4-carboxylic acid |
| 103 | 1-[6-(5-fluoro-2-(4-(4-fluoro-2-methylphenyl)phenylmethyloxy)-phenyl)pyridin-2-yl]-5-trifluoromethyl-pyrazole-4-carboxylic acid |
| 104 | 1-[6-(5-fluoro-2-(2-methyl-4-(4-methoxyphenyl)phenylmethyloxy)-phenyl)pyridin-2-yl]-5-trifluoromethyl-pyrazole-4-carboxylic acid |
| 105 | 1-[6-(5-fluoro-2-(2-methyl-4-(4-cyanophenyl)phenylmethyloxy)-phenyl)pyridin-2-yl]-5-trifluoromethyl-pyrazole-4-carboxylic acid |
| 106 | 1-[6-(5-fluoro-2-(2-methyl-4-(4-fluorophenyl)phenylmethyloxy)-phenyl)pyridin-2-yl]-5-trifluoromethyl-pyrazole-4-carboxylic acid |
| 107 | 1-[6-(5-fluoro-2-(2-methoxy-4-(4-carboxyphenyl)phenylmethyloxy)-phenyl)pyridin-2-yl]-5-trifluoromethyl-pyrazole-4-carboxylic acid |
| 108 | 1-[6-(5-fluoro-2-(2-fluoro-4-(4-fluorophenyl)phenylmethyloxy)-phenyl)pyridin-2-yl]-5-trifluoromethyl-pyrazole-4-carboxylic acid |
| 109 | 1-[6-(5-fluoro-2-(2-flu oro-4-(4-cyanophenyl)phenylmethyloxy)-phenyl)pyridin-2-yl]-5-trifluoromethyl-pyrazole-4-carboxylic acid |
| 110 | 1-[6-(5-fluoro-2-(2-flu oro-4-(4-methoxyphenyl)ph enylmethyloxy)-phenyl)pyridin-2-yl]-5-trifluoromethyl-pyrazole-4-carboxylic acid |
| 111 | 1-[6-(5-fluoro-2-(2-fluoro-4-(4-carboxyphenyl)phenylmethyloxy)-phenyl)pyridin-2-yl]-5-trifluoromethyl-pyrazole-4-carboxylic acid |
| 112 | 1-[6-(5-fluoro-2-(4-(5-trifluoromethylpyridin-2-yloxy)phenylmethyloxy)-phenyl)pyridin-2-yl]-5-trifluoromethyl-pyrazole-4-carboxylic acid |
| 113 | 1-[6-(5-fluoro-2-((6-(3,4-dichlorophenoxy)pyridin-3-yl)methyloxy)-phenyl)pyridin-2-yl]-5-trifluoromethyl-pyrazole-4-carboxylic acid |
| 114 | 1-[6-(3-(propen-2-yl)-2-(4-(4-trifluoromethylphenyl)phenylmethyloxy)-phenyl)pyridin-2-yl]-5-trifluoromethyl-pyrazole-4-carboxylic acid |
| 115 | 1-[6-(3-propyl-2-(4-(4-trifluoromethylphenyl)phenylmethyloxy)-phenyl)pyridin-2-yl]-5-trifluoromethyl-pyrazole-4-carboxylic acid |
| 116 | 1-[6-(3-(propen-2-yl)-2-(2-methyl-4-(4-trifluoromethylphenyl)phenylmethyloxy)-phenyl)pyridin-2-yl]-5-trifluoromethyl-pyrazole-4-carboxylic acid |
| 117 | 1-[6-(3-propyl-2-(2-methyl-4-(4-trifluoromethylphenyl)phenylmethyloxy)-phenyl)pyridin-2-yl]-5-trifluoromethyl-pyrazole-4-carboxylic acid |
| 118 | 1-[6-(3-(propen-2-yl)-2-(4-(4-methoxyphenyl)phenylmethyloxy)-phenyl)pyridin-2-yl]-5-trifluoromethyl-pyrazole-4-carboxylic acid |
| 119 | 1-[6-(3-propyl-2-(4-(4-methoxyphenyl)phenylmethyloxy)-phenyl)pyridin-2-yl]-5-trifluoromethyl-pyrazole-4-carboxylic acid |
| 120 | 1-[6-(5-methyl- 2-(4-(4-cyanophenyl)phenylmethyloxy)-phenyl)pyridin-2-yl]-5-trifluoromethyl-pyrazole-4-carboxylic acid |
| 121 | 1-[6-(5-methyl-2-(2-methyl-4-(4-cyanophenyl)phenylmethyloxy)-phenyl)pyridin-2-yl]-5-trifluoromethyl-pyrazole-4-carboxylic acid |
| 122 | 1-[6-(5-methyl-2-(2-methyl-4-(4-trifluoromethoxyphenyl)phenylmethyloxy)-phenyl)pyridin-2-yl]-5-trifluoromethyl-pyrazole-4-carboxylic acid |
| 123 | 4-[6-(2-(4-(4-cyanophenyl)phenylmethyloxy)-phenyl)pyridin-2-yl]-benzoic acid |
| 124 | 4-[6-(2-(4-(4-trifluoromethylphenyl)phenylmethyloxy)-phenyl)pyridin-2-yl]-benzoic acid |
| 125 | 1-[6-(2-(4-(phenethyl)phenylmethyloxy)-phenyl)pyridin-2-yl]-2-methyl-pyrrole-3-carboxylic acid |
| 126 | 1-[6-(2-(4-(4-methoxyphenyl)phenylmethyloxy)-phenyl)pyridin-2-yl]-2-methyl-pyrrole-3-carboxylic acid |
| 127 | 1-[6-(2-(2-methyl-4-(4-methoxyphenyl)phenylmethyloxy)-phenyl)pyridin-2-yl]-2-methyl-pyrrole-3-carboxylic acid |
| 128 | 1-[6-(5-fluoro-2-(4-(4-methoxyphenyl)phenylmethyloxy)-phenyl)pyridin-2-yl]-2-methyl-pyrrole-3-carboxylic acid |
| 129 | 1-[6-(2-(4-(4-methoxyphenoxy)phenylmethyloxy)-phenyl)pyridin-2-yl]-2-methyl-pyrrole-3-carboxylic acid |
| 130 | 1-[6-(2-(4-(phenethyl)phenylmethyloxy)-phenyl)pyridin-2-yl]-piperidine-4-carboxylic acid |
| 131 | 1-[6-(2-(4-(4-chlorophenyl)phenylmethyloxy)-phenyl)pyridin-2-yl]-piperidine-4-carboxylic acid |
| 132 | 1-[6-(2-(4-(4-cyanophenyl)phenylmethyloxy)-phenyl)pyridi n-2-yl]-piperidine-4-carboxylic acid |
| 133 | 1-[6-(2-(2-methyl-4-(4-trifluoromethylphenyl)phenylmethyloxy)-phenyl)pyridin-2-yl]-piperidine-4-carboxylic acid |
| 134 | 1-[6-(2-(2-propyloxy-4-(4-trifluoromethylphenyl)phenylmethyloxy)-phenyl)pyridin-2-yl]-piperidine-4-carboxylic acid |
| 135 | 1-[6-(2-(4-(5-trifluoromethylpyrid in-2-yloxy)phenyl methyloxy)-phenyl)pyridin-2-yl]-piperidine-4-carboxylic acid |
| 136 | 1-[6-(5-chloro-2-(2-methyl-4-(4-methoxyphenyl)phenylmethyloxy)-phenyl)pyridin-2-yl]-piperidine-4-carboxylic acid |
| 137 | 1-[6-(5-chloro-2-(2-methyl-4-(4-cyanophenyl)phenylmethyloxy)-phenyl)pyridin-2-yl]-piperidine-4-carboxylic acid |
| 138 | 1-[6-(5-chloro-2-(4-(4-trifluoromethylphenoxy)phenylmethyloxy)-phenyl)pyridin-2-yl]-piperidine-4-carboxylic acid |
| 139 | 1-[6-(5-chloro-2-(4-(5-trifluoromethylpyridin-2-yloxy)phenylmethyloxy)-phenyl)pyridin-2-yl]-piperidine-4-carboxylic acid |
| 140 | 1-[6-(5-chloro-2-(4-(4-cyanophenoxy)phenylmethyloxy)-phenyl)pyridin-2-yl]-piperidine-4-carboxylic acid |
| 141 | 1-[6-(3,5-difluoro-2-(2-methyl-4-(4-cyanophenyl)phenylm ethyloxy)-phenyl)pyridin-2-yl]-piperidine-4-carboxylic acid |
| 142 | 1-[6-(3,5-difluoro-2-(4-(4-trifluoromethylphenoxy)phenylmethyloxy)-phenyl)pyridin-2-yl]-piperidine-4-carboxylic acid |
| 143 | 1-[6-(3,5-difluoro-2-(4-(4-cyanophenoxy)phenylmethyloxy)-phenyl)pyridin-2-yl]-piperidine-4-carboxylic acid |
| 144 | 1-[6-(3,5-difluoro-2-(4-(5-trifluoromethylpyridin-2-yloxy)phenylmethyloxy)-phenyl)pyridin-2-yl]-piperidine-4-carboxylic acid |
| 145 | 1-[6-(3,5-difluoro-2-(4-(5-cyanopyridin-2-yloxy)phenylmethyloxy)-phenyl)pyridin-2-yl]-piperidine-4-carboxylic acid |
| 146 | 1-[6-(5-fluoro-2-(2-methyl-4-(4-methoxyphenyl)phenylmethyloxy)-phenyl)pyridin-2-yl]-piperidine-4-carboxylic acid |
| 147 | 1-[6-(5-fluoro-2-(2-methyl-4-(4-cyanophenyl)phenylmethyloxy)-phenyl)pyridin-2-yl]-piperidine-4-carboxylic acid |
| 148 | 1-[6-(5-fluoro-2-(2-methyl-4-(4-fluorophenyl)phenylmethyloxy)-phenyl)pyridin-2-yl]-piperidine-4-carboxylic acid |
| 149 | 1-[6-(5-fluoro-2-(4-(4-methoxyphenyloxy)phenylmethyloxy)-phenyl)pyridin-2-yl]-piperidine-4-carboxylic acid |
| 150 | 1-[6-(5-fluoro-2-(4-(4-cyanophenyloxy)phenylmethyloxy)-phenyl)pyridin-2-yl]-piperidine-4-carboxylic acid |
| 151 | 1-[6-(5-fluoro-2-(4-(4-fluorophenyloxy)phenylmethyloxy)-phenyl)pyridin-2-yl]-piperidine-4-carboxylic acid |
| 152 | 1-[6-(5-fluoro-2-(4-(4-trifluoromethylphenyloxy)phenylmethyloxy)-phenyl)pyridin-2-yl]-piperidine-4-carboxylic acid |
| 153 | 1-[6-(5-methyl-2-(2-methyl-4-(4-cyanophenyl)phenylmethyloxy)-phenyl)pyridin-2-yl]-piperidine-4-carboxylic acid |
| 154 | 1-[6-(5-methyl-2-(2-methyl-4-(4-trifluoromethylphenyl)phenylmethyloxy)-phenyl)pyridin-2-yl]-piperidine-4-carboxylic acid |
| 155 | 1-[6-(5-methyl-2-(2-methyl-4-(4-trifluoromethoxyphenyl)phenylmethyloxy)-phenyl)pyridin-2-yl]-piperidine-4-carboxylic acid |
| 156 | 1-[6-(5-methyl-2-(2-methyl-4-(4-cyano-2-methylphenyl)phenylmethyloxy)-phenyl)pyridin-2-yl]-piperidine-4-carboxylic acid |
| 157 | 1-[6-(5-methyl-2-(2-methyl-4-(4-methoxy-2-trifluoromethylphenyl)phenylmethyloxy)-phenyl)pyridin-2-yl]-piperidine-4-carboxylic acid |
| 158 | 1-[6-(5-methyl-2-(2-methyl-4-(4-ch loro-2-trifluoromethylphenyl)phenylmethyloxy)-phenyl)pyridin-2-yl]-piperidine-4-carboxylic acid |
| 159 | 1-[6-(5-methyl-2-(2-propyloxy-4-(4-cyanophenyl)phenylmethyloxy)-phenyl)pyridin-2-yl]-piperidine-4-carboxylic acid |
| 160 | 1-[6-(5-methyl-2-(2-methyl-4-(3-chloro-5-trifluoromethylpyridin-2-yloxy)phenylmethyloxy)-phenyl)pyridin-2-yl]-piperidine-4-carboxylic acid |
| 161 | 1-[6-(5-trifluoromethyl-2-(2-methyl-4-(4-cyanophenyl)phenylmethyloxy)-phenyl)pyridin-2-yl]-piperidine-4-carboxylic acid |
| 162 | 1-[6-(5-trifluoromethyl-2-(2-methyl-4-(3-ch loro-5-trifluoromethylpyridin-2-yl)phenylmethyloxy)-phenyl)pyridin-2-yl]-piperidine-4-carboxylic acid |
| 163 | 1-[6-(5-trifluoromethyl-2-(2-propyloxy-4-(4-chloro-2-trifluoromethylphenyl)phenylmethyloxy)-phenyl)pyridin-2-yl]-1-piperidine-4-carboxylic acid |
| 164 | 1-[6-(5-trifluoromethyl-2-(2-propyloxy-4-(2-m ethyl-4-trifluoromethylphenyl)phenylmethyloxy)-phenyl)pyridin-2-yl]-1-piperidine-4-carboxylic acid |
| 165 | 1-[6-(5-trifluoromethyl-2-(2-propyloxy-4-(4-cyano-2-methylphenyl)phenylmethyloxy)-phenyl)pyridin-2-yl]-1-piperidine-4-carboxylic acid |
| 166 | 1-[6-(5-trifluoromethyl-2-(2-propyloxy-4-(2-ch loro-4-methoxyphenyl)phenylmethyloxy)-phenyl)pyridin-2-yl]-1-piperidine-4-carboxylic acid |
**Example 167: 1-[6-(2-(4-(4-cyanophenyl)benzyloxy)-phenyl)pyridin-2-yl]-5-methylpyrazole-4-carboxylic acid** 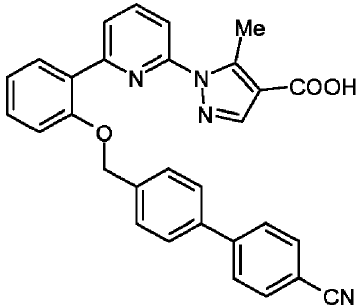 **Example 168: 1-[6-( 2-(4-(4-carboxamidophenyl)benzyloxy)-phenyl)pyridin-2-yl]-5-methyl-pyrazole-4-carboxylic acid** 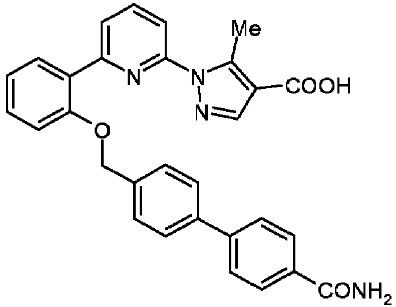 **Example 169: 1-[6-( 2-(4-(4-carboxamidophenoxy)benzyloxy)-phenyl)pyridin-2-yl]-5-trifluoromethyl-pyrazole-4-carboxylic acid** 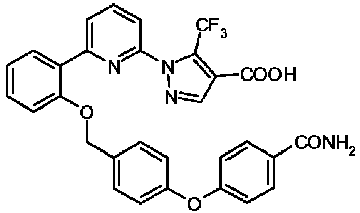 **Example 170: 1-[6-(2-(2-methyl-4-(5-carboxamidopyridin-2-yl)benzyloxy)-phenyl)pyridin-2-yl]-5-trifluoromethyl-pyrazole-4-carboxylic acid** 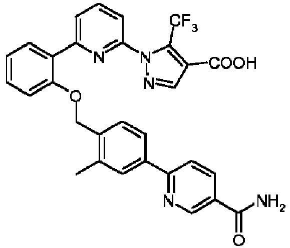 **Example 171: 1-[6-(5-methyl-2-(2-methyl-4-(5-cyanopyridin-2-yl)benzyloxy)-phenyl)pyridin-2-yl]-piperidine-4-carboxylic acid** 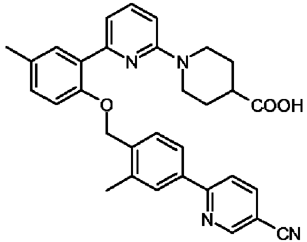 **Example 172: 1-[6-(5-trifluoromethyl-2-(2-methyl-4-(5-cyanopyridin-2-yl)benzyloxy)-phenyl)pyridin-2-yl]-piperidine-4-carboxylic acid** 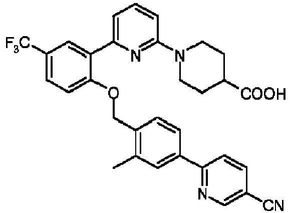 **Example 173: 1-[6-( 5-methyl-2-(2-methyl-4-(3-chloro-5-trifluoromethylpyridin-2-yl)benzyloxy)-phenyl)pyridin-2-yl]-piperidine-4-carboxylic acid** 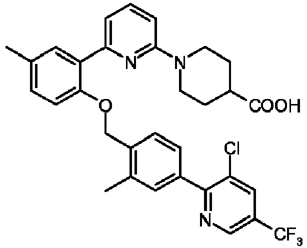 **Example 174: 1-[6-(2-(4-(4-cyanophenyl)benzyloxy)-phenyl)pyridin-2-yl]-2-methyl-pyrrole-3-carboxylic acid** 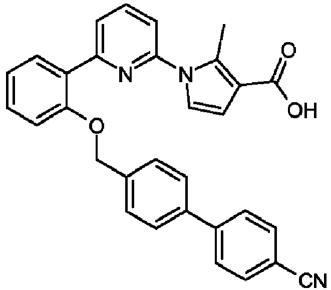 **Example 175: 1-[6-( 2-(2-methyl-4-(4-fluorophenyl)benzyloxy)-phenyl)pyridin-2-yl]-2-methyl-pyrrole-3-carboxylic** acid 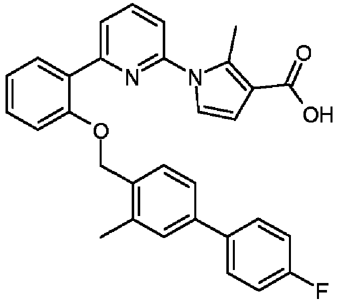
| **Example** | **Name** |
|---|---|
| 176 | 1-[6-(5-fluoro-2-(2-methyl-4-(4-cyanophenyl)phenylmethyloxy)-phenyl)pyridin-2-yl]-2-methyl-pyrrole-3-carboxylic acid |
| 177 | 1-[6-(5-fluoro-2-(2-methyl-4-(4-fluorophenyl)phenylmethyloxy)-phenyl)pyridin-2-yl]-2-methyl-pyrrole-3-carboxylic acid |
**Example 178: 1-[6-( 2-(4-(4-cyanophenoxy)benzyloxy)-phenyl)pyridin-2-yl]-2-methyl-pyrrole-3-carboxylic acid** 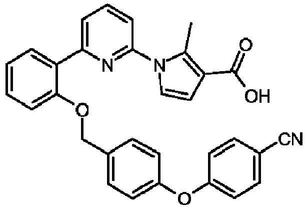 **Example 179: 1-[6-(3,5-difluoro-2-(2-methyl-4-(4-trifluoromethylphenyl)benzyloxy)-phenyl)pyridin-2-yl]-piperidine-4-carboxylic acid** 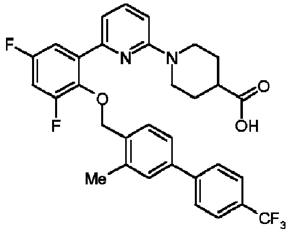 **Example 180: 1-[6-(5-fluoro-2-(2-methyl-4-(4-trifluoromethylphenyl)benzyloxy)-phenyl)pyridin-2-yl]-5-trifluoromethyl-pyrrazole-4-carboxylic acid** 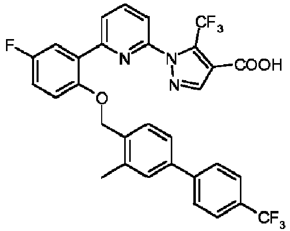 **Example 181: 1-[6-(5-chloro-2-(2-methyl-4-(4-fluorophenyl)benzyloxy)-phenyl)pyridin-2-yl]-piperidine-4-carboxylic acid** 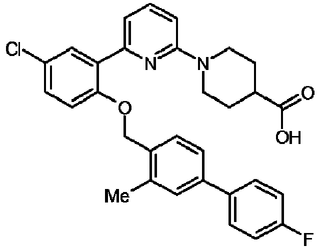 **Example 182: 1-[6-(2-(2-methyl-4-(3,4-dimethoxyphenyl)benzyloxy)-phenyl)pyridin-2-yl]-5-trifluoromethyl-pyrrazole-4-carboxylic acid** 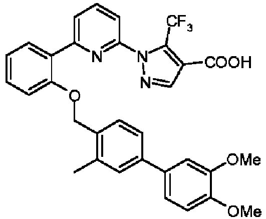
| **Example** | **Name** |
|---|---|
| 183 | 1-[6-(4-chloro-2-(2-methyl-4-(4-trifluoromethoxyphenyl)phenylmethyloxy)-phenyl)pyridin-2-yl]-5-trifluoromethyl-pyrazole-4-carboxylic acid |
| 184 | 1-[6-(6-chloro-2-(2-methyl-4-(4-trifluoromethoxyphenyl)phenylmethyloxy)-phenyl)pyridin-2-yl]-5-trifluoromethyl-pyrazole-4-carboxylic acid |
| 185 | 1-[6-(4-fluoro-2-(2-methyl-4-(4-trifluoromethoxyphenyl)phenylmethyloxy)-phenyl)pyridin-2-yl]-5-trifluoromethyl-pyrazole-4-carboxylic acid |
| 186 | 1-[6-(6-fluoro-2-(2-methyl-4-(4-trifluoromethoxyphenyl)phenylmethyloxy)-phenyl)pyridin-2-yl]-5-trifluoromethyl-pyrazole-4-carboxylic acid |
**Example 187: 1-[6-(2-(4-(4-methoxy-2-methyl-phenyl)benzyloxy)-phenyl)pyridin-2-yl]-5-trifluoromethyl-pyrazole-4-carboxylic acid** 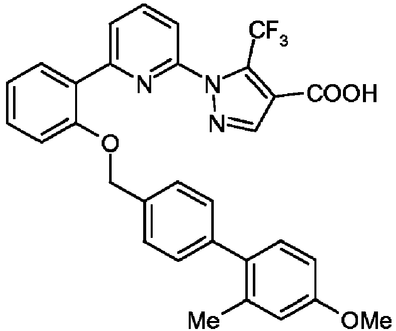 **Example 188: 1-[6-(5-fluoro-2-(2-methyl-4-(4-methoxy-2-methyl-phenyl)benzyloxy)-phenyl)pyridin-2-yl]-5-trifluoromethyl-pyrazole-4-carboxylic acid** 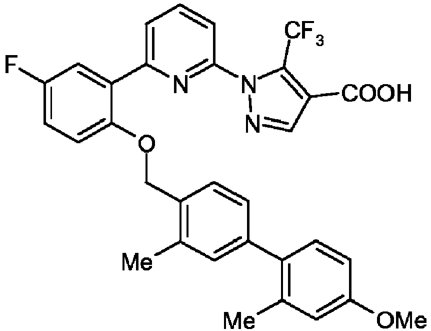
| **Example No.** | **Structure** | **Name** |
|---|---|---|
| **189** | 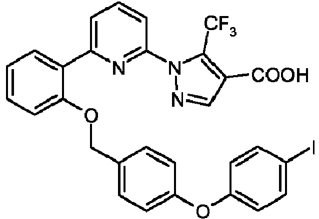 | 1-[6-(2-(4-(4-iodo-phenyloxy)benzyloxy)-phenyl)pyridin-2-yl]-5-trifluoromethyl-pyrazole-4-carboxylic acid |
| **190** | 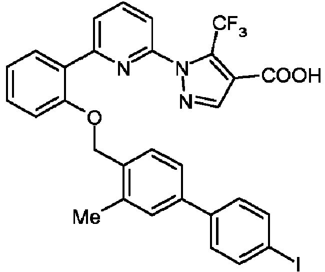 | 1-[6-(2-(2-methyl-4-(4-iodophenyl)benzyloxy)-phenyl)pyridin-2-yl]-5-trifluoromethyl-pyrazole-4-carboxylic acid |
| | | |
| **Example No.** | **Structure** | **Name** |
|---|---|---|
| **191** | 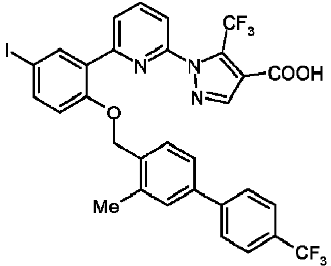 | 1-[6-(5-iodo-2-(2-methyl-4-(4-trifluoromethylphenyl)benzyloxy)-phenyl)pyridin-2-yl]-5-trifluoromethyl-pyrazole-4-carboxylic acid |
Compound Class VI
**Example 1: 1-[6-(3,4-dichlorophenyl)-2-pyridinyl]-5-(trifluoromethyl)-1*H*-pyrazole-4-carboxylic acid** 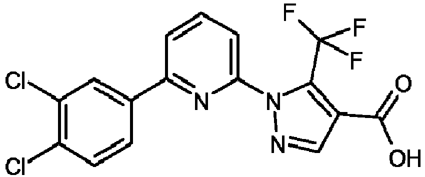 **Example 2: 1-{6-[4-(trifluoromethyl)phenyl]-2-pyridinyl}-5-(trifluoromethyl)-1*H-*pyrazole-4-carboxylic acid** 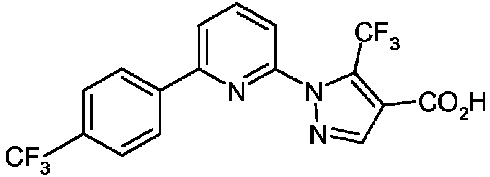 **Example 3: 1-[6-(2,3-dichlorophenyl)-2-pyridinyl]-5-(trifluoromethyl)-1*H*-pyrazole-4-carboxylic acid** 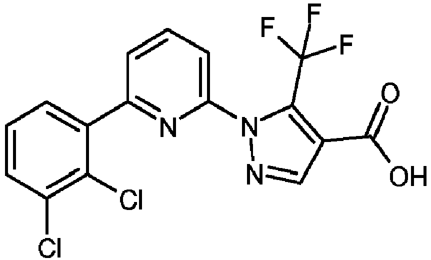 **Example 4: 1-{6-[3-(trifluoromethyl)phenyl]-2-pyridinyl}-5-(trifluoromethyl)-1*H-*pyrazole-4-carboxylic acid** 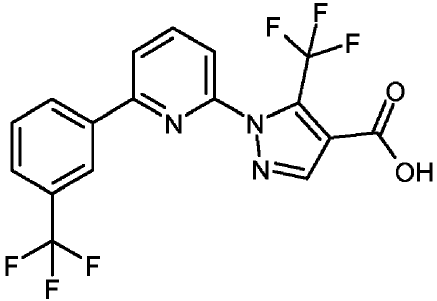 **Example 5: 1-[6-(3,4-difluorophenyl)-2-pyridinyl]-5-(trifluoromethyl)-1*H*-pyrazole-4-carboxylic acid** 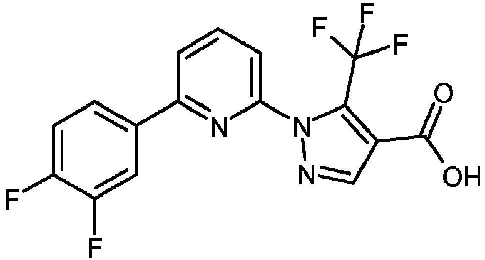 **Example 6: 1-{6-[3,5-bis(trifluoromethyl)phenyl]-2-pyridinyl}-5-(trifluoromethyl)-1*H-*pyrazole-4-carboxylic acid** 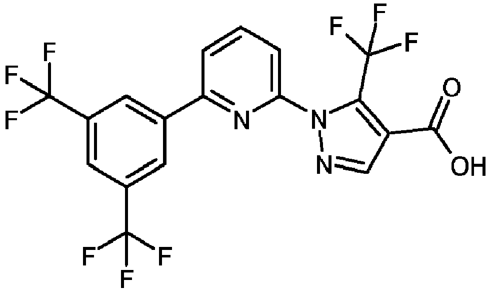 **Example 7: 1-[6-(3-chloro-4-fluorophenyl)-2-pyridinyl]-5-(trifluoromethyl)-1*H-*pyrazole-4-carboxylic acid** 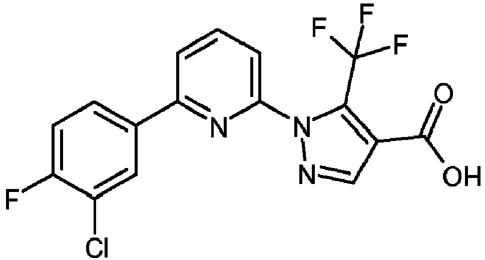 **Example 8: 1-[6-(4-bromo-3-fluorophenyl)-2-pyridinyl]-5-(trifluoromethyl)-1*H-*pyrazole-4-carboxylic acid** 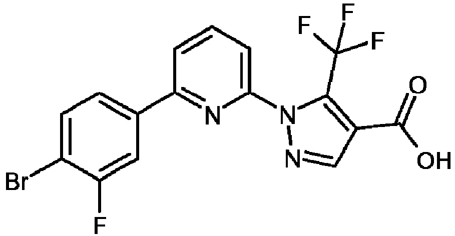 **Example 9: 1-[6-(3,5-dichlorophenyl)-2-pyridinyl]-5-(trifluoromethyl)-1*H*-pyrazole-4-carboxylic acid** 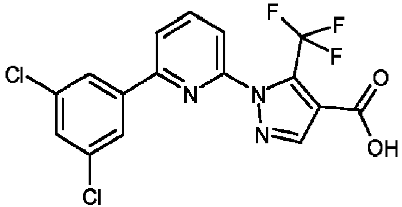 **Example 10: 1-[6-(3,4-dichlorophenyl)-4-methyl-2-pyridinyl]-5-(trifluoromethyl)-1*H-*pyrazole-4-carboxylic acid** 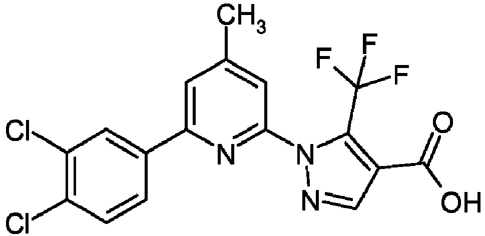 **Example 11: 1-(6-(3,4-dichlorophenyl)-pyridin-2-yl)-pyrrole-3-carboxylic acid** 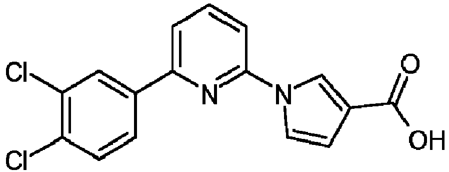 **Example 12:1-(6-(3,4-dichlorophenyl)-pyridin-2-yl)-2-methyl-pyrrole-3-carboxylic acid** 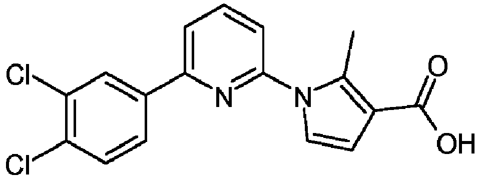 **Example 13**: **1-(6-(3,4-dichlorophenyl)-pyridin-2-yl)-piperidine-4-carboxylic acid** 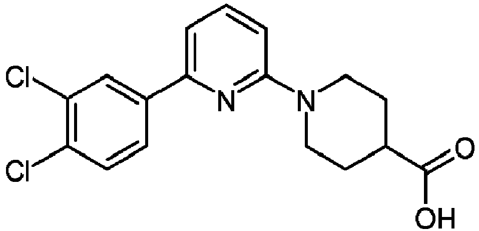 **Example 14: 4-(6-(3-trifluoromethylphenyl)-pyridin-2-yloxy)-benzoic acid** 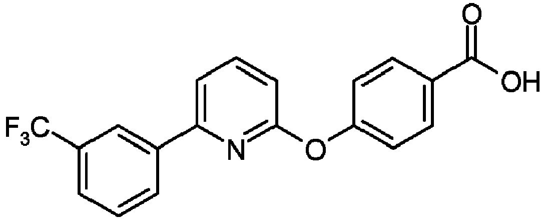 **Example 15: 5-(6-(3-trifluoromethylphenyl)-pyridin-2-yl)-thiophene-2-carboxylic acid** 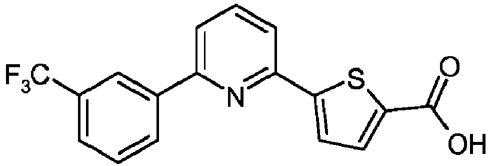 **Example 16: 1-[2-(3,4-dichlorophenyl)-4-pyrimidinyl]-5-(trifluoromethyl)-1*H*-pyrazole-4-carboxylic acid** 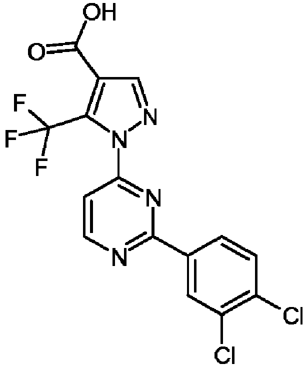 **Example 17: 5-(trifluoromethyl)-1-{2-[3-(trifluoromethyl)phenyl]-4-pyrimidinyl}-1*H-*pyrazole-4-carboxylic acid** 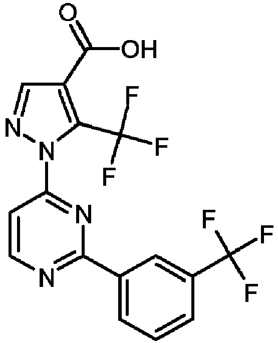 **Example 18: 5-(2-(3-trifluoromethylphenyl)-pyrimidin-4-yl)-thiophene-2-carboxylic acid** 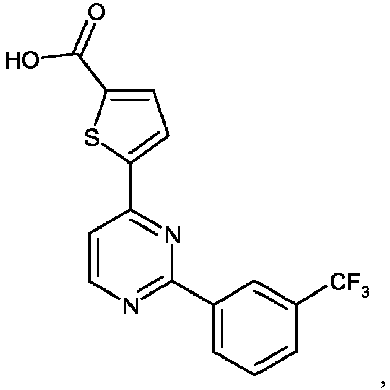 Compound Class VII
**Example 1: 1-[4-(2-{[(4'-cyano-2',3-dimethyl-4-biphenylyl)methyl]oxy}-5-methylphenyl)-1,3-thiazol-2-yl]-4-piperidinecarboxylic acid.** 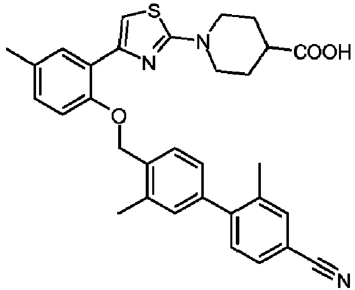
| **Example** | **Name** |
|---|---|
| **2** | 1-[4-(2-{[(4'-cyano-2',3-dimethyl-4-biphenylyl)methyl]oxy}-5-ethylphenyl)-1,3-thiazol-2-yl]-4-piperidinecarboxylic acid |
| **3** | 1-{4-[2-{[(4'-cyano-2',3-dimethyl-4-biphenylyl)methyl]oxy}-5-(trifluoromethyl)phenyl]-1,3-thiazol-2-yl}-4-piperidinecarboxylic acid |
| **4** | 1-{4-[2-({[4'-cyano-2'-methyl-3-(propyloxy)-4-biphenylyl]methyl}oxy)-5-methylphenyl]-1,3-thiazol-2-yl}-4-piperidinecarboxylic acid |
**Example 5: 1-{4-[2-({[2'-chloro-3-methyl-4'-(trifluoromethyl)-4-biphenylyl]methyl}oxy)-5-methylphenyl]-1,3-thiazol-2-yl}-4-piperidinecarboxylic acid** 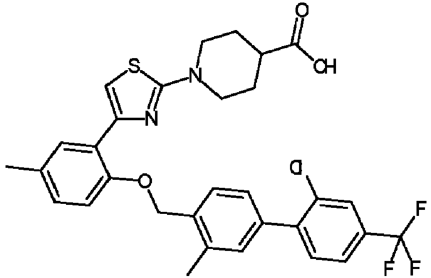 **Example 6: 1-{4-[2-({[4-(2-phenylethyl)phenyl]methyl}oxy)phenyl]-1,3-thiazol-2-yl}-4-piperidinecarboxylic acid** 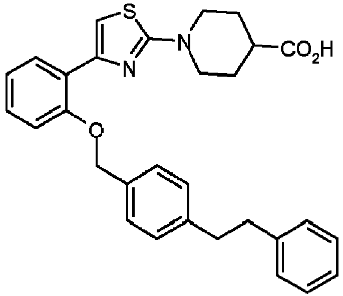
| **Example** | **Name** |
|---|---|
| **7** | 1-{4-[2-({[3'-(methyloxy)-4-biphenylyl]methyl}oxy)phenyl]-1,3-thiazol-2-yl}-4-piperidinecarboxylic acid |
| **8** | 1-[4-(2-{[(4'-fluoro-4-biphenylyl)methyl]oxy}phenyl)-1,3-thiazol-2-yl]-4-piperidinecarboxylic acid |
| **9** | 1-[4-(2-{[(4'-chloro-4-biphenylyl)methyl]oxy}phenyl)-1,3-thiazol-2-yl]-4-piperidinecarboxylic acid |
| **10** | 1-{4-[2-({[4'-(methyloxy)-4-biphenylyl]methyl}oxy)phenyl]-1,3-thiazol-2-yl}-4-piperidinecarboxylic acid |
| **11** | 1-{4-[2-({[4'-(trifluoromethyl)-4-biphenylyl]methyl}oxy)phenyl]-1,3-thiazol-2-yl}-4-piperidinecarboxylic acid |
| **12** | 1-[4-(2-{[(4'-cyano-4-biphenylyl)methyl]oxy}phenyl)-1,3-thiazol-2-yl]-4-piperidinecarboxylic acid |
| **13** | 1-{4-[2-({[3-methyl-4'-(trifluoromethyl)-4-biphenylyl]methyl}oxy)phenyl]-1,3-thiazol-2-yl}-4-piperidinecarboxylic acid |
| **14** | 1-[4-(2-{[(4'-chloro-3-methyl-4-biphenylyl)methyl]oxy}phenyl)-1,3-thiazol-2-yl]-4-piperidinecarboxylic acid |
| **15** | 1-{4-[2-({[3-methyl-4'-(methyloxy)-4-biphenylyl]methyl}oxy)phenyl]-1,3-thiazol-2-yl}-4-piperidinecarboxylic acid |
| **16** | 1-{4-[2-({[3-fluoro-4'-(methyloxy)-4-biphenylyl]methyl}oxy)phenyl]-1,3-thiazol-2-yl}-4-piperidinecarboxylic acid |
| **17** | 1-[4-(2-{[(4'-chloro-3-fluoro-4-biphenylyl)methyl]oxy}phenyl)-1,3-thiazol-2-yl]-4-piperidinecarboxylic acid |
| 18 | 1-(4-{2-[({4-[(4-chlorophenyl)oxy]phenyl}methyl)oxy]phenyl}-1,3-thiazol-2-yl)-4-piperidinecarboxylic acid |
| **19** | 1-[4-(2-{[(4-{[4-(trifluoromethyl)phenyl]oxy}phenyl)methyl]oxy}phenyl)-1,3-thiazol-2-yl]-4-piperidinecarboxylic acid |
| **20** | 1-(4-{2-[({4-[(4-fluorophenyl)oxy]phenyl}methyl)oxy]phenyl}-1,3-thiazol-2-yl)-4-piperidinecarboxylic acid |
| **21** | 1-[4-(2-{[(4'-carboxy-3-fluoro-4-biphenylyl)methyl]oxy}phenyl)-1,3-thiazol-2-yl]-4-piperidinecarboxylic acid |
| **22** | 1-(4-{2-[({4-[(4-cyanophenyl)oxy]phenyl}methyl)oxy]phenyl}-1,3-thiazol-2-yl)-4-piperidinecarboxylic acid |
| **23** | 1-{4-[2-({[3,4'-bis(methyloxy)-4-biphenylyl]methyl}oxy)phenyl]-1,3-thiazol-2-yl}-4-piperidinecarboxylic acid |
| **24** | 1-[4-(2-{[(4-{[4-(methyloxy)phenyl]oxy}phenyl)methyl]oxy}phenyl)-1,3-thiazol-2-yl]-4-piperidinecarboxylic acid |
| **25** | 1-{4-[2-({[4'-cyano-3-(methyloxy)-4-biphenylyl]methyl}oxy)phenyl]-1,3-thiazol-2-yl}-4-piperidinecarboxylic acid |
| **26** | 1-{4-[2-({[3-chloro-4'-(trifluoromethyl)-4-biphenylyl]methyl}oxy)phenyl]-1,3-thiazol-2-yl}-4-piperidinecarboxylic acid |
| **27** | 1-{4-[2-({[3-chloro-4'-(methyloxy)-4-biphenylyl]methyl}oxy)phenyl]-1,3-thiazol-2-yl}-4-piperidinecarboxylic acid |
| **28** | 1-[4-(2-{[(3-chloro-4'-fluoro-4-biphenylyl)methyl]oxy}phenyl)-1,3-thiazol-2-yl]-4-piperidinecarboxylic acid |
| **29** | 1-{4-[2-({[3-(methyloxy)-4'-(trifluoromethyl)-4-biphenylyl]methyl}oxy)phenyl]-1,3-thiazol-2-yl}-4-piperidinecarboxylic acid |
| **30** | 1-[4-(2-{[(4'-cyano-4-biphenylyl)methyl]oxy}-3,5-difluorophenyl)-1,3-thiazol-2-yl]-4-piperidinecarboxylic acid |
| **31** | 1-(4-{2-[({4-[(4-cyanophenyl)oxy]phenyl}methyl)oxy]-3,5-difluorophenyl}-1,3-thiazol-2-yl)-4-piperidinecarboxylic acid |
| **32** | 1-{4-[3,5-difluoro-2-({[4'-(methyloxy)-4-biphenylyl]methyl}oxy)phenyl]-1,3-thiazol-2-yl}-4-piperidinecarboxylic acid |
| ***33*** | 1-(4-{2-[({2-methyl-4-[6-(methyloxy)-3-pyridinyl]phenyl}methyl)oxy]phenyl}-1,3-thiazol-2-yl)-4-piperidinecarboxylic acid |
| **34** | 1-{4-[2-({[4'-(hydroxymethyl)-3-methyl-4-biphenylyl]methyl}oxy)phenyl]-1,3-thiazol-2-yl}-4-piperidinecarboxylic acid |
| **35** | 1-[4-(2-{[(4'-fluoro-3-methyl-4-biphenylyl)methyl]oxy}phenyl)-1,3-thiazol-2-yl]-4-piperidinecarboxylic acid |
| **37** | 1-[4-(2-{[(4'-cyano-3-methyl-4-biphenylyl)methyl]oxy}phenyl)-1,3-thiazol-2-yl]-4-piperidinecarboxylic acid |
| | |
| **Example 36** | 1-{4-[4-chloro-2-({[3-methyl-4'-(trifluoromethyl)-4-biphenylyl]methyl}oxy)phenyl]-1,3-thiazol-2-yl}-4-piperidinecarboxylic acid |
**Example 38: 1-[5-chloro-4-(2-([(4'-cyano-4-biphenylyl)methyl]oxy}phenyl)-1,3-thiazol-2-yl]-4-piperidinecarboxylic acid** 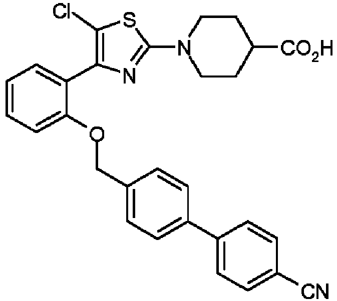
| **Example** | **Name** |
|---|---|
| **39** | 1-{5-chloro-4-[2-({[4-(2-phenylethyl)phenyl]methyl}oxy)phenyl]-1,3-thiazol-2-yl}-4-piperidinecarboxylic acid |
| **40** | 1-(5-chloro-4-{2-[({4-[(4-cyanophenyl)oxy]phenyl}methyl)oxy]phenyl}-1,3-thiazol-2-yl)-4-piperidinecarboxylic acid |
**Example 41: 1-{5-methyl-4-[2-({[4'-(methyloxy)-4-biphenylyl]methyl}oxy)phenyl]-1,3-thiazol-2-yl}-4-piperidinecarboxylic acid** 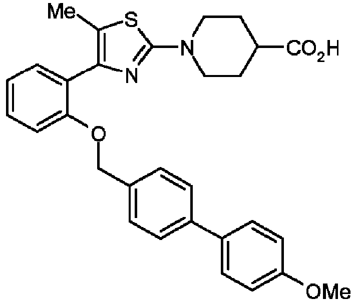
| | |
|---|---|
| **Example 42** | 1-{4-[3,5-difluoro-2-({[3-methyl-4'-(trifluoromethyl)-4-biphenylyl]methyl}oxy)phenyl]-1,3-thiazol-2-yl}-4-piperidinecarboxylic acid |
| | |
| **Example** | **Name** |
|---|---|
| **43** | 1-[4-(2-{[(4'-cyano-4-biphenylyl)methyl]oxy}phenyl)-5-methyl-1,3-thiazol-2-yl]-4-piperidinecarboxylic acid |
| **45** | 1-(4-{2-[({4-[(4-cyanophenyl)oxy]phenyl}methyl)oxy]phenyl}-5-methyl-1,3-thiazol-2-yl)-4-piperidinecarboxylic acid |
| | |
| **Example 44** | 1-{4-[2-fluoro-6-({[3-methyl-4'-(trifluoromethyl)-4-biphenylyl]methyl}oxy)phenyl]-1,3-thiazol-2-yl}-4-piperidinecarboxylic acid |
| **46** | 1-[4-(2-{[(4'-cyano-3-methyl-4-biphenylyl)methyl]oxy}-3,5-difluorophenyl)-1,3-thiazol-2-yl]-4-piperidinecarboxylic acid |
| **47** | 1-{4-[2-({[4'-(aminocarbonyl)-3-methyl-4-biphenylyl]methyl}oxy)-3,5-difluorophenyl]-1,3-thiazol-2-yl}-4-piperidinecarboxylic acid |
| **48** | 1-[4-(2-{[(4'-cyano-4-biphenylyl)methyl]oxy}-5-fluorophenyl)-1,3-thiazol-2-yl]-4-piperidinecarboxylic acid |
| **49** | 1-[4-(2-{[(4'-cyano-3-methyl-4-biphenylyl)methyl]oxy}-5-fluorophenyl)-1,3-thiazol-2-yl]-4-piperidinecarboxylic acid |
| **50** | 1-{4-[2-({[2',3-dimethyl-4'-(trifluoromethyl)-4-biphenylyl]methyl}oxy)-3,5-difluorophenyl]-1,3-thiazol-2-yl}-4-piperidinecarboxylic acid |
| **51** | 1-{4-[5-chloro-2-({[2',3-dimethyl-4'-(trifluoromethyl)-4-biphenylyl]methyl}oxy)phenyl]-1,3-thiazol-2-yl}-4-piperidinecarboxylic acid |
| **52** | 1-[4-(5-chloro-2-{[(4'-cyano-3-methyl-4-biphenylyl)methyl]oxy}phenyl)-1,3-thiazol-2-yl]-4-piperidinecarboxylic acid |
| **Example 53** | 1-[4-(2-{[(4'-cyano-2',3-dimethyl-4-biphenylyl)methyl]oxy}-3,5-difluorophenyl)-1,3-thiazol-2-yl]-4-piperidinecarboxylic acid |
**Example 54: 1-{4-[5-methyl-2-({[3-methyl-4'-(trifluoromethyl)-4-biphenylyl]methyl}oxy)phenyl]-1,3-thiazol-2-yl}-4-piperidinecarboxylic acid** 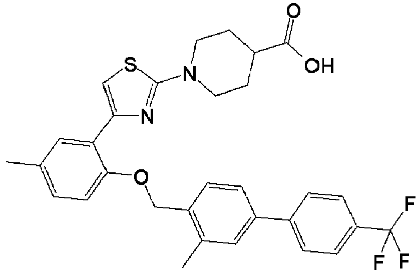 **Example 55: 1-{4-[5-methyl-2-({[3-methyl-4'-(trifluoromethyl)-4-biphenylyl]methyl}oxy)phenyl]-1,3-thiazol-2-yl}-4-piperidinecarboxylic acid-2-aminoethanol** (1:1) 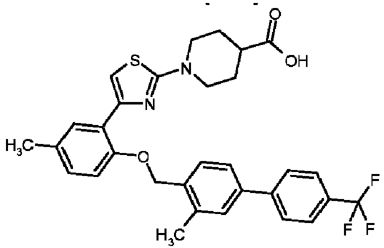 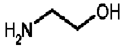 **Example 56**: **sodium 1-{4-[5-methyl-2-({[3-methyl-4'-(trifluoromethyl)-4-biphenylyl]methyl}oxy)phenyl]-1,3-thiazol-2-yl}-4-piperidinecarboxylate** 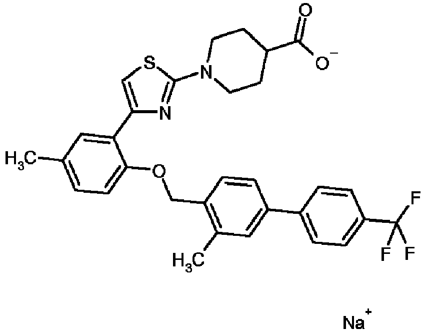
| | |
|---|---|
| **Example 57** | 1-[4-(2-{[(4'-cyano-3-methyl-4-biphenylyl)methyl]oxy}-5-methylphenyl)-1,3-thiazol-2-yl]-4-piperidinecarboxylic acid |
| | |
| **Example 58** | 1-[4-(2-{[(4'-cyano-3-methyl-4-biphenylyl)methyl]oxy}-5-methylphenyl)-1,3-thiazol-2-yl]-4-piperidinecarboxylic acid 2-aminoethanol (1:1) |
| | |
| **Example 59** | 1-(4-{2-{[(4'-cyano-3-methyl-4-biphenylyl)methyl]oxy}-5-[(trifluoromethyl)oxy]phenyl}-1,3-thiazol-2-yl)-4-piperidinecarboxylic acid |
| | |
| **Example** | **Name** |
| **60** | 1-(4-{5-methyl-2-[({3-methyl-4'-[(trifluoromethyl)oxy]-4-biphenylyl}methyl)oxy]phenyl}-1,3-thiazol-2-yl)-4-piperidinecarboxylic acid |
| **61** | 1-(4-{2-[({4'-[(dimethylamino)carbonyl]-3-methyl-4-biphenylyl}methyl)oxy]-5-methylphenyl}-1,3-thiazol-2-yl)-4-piperidinecarboxylic acid |
| | |
| **Example 62** | 1-(4-{5-chloro-2-[({3-methyl-4'-[(trifluoromethyl)oxy]-4-biphenylyl}methyl)oxy]phenyl}-1,3-thiazol-2-yl)-4-piperidinecarboxylic acid |
| | |
| **Example 63** | 1-{4-[2-{[(4'-cyano-3-methyl-4-biphenylyl)methyl]oxy}-5-(trifluoromethyl)phenyl]-1,3-thiazol-2-yl}-4-piperidinecarboxylic acid |
| **64** | 1-{4-[5-(methyloxy)-2-({[3-methyl-4'-(trifluoromethyl)-4-biphenylyl]methyl}oxy)phenyl]-1,3-thiazol-2-yl}-4-piperidinecarboxylic acid |
| **65** | 1-(4-{5-(methyloxy)-2-[({3-methyl-4'-[(trifluoromethyl)oxy]-4-biphenylyl}methyl)oxy]phenyl}-1,3-thiazol-2-yl)-4-piperidinecarboxylic acid |
| **66** | 1-{4-[2-{[(4'-cyano-3-methyl-4-biphenylyl)methyl]oxy}-5-(methyloxy)phenyl]-1,3-thiazol-2-yl}-4-piperidinecarboxylic acid |
| **72** | 1-(4-{2-[({4-[3-chloro-5-(trifluoromethyl)-2-pyridinyl]-2-methylphenyl}methyl)oxy]-5-methylphenyl}-1,3-thiazol-2-yl)-4-piperidinecarboxylic acid |
| **73** | 1-(4-{2-[({4-[3-chloro-5-(trifluoromethyl)-2-pyridinyi]-2-methylphenyl}methyl)oxy]-5-ethylphenyl}-1,3-thiazol-2-yl)-4-piperidinecarboxylic acid |
| **74** | 1-{4-[5-ethyl-2-({[3-methyl-4'-(trifluoromethyl)-4-biphenylyl]methyl}oxy)phenyl]-1,3-thiazol-2-yl}-4-piperidinecarboxylic acid |
| **75** | 1-(4-{5-ethyl-2-[({3-methyl-4'-[(trifluoromethyl)oxy]-4-biphenylyl}methyl)oxy]phenyl}-1,3-thiazol-2-yl)-4-piperidinecarboxylic acid |
| **76** | 1-{4-[2-({[3-methyl-4'-(trifluoromethyl)-4-biphenylyl]methyl}oxy)-3-(trifluoromethyl)phenyl]-1,3-thiazol-2-yl}-4-piperidinecarboxylic acid |
| **77** | 1-{4-[2-{[(4'-cyano-3-methyl-4-biphenylyl)methyl]oxy}-3-(trifluoromethyl)phenyl]-1,3-thiazol-2-yl}-4-piperidinecarboxylic acid |
| **78** | 1-[4-(2-{[(4'-cyano-3-methyl-4-biphenylyl)methyl]oxy}-3-methylphenyl)-1,3-thiazol-2-yl]-4-piperidinecarboxylic acid |
| **79** | 1-{4-[2-[({4-[3-chloro-5-(trifluoromethyl)-2-pyridinyl]-2-methylphenyl}methyl)oxy]-5-(trifluoromethyl)phenyl]-1,3-thiazol-2-yl}-4-piperidinecarboxylic acid |
| **80** | 1-{4-[2-{[(4'-cyano-2',3-dimethyl-4-biphenylyl)methyl]oxy}-3-(trifluoromethyl)phenyl]-1,3-thiazol-2-yl}-4-piperidinecarboxylic acid |
| **67** | 1-{4-[2-{[(4'-cyano-3-methyl-4-biphenylyl)methyl]oxy}-5-(1-methylethyl)phenyl]-1,3-thiazol-2-yl}-4-piperidinecarboxylic acid |
| **68** | 1-[4-(2-{[(4'-cyano-3-methyl-4-biphenylyl)methyl]oxy}-5-ethylphenyl)-1,3-thiazol-2-yl]-4-piperidinecarboxylic acid |
| **69** | 1-{4-[5-(1-methylethyl)-2-({[3-methyl-4'-(trifluoromethyl)-4-biphenylyl]methyl}oxy)phenyl]-1,3-thiazol-2-yl}-4-piperidinecarboxylic acid |
| **70** | 1-{4-[2-[({4-[3-chloro-5-(trifluoromethyl)-2-pyridinyl]-2-methylphenyl}methyl)oxy]-3-(trifluoromethyl)phenyl]-1,3-thiazol-2-yl}-4-piperidinecarboxylic acid |
| **71** | 1-{4-[3-methyl-2-({[3-methyl-4'-(trifluoromethyl)-4-biphenylyl]methyl}oxy)phenyl]-1,3-thiazol-2-yl}-4-piperidinecarboxylic acid |
| **72** | 1-(4-{2-[({4-[3-chloro-5-(trifluoromethyl)-2-pyridinyl]-2-methylphenyl}methyl)oxy]-5-methylphenyl}-1,3-thiazol-2-yl)-4-piperidinecarboxylic acid |
| **73** | 1-(4-{2-[({4-[3-chloro-5-(trifluoromethyl)-2-pyridinyi]-2-methylphenyl}methyl)oxy]-5-ethylphenyl}-1,3-thiazol-2-yl)-4-piperidinecarboxylic acid |
| **74** | 1-{4-[5-ethyl-2-({[3-methyl-4'-(trifluoromethyl)-4-biphenylyl]methyl}oxy)phenyl]-1,3-thiazol-2-yl}-4-piperidinecarboxylic acid |
| **75** | 1-(4-{5-ethyl-2-[({3-methyl-4'-[(trifluoromethyl)oxy]-4-biphenylyl}methyl)oxy]phenyl}-1,3-thiazol-2-yl)-4-piperidinecarboxylic acid |
| **76** | 1-{4-[2-({[3-methyl-4'-(trifluoromethyl)-4-biphenylyl]methyl}oxy)-3-(trifluoromethyl)phenyl]-1,3-thiazol-2-yl}-4-piperidinecarboxylic acid |
| **77** | 1-{4-[2-{[(4'-cyano-3-methyl-4-biphenylyl)methyl]oxy}-3-(trifluoromethyl)phenyl]-1,3-thiazol-2-yl}-4-piperidinecarboxylic acid |
| **78** | 1-[4-(2-{[(4'-cyano-3-methyl-4-biphenylyl)methyl]oxy}-3-methylphenyl)-1,3-thiazol-2-yl]-4-piperidinecarboxylic acid |
| **79** | 1-{4-[2-[({4-[3-chloro-5-(trifluoromethyl)-2-pyridinyl]-2-methylphenyl}methyl)oxy]-5-(trifluoromethyl)phenyl]-1,3-thiazol-2-yl}-4-piperidinecarboxylic acid |
| **80** | 1-{4-[2-{[(4'-cyano-2',3-dimethyl-4-biphenylyl)methyl]oxy}-3-(trifluoromethyl)phenyl]-1,3-thiazol-2-yl}-4-piperidinecarboxylic acid |
**Example 81:** GSK2236881A 1-{4-[2-({[4-(5-cyano-2-pyridinyl)-2-methylphenyl]methyl}oxy)-5-methylphenyl]-1,3-thiazol-2-yl}-4-piperidinecarboxylic acid (N7106-76-1) 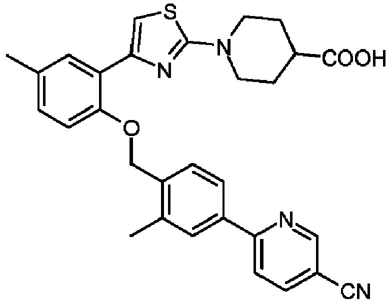
| **Example** | **Name** |
|---|---|
| **82** | 1-{4-[5-methyl-2-({[3-methyl-4'-(methyloxy)-2'-(trifluoromethyl)-4-biphenylyl]methyl}oxy)phenyl]-1,3-thiazol-2-yl}-4-piperidinecarboxylic acid |
| **83** | 1-{4-[5-ethyl-2-({[3-methyl-2',4'-bis(trifluoromethyl)-4-biphenylyl]methyl}oxy)phenyl]-1,3-thiazol-2-yl}-4-piperidinecarboxylic acid |
| **84** | 1-{4-[5-methyl-2-({[3-(propyloxy)-4'-(trifluoromethyl)-4-biphenylyl]methyl}oxy)phenyl]-1,3-thiazol-2-yl}-4-piperidinecarboxylic acid |
| **85** | 1-{4-[5-methyl-2-({[3-methyl-2',4'-bis(trifluoromethyl)-4-biphenylyl]methyl}oxy)phenyl]-1,3-thiazol-2-yl}-4-piperidinecarboxylic acid |
| **86** | 1-{4-[2-({[4-(5-cyano-2-pyridinyl)-2-methylphenyl]methyl}oxy)-5-(trifluoromethyl)phenyl]-1,3-thiazol-2-yl}-4-piperidinecarboxylic acid |
| **87** | 1-{4-[2-({[2'-chloro-3-methyl-4'-(trifluoromethyl)-4-biphenylyl]methyl}oxy)-3-(trifluoromethyl)phenyl]-1,3-thiazol-2-yl}-4-piperidinecarboxylic acid |
| **88** | 1-{4-[2-({[4-(5-cyano-2-pyridinyl)-2-methylphenyl]methyl}oxy)-3,5-dimethylphenyl]-1,3-thiazol-2-yl}-4-piperidinecarboxylic acid |
| | |
| **89** | 1-[4-(2-{[(4'-cyano-3-methyl-4-biphenylyl)methyl]oxy}-3,5-dimethylphenyl)-1,3-thiazol-2-yl]-4-piperidinecarboxylic acid |
| | |
| **Example 90** | 1-{4-[4-methyl-2-({[3-methyl-4'-(trifluoromethyl)-4-biphenylyl]methyl}oxy)phenyl]-1,3-thiazol-2-yl}-4-piperidinecarboxylic acid |
Compound Class VIII
**Example 1: 1-(4-(5-methyl-2-(2-methyl-4-(4-cyano-2-methylphenyl)benzyloxy)-phenyl)pyrimidin-2-yl)-piperidine-4-carboxylic acid** 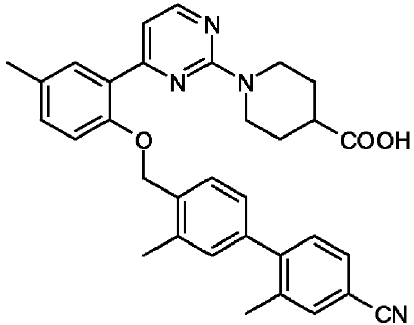 **Example 2: 1-(4-(5-methyl-2-(2-methyl-4-(4-methoxy-2-trifluoromethylphenyl)benzyloxy)-phenyl)pyrimidin-2-yl)-piperidine-4-carboxylic acid** 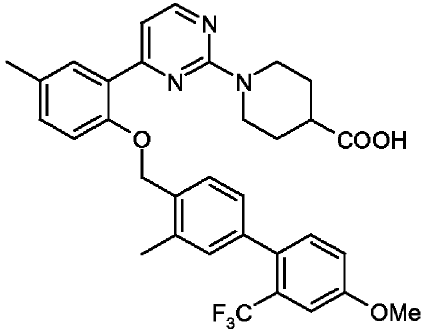
| **Example No.** | **Name** |
|---|---|
| 3 | 1-[4-(2-(4-(4-methoxyphenyl)phenylmethyloxy)-phenyl)pyrimidin-2-yl]-5-trifluoromethyl-pyrazole-4-carboxylic acid |
| 4 | 1-[4-(5-chloro-2-(4-(4-methoxyphenyl)phenylmethyloxy)-phenyl)pyrimidin-2-yl]-5-trifluoromethyl-pyrazole-4-carboxylic acid |
| 5 | 1-[4-(2-(4-(4-cyanophenyl)phenylmethyloxy)-phenyl)pyrimidin-2-yl]-5-trifluoromethyl-pyrazole-4-carboxylic acid |
| 6 | 1-[4-(2-(4-(4-trifluoromethylphenyl)phenylmethyloxy)-phenyl)pyrimidin-2-yl]-5-trifluoromethyl-pyrazole-4-carboxylic acid |
| 7 | 1-[4-(2-(2-methyl-4-(4-cyanophenyl)phenylmethyloxy)-phenyl)pyrimidin-2-yl]-5-trifluoromethyl-pyrazole-4-carboxylic acid |
| 8 | 1-[4-(2-(2-methyl-4-(4-methoxyphenyl)phenylmethyloxy)-phenyl)pyrimidin-2-yl]-5-trifluoromethyl-pyrazole-4-carboxylic acid |
| 9 | 1-[4-(2-(2-methyl-4-(4-trifluoromethylphenyl)phenylmethyloxy)-phenyl)pyrimidin-2-yl]-5-trifluoromethyl-pyrazole-4-carboxylic acid |
| 10 | 1-[4-(5-chloro-2-(2-methyl-4-(4-methoxyphenyl)phenylmethyloxy)-phenyl)pyrimidin-2-yl]-5-trifluoromethyl-pyrazole-4-carboxylic acid |
| 11 | 1-[4-(2-(4-(4-cyanophenyloxy)phenylmethyloxy)-phenyl)pyrimidin-2-yl]-5-trifluoromethyl-pyrazole-4-carboxylic acid |
| 12 | 1-[4-(5-chloro-2-(4-(4-cyanophenyloxy)phenylmethyloxy)-phenyl)pyrimidin-2-yl]-5-trifluoromethyl-pyrazole-4-carboxylic acid |
| 13 | 1-[4-(5-methyl-2-(4-(3-chloro-5-trifluoromethylpyridin-2-yloxy)phenylmethyloxy)phenyl)pyrimidin-2-yl]-5-trifluoromethyl-pyrazole-4-carboxylic acid |
| 14 | 1-[4-(5-methyl-2-(2-methoxy-4-(3-chloro-5-trifluoromethylpyridin-2-yloxy)phenylmethyloxy)phenyl)pyrimidin-2-yl]-5-trifluoromethyl-pyrazole-4-carboxylic acid |
| 15 | 1-[4-(5-methyl-2-(2-methyl-4-(3-chloro-5-trifluoromethylpyridin-2-yloxy)phenylmethyloxy)phenyl)pyrimidin-2-yl]-5-trifluoromethyl-pyrazole-4-carboxylic acid |
| 16 | 1-[4-(2-(2-methyl-4-(4-methoxyphenyl)phenylmethyloxy)-phenyl)pyrimidin-2-yl]-piperidine-4-carboxylic acid |
| 17 | 1-[4-(2-(2-methyl-4-(2,4-dimethoxy-phenyl)phenylmethyloxy)-phenyl)pyrimidin-2-yl]-piperidine-4-carboxylic acid |
| 18 | 1-[4-(2-(2-methyl-4-(4-methoxy-2-methyl-phenyl)phenylmethyloxy)-phenyl)pyrimidin-2-yl]-piperidine-4-carboxylic acid |
| 19 | 1-[4-(2-(2-methyl-4-(4-trifluoromethylphenyl)phenylmethyloxy)-phenyl)pyrimidin-2-yl-piperidine-4-carboxylic acid |
| 20 | 1-[4-(2-(2-methoxy-4-(4-trifluoromethylphenyl)phenylmethyloxy)-phenyl)pyrimidin-2-yl]-piperidine-4-carboxylic acid |
| 21 | 1-[4-(2-(2-methyl-4-(4-cyanophenyl)phenylmethyloxy)-phenyl)pyrimidin-2-yl]-piperidine-4-carboxylic acid |
| 22 | 1-[4-(5-fluoro-2-(2-methyl-4-(4-methoxyphenyl)phenylmethyloxy)-phenyl)pyrimidin-2-yl]-piperidine-4-carboxylic acid |
| 23 | 1-[4-(5-fluoro-2-(2-methyl-4-(4-fluorophenyl)phenylmethyloxy)-phenyl)pyrimidin-2-yl]-piperidine-4-carboxylic acid |
| 24 | 1-[4-(5-fluoro-2-(2-methyl-4-(4-cyanophenyl)phenylmethyloxy)-phenyl)pyrimidin-2-yl]-piperidine-4-carboxylic acid |
| 25 | 1-[4-(5-fluoro-2-(4-(4-cyanophenyloxy)phenylmethyloxy)-phenyl)pyrimidin-2-yl]-piperidine-4-carboxylic acid |
| 26 | 1-[4-(5-fluoro-2-(4-(5-trifluoromethylpyridin-2-yloxy)phenylmethyloxy)-phenyl)pyrimidin-2-yl]-piperidine-4-carboxylic acid |
| 27 | 1-[4-(5-methyl-2-(2-methyl-4-(4-trifluoromethoxyphenyl)phenylmethyloxy)-phenyl)pyrimidin-2-yl]-piperidine-4-carboxylic acid |
| 28 | 1-[4-(5-methyl-2-(2-methyl-4-(4-methoxyphenyl)phenylmethyloxy)-phenyl)pyrimidin-2-yl]-piperidine-4-carboxylic acid |
| 29 | 1-[4-(5-methyl-2-(2-methyl-4-(4-cyanophenyl)phenylmethyloxy)-phenyl)pyrimidin-2-yl]-piperidine-4-carboxylic acid |
| 30 | 1-[4-(5-methyl-2-(2-methyl-4-(2-methyl-4-trifluoromethylphenyl)phenylmethyloxy)-phenyl)pyrimidin-2-yl]-piperidine-4-carboxylic acid |
| 31 | 1-[4-(5-methyl-2-(2-methyl-4-(2-ch loro-4-methoxyphenyl)phenylmethyloxy)-phenyl)pyrimidin-2-yl]-piperidine-4-carboxylic acid |
| 32 | 1-[4-(5-methyl-2-(2-propyloxy-4-(4-trifluoromethylphenyl)phenylmethyloxy)-phenyl)pyrimidin-2-yl]-piperidine-4-carboxylic acid |
| 33 | 1-[4-(5-methyl-2-(2-propyloxy-4-(4-cyanophenyl)phenylmethyloxy)-phenyl)pyrimidin-2-yl]-piperidine-4-carboxylic acid |
| 34 | 1-[4-(5-methyl-2-(2-propyloxy-4-(2-chloro-4-methoxyphenyl)phenylmethyloxy)-phenyl)pyrimidin-2-yl]-piperidine-4-carboxylic acid |
| 35 | 1-[4-(5-methyl-2-(2-propyloxy-4-(4-methoxy-2-trifluoromethylphenyl)phenylmethyloxy)-phenyl)pyrimidin-2-yl]-piperidine-4-carboxylic acid |
| 36 | 1-[4-(5-methyl-2-(2-propyloxy-4-(4-chloro-2-trifluoromethylphenyl)phenylmethyloxy)-phenyl)pyrimidin-2-yl]-piperidine-4-carboxylic acid |
| 37 | 1-[4-(5-methyl-2-(2-methyl-4-(6-methoxypyridin-3-yl)phenylmethyloxy)-phenyl)pyrimidin-2-yl]-piperidine-4-carboxylic acid |
| 38 | 1-[4-(5-methyl-2-(2-methyl-4-(5-trifluoromethylpyridin-2-yl)phenylmethyloxy)-phenyl)pyrimidin-2-yl]-piperidine-4-carboxylic acid |
| 39 | 1-[4-(5-methyl-2-(2-methyl-4-(3-ch loro-5-trifluoromethylpyrid in-2-yl)phenylmethyloxy)-phenyl)pyrimidin-2-yl]-piperidine-4-carboxylic acid |
| 40 | 1-[4-(5-methyl-2-(2-methyl-4-(3-ch loro-5-trifluoromethylpyrid in-2-yloxy)phenylmethyloxy)-phenyl)pyrimidin-2-yl]-piperidine-4-carboxylic acid |
| 41 | 1-[4-(5-methyl-2-(2-methyl-4-(2-ch loro-4-cyanophenoxy)phenylmethyloxy)-phenyl)pyrimidin-2-yl]-piperidine-4-carboxylic acid |
| 42 | 1-[4-(5-trifluoromethyl-2-(2-methyl-4-(4-ch loro-2-trifluoromethylphenyl)phenylmethyloxy)-phenyl)pyrimidin-2-yl]-piperidine-4-carboxylic acid |
| 43 | 1-[4-(5-trifluoromethyl-2-(2-methyl-4-(3-chloro-5-trifluoromethylpyridin-2-yl)phenylmethyloxy)-phenyl)pyrimidin-2-yl]-piperidine-4-carboxylic acid |
| 44 | 1-[2-(2-(4-(4-cyanophenyl)phenylmethyloxy)-phenyl)pyrimidin-4-yl]-5-trifluoromethyl-pyrazole-4-carboxylic acid |
| 45 | 1-[2-(2-(4-(4-trifluoromethylphenyl)phenylmethyloxy)-phenyl)pyrimidin-4-yl]-5-trifluoromethyl-pyrazole-4-carboxylic acid |
| 46 | 1-[2-(2-(4-(4-methoxyphenyl)phenylmethyloxy)-phenyl)pyrimidin-4-yl]-5-trifluoromethyl-pyrazole-4-carboxylic acid |
**Example 47: 1-(4-(5-trifluoromethyl-2-(2-methyl-4-(4-cyanophenyl)benzyloxy)-phenyl)pyrimidin-2-yl)-piperidine-4-carboxylic acid** 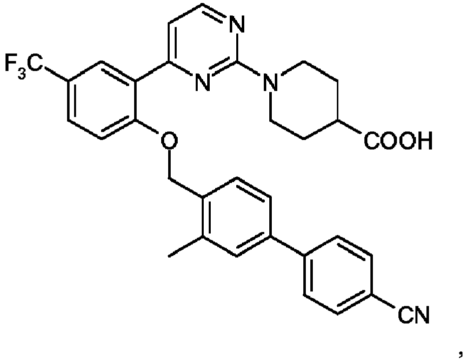 **Compound class IX**
Example 1 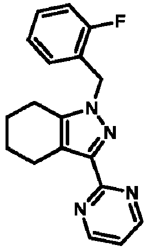 Example 2 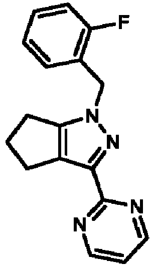 Example 3 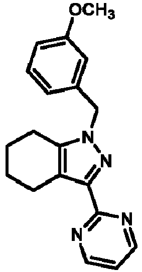 Example 4 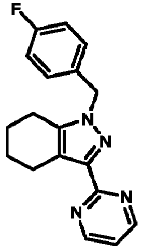 Example 5 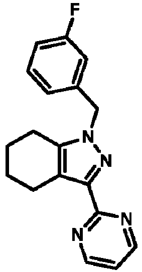 Example 6 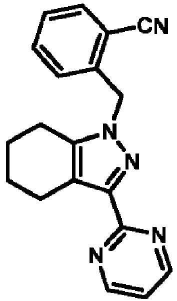 Example 7 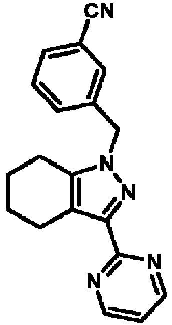 Example 8 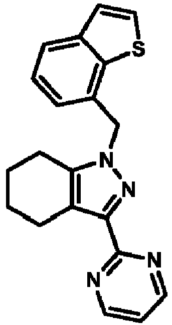 Example 9 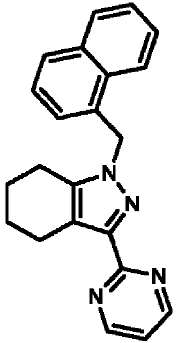 Example 10 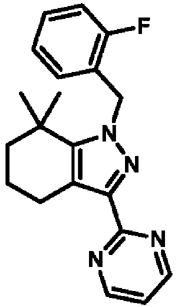 Example 11 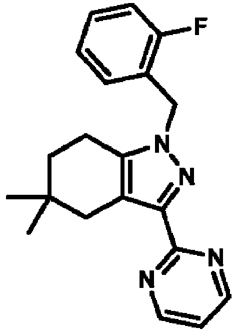 Example 12 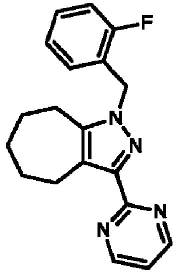 Example 13 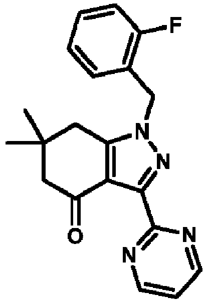 Example 14 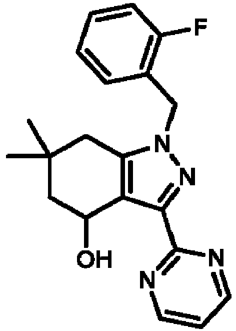 Example 15 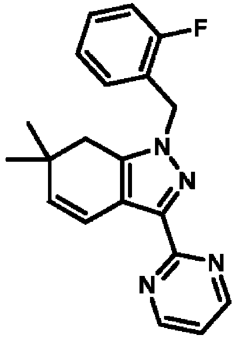 Example 16 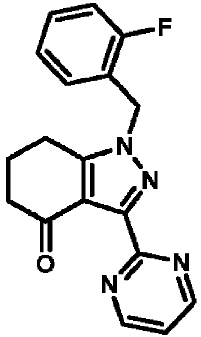 Example 17 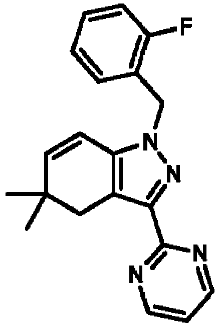 Example 18 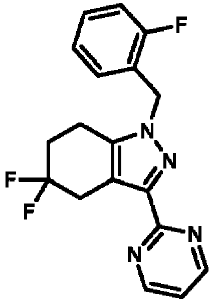 Example 19 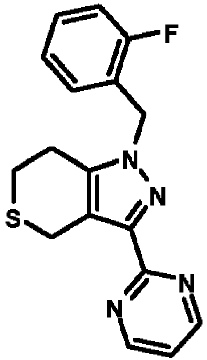 Example 20 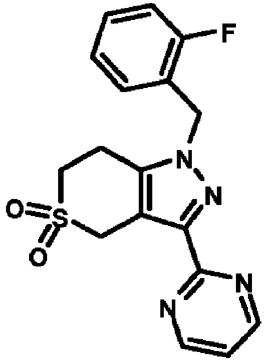 Example 21 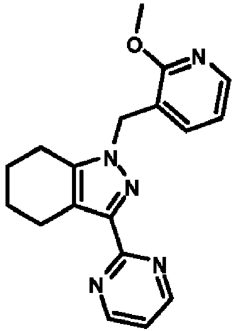 Example 22 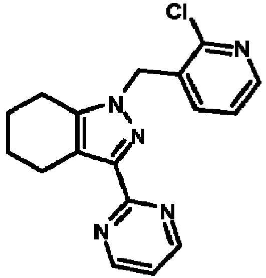 Example 23 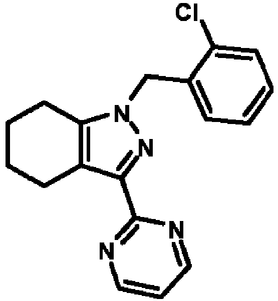 Example 24 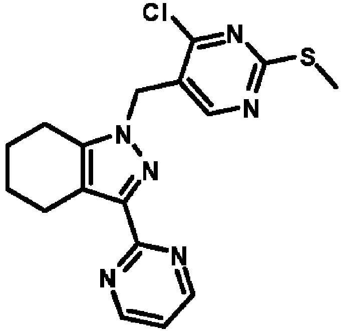 Example 25 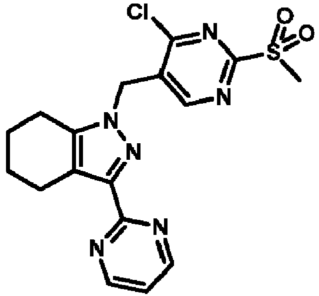 Example 26 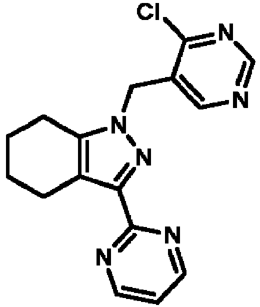 Example 27 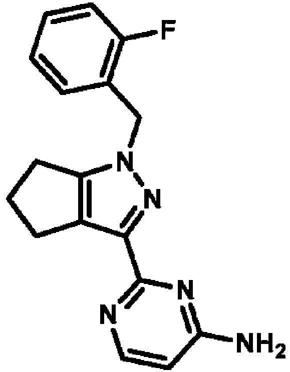 Example 28 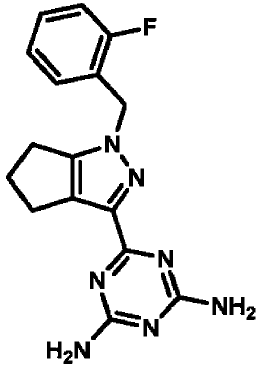 Example 29 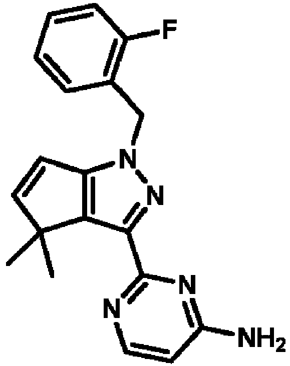 Example 30 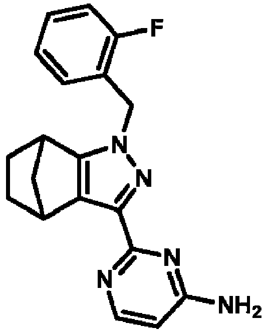 Example 31 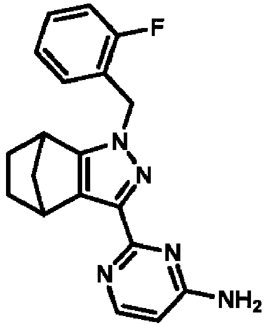 Example 32 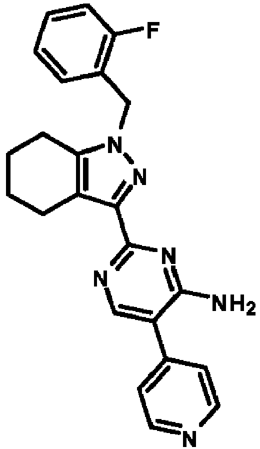 Example 33 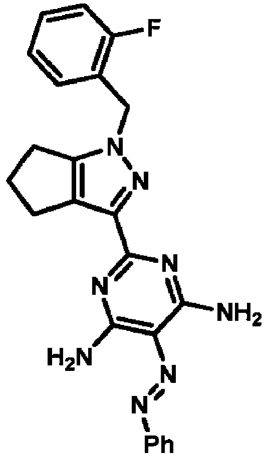 Example 34 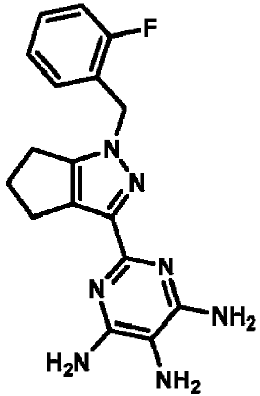 Example 35 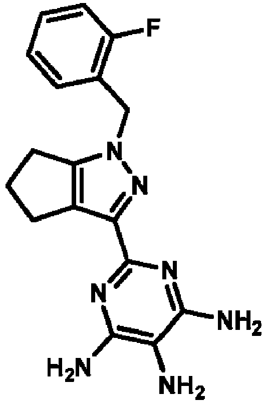 Example 36 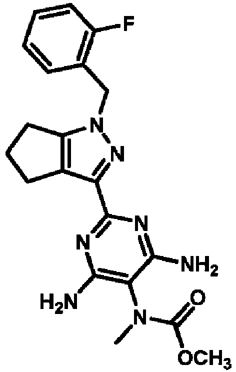 Example 37 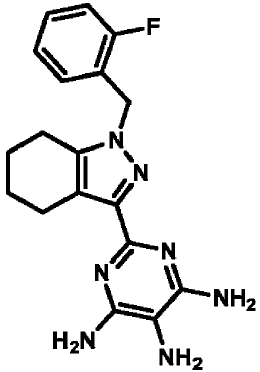 Example 38 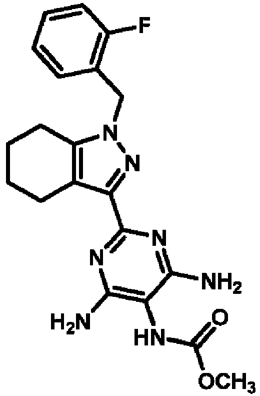 Example 39 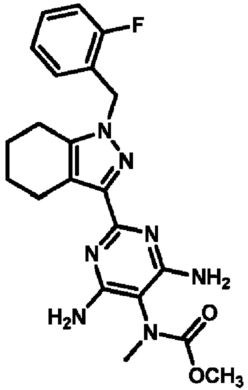 Example 40 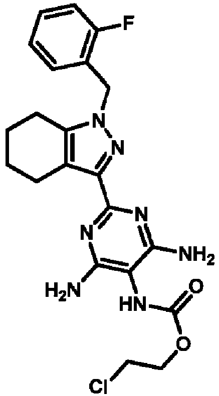 Example 41 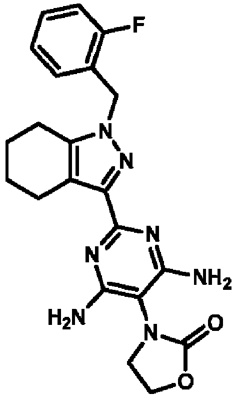 Example 42 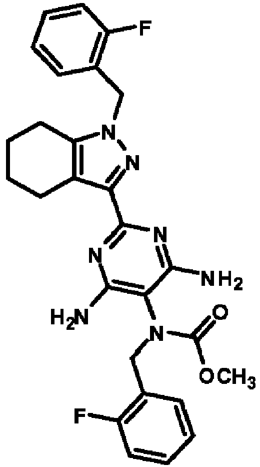 Example 43 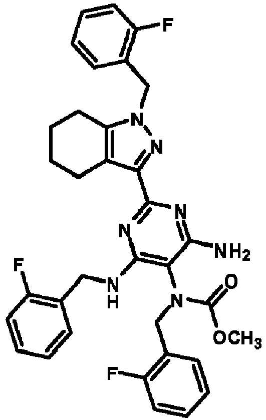 Example 44 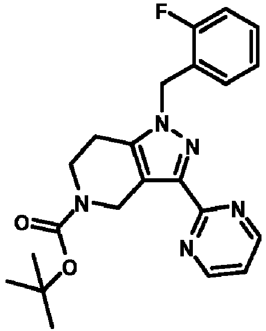 Example 45 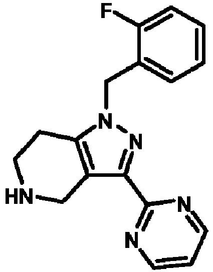 Example 46 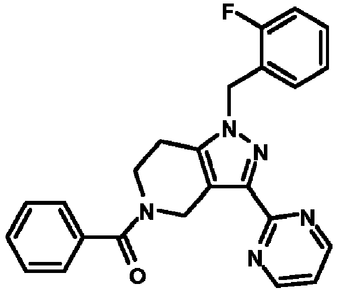 Example 47 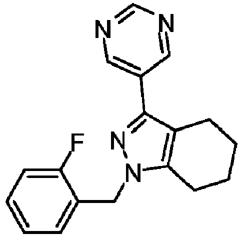 Example 48 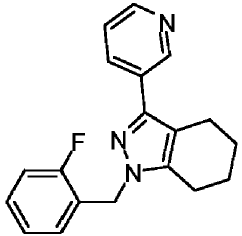 Example 49 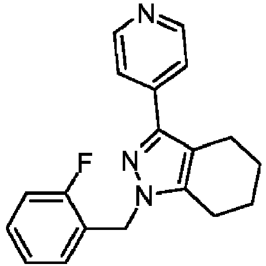 Example 50 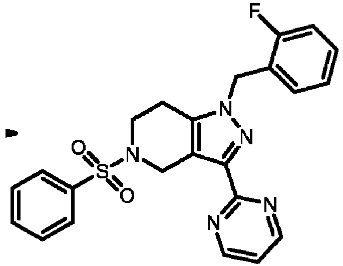 Example 51 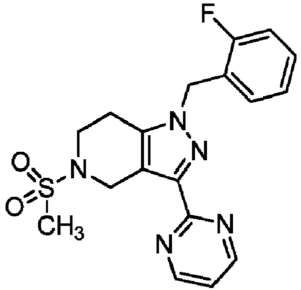 Example 52 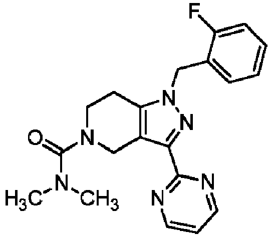 Example 53 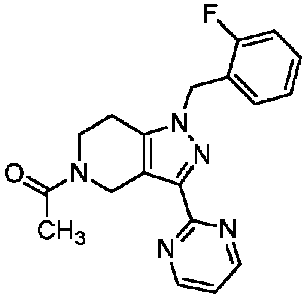 Example 54 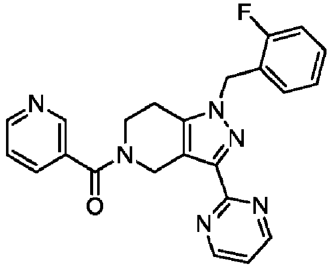 Example 55 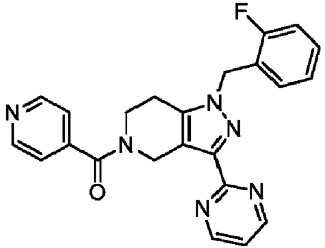 Example 56 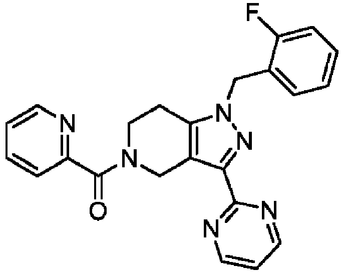 Example 57 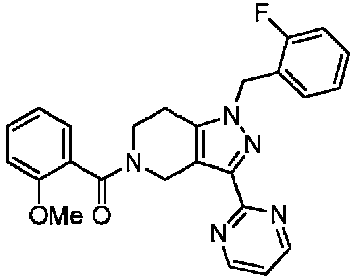 Example 58 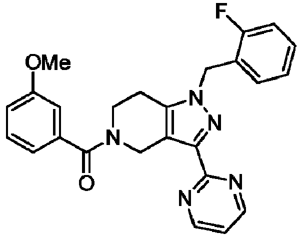 Example 59 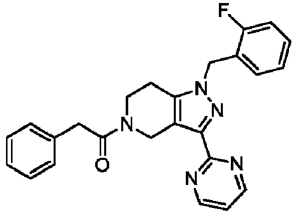 Example 60
1-(2-fluorobenzyl)-5-phenyl-3-(pyrimidin-5-yl)-4,5,6,7-tetrahydro-1H-pyrazolo[4,3-c]pyridine
Example 61
1-(2-fluorobenzyl)-5-(pyridine-3-yl)-3-(pyrimidin-5-yl)-4,5,6,7-tetrahydro-1H-pyrazolo[4,3-c]pyridine
Example 62
1-(2-fluorobenzyl)-5-(pyridine-4-yl)-3-(pyrimidin-5-yl)-4,5,6,7-tetrahydro-1H-pyrazolo[4,3-c]pyridine
Example 63
1-(2-fluorobenzyl)-5-(pyridine-2-yl)-3-(pyrimidin-5-yl)-4,5,6,7-tetrahydro-1H-pyrazolo[4,3-c]pyridine,
Compound class X
| EXAMPLE | IUPAC NAME |
|---|---|
| 2 | 2-[7-(2,3,6-trifluorobenzyl)imidazo[1,5-b]pyridazin-5-yl]-5-(1,3,5-trimethyl-1H-pyrazol-4-yl)pyrimidin-4-amine |
| 3 | 5-[1,5-dimethyl-3-(trifluoromethyl)-1*H*-pyrazol-4-yl]-2-[7-(2,3,6-trifluorobenzyl)imidazo[1,5-*b*]pyridazin-5-yl]pyrimidin-4-amine |
| 4 | 5-(2-methylpyridin-4-yl)-2-[7-(2,3,6-trifluorobenzyl)imidazo[1,5-*b*]pyridazin-5-yl]pyrimidin-4-amine |
| 5 | methyl {4,6-diamino-2-[7-(2,3,6-trifluorobenzyl)imidazo[1,5-*b*]pyridazin-5-yl]pyrimidin-5-yl}carbamate |
| 6 | methyl {4,6-diamino-2-[7-(2,3,6-trifluorobenzyl)imidazo[1,5-*b*]pyridazin-5-yl]pyrimidin-5-yl}methylcarbamate |
| 7 | 5-quinolin-5-yl-2-[7-(2,3,6-trifluorobenzyl)imidazo[1,5-*b*]pyridazin-5-yl]pyrimidin-4-amine |
| 8 | 5-pyridin-4-yl-2-[7-(2,3,6-trifluorobenzyl)imidazo[1,5-*b*]pyridazin-5-yl]pyrimidin-4-amine |
| 9 | 5-pyridin-3-yl-2-[7-(2,3,6-trifluorobenzyl)imidazo[1*,*5*-b*]pyridazin-5-yl]pyrimidin-4-amine |
| 10 | 2-[7-(2,3,6-trifluorobenzyl)imidazo[1,5-*b*]pyridazin-5-yl]-5-[5-(trifluoromethyl)pyridin-3-yl]pyrimidin-4-amine |
| 11 | 5-(5-fluoropyridin-3-yl)-2-[7-(2,3,6-trifluorobenzyl)imidazo[1,5-*b*]pyridazin-5-yl]pyrimidin4-amine |
| 12 | 5-(2-methoxypyridin-3-yl)-2-[7-(2,3,6-trifluorobenzyl)imidazo[1,5-*b*]pyridazin-5-yl]pyrimidin-4-amine |
| 13 | 5-(4-methylpyridin-3-yl)-2-[7-(2,3,6-trifluorobenzyl)imidazo[1,5-*b*]pyridazin-5-yl]pyrimidin-4-amine |
| 14 | 5-(4-methoxypyridin-3-yl)-2-[7-(2,3,6-trifluorobenzyl)imidazo[1,5-*b*]pyridazin-5-yl]pyrimidin-4-amine |
| 15 | 5-(1-methyl-1*H*-pyrazol-4-yl)-2-[7-(2,3,6-trifluorobenzyl)imidazo[1,5-*b*]pyridazin-5-yl]pyrimidin-4-amine |
| 16 | 5-(1-methyl-1*H*-pyrazol-5-yl)-2-[7-(2,3,6-trifluorobenzyl)imidazo[1,5-*b*]pyridazin-5-yl]pyrimidin-4-amine |
| 17 | 5-(7-fluoroquinolin-4-yl)-2-[7-(2,3,6-triftuorobenzyl)imidazo[1,5-*b*]pyridazin-5-yl]pyrimidin-4-amine |
| 18 | 2-[7-(2,3,6-trifluorobenzyl)imidazo[1,5-*b*]pyridazin-5-yl]-5-[2-(trifluoromethyl)quinolin-4-yl]pyrimidin-4-amine |
| 19 | 5-(3-fluoropyridin-4-yl)-2-[7-(2,3,6-trifluorobenzyl)imidazo[1,5-*b*]pyridazin-5-yl]pyrimidin-4-amine |
| 20 | 2-[7-(2,3,6-trifluorobenzyl)imidazo[1,5-*b*]pyridazin-5-yl]-5-[2-(trifluoromethyl)pyridin4-yl]pyrimidin-4-amine |
| 21 | 2-[7-(2,3,6-trifluorobenzyl)imidazo[1,5-*b*]pyridazin-5-yl]-5-[2-(trifluoromethyl)quinolin-4-yl]pyrimidin-4-amine |
| 22 | 5-[1-methyl-3-(trifluoromethyl)-1*H*-pyrazol-4-yl]-2-[7-(2,3,6-trifluorobenzyl)imidazo[1,5-*b*]-pyridazin-5-yl]pyrimidin-4-amine |
| 23 | 5-pyridin-4-yl-2-[7-(2,3,5-trifluorobenzyl)imidazo[1,5-*b*]pyridazin-5-yl]pyrimidin-4-amine |
| 24 | 5-pyridin-3-yl-2-[7-(2,3,5-trifluorobenzyl)imidazo[1,5-*b*]pyridazin-5-yl]pyrimidin-4-amine |
| 25 | 5-(4-methoxypyridin-3-yl)-2-[7-(2,3,5-trifluorobenzyl)imidazo[1,5-*b*]pyridazin-5-yl]pyrimidin-4-amine |
| 26 | 5-(4-methylpyridin-3-yl)-2-[7-(2,3,5-trifluorobenzyl)imidazo[1,5-*b*]pyridazin-5-yl]pyrimidin-4-amine |
| 27 | 5-quinolin-5-yl-2-[7-(2,3,5-trifluorobenzyl)imidazo[1,5-*b*]pyridazin-5-yl]pyrimidin-4-amine |
| 28 | 5-(2-methylpyridin-4-yl)-2-[7-(2,3,5-trifluorobenzyl)imidazo[1,5-*b*]pyridazin-5-yl]pyrimidin-4-amine |
| 29 | 2-[7-(2-chloro-3,6-difluorobenzyl]imidazo[1,5-*b*]pyridazin-5-yl]-5-pyridin-4-ylpyrimidin-4-amine |
| 30 | 2-[7-(2-chloro-3,6-difluorobenzyl)imidazo[1,5-*b*]pyridazin-5-yl]-5-(1,3,5-trimethyl-1*H*-pyrazol-4-yl)pyrimidin-4-amine |
| 31 | 2-[7-(2-chloro-3,6-difluorobenzyl)imidazo[1,5-*b*]pyridazin-5-yl]-5-(2-methylpyridin-4-yl)pyrimidin-4-amine |
| 32 | 2-[7-(2-chloro-3,6-difluorobenzyl)imidazo[1,5-*b*]pyridazin-5yl]-5-quinolin-5-ylpyrimidin-4-amine |
| 33 | 2-[7-(2-chloro-3,6-difluorobenzyl)imidazo[1,5-*b*]pyridazin-5-yl]-5-[2-(trifluoromethyl)pyridin-4-yl]pyrimidin-4-amine |
| 34 | 2-[7-(2-chloro-3,6-difluorobenzyl)imidazo[1,5-*b*]pyridazin-5-yl]-5-pyridin-3-ylpyrimidin-4-amine |
| 35 | 2-[7-(2-chloro-3,6-difluorobenzyl)imidazo[1,5-*b*]pyridazin-5-yl]-5-(4-methoxypyridin-3-yl)pyrimidin-4-amine |
| 36 | 2-[7-(2-chloro-3,6-difluorobenzyl)imidazo[1,5-*b*]pyridazin-5-yl]-5-[5-(trifluoromethyl)pyridin-3-yl]pyrimidin-4-amine |
| 37 | 2-[7-(2-chloro-3,6-difluorobenzyl)imidazo[1,5-*b*]pyridazin-5-yl]-5-(4-methylpyridin-3-yl)pyrimidin-4-amine |
| 38 | 2-[7-(2-chloro-3,6-difluorobenzyl)imidazo[1,5-*b*]pyridazin-5-yl]-5-(1-methyl-1*H*-pyrazol-4-yl)pyrimidin-4-amine |
| 39 | 2-[7-(2-chloro-3,6-difluorobenzyl)imidazo[1,5-*b*]pyridazin-5-yl]-5-[1,5-dimethyl-3-(trifluoromethyl)-1*H*-pyrazol-4-yl]pyrimidin-4-amine |
| 40 | 2-[7-(2,3,5-trifluorobenzyl)imidazo[1,5-*b*]pyridazin-5-yl]-5-(1,3,5-trimethyl-1*H*-pyrazol-4-yl)pyrimidin-4-amine |
| 41 | 5-[1,5-dimethyl-3-(trifluoromethyl)-1*H*-pyrazol-4-yl]-2-[7-(2,3,5-trifluorobenzyl)imidazo[1,5-*b*]pyridazin-5-yl]pyrimidin-4-amine |
| 42 | 2-[7-(3-chloro-2,6-difluorobenzyl)imidazo[1,5-*b*]pyridazin-5-yl]-5-(1,3,5-trimethyl-1*H*-pyrazol-4-yl)pyrimidin-4-amine |
| 43 | 2-[7-(2,3,5-trifluorobenzyl)imidazo(1,5-*b*]pyridazin-5-yl]-5-[2-(trifluoromethyl)pyridin-4-yl]pyrimidin-4-amine |
| 44 | 2-[7-(2,3,5-trifluorobenzyl)imidazo[1,5-*b*]pyridazin-5-yl]-5-[5-(trifluoromethyl)pyridin-3-yl]pyrimidin-4-amine |
| 45 | 5-[3-(methylsulfonyl)phenyl]-2-[7-(2,3,5-trifluorobenzyl)imidazo[1,5-*b*]pyridazin-5-yl]pyrimidin-4-amine |
| 46 | 2-[7-(2-chloro-3,5-difluorobenzyl)imidazo[1,5-*b*]pyridazin-5-yl]-5-[3-(methylsulfonyl)phenyl]pyrimidin-4-amine |
| 47 | 5-[2-(methylsulfonyl)phenyl]-2-[7-(2,3,5-trifluorobenzyl)imidazo[1,5-*b*]pyridazin-5-yl]pyrimidin-4-amine |
| 48 | 5-[4-(methylsulfonyl)phenyl]-2-[7-(2,3,5-trifluorobenzyl)imidazo[1,5-*b*]pyridazin-5-yl]pyrimidin-4-amine |
| 49 | 5-(3-methoxyphenyl)-2-[7-(2,3,5-trifluorobenzyl)imidazo[1,5-*b*]pyridazin-5-yl]pyrimidin-4-amine |
| 50 | 5-(2-methoxypyridn-3-yl)-2-[7-(2,3,4-trifluorobenzyl)imidazo[1,5-*b*]pyridazin-5-yl]pyrimidin-4-amine |
| 51 | 5-(4-methoxypyridin-3-yl)-2-[7-(2,3,4-trifluorobenzyl)imidazo[1,5-*b*]pyridazin-5-yl]pyrimidin-4-amine |
| 52 | 2-[7-(2,3,4-trifluorobenzyl)imidazo[1,5-*b*]pyridazin-5-yl]-5-[5-(trifluoromethyl)pyridin-3-yl]pyrimidin-4-amine |
| 53 | 5-pyridin-4-yl-2-[7-(2,3,4-trifluorobenzyl)imidazo[1,5-*b*]pyridazin-5-yl]pyrimidin-4-amine |
| 54 | 5-(1-methyl-1*H*-pyrazol-5-yl)-2-[7-(2,3,6-trifluorobenzyl)imidazo[1,5-*b*]pyridazin-5-yl]pyrimidin-4-amine |
| 55 | 5-[3-(methylsulfonyl)phenyl]-2-[7-(2,3,4-trifluorobenzyl)imidazo[1,5-*b*]pyridazin-5-yl]pyrimidin-4-amine |
| 56 | 2-[6-fluoro-3-(2,3,6-trifluorobenzyl)imidazo[1,5-*a*]pyridin-1-yl]-5-pyridin-3-ylpyrimidin-4-amine |
| 57 | 2-[6-fluoro-3-(2,3,6-trifluorobenzyl)imidazo[1,5-*a*]pyridin-1-yl]-5-quinolin-5-ylpyrimidin-4-amine |
| 58 | 2-[6-fluoro-3-(2,3,6-trifluorobenzyl)imidazo[1,5-*a*]pyridin-1-yl]-5-pyridin-4-ylpyrimidin-4-amine |
| 59 | 2-[6-fluoro-3-(2,3,6-trifluorobenzyl)imidazo[1,5-*a*]pyridin-1-yl]-5-(1,3,5-trimethyl-1*H*-pyrazol-4-yl)pyrimidin-4-amine |
| 60 | 2-[6-fluoro-3-(2,3,6-trifluorobenzyl)imidazo[1,5-*a*]pyridin-1-yl]-5-[5-(trifluoromethyl)pyridin-3-yl]pyrimidin-4-amine |
| 61 | 5-(5-fluoropyridin-3-yl)-2-[6-fluoro-3-(2,3,6-trifluorobenzyl)imidazo[1,5-*a*]pyridin-1-yl]pyrimidin-4-amine |
| 62 | 2-[6-fluoro-3-(2,3,6-trifluorobenzyl)imidazo[1,5-*a*]pyridin-1-yl]-5-(2-methoxypyridin-3-yl)pyrimidin-4-amine |
| 63 | 2-[6-fluoro-3-(2,3,6-trifluorobenzyl)imidazo[1,5-*a*]pyridin-1-yl]-5-(4-methylpyridin-3-yl)pyrimidin-4-amine |
| 64 | 2-[6-fluoro-3-(2,3,6-trifluorobenzyl)imidazo[1,5-*a*]pyridin-1-yl]-5-(2-methylpyridin-4-yl)pyrimidin-4-amine |
| 65 | 2-[6-fluoro-3-(2,3,6-trifluorobenzyl)imidazo[1,5-*a*]pyridin-1-yl]-5-(4-methoxypyridin-3-yl)pyrimidin-4-amine |
| 66 | 2-[6-fluoro-3-(2,3,6-trifluorobenzyl)imidazo[1,5-*a*]pyridin-1-yl]-5,5'-bipyrimidin-4-amine |
| 67 | 2-[6-fluoro-3-(2,3,6-trifluorobenzyl)imidazo[1,5-*a*]pyridin-1-yl]-5-(1-methyl-1*H*-pyrazol-4-yl)pyrimidin-4-amine |
| 68 | 2-[6-fluoro-3-(2,3,6-trifluorobenzyl)imidazo[1,5-*a*]pyridin-1-yl]-5-(1-methyl-1*H*-pyrazol-5-yl)pyrimidin-4-amine |
| 69 | 5-[1,5-dimethyl-3-(trifluoromethyl)-1*H*-pyrazol-4-yl]-2-[6-fluoro-3-(2,3,6-trifluorobenzyl)imidazo[1,5-*a*]pyridin-1-yl]pyrimidin-4-amine |
| 70 | 2-[6-fluoro-3-(2,3,6-trifluorobenzyl)imidazo[1,5-*a*]pyridin-1-yl]-5-[2-(trifluoromethyl)pyridin-4-yl]pyrimidin-4-amine |
| 71 | 5-(3-fluoropyridin-4-yl)-2-[6-fluoro-3-(2,3,6-trifluorobenzyl)imidazo[1,5-*a*]pyridin-1-yl]pyrimidin-4-amine |
| 72 | 2-[6-fluoro-3-(2,3,6-trifluorobenzyl)imidazo[1,5-*a*]pyridin-1-y]]-5-[2-(trifluoromethyl)quinolin-4-yl]pyrimidin-4-amine |
| 73 | 5-(6-fluoroquinolin-4-yl)-2-[6-fluoro-3-(2,3,6-trifluorobenzyl)imidazo[1,5-*a*]pyridin-1-yl]pyrimidin-4-amine |
| 74 | 2-[6-fluoro-3-(2,3,6-trifluorobenzyl)imidazo[1,5-*a*]pyridin-1-yl]-5-quinolin-4-ylpyrimidin-4-amine |
| 75 | 2-[6-fluoro-3-(2,3,6-trifluorobenzyl)imidazo[1,5-*a*]pyridin-1-yl]-5-[1-methyl-3-(trifluoromethyl)-1*H*-pyrazol-4-yl]pyrimidin-4-amine |
| 76 | 5-[5-(methoxymethyl)-1,3-dimethyl-1*H*-pyrazol-4yl]-2-[7-(2,3,6-trifluorobenzyl)imidazo[1,5-*b*]pyridazin-5-yl]pyrimidin-4-amine |
| 77 | 2-[6-fluoro-3-(2,3,6-trifluorobenzyl)imidazo[1,5-*a*]pyridin-1-yl]-5-(1-methyl-1*H*-pyrrolo[2,3-*b*]pyridin-4-yl)pyrimidin-4-amine |
| 78 | 2-[6-fluoro-3-(2,3,6-trifluorobenzyl)imidazo[1,5-*a*]]pyridin-1-yl]-5-(3-methylpyridin-4-yl)pyrimidin-4-amine |
| 79 | 2-[6-fluoro-3-(2,3,6-trifluorobenzyl)imidazo[1,5-*a*]pyridin-1-yl]-5-(2-methoxypyridin-4-yl)pyrimidin-4-amine |
| 80 | 5-(3,5-dimethylisoxazol-4-yl)-2-[6-fluoro-3-(2,3,6-trifluorobenzyl)midazo[1,5-*a*])pyridin-1-yl]pyrimidin-4-amine |
| 81 | 2-[6-fluoro-3-(2,3,6-trifluorobenzyl)imidazo[1,5-*a*]pyridin-1-yl]-5-(1-methyl-1*H*-imidazol-5-yl)pyrimidin-4-amine |
| 82 | 2-[6-fluoro-3-(2,3,6-trifluorobenzyl)imidazo[1,5-*a*]pyridin-1-yl]-5-[3-(methylsulfonyl)phenyl]pyrimidin-4-amine |
| 83 | 2-[5-chloro-3-(2,3,6-trifluorobenzyl)-1*H*-indazol-1-yl]-5-(1-methyl-1*H-*pyrazol-4-yl)pyrimidine-4,6-diamine |
| 84 | 2-[5-chloro-3-(2,3,6-trifluorobenzyl)-1*H*-indazol-1-yl]-2'-methyl-5,5'-bipyrimidine-4,6-diamine |
| 85 | 2-[5-chloro-3-(2,3,6-trifluorobenzyl)-1*H*-indazol-1-yl]-5-pyridin-4-ylpyrimidine-4,6-diamine |
| 86 | 2-[5-chloro-3-(2,3,6-trifluorobenzyl)-1*H*-indazol-1-yl]-5-(1-isobutyl-1*H-*pyrazol-4-yl)pyrimidine-4,6-diamine |
| 87 | 2-[5-chloro-3-(2,3,6-trifluorobenzyl)-1*H*-indazol-1-yl]-5-[1-(3-methylbutyl)-1*H*-pyrazol-4-yl]pyrimidine-4,6-diamine |
| 88 | 5-(1-*tert*-butyl-3-methyl-1*H*-pyrazol-4-yl)-2-[5-chloro-3-(2,3,6-trifluorobenzyl)-1*H*-indazol-1-yl]pyrimidine-4,6-diamine |
| 89 | 2-[5-chloro-3-(2,3,6-trifluorobenzyl)-1*H*-indazol-1-yl]-5-[1-(cyclopropylmethyl)-3-methyl-1*H*-pyrazol-4-yl]pyrimidine-4,6-diamine |
| 90 | 2-[5-chloro-3-(2,3,6-trifluorobenzyl)-1*H*-indazol-1-yl]-5-[5-(methoxymethyl)-1,3-dimethyl-1*H*-pyrazol-4-yl]pyrimidine-4,6-diamine |
| 91 | 2-[5-chloro-3-(2,3,6-trifluorobenzyl)-1*H*-indazol-1-yl]-5-(1,3,5-trimethyl-1*H*-pyrazol-4-yl)pyrimidine-4,6-diamine |
| 92 | 2-[5-chloro-3-(2,3,6-trifluorobenzyl)-1*H*-indazol-1-yl]-5-pyridin-3-ylpyrimidine-4,6-diamine |
| 93 | 2-[5-chloro-3-(2,3,6-trifluorobenzyl)-1*H*-indazol-1-yl]-5-(6-fluoropyridin-3-yl)pyrimidine4,6-diamine |
| 94 | 2-[5-chloro-3-(2,3,6-trifluorobenzyl)-1*H*-indazol-1-yl]-5-[3-(methylsulfonyl)phenyl]pyrimidine-4,6-diamine |
| 95 | 2-[5-chloro-3-(2,3,6-trifluorobenzyl)-1*H*-indazol-1-yl]-5-(2-methylpyridin-4-yl)pyrimidine-4,6-diamine |
| 96 | 2-[5-chloro-3-(2,3,6-trifluorobenzyl)-1*H*-indazol-1-yl]-5-(5-fluoropyridin-3-yl]pyrimidine-4,6-diamine |
| 97 | 2-[5-chloro-3-(2,3,6-trifluorobenzyl)-1*H*-indazol-1-yl]-5-[1-methyl-5-(trifluoromethyl)-1*H*-pyrazol-yl]pyrimidine-4,6-diamine |
| 98 | 2-[5-chloro-3-(2,3,6-trifluorobenzyl)-1*H*-indazol-1-yl]-5-(5-methoxypridin-3-yl)pyrimidine-4,6-diamine |
| 99 | 2-[5-chloro-3-(2,3,6-trifluorobenzyl)-1*H*-indazol-1-yl]-5-(3,5-dimethylisoxazol-4-yl)pyrimidine-4,6-diamine |
| 100 | 2-[5-chloro-3-(2,3,6-trifluorobenzyl)-1*H*-indazol-1-yl]-5-(2-methylpyridin-3-yl)pyrimidine-4,6-diamine |
| 101 | 2-[5-chloro-3-(2,3,6-trifluorobenzyl)-1*H*-indazol-1-yl]-5-(6-methylpyridin-3-yl)pyrimidine-4,6-diamine |
| 102 | 2-[5-chloro-3-(2,3,6-trifluorobenzyl)-1*H*-indazol-1-yl]-5-(5-methylpyridin-3-yl)pyrimidine-4,6-diamine |
| 103 | 2-[5-chloro-3-(2,3,6-trifluorobenzyl)-1*H*-indazol-1-yl]-5-(1-methyl-1*H-*pyrazol-5-yl)pyrimidine-4,6-diamine |
| 104 | 2-[5-chloro-3-(2,3,6-trifluorobenzyl)-1*H*-indazol-1-yl]-5-(6-methoxypyridin-3-yl)pyrimidine-4,6-diamine |
| 105 | 2-[5-chloro-3-(2,3,6-trifluorobenzyl)-1*H*-indazol-1-yl]-5-(2-methoxypyridin-4-yl)pyrimidine-4,6-diamine |
| 106 | 5-(2-aminopyridin-4-yl)-2-[5-chloro-3-(2,3,6-trifluorobenzyl)-1*H-*indazol-1-yl]pyrimidine-4,6-diamine |
| 1.07 | 2-[5-chloro-3-(2,3,6-trifluorobenzyl)-1*H*-indazol-1-yl]-5-(3,5-dimethyl-1*H*-pyrazol-4-yl)pyrimidine-4,6-diamine |
| 108 | methyl (4-{4,6-diamino-2-[5-chloro-3-(2,3,6-trifluorobenzyl)-1*H-*indazol-1-yl]pyrimidin-5-yl}pyridin-2-yl)carbamate |
| 109 | 2-[5-chloro-3-(2,3,6-trifluorobenzyl)-1*H*-indazol-1-yl]-5-(6-methoxypyridin-2-yl)pyrimidine-4,6-diamine |
| 110 | methyl (5-{4,6-diamino-2-[5-chloro-3-(2,3,6-trifluorobenzyl)-1*H-*indazol-1-yl]pyrimidin-5-yl}pyridin-2-yl)carbamate |
| 111 | 2-[5-chloro-3-(2,3,6-trifluorobenzyl)-1*H*-indazol-1-yl]-5-(2-methoxypyridin-3-yl)pyrimidine-4,6-diamine |
| 112 | 2-[5-chloro-3-(2,3,6-trifluorobenzyl)-1*H*-indazol-1-yl]-5-(3-fluoro-2-morpholin-4-ylpyridin-4-yl)pyrimidine-4,6-diamine |
| 113 | 2-[5-chloro-3-(2,3,6-trifluorobenzyl)-1*H*-indazol-1-yl]-5-pyrazin-2-ylpyrimidine-4,6-diamine |
| 114 | *N*-(5-{4,6-diamino-2-[5-chloro-3-(2,3,6-trifluorobenzyl)-1*H*-indazol-1-yl]pyrimidin-5-yl}pyridin-2-yl)acetamide |
| 115 | 2-[5-fluoro-3-(2,3,6-trifluorobenzyl)-1*H*-indazol-1-yl]-5-(2-methoxypyridin-4-yl)pyrimidine-4,6-diamine |
| 116 | 2-[5-fluoro-3-(2,3,6-trifluorobenzyl)-1*H*-indazol-1-yl]-5-[5-(trifluoromethyl)pyridin-3-yl]pyrimidine-4,6-diamine |
| 117 | 2-[5-fluoro-3-(2,3,6-trifluorobenzyl)-1*H*-indazol-1-yl]-5-pyridin-3-ylpyrimidine-4,6-diamine |
| 118 | 5-(6-fluoropyridin-3-yl)-2-[5-fluoro-3-(2,3,6-trifluorobenzyl)-1*H-*indazol-1-yl]pyrimidine-4,6-diamine |
| 119 | 5-(6-aminopyridin-3-yl)-2-[5-fluora-3-(2,3,6-trifluorobenzyl)-1*H-*indazol-1-yl]pyrimidine-4,6-diamine |
| 120 | 2-[5-fluoro-3-(2,3,6-trifluorobenzyl)-1*H*-indazol-1-yl]-5-[3-(methylsulfonyl)phenyl]pyrimidine-4,6-diamine |
| 121 | 2-[5-fluoro-3-(2,3,6-trifluorobenzyl)-1*H*-indazol-1-yl]-5-quinolin-5-ylpyrimidine-4,6-diamine |
| 122 | 2-[5-fluoro-3-(2,3,6-trifluorobenzyl)-1*H*-indazol-1-yl]-5-(2-methylpyridin-4-yl)pyrimidine4,6-diamine |
| 123 | 3-{4,6-diamino-2-[5-fluoro-3-(2,3,6-trifluorobenzyl)-1*H*-indazol-1-yl]pyrimidin-5-yl}quinolin-2-ol |
| 124 | 5-(5-fluoropyridin-3-yl)-2-[5-fluoro-3-(2,3,6-trifluorobenzyl)-1*H-*indazol-1-yl]pyrimidine-4,6-diamine |
| 125 | 5-(2-fluoroquinolin-3-yl)-2-[5-fluoro-3-(2,3,6-trifluorobenzyl)-1*H-*indazol-1-yl]pyrimidine-4,6-diamine |
| 126 | 2-[5-fluoro-3-(2,3,6-trifluorobenzyl)-1*H*-indazol-1-yl]-5-pyridin-4-ylpyrimidine-4,6-diamine |
| 127 | 2-[5-fluoro-3-(2,3,6-trifluorobenzyl)-1*H*-indazol-1-yl]-5-(1,3,5-trimethyl-1*H*-pyrazol-4-yl)pyrimidine-4,6-diamine |
| 128 | 2-[5-fluoro-3-(2,3,6-trifluorobenzyl)-1*H*-indazol-1-yl]-5-[1-methyl-5-(trifluoromethyl)-1*H*-pyrazol-4-yl]pyrimidine-4,6-diamine |
| 129 | 2-[5-fluoro-3-(2,3,6-trifluorobenzyl)-1*H*-indazol-1-yl]-5-[1-methyl-3-(trifluoromethyl)-1*H*-pyrazol-5-yl]pyrimidine-4,6-diamine |
| 130 | 5-(3,5-dimethylisoxazol-4-yl)-2-[5-fluoro-3-(2,3,6-trifluorobenzyl)-1*H-*indazol-1-yl]pyrimidine-4,6-diamine |
| 131 | 2-[5-fluoro-3-(2,3,6-trifluorobenzyl)-1*H*-indazol-1-yl]-5-(2-methoxypyridin-4-yl)pyrimidine-4,6-diamine |
| 132 | 2-[5-fluoro-3-(2,3,6-trifluorobenzyl)-1*H*-indazol-1-yl]-2',4'-dimethoxy-5,5'-bipyrimidine-4,6-diamine |
| 133 | 2-[5-fluoro-3-(2,3,6-trifluorobenzyl)-1*H*-indazol-1-yl]-5-(2-methoxypyridin-3-yl)pyrimidine-4,6-diamine |
| 134 | 2-[5-fluoro-3-(2,3,6-trifluorobenzyl)-1*H*-indazol-1-yl]-5-(5-methoxypyridin-3-yl)pyrimidine-4,6-diamine |
| 135 | 2-[5-fluoro-3-(2,3,6-trifluorobenzyl)-1*H*-indazol-1-yl]-2-methyl-5,5'-bipyrimidine-4,6-diamine |
| 136 | 2-[5-fluoro-3-(2,3,6-trifluorobenzyl)-1*H*-indazol-1-yl]-2'-methoxy-5,5'-bipyrimidine-4,6-diamine |
| 137 | 2-[5-fluoro-3-(2,3,6-trifluorobenzyl)-1*H*-indazol-1-yl]-5-(6-methoxypyridin-2-yl)pyrimidine-4,6-diamine |
| 138 | 5-(2,6-dimethoxypyridin-3-yl)-2-[5-fluoro-3-(2,3,6-trifluorobenzyl)-1*H-*indazol-1-yl]pyrimidine-4,6-diamine |
| 139 | 2-[5-fluoro-3-(2,3,6-trifluorobenzyl)-1*H*-indazol-1-yl]-5-(4-methox_ypyridin-3-yl)pyrimidine-4,6-diamine |
| 140 | 2-[5-fluoro-3-(2,3,6-trifluorobenzyl)-1H-indazol-1-yl]-5,5¹-bipyrimidine-4,6-diamine |
| 141 | 5-(4-fluoropyridin-3-yl)-2-[5-fluoro-3-(2,3,6-trifluorobenzyl)-1H-indazol-1-y!J_Qyrimidine-4,6-diamine |
| 142 | 2-[5-fluoro-3-(2,3,6-trifluorobenzyl)-1H-indazol-1-yl]-5-(1,3-thiazol-2-yl)pyrimidine-4,6-diamine |
| 143 | 2-[5-fluoro-3-(2,3,6-trifluorobenzyl)-1H-indazol-1-yl]-5-(4-methyl-2-phenyl-1,3-thiazol-5.,rl)_pyrimidine-4,6-diamine |
| 144 | *N*-{4,6-diamino-2-[5-fluoro-3-(2,3,6-trifluorobenzyl)-1H-indazol-1-yl]pyrimidin-5-yl}-2-methylpropanamide |
| 145 | 2-(5-fluoro-3-(2,3,6-trifluorobenzyl)-1H-indazol-1-yl]-5-(1,3-oxazol-2-yl)_Nrimidine-4,6-diamine |
| 146 | 5-(2-methylpyridin-4-yl)-2-[3-(2,3,6-trifluorobenzyl)-1H-indazol-1-yl]pyrimidine-4,6-diamine |
| 147 | 5-(6-fluoropyridin-3-yl)-2-[3-(2,3,6-trifluorobenzyl)-1H-indazol-1-y!]QYrimidine-4,6-diamine |
| 148 | 2-[3-(2,3,6-trifluorobenzyl)-1H-indazol-1-yl]-5-(1,3,5-trimethyl-1H-pyrazol-4-yl):pyrimidine-4,6-diamine |
| 149 | 5-(5-fluoropyridin-3-yl)-2-[3-(2,3,6-trifluorobenzyl)-1H-indazol-1-yl]pyrimidine-4,6-diamine |
| 150 | 5-(1-methyl-1H-pyrazol-4-yl)-2-[3-(2,3,6-trifluorobenzyl)-1H-indazol-1-_yl]_Qyrimidine-4,6-diamine |
| 151 | 5-(2-methylpyridin-4-yl)-2-[3-(2,3,6-trifluorobenzyl)-1H-indazol-1-yl]pyrimidine-4,6-diamine |
| 152 | 5-pyridin-4-yl-2-[3-(2,3,6-trifluorobenzyl)-1H-indazol-1-yl]pyrimidine-4,6-diamine |
| 153 | 5-pyridin-3-yl-2-[3-(2,3,6-trifluorobenzyl)-1H-indazol-1-yl]pyrimidine-4,6-diamine |
| 154 | 5-(1-methyl-5-(trifluoromethyl)-1H-pyrazol-4-yl]-2-[3-(2,3,6-trifluorobenzyl)-1H-indazol-1-yl]pyrimidine-4,6- |
| 155 | 5-(2-methoxypyridin-4-yl)-2-[3-(2,3,6-trifluorobenzyl)-1H-indazol-1-yl]pyrimidine-4,6-diamine |
| 156 | 5-(3,5-dimethylisoxazol-4-yl)-2-[3-(2,3,6-trifluorobenzyl)-1H-indazol-1-yl]pyrimidine-4,6-diamine |
| 157 | 5-(2-methylpyridin-3-yl)-2-[3-(2,3,6-trifluorobenzyl)-1H-indazol-1-yl]pyrimidine-4,6-diamine |
| 158 | 5-(6-methylpyridin-3-yl)-2-[3-(2,3,6-trifluorobenzyl)-1*H*-indazol-1-yl]pyrimidme-4,6-diamine |
| 159 | 2'-methyl-2-[3-(2,3,6-trifluorobenzyl)-1*H*-indazol-1-yl]-5,5'-bipyrimidine-4,6-diamine |
| 160 | 5-(3-fluoropyridin-4-yl)-2-[3-(2,3,6-trifluorobenzyl)-1*H*-indazol-1-yl]pyrimidine-4,6-diamine |
| 161 | 5-[6-(dimethylamino)pyridin-3-yl]-2-[3-(2,3,6-trifluorobenzyl)-1*H-*indazol-1-yl]pyrimidine-4,6-diamine |
| 162 | 5-(2-methoxypyridin-3-yl)-2-[3-(2,3,6-trifluorobenzyl)-1*H*-indazol-1-yl]pyrimidine-4,6-diamine |
| 163 | 5-(6-methoxypyridin-3-yl)-2-[3-(2,3,6-trifluorobenzyl)-1*H*-indazol-1-yl]pyrimidine-4,6-diamine |
| 164 | 5-(4-methyl-2-phenyl-1,3-thiazol-5-yl)-2-[3-(2,3,6-trifluorobenzyl)-1*H-*indazol-1-yl]pyrimidine-4,6-diamine |
| 165 | 5-pyrazin-2-yl-2-[3-(2,3,6-trifluorobenzyl)-1*H*-indazol-1-yl]pyrimidine-4,6-diamine |
| 166 | 5-(4-methoxypyridin-3-yl)-2-[3-(2,3,6-trifluorobenzyl)-1*H*-indazol-1-yl]pyrimidine-4,6-diamine |
| 167 | 5-(2,6-dimethoxypyridin-3-yl)-2-[3-(2,3,6-trifluorobenzyl)-1*H*-indazol-1-yl]pyrimidine-4,6-diamine |
| 168 | 5-(6-methoxypyridin-2-yl)-2-[3-(2,3,6-trifluorobenzyl)-1*H*-indazol-1-yl]pyrimidine-4,6-diamine |
| 169 | 5-pyridin-2-yl-2-[3-(2,3,6-trifluorobenzyl)-1*H*-indazol-1-yl]pyrimidine-4,6-diamine |
| 170 | 2-[5-methoxy-3-(2,3,6-trifluorobenzyl)-1*H*-indazol-1-yl]-5-(1-methyl-1*H*-pyrazol-4-yl)pyrimidine-4,6-diamine |
| 171 | 2-[5-methoxy-3-(2,3,6-trifluorobenzyl)-1*H*-indazol-1-yl]-5-pyridin-3-ylpyrimidine-4,6-diamine |
| 172 | 2-[5-methoxy-3-(2,3,6-trifluorobenzyl)-1*H*-indazol-1-yl]-5-[5-(trifluoromethyl)pyridin-3-yl]pyrimidine-4,6-diamine |
| 173 | 2-[5-methoxy-3-(2,3,6-trifluorobenzyl)-1*H*-indazol-1-yl]-5-(1,3,5-trimethyl-1*H*-pyrazol-4-yl)pyrimidine-4,6-diamine |
| 174 | 5-pyridin4-yl-2-[3-(2,3,6-trifluorobenzyl)-1*H*-pyrazolo[4,3-*b*]pyridin-1-yl]pyrimidine-4,6-diamine |
| 175 | 5-(1-methyl-1*H*-pyrazol-4-yl)-2-[3-(2,3,6-trifluorobenzyl)-1*H-*pyrazolo[4,3-*b*]pyridin-1-yl]pyrimidine-4,6-diamine |
| 176 | methyl {4,6-diamino-2-[5-chloro-3-(2,3,6-trifluorobenzyl)-1*H*-indazol-1-yl]pyrimidin-5-yl}carbamate |
| 177 | methyl {4,6-diamino-2-[5-chloro-3-(2,3,6-trifluorobenzyl)-1*H*-indazol-1-yl]pyrimidin-5-yl}methylcarbamate |
| 178 | *N-*{4,6-diamino-2-[5-chloro-3-(2,3,6-trifluorobenzyl)-1*H*-indazol-1-yl]pyrimidin-5-yl}cyclopropanecarboxamide |
| 179 | S-methyl {4,6-diamino-2-[5-chloro-3-(2,3,6-trifluorobenzyl)-1*H*-indazol-1-yl]pyrimidin-5-yl}thiocarbamate |
| 180 | *S*-methyl {4,6-dimino-2-[5-fluoro-3-(2,3,6-trifluorobenzyl)-1*H*-indazol-1-yl]pyrimidin-5-yl}thiocarbamate |
| 181 | methyl {4,6-diamino-2-[5-fluoro-3-(2,3,6-trifluorobenzyl)-1*H*-indazol-1-yl]pyrimidin-5-yl}methylcarbamate |
| 182 | methyl {4,6-diamtno-2-[3-(2,3,6-trifluorobenzyl)-1*H*-indazol-1-yl]pyrimidin-5-yl}carbamate |
| 183 | *S*-methyl {4,6-diamino-2-[3-(2,3,6-trifluorobenzyl)-1*H*-indazol-1-yl]pyrimidin-5-yl}thiocarbamate |
| 184 | methyl {4,6-diamino-2-[3-(2,3,6-trifluorobenzyl)-1*H*-indazol-1-yl]pyrimidin-5-yl}methylcarbamate |
| 185 | ethyl {4,6-diamino-2-[3-(2,3,6-trifluorobenzyl)-1*H*-indazol-1-yl]pyrimidin-5-yl}carbamate |
| 186 | methyl {4,6-diamino-2-[5-chloro-3-(2,3,5-trifluorobenzyl)-1*H*-indazol-1-yl]pyrimidin-5-yl}carbamate |
| 187 | S-methyl {4,6-diamino-2-[5-chloro-3-(2,3,5-trifluorobenzyl)-1*H*-indazol-1-yl]pyrimidin-5-yl}thiocarbamate |
| 188 | methyl {4,6-diamino-2-[5-chloro-3-(2,3,5-trifluorobenzyl)-1*H*-indazol-1-yl]pyrimidin-5-yl}methylcarbamate |
| 189 | methyl {4,6-diamino-2-[3-(2,3,5-trifluorobenzyl)-1*H*-indazol-1-yl]pyrimidin-5-yl}carbamate |
| 190 | methyl {4,6-diamino-2-[3-(2,3,5-trifluorobenzyl)-1*H*-indazol-1-yl]pyrimidin-5-yl}methylcarbamate |
| 191 | *S*-methyl {4,6-diamino-2-[3-(2,3,5-trifluorobenzyl)-1*H*-indazol-1-yl]pyrimidin-5-yl}methylthiocarbamate |
| 192 | ethyl {4,6-diamino-2-[3-(2,3,5-trifluorobenzyl)-1*H*-indazol-1-yl]pyrimidin-5-yl}carbamate |
| 193 | ethyl {4,6-diamino-2-[3-(2,3,5-trifluorobenzyl)-1*H*-indazol-1-yl]pyrimidin-5-yl}methylcarbamate |
| 194 | methyl {4,6-diamino-2-[5-phenyl-3-(2,3,6-trifluorobenzyl)-1*H*-indazol-1-yl]pyrimidin-5-yl}methylcarbamate |
| 195 | methyl {4,6-diamino-2-[5-fluoro-3-(2,3,5-trifluorobenzyl)-1*H*-indazol-1-yl]pyrimidin-5-yl}carbamate |
| 196 | methyl {4,6-diamino-2-[5-fluoro-3-(2,3,5-trifluorobenzyl)-1*H*-indazol-1-yl]pyrimidin-5-yl}methylcarbamate |
| 197 | ethyl {4,6-diamino-2-[5-chloro-3-(2,3,6-trifluorobenzyl)-1*H*-indazol-1-yl]pyrimidin-5-yl}carbamate |
| 198 | ethyl {4,6-diamino-2-[5-chloro-3-(2,3,5-trifluorobenzyl)-1*H*-indazol-1-yl]pyrimidin-5-yl}carbamate |
| 199 | methyl {4,6-diamino-2-[5-chloro-3-(2,3,6-trifluorobenzyl)-1*H-*pyrazolo[4,3-*b*]pyridin-1-yl]pyrimidin-5-yl}carbamate |
| 200 | methyl {4,6-diamino-2-[5-chloro-3-(2,3,6-trifluorobenzyl)-1*H-*pyrazolo[4,3-*b*]pyridin-1-yl]pyrimidin-5-yl}methylcarbamate |
| 201 | methyl {4,6-diamino-2-[3-(2,3,6-trifluorobenzyl)-1*H*-pyrazolo[4,3-b]pyridin-1-yl]pyrimidin-5-yl}carbamate |
| 202 | methyl {4,6-diamino-2-[3-(3-chloro-2,5-difluorobenzyl)-5-fluoro-1*H-*indazol-1-yl]pyrimidin-5-yl}carbamate |
| 203 | methyl {4,6-diamino-2-[3-(2-chloro-3,6-difluorobenzyl)-5-fluoro-1*H-*indazol-1-yl]pyrimidin-5-yl}carbamate |
| 204 | methyl {4,6-diamino-2-[3-(2,6-difluoro-3-methylbenzyl)-5-fluoro-1*H-*indazol-1-yl]pyrimidin-5-yl}carbamate |
| 205 | *S*-methyl {4,6-diamino-2-[5-fluoro-3-(2,3,6-trichlorobenzyl)-1*H*-indazol-1-yl]pyrimidin-5-yl}thiocarbamate |
| 206 | *N-*{4,6-diamino-2-[5-fluoro-3-(2,3,6-trifluorobenzyl)-1*H*-indazol-1-yl]pyrimidin-5-yl}cyclopropanecarboxamide |
| 207 | *N-*{4,6-diamino-2-[3-(2,3,6-trifluorobenzyl)-1*H*-indazol-1-yl]pyrimidin-5-yl}cyclopropanecarboxamide |
| 208 | 2-[3-(2-cyclopentylethyl)-1*H*-indazol-1-yl]-5-pyridin-3-ylpyrimidine-4,6-diamine |
| 209 | 2-[3-(2-cyclopentylethyl)-1*H*-indazol-1-yl]-5-(1-methyl-1*H*-pyrazol-4-yl)pyrimidine-4,6-diamine |
| 210 | 2-[3-(2-cyclopentylethyl)-1*H*-indazol-1-yl]-5-(2-methylpyridin-4-yl)pyrimidine-4,6-diamine |
| 211 | 2-[3-(2-cyclopentylethyl)-1*H*-indazol-1-yl]-5-pyridin-4-ylpyrymidine-4,6-diamine |
| 212 | 2-[3-(2-cyclopentylethyl)-1*H*-indazol-1-yl]-5-(1,3,5-trimethyl-1*H-*pyrazol-4-yl)pyrimidine-4,6-diamine |
| 213 | 2-[3-(2-cyclopentylethyl)-1*H*-indazol-1-yl]-5-[5-(trifluoromethyl)pyridin-3-yl]pyrimidine-4,6-diamine |
| 214 | 2-[3-(2-cyclopentylethyl)-1*H*-indazol-1-yl]-5-[1-methyl-3-(trifluoromethyl)-1*H*-pyrazol-4-yl]pyrimidine-4,6-diamine |
| 215 | 2-[5-chloro-3-(2-cyclopentylethyl)-1*H*-indazol-1-yl]-5-pyridin-3-ylpyrimidine-4,6-diamine |
| 216 | 2-[5-chloro-3-(2-cyclopentylethyl)-1*H*-indazol-1-yl]-5-(2-methylpyridin-4-yl)pyrimidine-4,6-diamine |
| 217 | 2-[5-chloro-3-(2-cyclopentylethyl)-1*H*-indazol-1-yl]-5-(1-methyl-1*H-*pyrazol-4-yl)pyrimidine-4,6-diamine |
| 218 | 2-[5-chloro-3-(2-cyclopentylethyl)-1*H*-indazol-1-yl]-5-(1,3,5-trimethyl-1*H*-pyrazol-4-yl)pyrimidine-4,6-diamine |
| 219 | 2-{3-(2-cyclopentylethyl)-5-[5-(trifluoromethyl)pyridin-3-yl]-1*H*-indazol-1-yl}-5-[5-(trifluoromethyl)pyridin-3-yl]pyrimidine-4,6-diamine |
| 220 | 2-[3-(2-cyclopentylethyl)-5-fluoro-1*H*-indazol-1-yl]-5-pyridin-3-ylpyrimidine-4,6-diamine |
| 221 | 2-[3-(2-cyclopentylethyl)-5-fluoro-1*H*-indazol-1-yl]-5-(2-methylpyridin-4-yl)pyrimidine-4,6-diamine |
| 222 | 2-[3-(2-cyclopentylethyl)-5-fluoro-1*H*-indazol-1-yl]-5-(1-methyl-1*H-*pyrazol-4-yl)pyrimidine-4,6-diamine |
| 223 | 2-[3-(2-cyclopentylethyl)-5-fluoro-1*H*-indazol-1-yl]-5-(1,3,5-trimethyl-1*H*-pyrazol-4-yl)pyrimidine-4,6-diamine |
| 224 | 2-(5-chloro-3-pentyl-1*H*-indazol-1-yl)-5-(1-methyl-1*H*-pyrazol-4-yl)pyrimidine-4,6-diamine |
| 225 | 2-(5-chloro-3-pentyl-1*H*-indazol-1-yl)-5-(2-methylpyridin-4-yl)pyrimidine-4,6-diamine |
| 226 | 2-(5-chloro-3-pentyl-1*H*-indazol-1-yl)-5-(1,3,5-trimethyl-1*H*-pyrazol-4-yl)pyrimidine-4,6-diamine |
| 227 | 2-(3-pentyl-1*H*-indazol-1-yl)-5-(1,3,5-trimethyl-1*H*-pyrazol-4-yl)pyrimidine-4,6-diamine |
for use in the prevention and/or treatment of Systemic Sclerosis (SSc)

2. At least one compound according to claim 1 in combination with at least one PDE5 Inhibitor selected from the group of:
*Tadalafil* ((6R,12aR) -2,3,6,7,12,12a - Hexahydro - 2 - methyl - 6 - (3,4-methylene - dioxyphenyl) pyrazino(1',2':1,6) pyrido(3,4-b)indole-1,4-dione), *Vardenafil* (2-(2-Ethoxy-5-(4-ethylpiperazin-1-yl-1-sulfonyl)phenyl)-5-methyl-7-propyl-3H-imidazo (5,1-f) (1,2,4)triazin-4-one), *Sildenafil* (3-[2-ethoxy-5-(4-methylpiperazin-1-yl)sulfonyl-phenyl]-7- methy 1- 9- propy 1-2,4,7,8- tetrazabicyclo [4.3.0]nona -3,8,10-trien-5-one), *Udenafil* 5-[2-propyloxy-5-(1-methyl-2-pyrrolidinylethylamidosulfonyl)phenyl]-methyl-3-propyl-1,6-dihydro-7H-pyrazolo(4,3-d)pyrimidine-7-one, *Dasantafil* 7-(3-Bromo-4-methoxybenzyl)-1-ethyl-8-[[(1,2)-2-hydroxycyclopentyl]amino]-3-(2-hydroxyethyl)-3,7-dihydro-1-purine-2,6-dione, *Avanafil* 4-{[(3-chloro-4-methoxyphenyl)methyl]amino}-2-[(2S)-2-(hydroxymethyl)pyrrolidin-1-yl]-N-(pyrimidin-2-ylmethyl)pyrimidine-5-carboxamide, Mirodenafil, Lodenafil, UK 369.003, UK 371.800, *SLx 2101* of Surface Logix, *LAS 34179*Triazolo[1,2-]xanthine,6-methyl-4-propyl-2-[2-propoxy-5-(4-methylpiperazino)sulfonyl]phenyl or salts, hydrates or hydrates of the salts
for use in the prevention and/or treatment of Systemic Sclerosis (SSc).

3. At least one compound according to claim 1 in combination with sildenafil or vardenafil for use in the prevention and/or treatment of Systemic Sclerosis (SSc).

4. Compounds according to claim 1 to 3 for use in the prevention and/or treatment of Systemic Sclerosis (SSc), diffuse Systemic Sclerosis (dSSc), limited Systemic Sclerosis (ISSc), overlap type of Systemic Sclerosis, undifferentiated type of Systemic Sclerosis, Systemic Sclerosis sine scleroderma, skin fibrosis, scleroderma, nephrogenic fibrosing dermopathy (NFD), nephrogenic systemic fibrosis (NSF), keloid formation.

5. Compounds according to claim 1 to 3 for use in the prevention and/or treatment of Systemic Sclerosis SSc concomitant fibrosis of internal organs, comprising the gut, the lung, the kidney and the blood vessels.

6. Compounds according to examples of compound classes I to X according to claim 1 for use in the prevention and/or treatment of Systemic Sclerosis SSc.

7. At least one compound according to examples of compound classes I to X according to claim 1 in combination with vardenafil or sildenafil for use in the prevention and/or treatment of Systemic Sclerosis SSc.

8. Use for the production of a medicament for prevention and/or treatment of Systemic Sclerosis (SSc) comprising an effective amount of a compound according to claim 1 to 3.

9. Use for the production of a medicament for prevention and/or treatment of Systemic Systemic Sclerosis (SSc), diffuse Systemic Sclerosis (dSSc), limited Systemic Sclerosis (lSSc), overlap type of Systemic Sclerosis, undifferentiated type of Systemic Sclerosis, Systemic Sclerosis sine scleroderma, skin fibrosis, scleroderma, nephrogenic fibrosing dermopathy (NFD), keloid formation comprising an effective amount of a compound according to 1 to 3.

10. Use for the production of a medicament for prevention and/or treatment of Systemic Sclerosis SSc concomitant fibrosis of internal organs, comprising the gut, the lung, the kidney and the blood vessels comprising an effective amount of a compound according to 1 to 3.

11. Pharmaceutical formulation comprising at least one compound or one combination according to claims 1 to 3 for the use in the prevention and/or treatment of Systemic Sclerosis (SSc).

12. Pharmaceutical formulation comprising at least one compound or one combination according to claims 1 to 3 for the use in the prevention and/or treatment of Systemic Sclerosis (SSc), diffuse Systemic Sclerosis (dSSc), limited Systemic Sclerosis (ISSc), overlap type of Systemic Sclerosis, undifferentiated type of Systemic Sclerosis, Systemic Sclerosis sine scleroderma, skin fibrosis, scleroderma, nephrogenic fibrosing dermopathy (NFD), keloid formation.

13. Pharmaceutical formulation comprising at least one compound or one combination according to claims 1 to 3 for the use in the prevention and/or treatment of Systemic Sclerosis SSc concomitant fibrosis of internal organs, comprising the gut, the lung, the kidney and the blood vessels.

14. Kit comprising at least one sGC stimulator and/or activator according to claims 1 or a combination according to claims 2 to 3 for the use in the prevention and/or treatment of Systemic Sclerosis (SSc).

15. Kit according to claim 14 for the use in the prevention and/or treatment of Systemic Sclerosis (SSc), diffuse Systemic Sclerosis (dSSc), limited Systemic Sclerosis (ISSc), overlap type of Systemic Sclerosis, undifferentiated type of Systemic Sclerosis, Systemic Sclerosis sine scleroderma, skin fibrosis, scleroderma, nephrogenic fibrosing dermopathy (NFD), keloid formation.

16. Kit according to claims 14 and 15 for the use in the prevention and/or treatment of Systemic Sclerosis SSc concomitant fibrosis of internal organs, comprising the gut, the lung, the kidney and the blood vessels.
